# EUROPEAN PATENT APPLICATION

(11) **EP 2 716 751 A1**
(43) Date of publication of application: **09.04.2014**
(21) Application number: 13187756.5
(22) Date of filing: 08.10.2013
(51) Int. Cl.: C12N 5/077, C12N 5/0775

(54) **Differentiated progeny of clonal progenitor cell lines**

(30) Priority: 08.10.2012 US 201261711161 P; 25.10.2012 US 201261718647 P; 13.11.2012 US 201261725866 P; 04.04.2013 US 201361808578 P; 29.04.2013 US 201361817260 P; 05.09.2013 US 201361874316 P
(71) Applicant: BioTime, Inc., Alameda, CA 94502 (US)
(72) Inventor: West, Michael, Alameda, CA 94510 (US); Chapman, Karen, Alameda, CA 94510 (US); Sternberg, Hal, Alameda, CA 94510 (US); Binette, Francois, Alameda, CA 94510 (US)
(74) Representative: Williams, Gareth Owen

(57) **Abstract**

Aspects of the present invention include methods and compositions related to the production and use of embryonic progenitor cell types useful in the generation of cartilage, bone, choroid plexus, tendon, lipasin-secreting adipocytes, and other differentiated cell types useful in research and therapy.

## Description

### CROSS-REFERENCE TO RELATED APPLICATIONS

This application claims priority to U.S. Provisional Application No. 61/711,161 filed on October 8, 2012, U.S. Provisional Application No. 61/718,647 filed on October 25, 2012, U.S. Provisional Application No. 61/725,866 filed on November 13, 2012, U.S. Provisional Application No. 61/808,578 filed on April 4, 2013, U.S. Provisional Application No. 61/817,260 filed on April 29, 2013 and U.S. Provisional Application No. 61/874,316 filed on September 5, 2013. The disclosures of all of the aforementioned priority documents are hereby incorporated by reference in their entirety.

### FIELD OF THE INVENTION

The invention relates to the field of stem cell biology.

### BACKGROUND OF THE INVENTION

Advances in stem cell technology, such as the isolation and propagation *in vitro* of primordial stem cells, including embryonic stem cells ("ES" cells including human ES cells ("hES" cells)) and related primordial stem cells including but not limited to, iPS, EG, EC, ICM, epiblast, or ED cells (including human iPS, EG, EC, ICM, epiblast, or ED cells), constitute an important new area of medical research. hES cells have a demonstrated potential to be propagated in the undifferentiated state and then to be induced subsequently to differentiate into any and all of the cell types in the human body, including complex tissues. Many of these primordial stem cells are naturally telomerase positive in the undifferentiated state, thereby allowing the cells to be expanded extensively and subsequently genetically modified and clonally expanded. Since the telomere length of many of these cells is comparable to that observed in sperm DNA (approximately 10-18 kb TRF length), differentiated cells derived from these immortal lines, generally is associated with the repression of the expression of the catalytic component of telomerase (*TERT*)*,* while displaying a long initial telomere length providing the cells with a long replicative capacity compared to fetal or adult-derived tissue. This has led, for example, to the suggestion that many diseases resulting from the dysfunction of cells may be amenable to treatment by the administration of hES-derived cells of various differentiated types (Thomson et al., Science 282:1145-1147 (1998)).

Nuclear transfer studies have demonstrated that it is possible to transform a somatic differentiated cell back to a primordial stem cell state such as that of embryonic stem ("ES") cells (Cibelli et al., Nature Biotech 16:642-646 (1998)) or embryo-derived ("ED") cells. Technologies to reprogram somatic cells back to a totipotent ES cell state, such as by the transfer of the genome of the somatic cell to an enucleated oocyte and the subsequent culture of the reconstructed embryo to yield ES cells, often referred to as somatic cell nuclear transfer ("SCNT") or through analytical reprogramming technology wherein somatic cells are reprogrammed using transcriptional regulators (see PCT application Ser. No. PCT/US2006/030632 filed on August 3, 2006 and titled "Improved Methods of Reprogramming Animal Somatic Cells") have been described. These methods offer potential methods to transplant primordial-derived somatic cells with a nuclear genotype of the patient (Lanza et al., Nature Medicine 5:975-977 (1999)).

In addition to SCNT and analytical reprogramming technologies, other techniques exist to address the problem of transplant rejection, including the use of gynogenesis and androgenesis (see U.S. application nos. 60/161,987, filed October 28, 1999; 09/697,297, filed October 27, 2000; 09/995,659, filed November 29, 2001; 10/374,512, filed February 27, 2003; PCT application no. PCT/US00/29551, filed October 27, 2000). In the case of a type of gynogenesis designated parthenogenesis, pluripotent stem cells may be manufactured without antigens foreign to the gamete donor and therefore useful in manufacturing cells that can be transplanted without rejection. In addition, parthenogenic stem cell lines can be assembled into a bank of cell lines homozygous in the HLA region (or corresponding MHC region of nonhuman animals) to reduce the complexity of a stem cell bank in regard to HLA haplotypes.

Cell lines or a bank of said cell lines can be produced that are hemizygous in the HLA region (or corresponding MHC region of nonhuman animals; see PCT application Ser. No. PCT/US2006/040985 filed October 20, 2006 entitled "Totipotent, Nearly Totipotent or Pluripotent Mammalian Cells Homozygous or Hemizygous for One or More Histocompatibility Antigen Genes", incorporated herein by reference). A bank of hemizygous cell lines provides the advantage of not only reducing the complexity inherent in the normal mammalian MHC gene pool, but it also reduces the gene dosage of the antigens to reduce the expression of said antigens without eliminating their expression entirely, thereby not stimulating a natural killer response.

In regard to differentiating primordial stem cells into desired cell types, the potential to clonally isolate lines of human embryonic progenitor cell lines provides a means to propagate novel highly purified cell lineages with a prenatal pattern of gene expression useful for regenerating tissues such as skin in a scarless manner. Such cell types have important applications in research, and for the manufacture of cell-based therapies (see PCT application Ser. No. PCT/US2006/013519 filed on April 11, 2006 and titled "Novel Uses of Cells With Prenatal Patterns of Gene Expression"; U.S. patent application Ser. No. 11/604,047 filed on November 21, 2006 and titled "Methods to Accelerate the Isolation of Novel Cell Strains from Pluripotent Stem Cells and Cells Obtained Thereby"; and U.S. patent application Ser. No. 12/504,630 filed on July 16, 2009 and titled "Methods to Accelerate the Isolation of Novel Cell Strains from Pluripotent Stem Cells and Cells Obtained Thereby"). Nevertheless, there remains a need for improved methods to differentiate pluripotent stem cells in directed manner.

The limited regenerative capacity of cartilage, tendon, and aged bone underlies the chronic nature of many age-related degenerative diseases of skeletal tissues. Bone maintains a regenerative capacity through the activation, proliferation, and differentiation of bone mesenchymal stem cells (MSCs), however, this activity declines with age¹. On the other end of the spectrum, cartilage shows almost no postnatal regenerative capacity. The rising incidence of age-related degenerative diseases in these tissues including osteoarthritis, intervertebral disc (IVD) disease, and osteoporosis has led to an increased focus on strategies for repairing defects resulting from these conditions using exogenous cell-based formulations.

Current challenges include the need for protocols that can generate cells of the connective tissues in a reproducible and scalable manner with a high level of purity and site-specific identity. The use of MSCs to supply these needs has been the focus of extensive study to date^{2,3}, however, despite reports of the transgermal plasticity of MSCs⁴⁻⁶, rigorous clonal data demonstrating differentiation beyond hypertrophic chondrocytes, osteoblasts, adipocytes, fibroblasts, and reticular cells is largely lacking⁷. In addition, MSCs also typically differentiate into transient hypertrophic chondrocytes as opposed to permanent definitive chondrocytes when differentiated using TGFβ family members and dexamethasone⁸. Hypertrophic markers include *COL10A1* and *IHH,* genes normally expressed in the growth plate of developing bones or in the callus formed following bone fracture.⁹⁻¹² This hypertrophy and the high levels of bone-forming factors expressed by differentiating MSCs such as bone sialoprotein II (IBSP) and tissue-nonspecific alkaline phosphatase (*ALPL*) commonly lead to mineralization of the resulting tissue.

Human pluripotent stem (hPS) cells such as human embryonic stem (hES) and induced pluripotent stem (iPS) cells have the potential to replicate without limit in the undifferentiated state, and also are believed to have the ability to differentiate into all mortal somatic cell lineages. hPS cells could provide a means of manufacturing all skeletal cell types to scale including mesodermal connective tissues of the developing limbs and axial skeleton, and neural crest-derived mesenchymal cells, such as those of craniofacial bone, dermis, meninges, peripheral nerves, tendons and ligaments (including those associated with the teeth), and cartilages with diverse mechanical properties such as elastic, hyaline, and fibrocartilage¹³.

Unlike the skeletal tissues of the limbs and trunk, cranial neural crest cells commonly lack *HOX* gene expression¹⁴, but express complex combinations of other homeobox genes including members of the *DLX* and *MSX* families¹⁵⁻¹⁷. Current protocols for generating skeletal cell types from hPS cells generally do not define whether the cells are of neural crest of mesodermal origin, and therefore the resulting formulations may include diverse cell types, and could respond to terminal differentiation signals such BMP2, BMP4, BMP6, and BMP7 by differentiating in an unpredictable manner¹⁸.

In certain embodiments described herein the invention provides methods and compositions directed to cell compositions and methods of deriving cell based compositions that are directed to soft tissue such as connective tissue, fat and mineralized tissue such as bone which may be useful in both the clinical and research setting.

### SUMMARY

The present invention provides compounds, compositions, kits, reagents and methods useful for the differentiation and use of human embryonic progenitor cell types.

In certain embodiments the invention provides an osteochondral cell, and methods of making and using the same.

In some embodiments, the invention provides methods of generating novel and diverse cartilage, and bone-forming cells, compositions comprising the same, and methods of using the same.

In other embodiments, the invention provides methods of generating novel types of adipocytes, including lipasin-expressing adipocytes, compositions regarding the same and methods of using the same.

In still other embodiments the invention provides a method of treating a tissue defect in a subject comprising administering to the subject one or more of the cells described infra. The tissue defect may be a soft tissue defect such as connective tissue defect, e.g. a tendon, or collagen or the tissue defect may be a mineralized tissue defect such as bone. The cells may be administered to the subject in hydrogel as described infra.

Yet other embodiments of the invention include kits and reagents comprising cells described herein and reagents useful for obtaining and/or growing the cells described herein.

In a particularly preferred aspect, the present invention provides a method of differentiating a human clonal progenitor cell into an osteochondral cell comprising contacting the human clonal progenitor cell with one or more TGFβ superfamily members.

Preferably, the human clonal progenitor cell is cultured under micromass conditions. Preferably, the human clonal progenitor cell is cultured in contact with a hydrogel. It is also preferred that the human clonal progenitor cell is encapsulated in the hydrogel. Preferably, the hydrogel comprises hylauronate and gelatin. It is preferred that the hyaluronate is thiolated. It is also preferred that the gelatin is thiolated. Both the hyaluronate and the gelatin may be thiolated alone or in combination with the other.

The hydrogel may preferably comprise acrylate, preferably PEG Diacrylate.

Preferably, the one or more TGFβ super family member may be chosen from TGFβ-3, BMP2, BMP-4, BMP-6, BMP-7 and GDF-5. Combinations thereof are also preferred. Particularly preferred combinations are: TGFβ-3 and BMP-2; TGFβ-3 and BMP-4; and TGFβ-3 and GDF5.

In a further preferred aspect, the invention also provides a cellular composition comprising a hydrogel and the in vitro differentiated progeny of a human clonal progenitor cell, wherein the progeny of the human clonal progenitor cell is an osteochondral cell. As above, it is preferred that the hydrogel comprises hylauronate and gelatin. It is preferred that the hyaluronate is thiolated. It is also preferred that the gelatin is thiolated. Both the hyaluronate and the gelatin may be thiolated alone or in combination with the other.

It is preferred that, the composition further comprises one or more members of the TGFβ super family. Preferably, the one or more members of the TGFβ super family are chosen from TGFβ-3, BMP-2, BMP-4, BMP-6, BMP-7 and GDF-5. Combinations thereof are also preferred. Particularly preferred combinations are: TGFβ-3 and BMP-2; TGFβ-3 and BMP-4; and TGFβ-3 and GDF5.

It is also preferred that the differentiated progeny of the clonal human progenitor cell expresses one or more genes chosen from COL2A1, COL10A1, ACAN, CRTAC1, TNMD, ALPL, PENK, BGLAP, BMP-2, DLX5, GPC3, IHH, PRG4, CILP, EPYC,SPP1, TTR LPL, CEBPD, PPARG, FABP4, PLIN1, DLK1, PPARGC1A, CEBPD, PPARG, FABP4, PRDM16, FOXC2, CRLF1, FOXF2, FBLN5, DPT, ITGBL1, COL6A3, DUSP1, FOXF1, TGFB3, PAX9, GSN, FMOD, PDE8B, COMP, ITGA10, SAA1, DTNA, PCDH9, EBF1, SORBS1, SORBS2, ZNF503, MGST2, PNMT, DPT, OGN, FBLN5, FMOD, CRLF1, ITGBL1, TGFB3, GSN, HHIP, LRIG1, TRPS1, COMP, LECT1, COL9A1, HAPLN1, ITGA10, OLFML3, PKDCC, FGFR3, CSPG4, COL9A3, ITGB5, DNM1; OGN, COL9A2, EPYC, CHAD, PPIB, SRPX2, MATN3, LUM, COL13A1, FKBP11, MXRA8, COL27A1, PELI2, GPX7, ANGPTL2, GXYLT2, KLF4, STEAP3, SLC39A14, PTH1R, FAM46A, FAM180A, SLC26A2, RUNX1, CTGF, PLEKHB1, FKBP7, TNC, JAK2, CYTL1, KDELR3, ATP8B2, TRPS1; ELN, PPIB, PHEX, SOX9, COL27A1, PELI2, ANGPTL2, GXYLT2, SLC39A14, P4HA3, SLC26A2, CTGF, MRC2, COL9A3, PLEKHB1, TNC, FRMD8, CYTL1, ATP8B2; SCRG1, MATN3, SMOC1, PELI2, PTH1R, HTRA1, RUNX1, CTGF, PLEKHB1, RG9MTD1, CYTL1, and KLF2, LPL, CEBPB, PLIN1, PPARG, PPARGC1A,, PPARG, GSN, WIF1, TGFB3, , COR02B, ADAMTS15, and IGFBP7COMP, SPP1, KAZALD1, LECT1, MMP13, HAPLN1, PHEX, PTH1R, COL11A1, and IP6K2 PKDCC, LTBP3, HAPLN1, OLFML3, ITGB5, IP6K2, ELN, CYTL1, LTBP3, PELI2, EPYC, PHEX, MRC2, CALY, GXYLT2, COL27A1, ANGPTL2, SOX9, GAA, TNC, LEPRE1, LTBP2, ARFGAP1, SRPX2, CYTL1, FAM46A, LUM, LTBP3, MXRA8, RUNX1, CALY, PTH1R, FKBP11, STEAP3, CFH, SLC40A1, GXYLT2, COL27A1, GPX7, ANGPTL2, MATN3, FKBP7, GAA, TNC, LEPRE1, and LTBP2, PPARG, PLIN1, LPL, CSPG4, LTBP3, CA12, PPIB, IRX5, ARHGAP24, FBLN5, DPT, CRLF1, ITGBL1, TGFB3, COL6A3, VCAN, DUSP1, TRPS1, GSN, ADAMTS6, ERG; EPYC, ELN, COL8A1, COL9A3, CYTL1, PPIB, CTHRC1, PLEKHB1, SLC26A2, KANK1, SLC39A14, ATP8B2, TNC, LTBP2, GPC1, WWP2, P4HA3, BAMBI, FGFRL1, SDC2; SCRG1, MEF2C, MATN3, PTH1R, ENPP2, CYTL1, CTHRC1, PLEKHB1, RUNX3, RUNX1, PRICKLE1, WWP2, HTRA1, CTGF, FGFRL1, ERG, FAT3; COL9A2, PCOLCE2, MEF2C, MATN3, LUM, HHIP, PTH1R, SLC40A1, KLF4, SRPX2, CYTL1, FKBP11, CTHRC1, STEAP3, LOXL4, DUSP1, LTBP2, TRPS1, GPC1, CFH, BAMBI, CDH2, COL27A1, FAM46A, CTGF, SDC2, and PLCD1, FRZB, HAND2, DLX5, DLX6, FOXD1, MSX2, TFAP2A, ALPL, BMP2, MSX2, DCN, SPP1, SATB2, MSX2, POSTN PCDH20, SERPINA3, TAC1, EDNRA, SCRG1, CRABP2, SPOCK3, VCAN, BOC, ECM2, CRLF1, GAS1, TSPAN8, MFAP4, NFIA, RASSF9, DCN, SATB2, NKX3-2, EBF1, SORBS2, PCDH17, SOBP, ST8SIA4; MEF2C, ALPL, PTH1R, STEAP3, PELI2, SLC40A1, MXRA8, MATN3, PARD6G, GPX7, FAM180A, CTHRC1, ATP8B2, BAMBI, PLCD1, LTBR, SLC26A2, ANGPTL2, SATB2, FAM46A, DUSP1, CSPG4,, OLFML3, PKDCC, LTBP3, FUS, FBLN5, GAS1, DUSP1, GSN, ARHGAP24, VCAN, EMILIN3; PDE8B, SORBS2, PHACTR2, EBF1, PCDH9, MATN3, LTBP2, DUSP1, CDH2, SLC40A1, MXRA8, STEAP3, ANGPTL2, GPX7, TSPAN3, SDC2, ATP8B2, CD36, CTGF, ARHGAP24, FBLN2, SPP1, CSPG4, CSPG4, ENPP1, CA12, DNM1; GXYLT2, ANGPTL2, METRNL, KDELR3, PARD6G, and LEPRE1, ACTC1, CRYAB, EFHD1, MFAP5, DLK1, ACTA2, OCA2, AIF1L, GPC4, SCRG1, CNN1, HES6, KRT17, RASL11B, IGFBP3, EDN1, ENC1, SFRP2, ACTG2, CKB, CSRP1, CSRP2, C5orf46, COL3A1, HEY1, TUBB2B, CALD1, KAL1, CD24, CDH2, MAMDC2, EFR3B, CDKN2B, HES4, TAGLN, CAP2, PMEPA1, CTGF, TPM1, TGFB2, CXXC5, COL4A1, PALLD, SOX11, OCIAD2, EML1, CCDC99, TTR, CCDC3, TGFB3, ZIC2, GPC4, POSTN, ID3, PODXL, FBLN5, KRT7, TINAGL1, LGMN, GPX7, ACTG2, ANGPTL4, TUBB2B, SLC4A2, SULF1, SLC16A9, CDH2, MAMDC2, , ENPP2, NPR3, CDKN2B, TAGLN, IN080C, HTRA1, DHRS3, CTGF, IGFBP7, , PMEPA1, MRPS6, APOE, SMPD1, TPM1, STXBP2, TMEM108, IQCG, COL4A1, SPINT2, MXD3, TPD52L1, HEYL, CDH6, CYR61, EFHD1, CCDC3: , CFH , ZIC2, TIMP4 , SYNM , AIF1L, EBF1, H19 , DYSF , EDNRA , NDUFA4L2 , ACTG2 , COL3A1, CSPG4 , PRRX1, TMEFF2 , TAGLN , ZBTB46 , HTRA1, IGFBP7 , APOE, OLAH , IGDCC4 , FBLN1, GGT5, MAN1C1, RFTN2 , STC2 , IGFBP1, TMEM119, CDH6, DKK2, DKK3, FAM198B, ACTG2, FILIP1L, MYH11; CSRP2 PRG4, AMELX, ENAM, SILV1, PITX1, FABP4, AMBN, CNN1, MYH11, ORM1 and LIPASIN.

In a further preferred aspect, the invention also provides a kit for differentiating human clonal progenitor cells into an osteochondral cell comprising a hydrogel and one or more members of the TGFβ super family. Preferably, the one or more members of the TGFβ super family are chosen from TGFβ-3, BMP-2, BMP-4, BMP-6, BMP-7 and GDF-5. Combinations thereof are also preferred. Particularly preferred combinations are: TGFβ-3 and BMP-2; TGFβ-3 and BMP-4; and TGFβ-3 and GDF5. Preferably, the hydrogel comprises hylauronate and gelatin. It is preferred that the hyaluronate is thiolated. It is also preferred that the gelatin is thiolated. Both the hyaluronate and the gelatin may be thiolated alone or in combination with the other. Preferably, the hydrogel may preferably comprise acrylate, preferably PEG Diacrylate.

In a further preferred aspect, the invention also provides a cryo-preserved human clonal progenitor cell and a hydrogel.

In a further preferred aspect, the invention also provides a cryo-preserved in vitro differentiated progeny of a human clonal progenitor cell and a hydrogel.

### BRIEF DESCRIPTION OF THE DRAWINGS

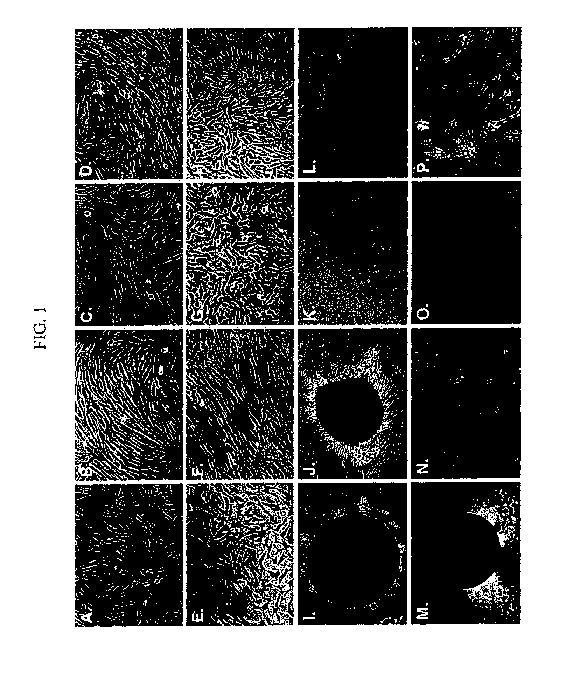

Figure 1. Phase contrast images of MSCs and the seven chondrogenic hEP cell lines in the undifferentiated state and day 14 micromass conditions. A) MSCs (P4); the chondrogenic hEP cell lines: B). 4D20.8 (P11); C). 7PEND24 (P20); D). 7SMOO32 (P9); E). E15 (P13); F).MEL2 (P21); G). SK11 (P11); and H). SM30 (P20) are shown in an undifferentiated state in log growth conditions. Day 14 micromass cultures comprised of: I). MSCs (P8); J). 4D20.8 (P17); K). 7PEND24 (P26); L). 7SMOO32 (P13); M). E15 (P17); N). MEL2 (P21); O). SK11 (P17); and P). SM30 (P13). (Scale bar, 100 microns).

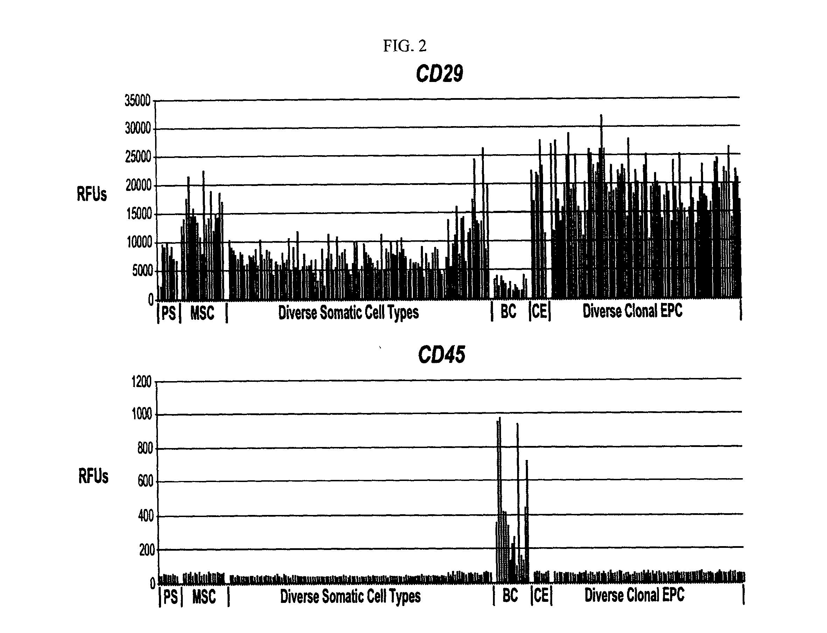

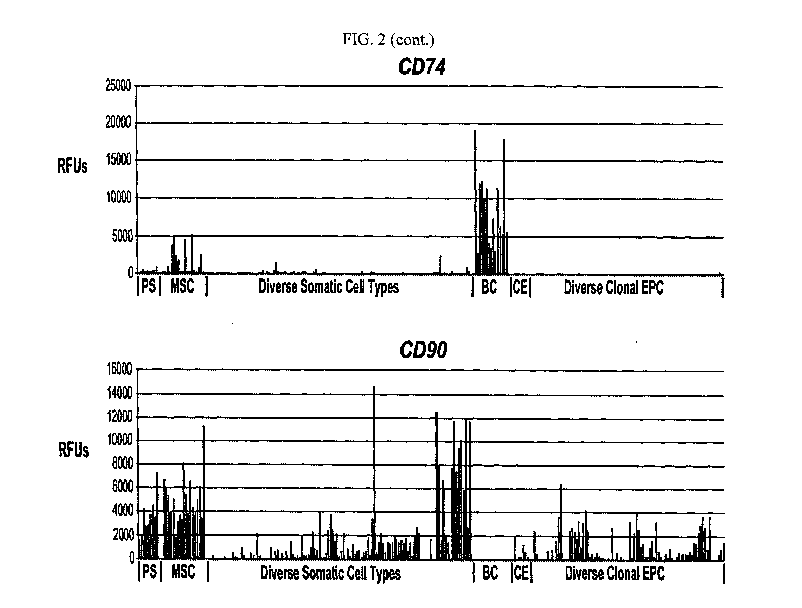

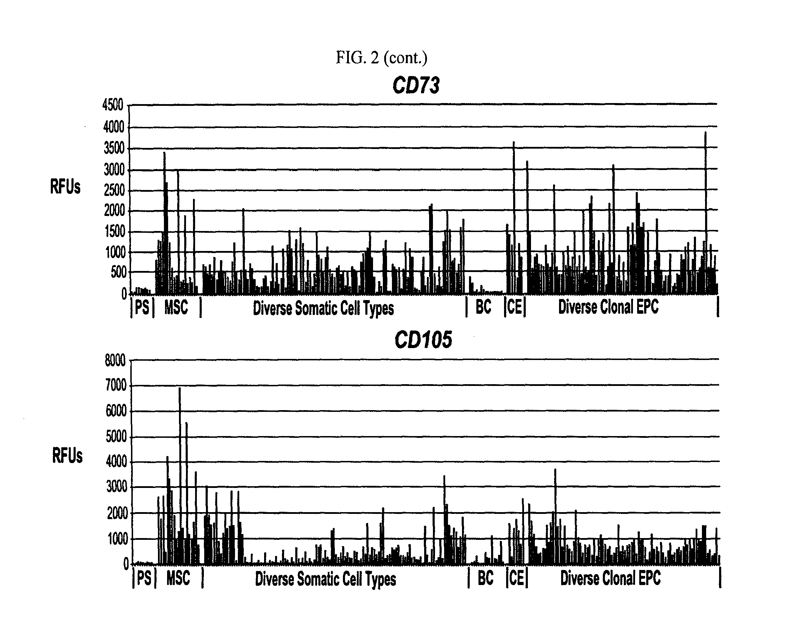

Figure 2. Microarray-based analysis of commonly-used MSC-associated CD antigen gene expression in hPS cells, MSCs, diverse cultured somatic cell types, and clonal hEP cell lines. Expression of the genes *CD29, CD45, CD73, CD74, CD90,* and *CD105* are shown as relative fluorescence units (RFUs) in: PS) 5 human ES and 4 iPS cell lines; MSCs; Diverse Somatic Cell Types including 111 epithelial and mesenchymal cell types; BC) 15 diverse blood cell types; CE) the seven clonal chondrogenic hEP cell lines described infra; and 86 Diverse Clonal EPC lines that are not observed to express *COL2A1* in micromass conditions in the presence of TGFβ3.

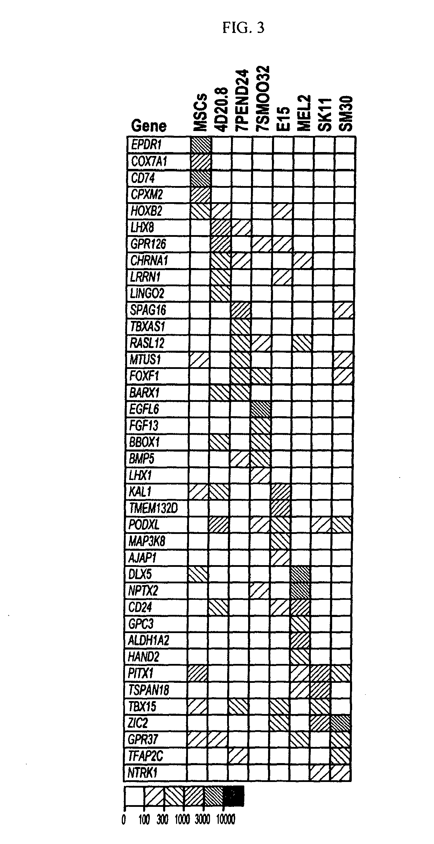

Figure 3. Heat map of unique gene expression markers distinguishing MSCs and chondrogenic hEP cell lines in the undifferentiated state. The relative expression of differentially-expressed marker genes detected by Illumina microarray analysis by comparing RNA from MSCs (mean of P6 and P8), and hEP lines: 4D20.8 (mean of P11 and P17), 7PEND24 (P15 and 26), 7SMOO32 (P11 and P18), E15 (P14 and P15), MEL2 (P17 and P22), SK11 (P12 and P17), and SM30 (P13 and P15) in the undifferentiated state synchronized in quiescence. (Color key displays microarray signal in RFU values where < 100 was considered nonspecific background signal, and values of >110 were considered detectable signal).

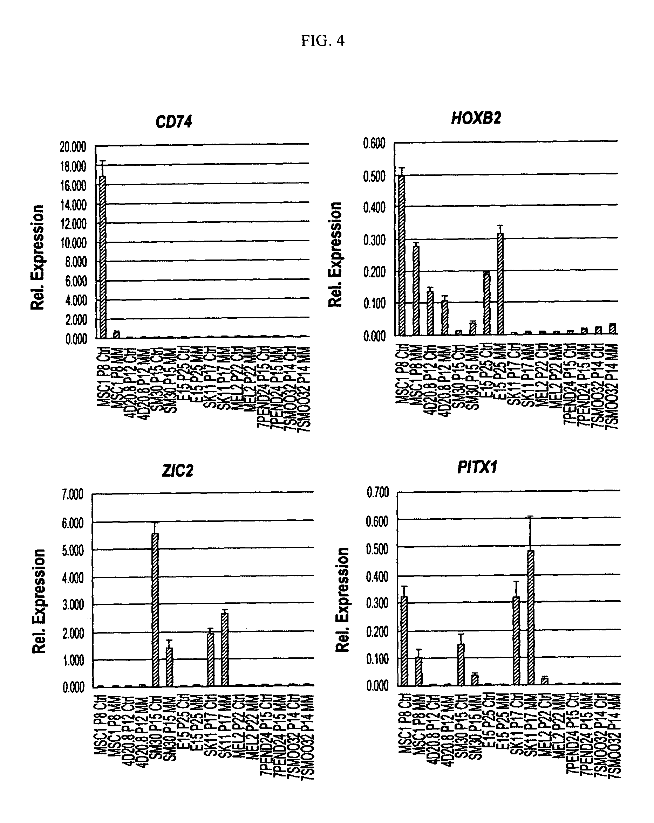

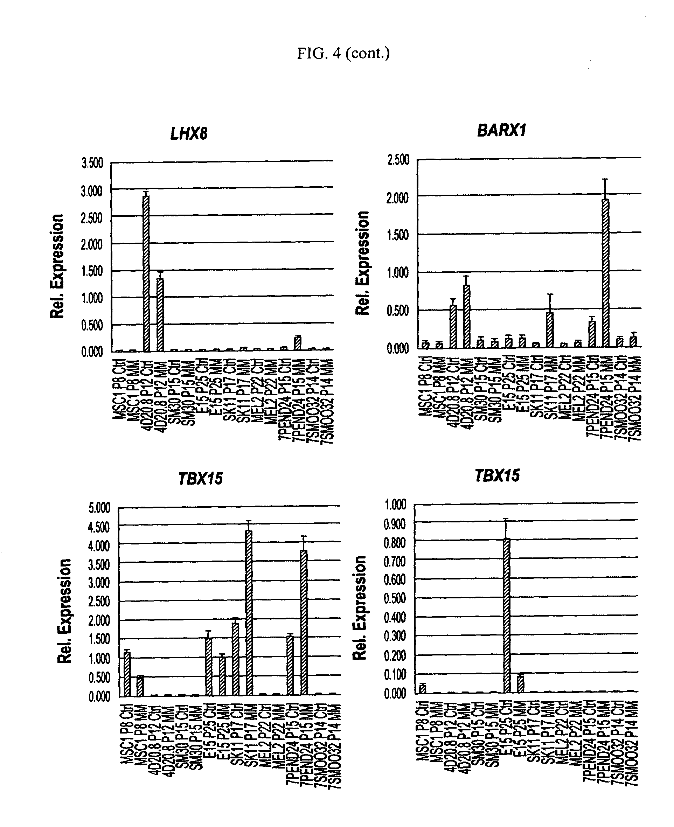

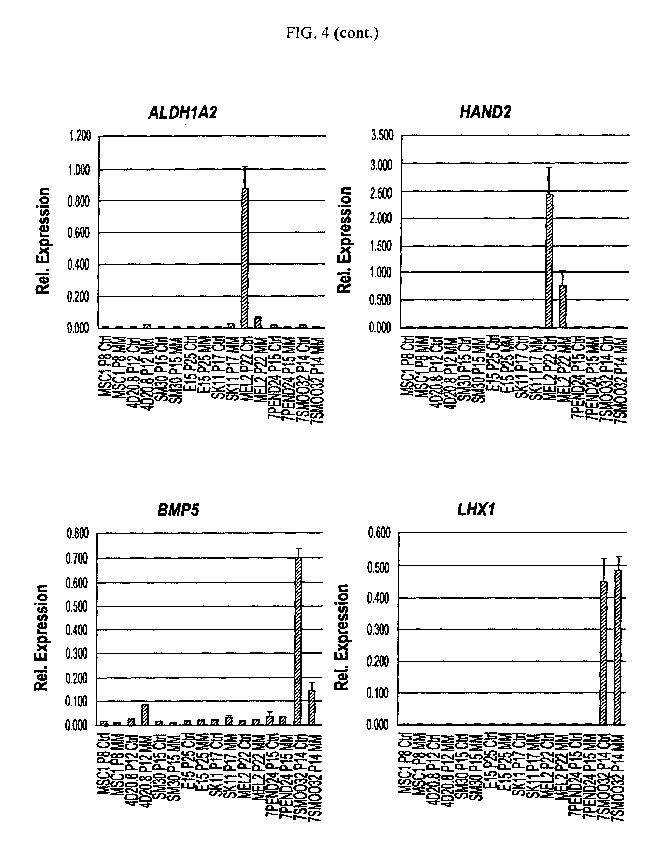

Figure 4: qPCR assay of a subset of site-specific gene expression markers in MSCs and chondrogenic hEP cell lines in the undifferentiated state. The relative expression of a subset of the differentially-expressed marker genes detected by Illumina microarray analysis was confirmed by qPCR from RNA from MSCs (P8), 4D20.8 (P12), 7PEND24 (P15), 7SMOO32 (P14), E15 (P25), MEL2 (P22), SK11 (P17), and SM30 (P15) in the undifferentiated state synchronized in quiescence. (Error bar represents standard deviation of quadruplicate technical replicates).

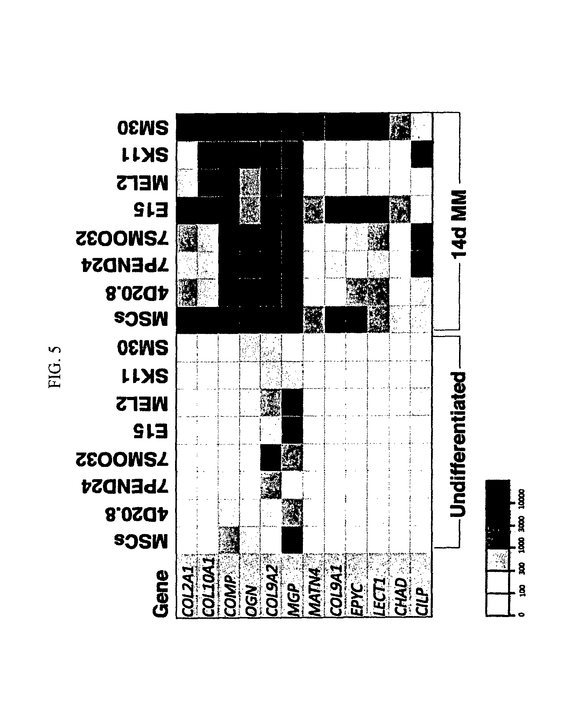

Figure 5. Heat map of the relative expression levels of chondrocyte-related gene expression in MSCs and chondrogenic hEP cell lines in the undifferentiated state and d14 micromass conditions. MSCs (mean of P6 and P8), 4D20.8 (mean of P11 and P17), 7PEND24 (P15 and 26), 7SMOO32 (P11 and P18), E15 (P14 and P15), MEL2 (P17 and P22), SK11 (P12 and P17), and SM30 (P13 and P15) were cultured either in the undifferentiated or d14 micromass (MM) conditions and relative expression of chondrocyte-related mRNA expression is displayed as a heat map. (Color key displays microarray signal in RFU values where < 100 was considered nonspecific background signal).

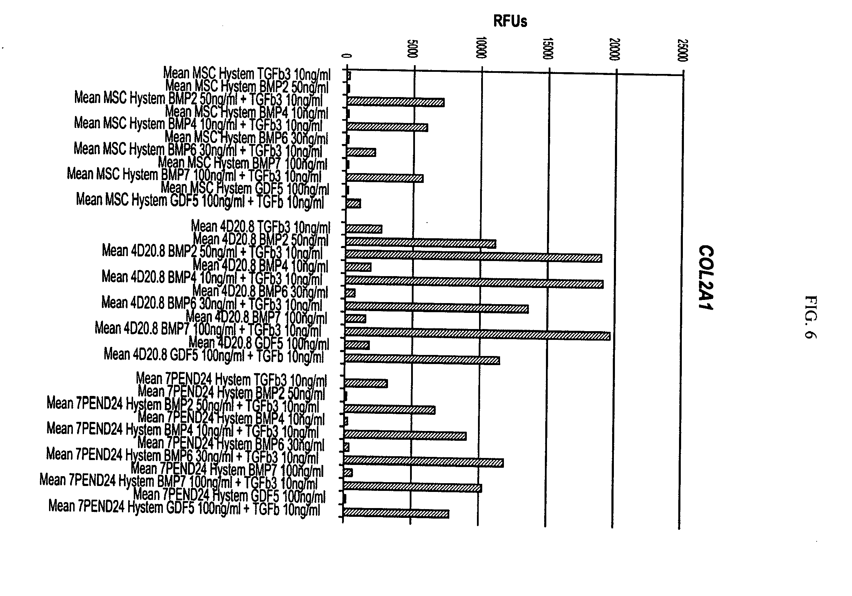

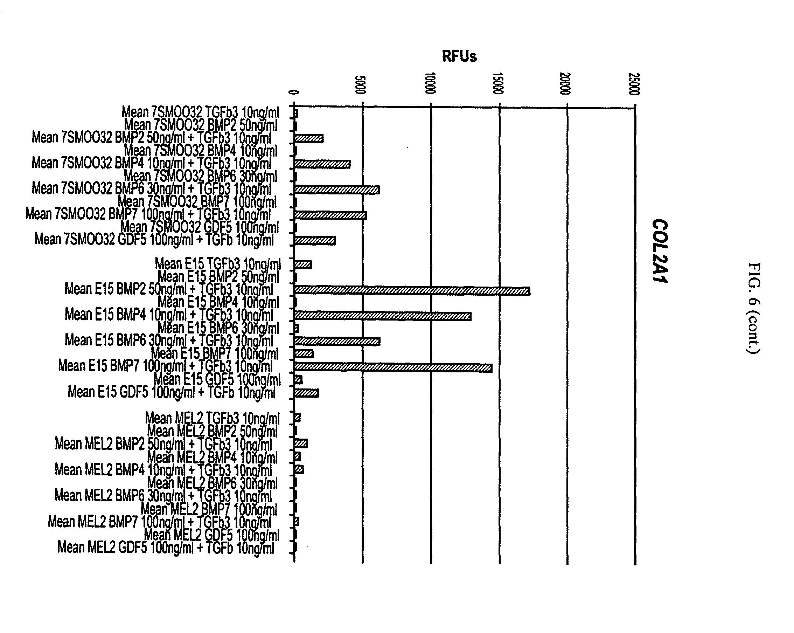

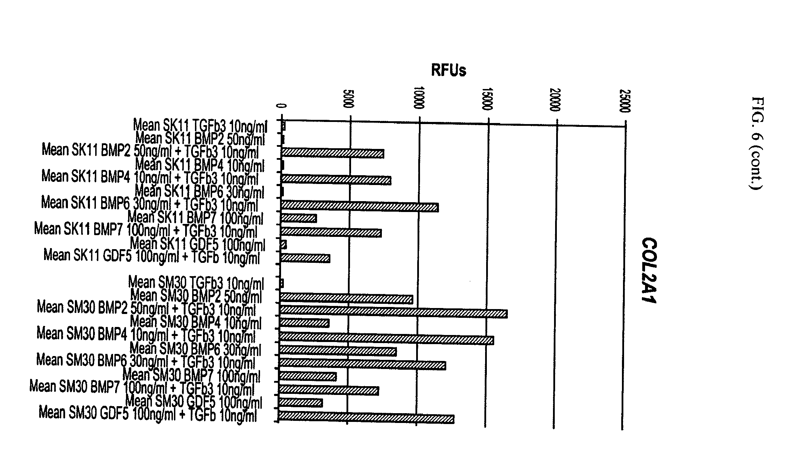

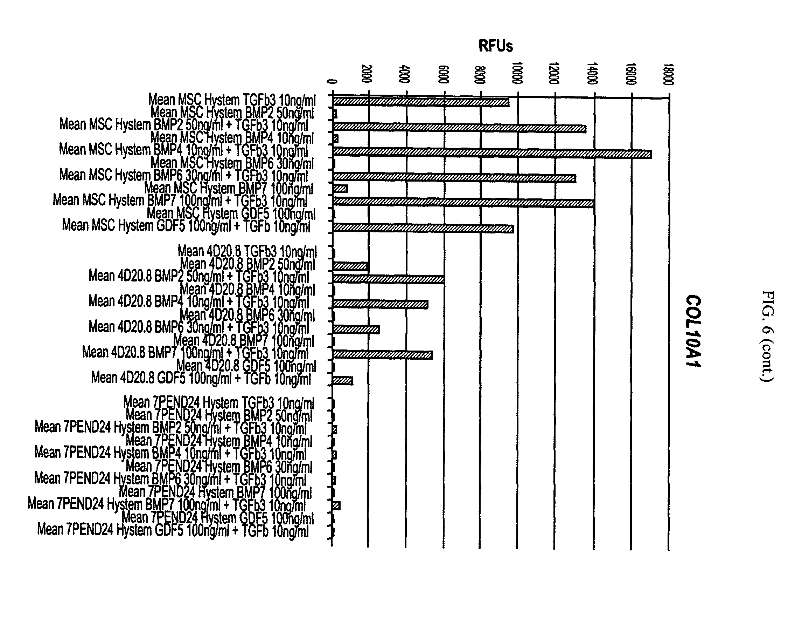

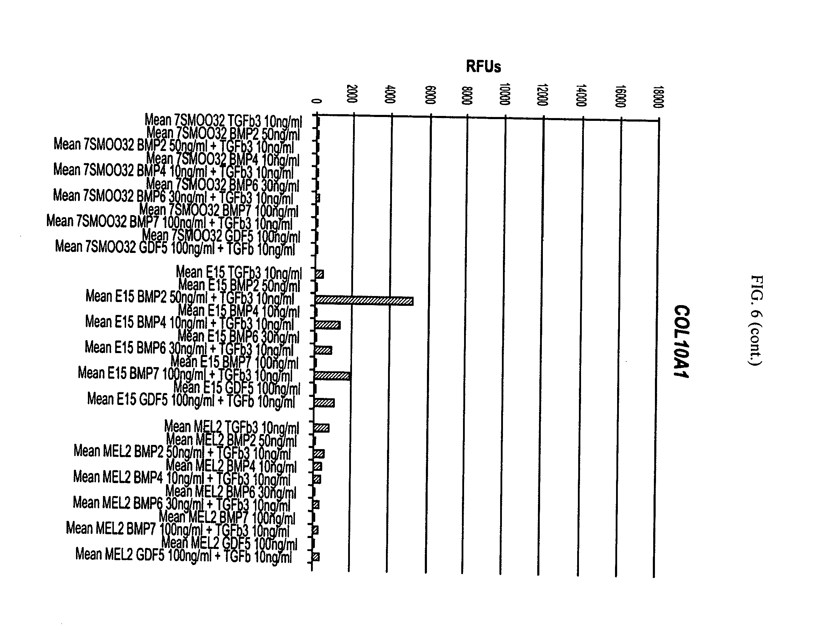

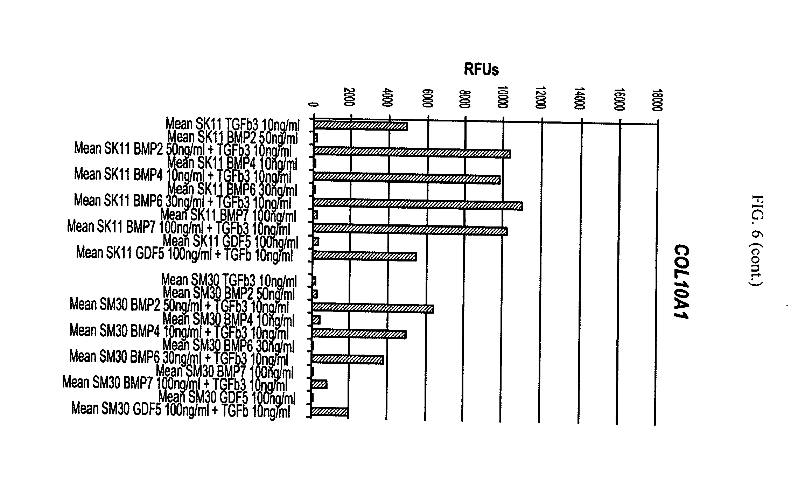

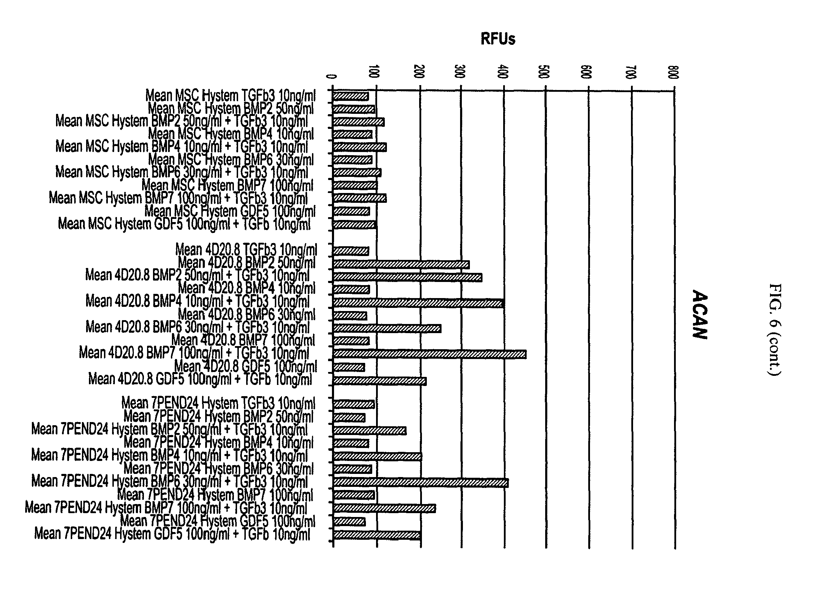

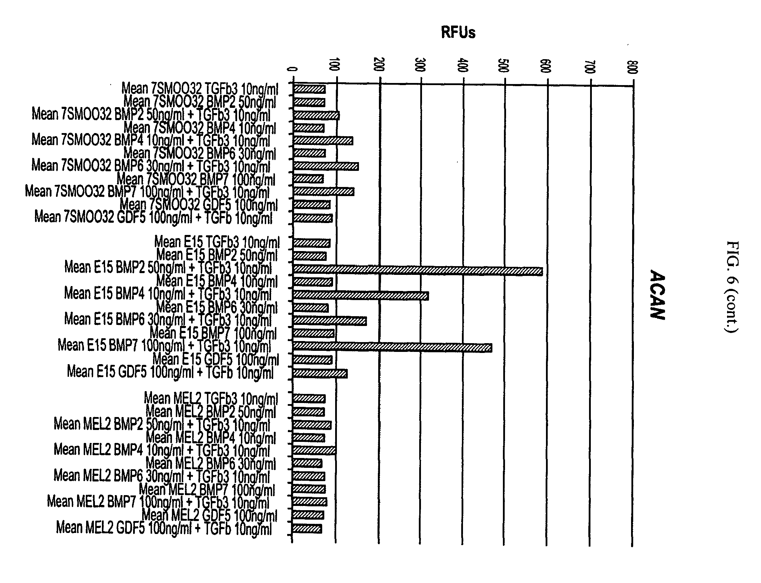

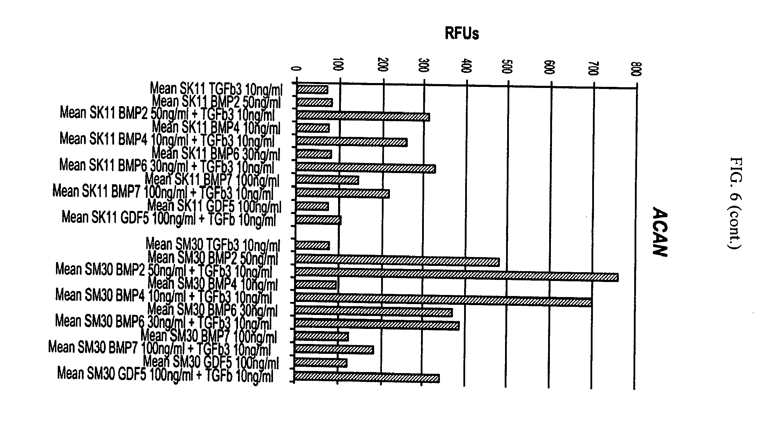

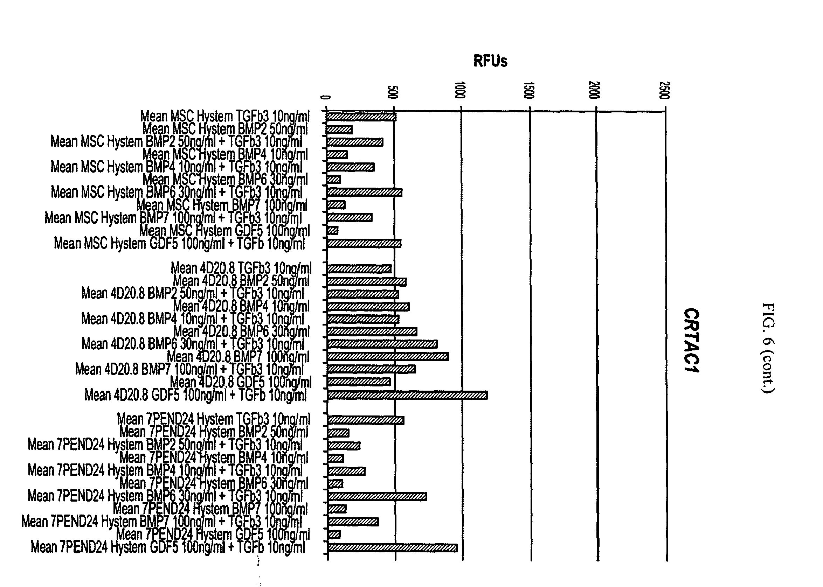

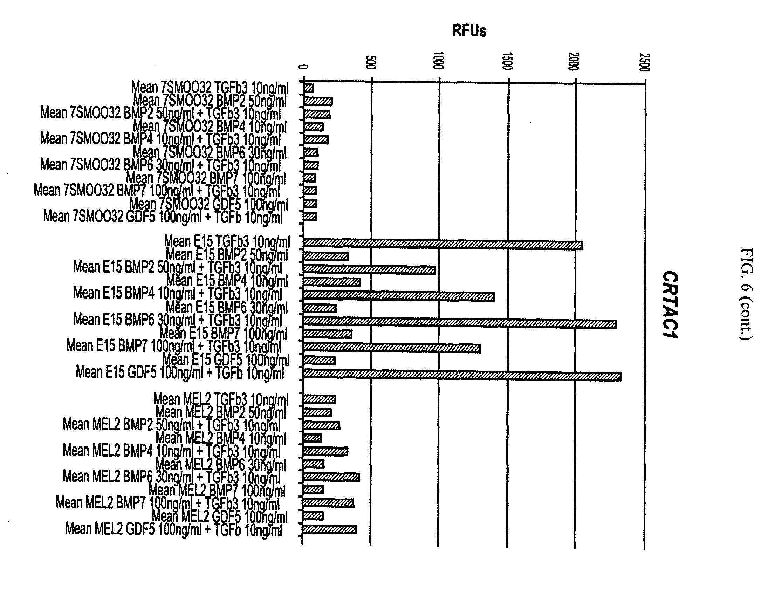

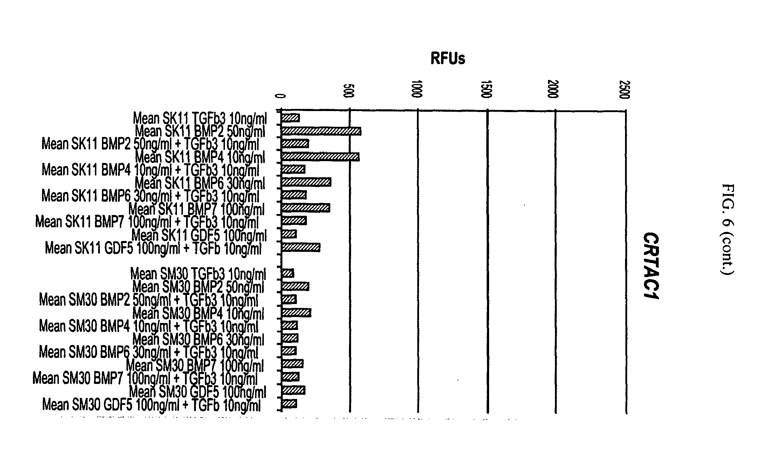

Figure 6: Relative expression levels of *COL2A1, COL10A1, ACAN,* and *CRTAC1* in MSCs and diverse chondrogenic hEP cell lines in the presence of diverse members of the TGF Beta family of growth factors measured by microarray analysis. RNA from MSCs and seven chondrogenic hEP cell lines cultured in D14 HyStem differentiation conditions with TGFβ3 in combination with other TGFβ family members was analyzed by Illumina microarray with values for the genes *COL2A1, COL10A1, ACAN,* and *CRTAC1.* Data are displayed as mean values of two or more biological replicates. (RFU values <100 considered background signal).

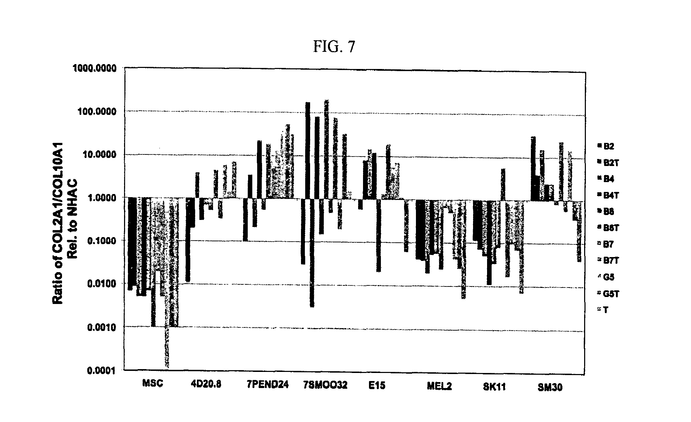

Figure 7. Comparison of the ratios of *COL2A1*/*COL10A1* in MSCs and the chondrogenic hEP cell lines in combinations of TGFβ family members as determined by qPCR. The ratios of *COL2A1*/*COL10A1* expression measured as fold expression compared to cultured NHACs are shown for MSCs and the seven chondrogenic hEP cell lines in the presence of TGFβ3 and other TGFβ family members. (Values shown are the mean of 2 or more biological replicates).

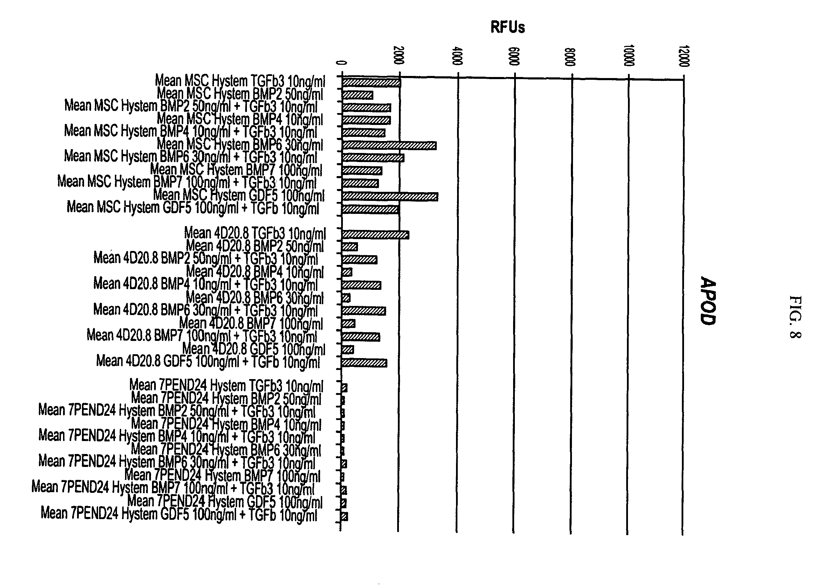

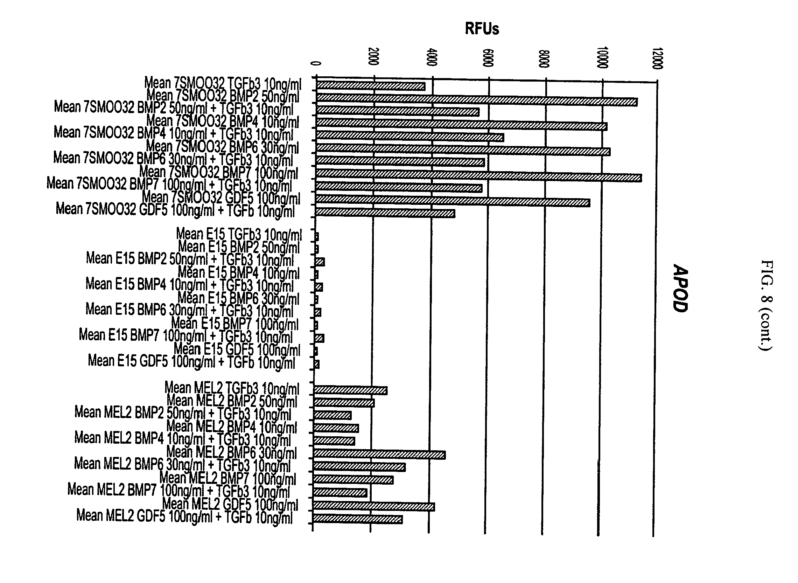

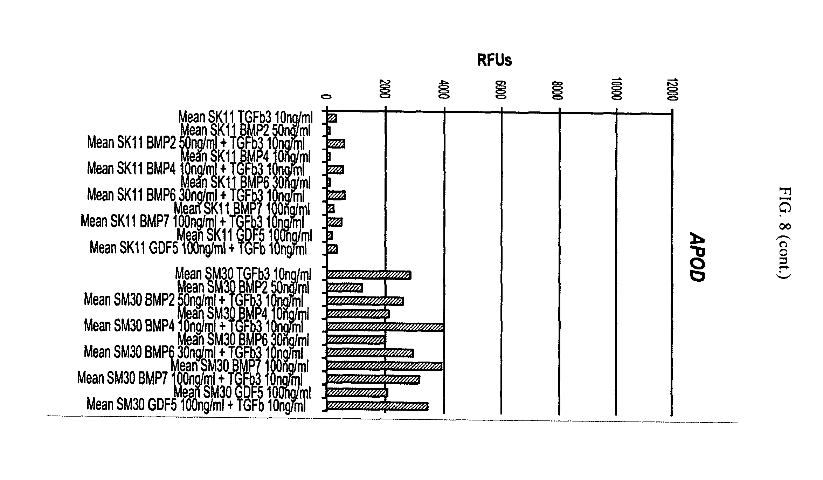

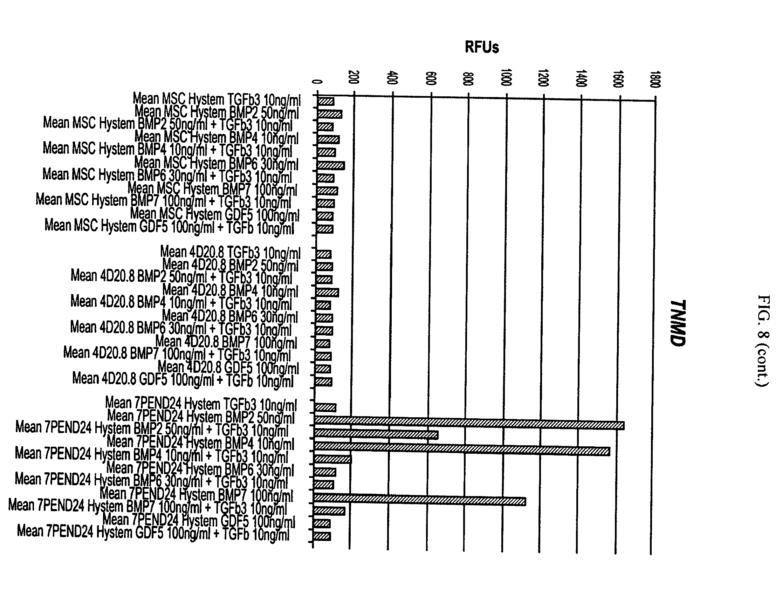

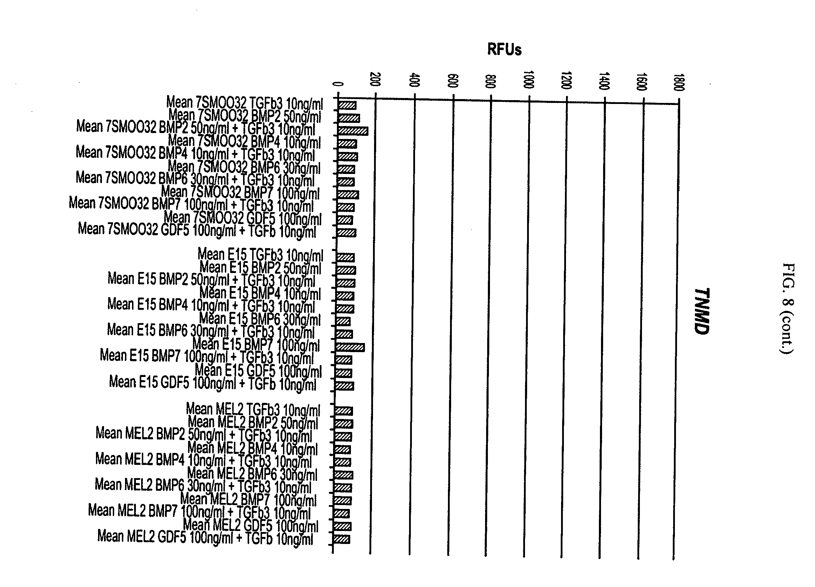

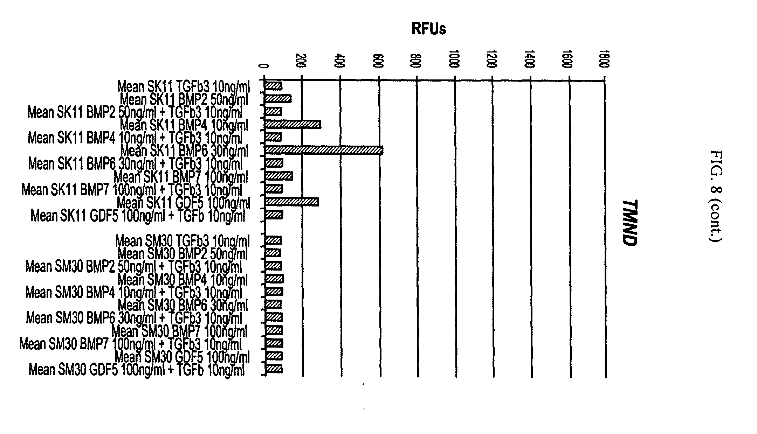

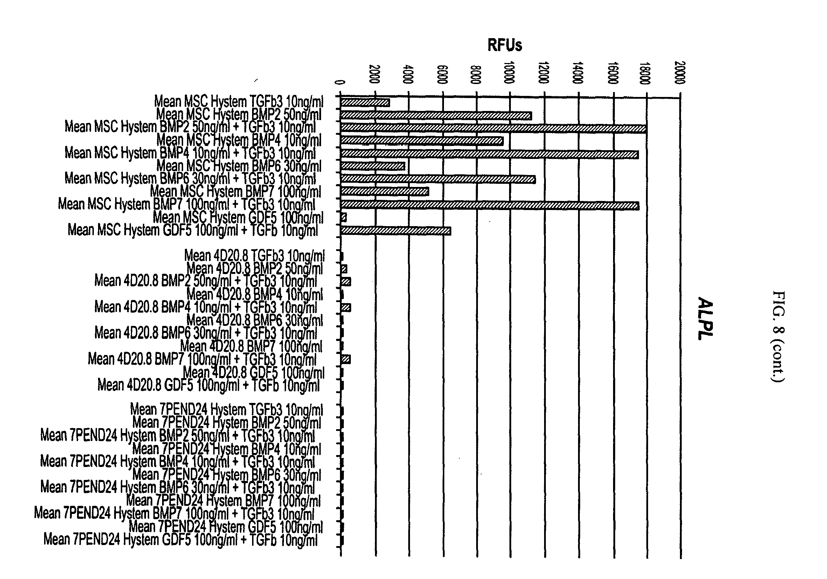

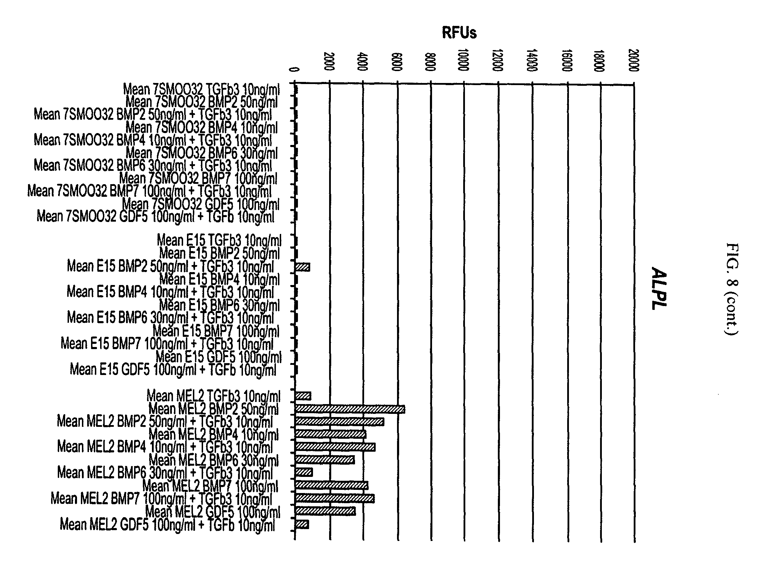

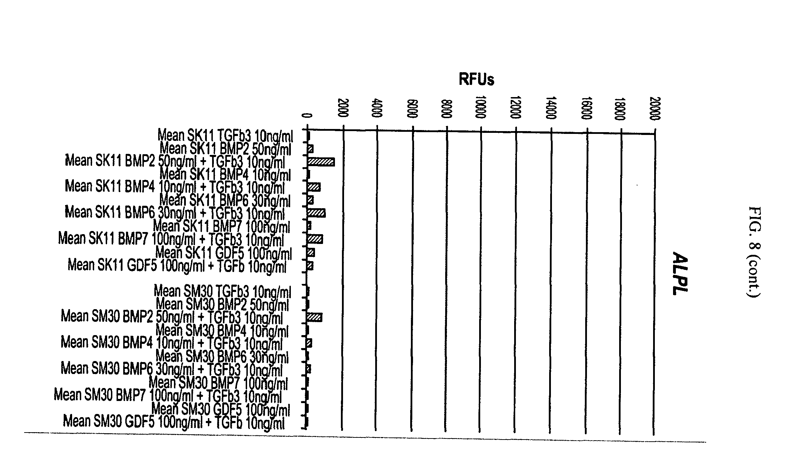

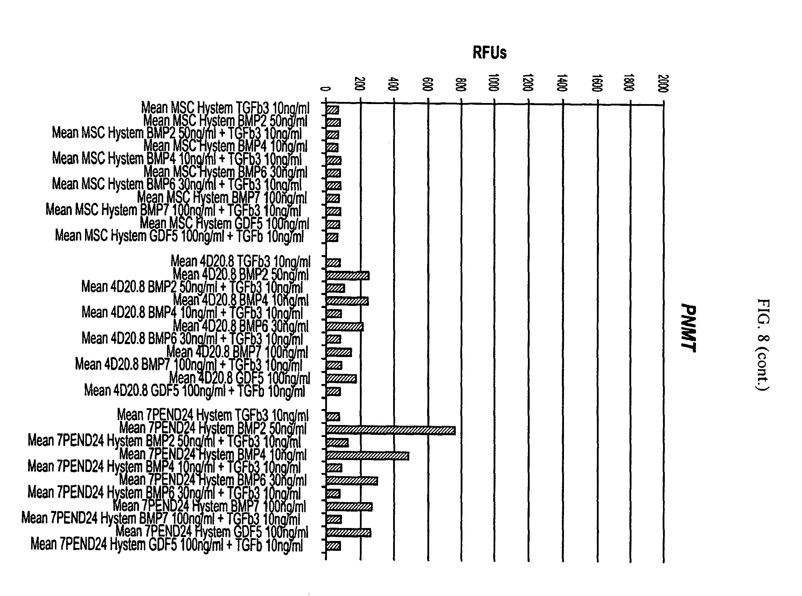

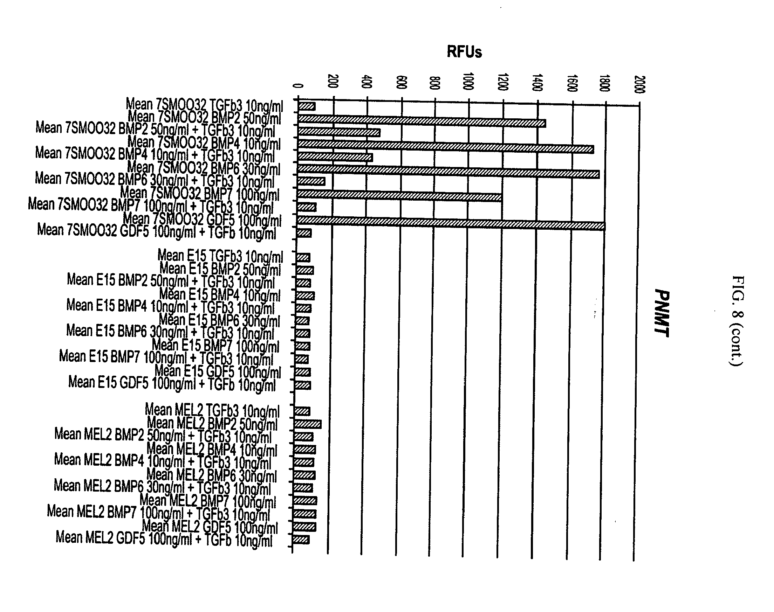

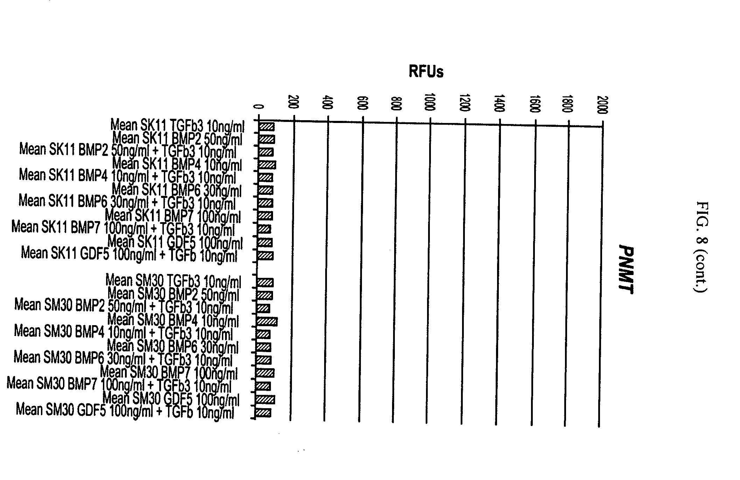

Figure 8: Relative expression levels of APOD, ALPL, TNMD, and PNMT in MSCs and diverse chondrogenic hEP cell lines in the presence of diverse members of the TGF Beta family of growth factors measured by microarray analysis. RNA from MSCs and seven chondrogenic hEP cell lines cultured in D14 HyStem differentiation conditions with TGFβ3 in combination with other TGFβ family members was analyzed by Illumina microarray with values for the genes *APOD, ALPL, TNMD,* and *PNMT.* Data are displayed as mean values of two or more biological replicates. (RFU values <100 considered background signal).

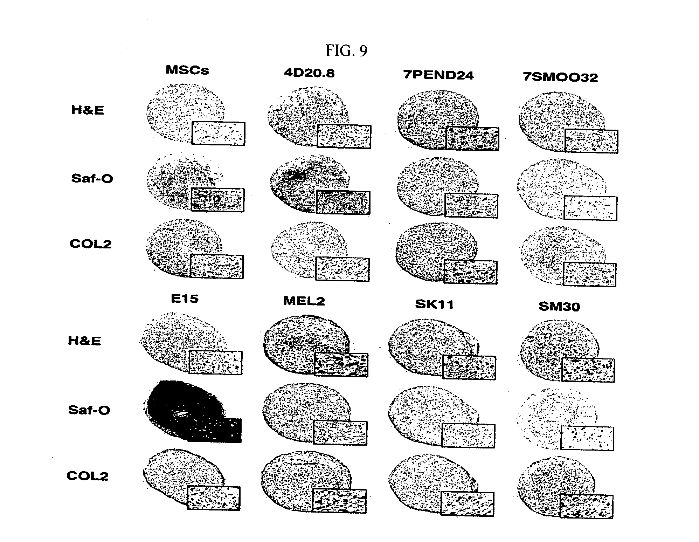

Figure 9. H&E staining, Safranin O and collagen II immunohistochemical staining of differentiated pellet cultures of MSCs and seven chondrogenic hEP cell lines. Differentiated pellets of MSCs, 4D20.8, 7PEND24, 7SMOO32, E15, MEL2, SK11, and SM30 treated for 21 days with TGFβ3 plus GDF5 were stained with H&E, Safranin O, and with anti-collagen II antibody. Pellet diameters approximated 0.8 microns in diameter.

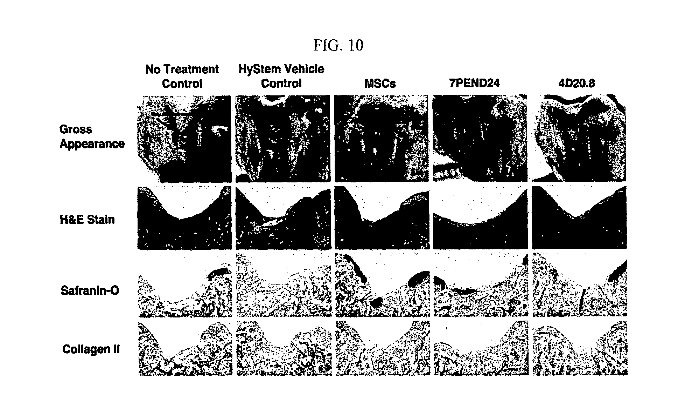

Figure 10. Cartilage repair in vivo using MSCs and chondrogenic hEP cell lines in Hystem hydrogel. (A) Gross appearance of the joint surface at four weeks post-implantation of MSC, 7PEND24, and E15 hydrogel constructs into trochlear groove osteochondral defects. (B) H&E, Safranin-O, and anti-collagen II staining of histological sections.

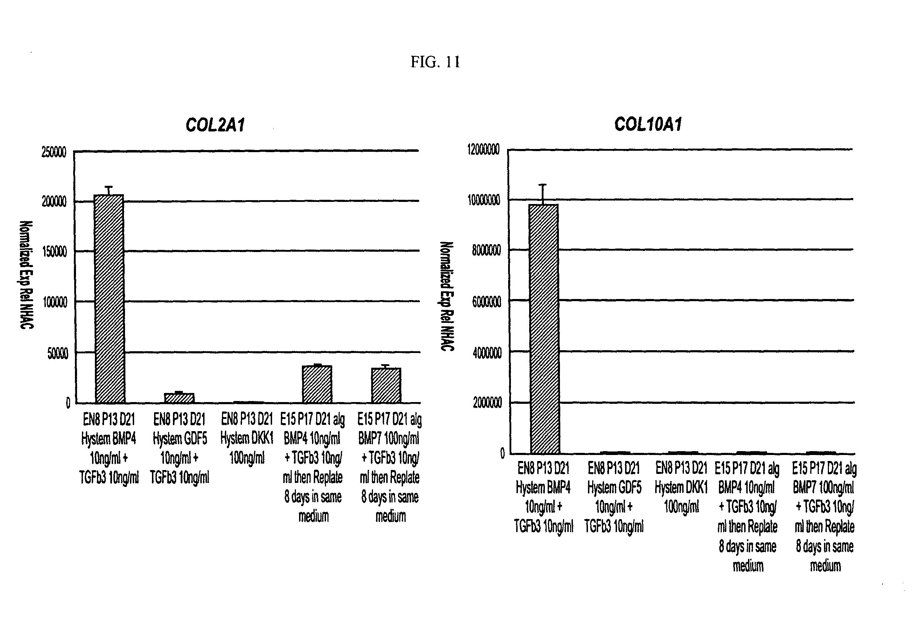

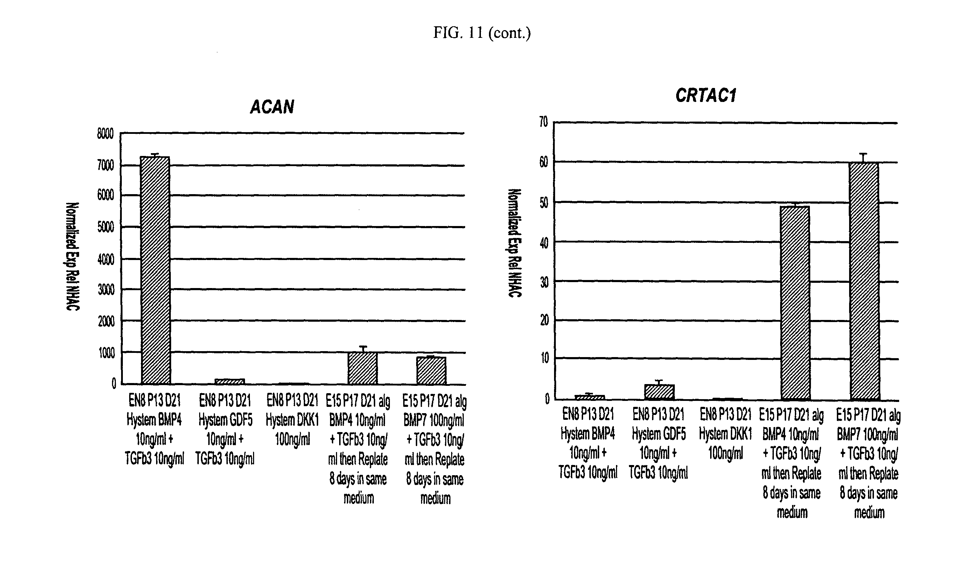

Figure 11: qPCR values for in relative values in the cell line EN8 and E15 compared to culture normal human articular chondrocytes (NHACs) for the genes *COL2A1, COL10A1, ACAN* and *CRTAC1* differentiated for 21 days in HyStem.

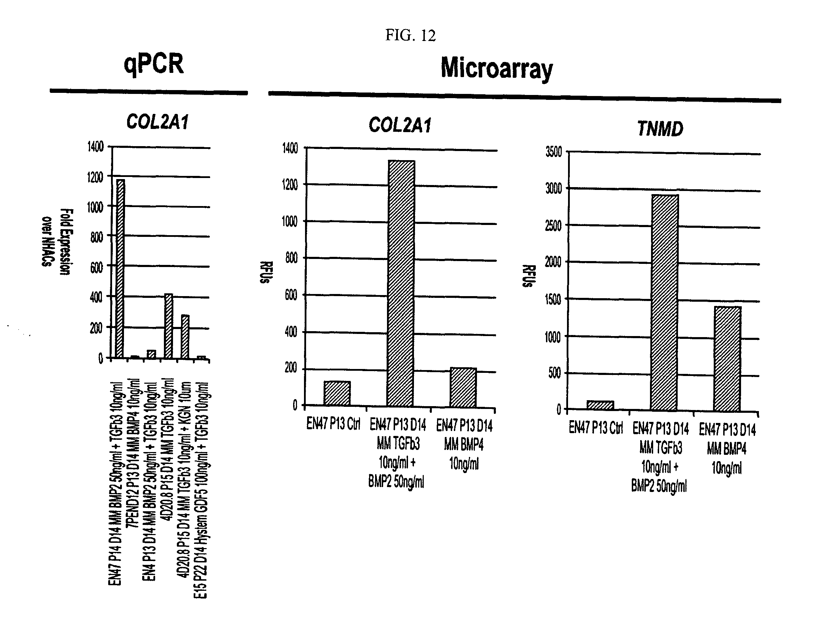

Figure 12: qPCR and Illumina microarray values for *COL2A1* expression (Accession number NM_001844.3) in relative values for EN47 compared to culture normal human articular chondrocytes (NHACs) in the case of qPCR and relative fluorescence units (RFUs) and microarray expression of TNMD. Values below 150 RFUs are considered background signal and therefore negative expression.

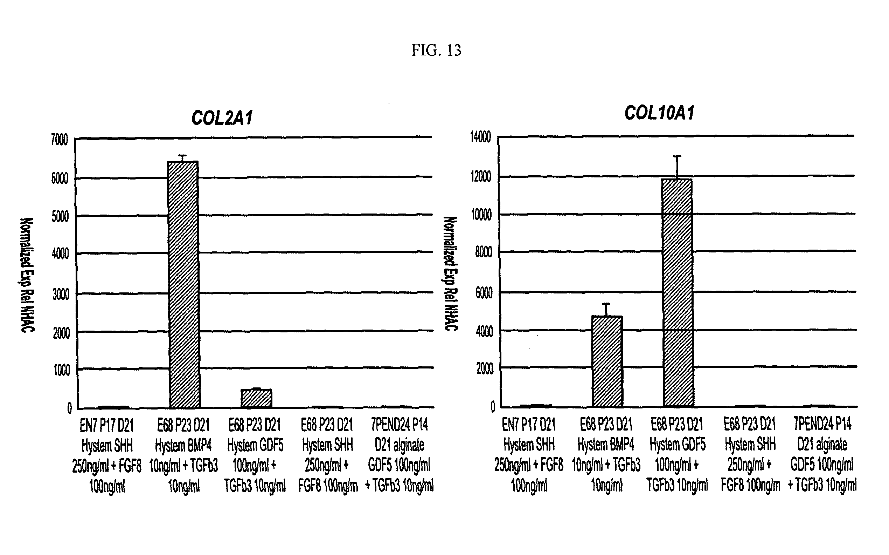

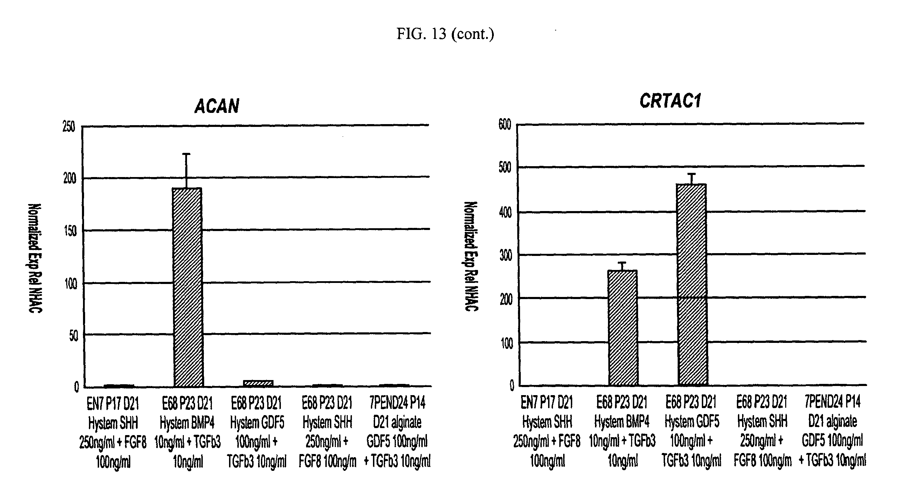

Figure 13: qPCR values for in relative values in the cell line EN68 compared to culture normal human articular chondrocytes (NHACs) for the genes *COL2A1, COL10A1, ACAN* and *CRTAC1* differentiated for 21 days in HyStem.

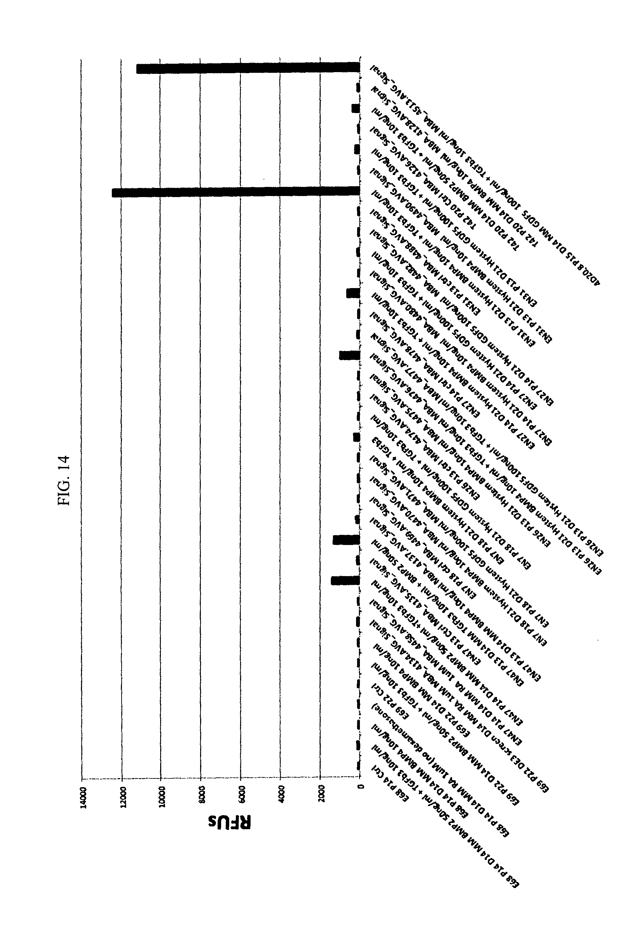

Figure 14: Illumina microarray data for COL2A1 (accession number NM_001844.3, Probe ID 4010136) expression in E68, E69, EN26, EN27, EN31, and T42 compared to 4D20.8.

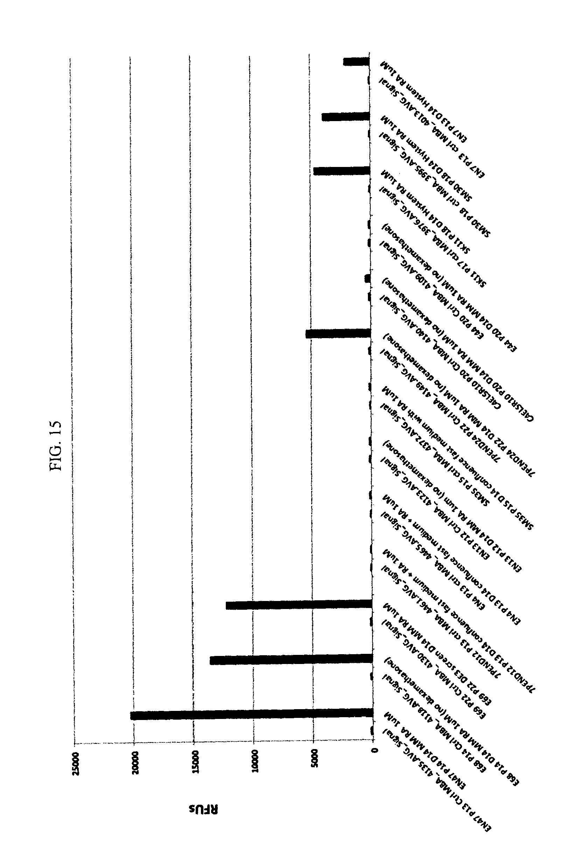

Figure 15: *SFRP4* expression in the indicated cell lines in undifferentiated state vs differentiation with retinoic acid.

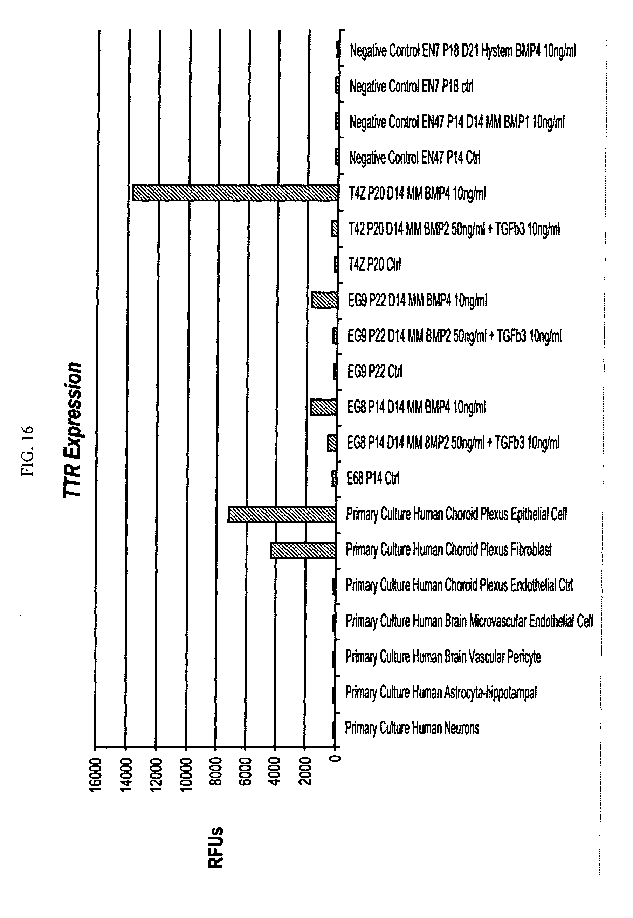

Figure 16: Microarray-based *TTR* expression data in the cell lines E68, E69, and T42 when differentiated as micromasses in the presence of 10ng/mL of BMP4 vs control undifferentiated cells.

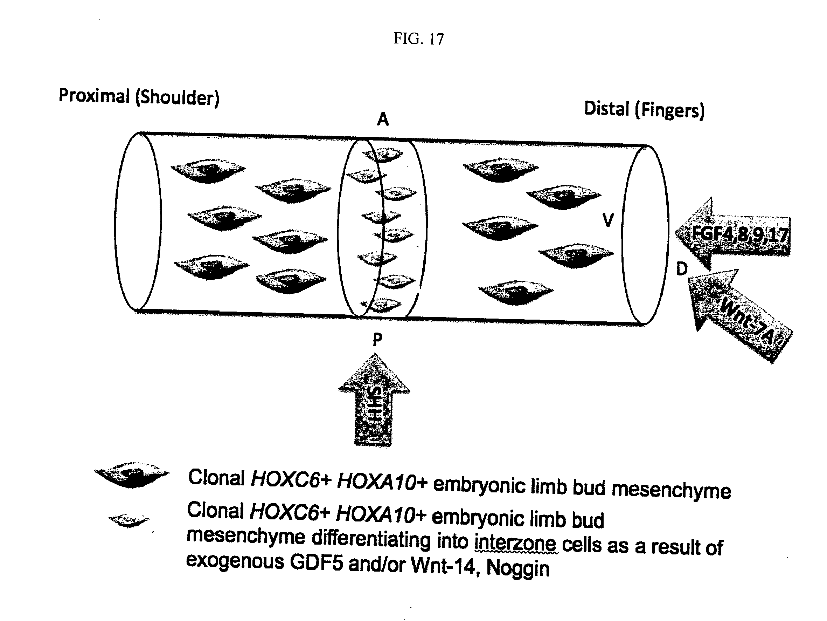

Figure 17: Constructs containing purified human embryonic limb bud progenitor cells embedded in semisolid matrices useful for modeling human limb embryology, screening for teratogens, and for modeling potential strategies for limb regeneration in the human species.

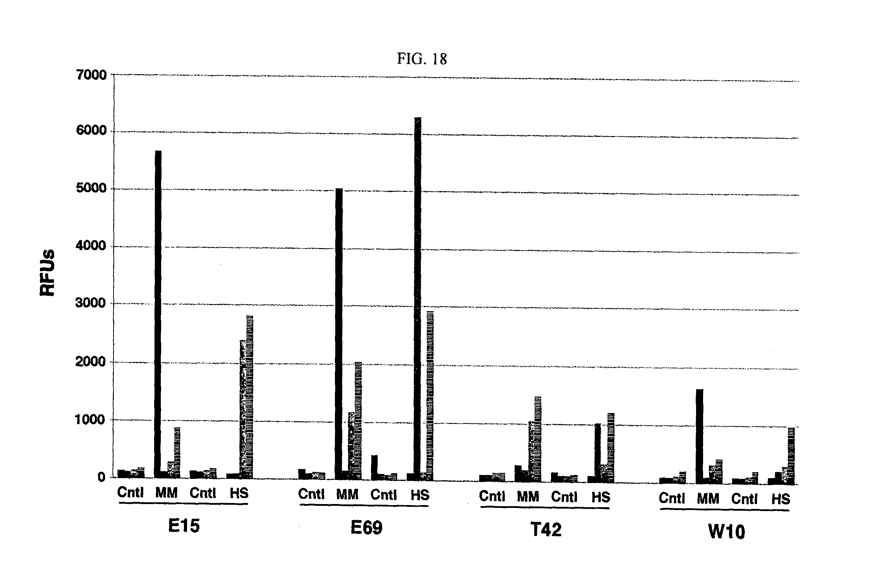

Figure 18: Relative expression levels of MYH11, FABP4, DCN, and TIMP4 in hEP cell lines differentiated in BMP4 in both micromass and HyStem-4D bead arrays. Expression levels of *MYH11* (solid black), *FABP4* (stippled), *DCN* (gray), and *TIMP4* (cross-hatched) are shown for the hEP cell lines E15, E69, T42, and W10 in both micromass and HyStem-4D bead arrays both being supplemented with BMP4. (RFUs, relative fluorescence units; RFU values of <100 considered as background signal). Cntl, Control; MM, micromass; HS, HyStem-C (BioTime, Inc. Alameda, CA) constructs.

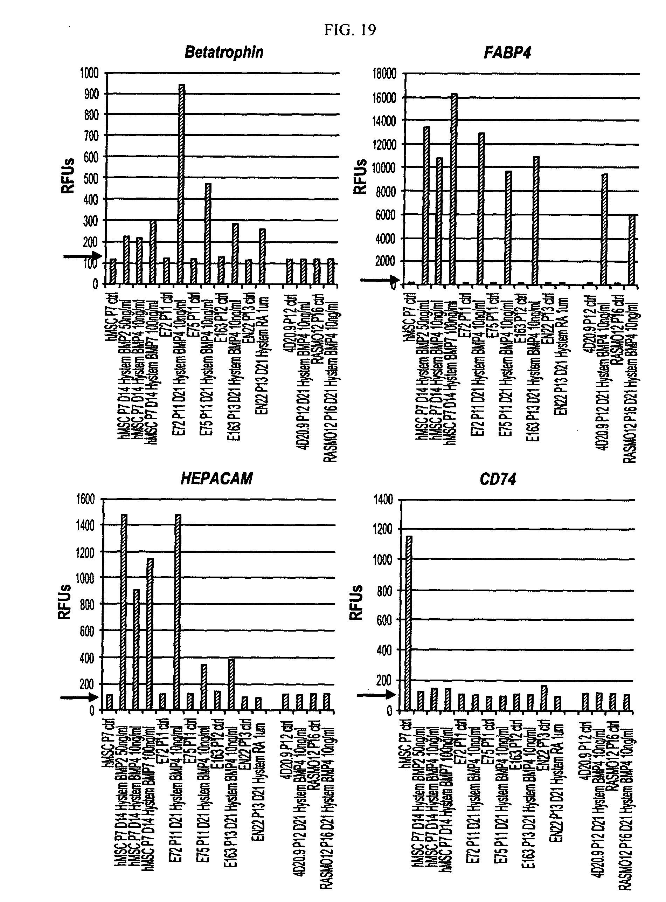

Figure 19: Illumina microarray-based gene expression data for the genes *LOC55908* (also known as *LIPASIN,* also known as *BETATROPHIN* (Accession number NM_018687.3), *FABP4* (Accession number NM_001442.1), *HEPACAM* (Accession number NM_152722.3), and *CD74* (Accession number NM_001025159.1). Relative Fluorescence Units (RFUs) < 130 were considered background signal (designated by red arrow).

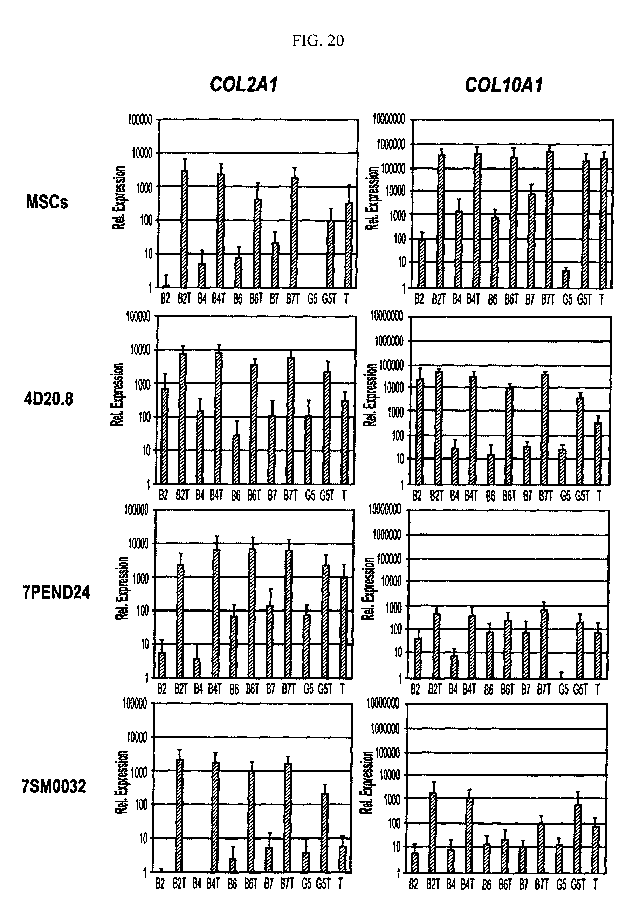

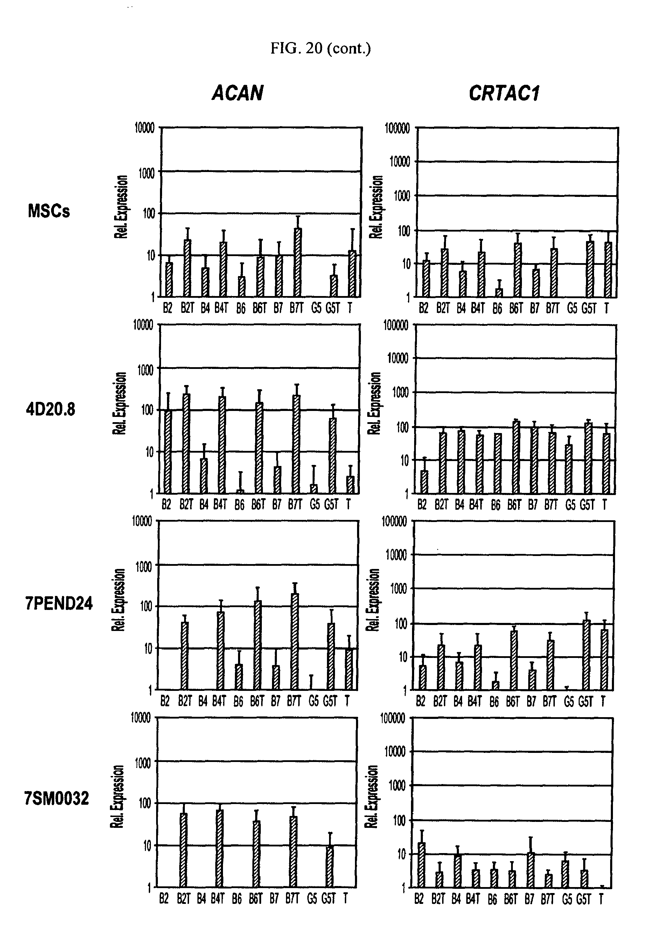

Figure 20: qPCR validation of *COL2A1, COL10A1, ACAN,* and *CRTAC1* gene expression in MSCs and 4D20.8, 7PEND24, and 7SMOO32. Expression is shown as levels relative to cultured NHACs. (Error bar represents standard deviation of 2 or more biological replicates).

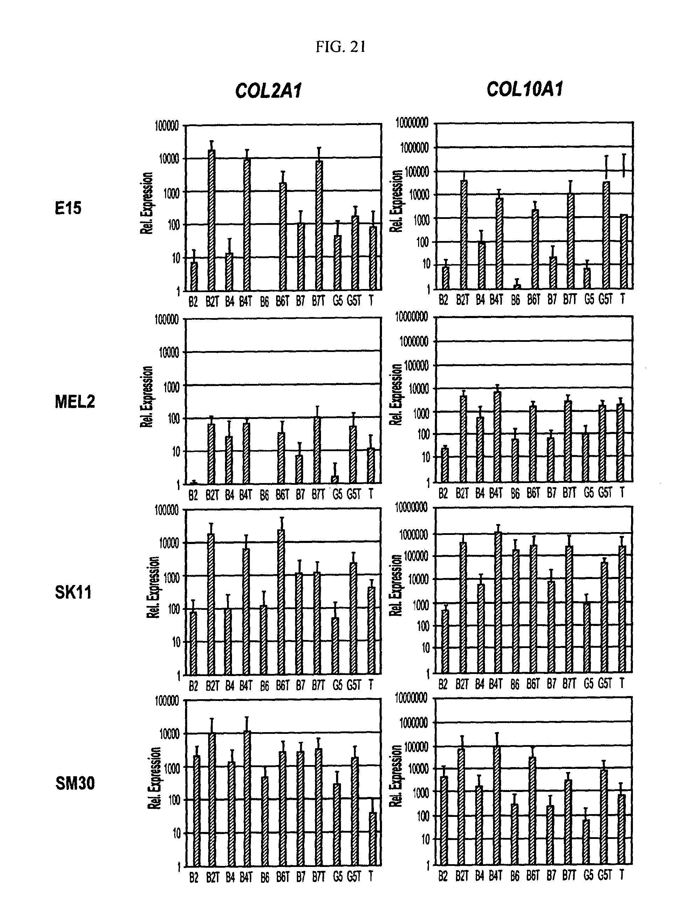

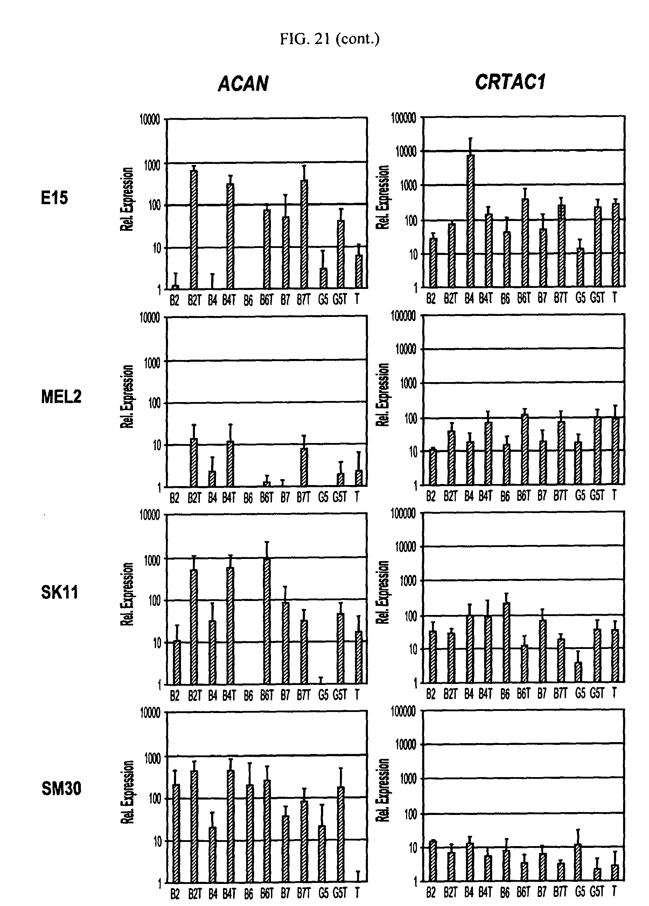

Figure 21: qPCR validation of *COL2A1, COL10A1, ACAN,* and *CRTAC1* gene expression in E15, MEL2, SK11, and SM30. Expression is shown as levels relative to cultured NHACs. (Error bar represents standard deviation of two or more biological replicates).

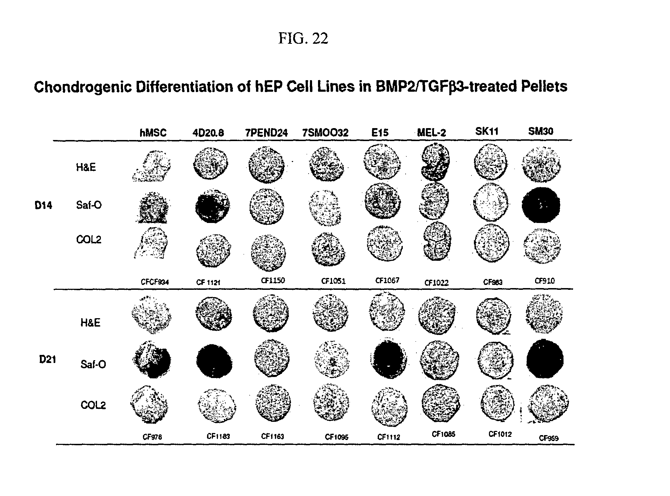

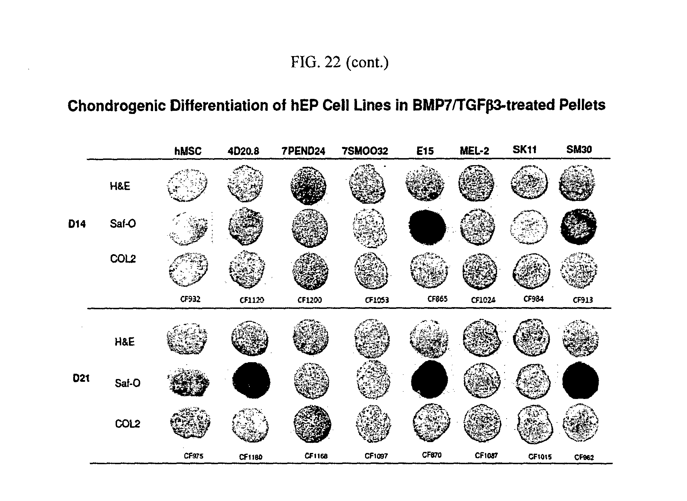

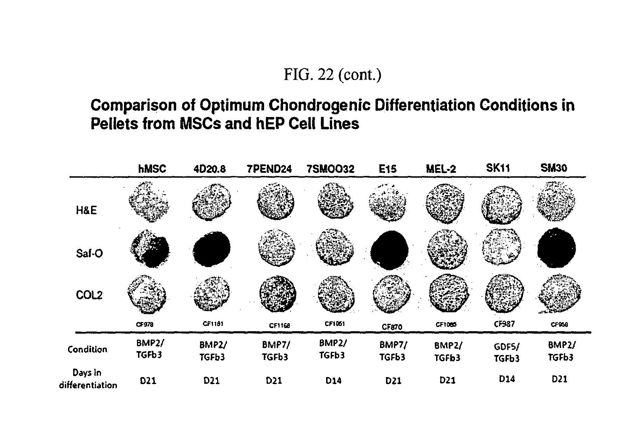

Figure 22: H&E, Safranin-O, and anti-collagen II staining of MSC and chondrogenic hEP cell lines in pellet culture with TGFβ3 in combination with other TGFβ family members. Histology of cell line pellets stained for H&E, Safranin-O, and anti-collagen II reactivity when differentiated in the presence of 10 ng/mL TGFβ3 in various combination with 50 ng/mL BMP-2, 100 ng/mL BMP-7 or 100 ng/ml GDF5. Pellet diameters are approximately 0.8 mm.

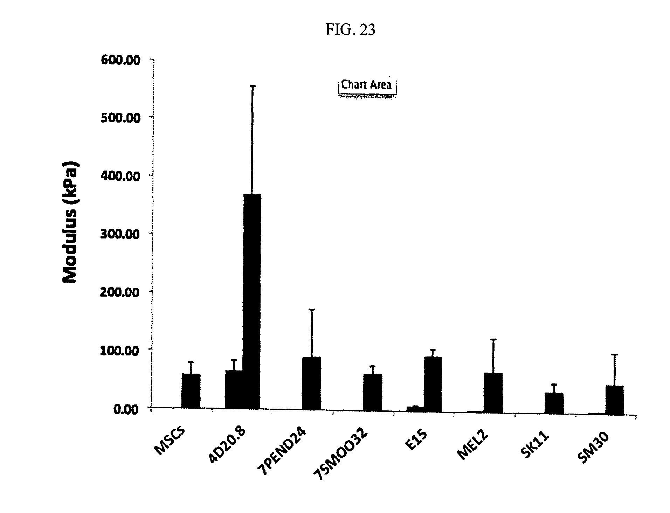

Figure 23. Mechanical properties of MSCs and chondrogenic hEP cell-seeded HyStem-C (BioTime, Inc. Alameda, CA) hydrogel constructs. The equilibrium modulus and dynamic modulus were tested in HyStem-C (BioTime, Inc. Alameda, CA) hydrogel-cell constructs following 42 days of differentiation in 100 ng/mL GDF5 and 10 ng/mL of TGFβ3-containing medium. (mean ± standard deviation of the mean; n=3).

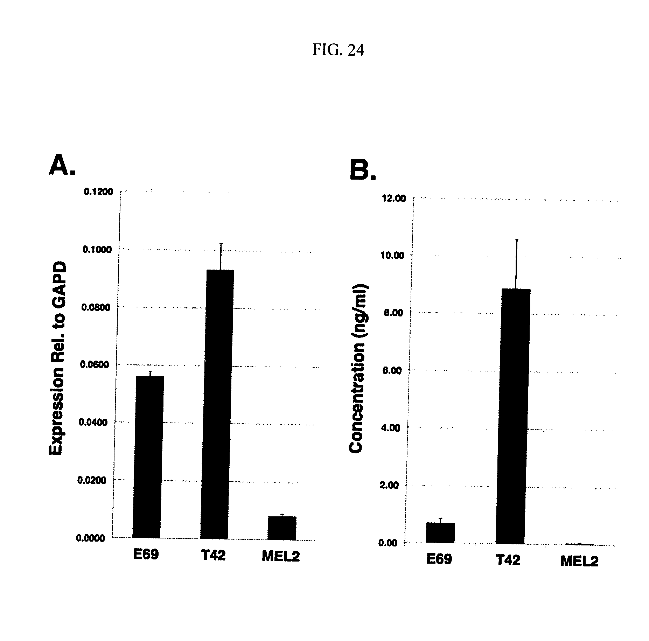

Figure 24. Relative levels of expression of transthyretin in cells as determined by RT-qPCR and ELISA. A) RNA from the hEP cell clones E69, T42, and MEL2 differentiated for 14 days in HyStem-4D beads in the presence of 10 ng/mL BMP4 was analyzed by RT-qPCR for TTR transcript. B) Three day serum-free conditioned medium from 14 day HyStem-4D bead arrays was analyzed by ELISA to quantitate the concentration of transthyretin. (Error bars represent standard deviation).

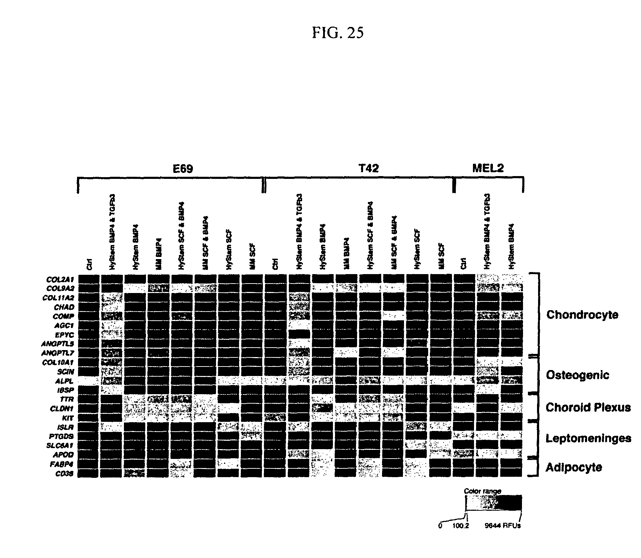

Figure 25. Heat map of osteochondral, meningeal, adipose, and choroid plexus markers in E69, T42, and MEL2 cells cultured in diverse differentiation conditions. RFU values of select markers of osteochondral, meningeal, adipose, and choroid plexus differentiation obtained by microarray analysis are displayed. E69, T42, and MEL2 cells were differentiated for 14 days in either HyStem-4D bead arrays or MM conditions in the presence of added growth factors shown. (Color key shows associated ranges of RFU values).

### DEFINITIONS AND ABBREVIATIONS

### Definitions

The term "analytical reprogramming technology" refers to a variety of methods to reprogram the pattern of gene expression of a somatic cell to that of a more pluripotent state, such as that of an iPS, ES, ED, EC or EG cell, wherein the reprogramming occurs in multiple and discrete steps and does not rely simply on the transfer of a somatic cell into an oocyte and the activation of that oocyte (see U.S. application nos. 60/332,510, filed November 26, 2001; 10/304,020, filed November 26, 2002; PCT application no. PCT/US02/37899, filed November 26, 2003; U.S. application no. 60/705625, filed August 3, 2005; U.S. application no. 60/729173, filed August 20, 2005; U.S. application no. 60/818813, filed July 5, 2006, PCT/US06/30632, filed August 3, 2006).

The term "blastomere/morula cells" refers to blastomere or morula cells in a mammalian embryo or blastomere or morula cells cultured in vitro with or without additional cells including differentiated derivatives of those cells.

The term "cell expressing a gene" as used herein means that the expression of the gene was at least above the background signal obtained by at least one assay for measuring gene expression.

The term "cell line" refers to a mortal or immortal population of cells that is capable of propagation and expansion in vitro.

The term "cellular reconstitution" refers to the transfer of a nucleus of chromatin to cellular cytoplasm so as to obtain a functional cell.

The term "clonal" refers to a population of cells obtained the expansion of a single cell into a population of cells all derived from that original single cells and not containing other cells.

The term "colony in situ differentiation" refers to the differentiation of colonies of cells (e.g., hES, hEG, hiPS, hEC or hED) in situ without removing or disaggregating the colonies from the culture vessel in which the colonies were propagated as undifferentiated stem cell lines. Colony in situ differentiation does not utilize the intermediate step of forming embryoid bodies, though embryoid body formation or other aggregation techniques such as the use of spinner culture may nevertheless follow a period of colony in situ differentiation.

The term "cytoplasmic bleb" refers to the cytoplasm of a cell bound by an intact or permeabilized but otherwise intact plasma membrane, but lacking a nucleus.

The term "differentiated cells" when used in reference to cells made by methods of this invention from pluripotent stem cells refer to cells having reduced potential to differentiate when compared to the parent pluripotent stem cells. The differentiated cells of this invention comprise cells that could differentiate further (i.e., they may not be terminally differentiated).

The term "direct differentiation" refers to process of differentiating. e.g., blastomere cells, morula cells, ICM cells, ED cells, or somatic cells reprogrammed to an undifferentiated state (such as in the process of making iPS cells but before such cells have been purified in an undifferentiated state) directly without the intermediate state of propagating isolated undifferentiated stem cells such as hES cells as undifferentiated cell lines. A nonlimiting example of direct differentiation would be the culture of an intact human blastocyst into culture and the derivation of ED cells without the generation of a human ES cell line as was described (Bongso et al, 1994. Human Reproduction 9:2110).

The term "embryonic stem cells" (ES cells) refers to cells derived from the inner cell mass of blastocysts, blastomeres, or morulae that have been serially passaged as cell lines while maintaining an undifferentiated state (e.g. expressing TERT, OCT4, and SSEA and TRA antigens specific for ES cells of the species). The ES cells may be derived from fertilization of an egg cell with sperm or DNA, nuclear transfer, parthenogenesis, or by means to generate hES cells with hemizygosity or homozygosity in the MHC region. While ES cells have historically been defined as cells capable of differentiating into all of the somatic cell types as well as germ line when transplanted into a preimplantation embryo, candidate ES cultures from many species, including human, have a more flattened appearance in culture and typically do not contribute to germ line differentiation, and are therefore called "ES-like cells." It is commonly believed that human ES cells are in reality "ES-like", however, in this application we will use the term ES cells to refer to both ES and ES-like cell lines.

The term "human embryo-derived" ("hED") cells refers to blastomere-derived cells, morula-derived cells, blastocyst-derived cells including those of the inner cell mass, embryonic shield, or epiblast, or other totipotent or pluripotent stem cells of the early embryo, including primitive endoderm, ectoderm, mesoderm, and neural crest and their derivatives up to a state of differentiation correlating to the equivalent of the first eight weeks of normal human development, but excluding cells derived from hES cells that have been passaged as cell lines (see, e.g., U.S. Patents 7,582,479; 7,217,569; 6,887,706; 6,602,711; 6,280,718; and 5,843,780 to Thomson). The hED cells may be derived from preimplantation embryos produced by fertilization of an egg cell with sperm or DNA, nuclear transfer, or chromatin transfer, an egg cell induced to form a parthenote through parthenogenesis, analytical reprogramming technology, or by means to generate hES cells with hemizygosity or homozygosity in the HLA region. The term "human embryonic germ cells" (hEG cells) refer to pluripotent stem cells derived from the primordial germ cells of fetal tissue or maturing or mature germ cells such as oocytes and spermatogonial cells, that can differentiate into various tissues in the body. The hEG cells may also be derived from pluripotent stem cells produced by gynogenetic or androgenetic means, i.e., methods wherein the pluripotent cells are derived from oocytes containing only DNA of male or female origin and therefore will comprise all female-derived or male-derived DNA (see U.S. application nos. 60/161,987, filed October 28, 1999; 09/697,297, filed October 27, 2000; 09/995,659, filed November 29,2001; 10/374,512, filed February 27, 2003; PCT application no. PCT/US/00/29551, filed October 27, 2000).

The term "human embryonic stem cells" (hES cells) refers to human ES cells.

The term "human iPS cells" refers to cells with properties similar to hES cells, including the ability to form all three germ layers when transplanted into immunocompromised mice wherein said iPS cells are derived from cells of varied somatic cell lineages following exposure to de-differentiation factors, for example hES cell-specific transcription factor combinations: KLF4, SOX2, MYC, and OCT4 or SOX2, OCT4, NANOG, and LIN28. Any convenient combination of de-differentiation factors may be used to produce iPS cells. Said iPS cells may be produced by the expression of these genes through vectors such as retroviral, lentiviral or adenoviral vectors as is known in the art, or through the introduction of the factors as proteins, e.g., by permeabilization or other technologies. For descriptions of such exemplary methods see: PCT application number PCT/US2006/030632, filed on August 3, 2006; U.S. Application Ser. No. 11/989,988; PCT Application PCT/US2000/018063, filed on June 30, 2000; U.S. Application Ser. No. 09,736,268 filed on December 15, 2000; U.S. Application Ser. No. 10/831,599, filed April 23, 2004; and U.S. Patent Publication 20020142397 (App. Ser. No. 10/015,824, entitled "Methods for Altering Cell Fate"); U.S. Patent Publication 20050014258 (App. Ser. No. 10/910,156, entitled "Methods for Altering Cell Fate"); U.S. Patent Publication 20030046722 (App. Ser. No. 10/032,191, entitled "Methods for cloning mammals using reprogrammed donor chromatin or donor cells"); and U.S. Patent Publication 20060212952 (App. Ser. No. 11/439,788, entitled "Methods for cloning mammals using reprogrammed donor chromatin or donor cells").

The term "ICM cells" refers to the cells of the inner cell mass of a mammalian embryo or the cells of the inner cell mass cultured in vitro with or without the surrounding trophectodermal cells.

The term "oligoclonal" refers to a population of cells that originated from a small population of cells, typically 2-1000 cells, that appear to share similar characteristics such as morphology or the presence or absence of markers of differentiation that differ from those of other cells in the same culture. Oligoclonal cells are isolated from cells that do not share these common characteristics, and are allowed to proliferate, generating a population of cells that are essentially entirely derived from the original population of similar cells.

The term "osteochondral cell," as used herein, refers to a cell expressing at least one gene expressed by a chondrocyte, a chondrocyte precursor, an osteoblast, an osteoblast precursor, or a mature bone forming cell and having the capability of differentiating into an osteoblast, a mature bone forming cell and/or a chondrocyte.

The term "pluripotent stem cells" refers to animal cells capable of differentiating into more than one differentiated cell type. Such cells include hES cells, blastomere/morula cells and their derived hED cells, hiPS cells, hEG cells, hEC cells, and adult-derived cells including mesenchymal stem cells, neuronal stem cells, and bone marrow-derived stem cells. Pluripotent stem cells may be genetically modified or not genetically modified. Genetically modified cells may include markers such as fluorescent proteins to facilitate their identification within the egg.

The term "pooled clonal" refers to a population of cells obtained by combining two or more clonal populations to generate a population of cells with a uniformity of markers such as markers of gene expression, similar to a clonal population, but not a population wherein all the cells were derived from the same original clone. Said pooled clonal lines may include cells of a single or mixed genotypes. Pooled clonal lines are especially useful in the cases where clonal lines differentiate relatively early or alter in an undesirable way early in their proliferative lifespan.

The term "primordial stem cells" refers collectively to pluripotent stem cells capable of differentiating into cells of all three primary germ layers: endoderm, mesoderm, and ectoderm, as well as neural crest. Therefore, examples of primordial stem cells would include but not be limited by human or non-human mammalian ES cells or cell lines, blastomere/morula cells and their derived ED cells, iPS, and EG cells.

"Subject" as used herein includes, but is not limited to, humans, non-human primates and non-human vertebrates such as wild, domestic and farm animals including any mammal, such as cats, dogs, cows, sheep, pigs, horses, rabbits, rodents such as mice and rats. In some embodiments, the term "subject," "patient" or "animal" refers to a male. In some embodiments, the term "subject," "patient" or "animal" refers to a female.

The terms "treat," "treated," or "treating" as used herein can refer to both therapeutic treatment or prophylactic or preventative measures, wherein the object is to prevent or slow down (lessen) an undesired physiological condition, symptom, disorder or disease, or to obtain beneficial or desired clinical results. In some embodiments, the term may refer to both treating and preventing. For the purposes of this disclosure, beneficial or desired clinical results include, but are not limited to, alleviation of symptoms; diminishment of the extent of the condition, disorder or disease; stabilization (*i.e*., not worsening) of the state of the condition, disorder or disease; delay in onset or slowing of the progression of the condition, disorder or disease; amelioration of the condition, disorder or disease state; and remission (whether partial or total), whether detectable or undetectable, or enhancement or improvement of the condition, disorder or disease. Treatment includes eliciting a clinically significant response without excessive levels of side effects. Treatment also includes prolonging survival as compared to expected survival if not receiving treatment.

The term "tissue regeneration" refers to at least partial regeneration, replacement, restoration, or regrowth of a tissue, organ, or other body structure, or portion thereof, following loss, damage, or degeneration, where said tissue regeneration but for the methods described in the present invention would not take place. Examples of tissue regeneration include the regrowth of severed digits or limbs including the regrowth of cartilage, bone, muscle, tendons, and ligaments, the scarless regrowth of bone, cartilage, skin, or muscle that has been lost due to injury or disease, with an increase in size and cell number of an injured or diseased organ such that the tissue or organ approximates the normal size of the tissue or organ or its size prior to injury or disease. Depending on the tissue type, tissue regeneration can occur via a variety of different mechanisms such as, for example, the rearrangement of pre-existing cells and/or tissue (e.g., through cell migration), the division of adult somatic stem cells or other progenitor cells and differentiation of at least some of their descendants, and/or the dedifferentiation, transdifferentiation, and/or proliferation of cells.

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

### Abbreviations

- cGMP: - Current Good Manufacturing Processes
- CNS: - Central Nervous System
- DMEM: - Dulbecco's modified Eagle's medium
- DMSO: - Dimethyl sulphoxide
- DPBS: - Dulbecco's Phosphate Buffered Saline
- EC: - Embryonal carcinoma
- EC Cells: - Embryonal carcinoma cells; hEC cells are human embryonal carcinoma cells
- ECM: - Extracellular Matrix
- ED Cells: - Embryo-derived cells; hED cells are human ED cells
- EDTA: - Ethylenediamine tetraacetic acid
- EG Cells: - Embryonic germ cells; hEG cells are human EG cells
- ES Cells: -Embryonic stem cells; hES cells are human ES cells. ES cells, including hES cells for the purposes of this invention may be in a naive state corresponding to ICM cells of the human blastocyst, or the primed state corresponding to flattened epiblast cells (sometimes referred to as "ES-like" cells).
- FACS: - Fluorescence activated cell sorting
- FBS: - Fetal bovine serum
- GMP: - Good Manufacturing Practices
- H&E: - Hematoxylin & Eosin
- hED Cells: - Human embryo-derived cells
- hEG Cells: - Human embryonic germ cells are stem cells derived from the primordial germ cells of fetal tissue.
- hEP Cells: - Human embryonic progenitor cells
- hiPS Cells: - Human induced pluripotent stem cells are cells with properties similar to hES cells obtained from somatic cells after exposure to hES-specific transcription factors such as *SOX2, KLF4, OCT4, MYC,* or *NANOG, LIN28, OCT4,* and *SOX2.*

- HSE: - Human skin equivalents are mixtures of cells and biological or synthetic matrices manufactured for testing purposes or for therapeutic application in promoting wound repair.
- ICM: - Inner cell mass of the mammalian blastocyst-stage embryo.
- iPS Cells: - Induced pluripotent stem cells are cells with properties similar to hES cells obtained from somatic cells after exposure to ES-specific transcription factors such as *SOX2, KLF4, OCT4, MYC,* or *NANOG, LIN28, OCT4,* and *SOX2.*
- MEM: - Minimal essential medium
- MSCs: - Mesenchymal Stem Cells
- NHACs: - Normal human articular chondrocytes
- NT: - Nuclear Transfer
- PBS: - Phosphate buffered saline
- PCR: - Polymerase Chain Reaction
- PNS: - Peripheral Nervous System
- qRT-PCR: - quantitative real-time polymerase chain reaction
- RFU: - Relative Fluorescence Units
- SCNT: - Somatic Cell Nuclear Transfer
- SFM: - Serum-Free Medium

### DETAILED DESCRIPTION

There is a growing need for improved methods of generating progenitor cell types from ES and iPS cells that maintain a uniform differentiated state, and preserve site-specific homeobox gene expression. Techniques such as the clonal propagation of human embryonic progenitor (hEP) cell lines may facilitate the derivation of purified and scalable cell lines corresponding to regional anlagen of diverse tissue types for use in research and therapy. In addition, the standardization of research around such defined and scalable progenitors may improve the reproducibility of differentiation studies from laboratory-to-laboratory. A future trend may therefore be the increased utilization of hEP cell lines in research and as a means of scaling clinical-grade cellular formulations.

Provided herein are compositions comprising hEP cells and their differentiated progeny as well as methods for directing the differentiation of hEP cells to a specific phenotype and genotype.

### Progenitor Cell Lines

Progenitor cells and progenitor cell lines are used interchangeably herein and refer to cultures of cells that can be propagated for at least 5 passages, but nevertheless are mortal and eventually senesce due to telomere shortening.

Certain embodiments of the invention provide progenitor cell lines, methods of making progenitor cell lines and methods of using progenitor cell lines. Progenitor cell lines may, in some embodiments, be the progeny, such as the in vitro progeny, of an embryonic stem cell (e.g. an ES cell such as a hES cell) or an iPS cell. The ES cell or iPS cell may be obtained from a mammal, such as a primate. In one embodiment the ES or iPS cell is of human origin. The progenitor cell may be obtained from an established ES cell line available from cell bank, such as WiCell or BioTime, Inc.. The progenitor cell may be obtained from ES cell line generated without destroying an embryo or an in vitro fertilized egg (Chung et al. Cell Stem Cell (2008) 2:113).

Progenitor cells may include clonal or oligo-clonal progenitor cell lines. Progenitior cells may have the ability to replicate in culture through multiple passages. In some embodiments of the invention the progenitor cells may be passaged about 1-100 times, about 5-90 times, about 10-80 times, about 20-70 times, about 30-60 times, about 40-50 times. In some embodiments the progenitor cells may be passaged about 5 times, about 10 times, about 11 times, about 12 times, about 13 times, about 14 times, about 15 times, about 16 times, about 17 times, about 18 times, about 19 times, about 20 times, about 21 times, about 22 times, about 23 times, about 24 times, about 25 times, about 30 times, about 40 times, about 50 times or more.

In certain embodiments the invention provides progenitor cell lines that have the ability to differentiate into cells found in an animal body, such as a human. Differentiation may be induced for example, by altering the culture conditions in which the progenitor cells are typically maintained. For example, growth factors, cytokines, mitogens or the like may be added or removed from the culture media.

In some embodiments the progenitor cells are multipotent cells. In some embodiments the progenitor cells are not pluripotent cells. In some embodiments the progenitor cells are not mesenchymal stem cells (MSC). In some embodiments the progenitor cells do not express one or more markers found on a mesenchymal stem cell. In some embodiments the progenitor cells express one or more markers found on an MSC at level that is lower than the expression level found on an MSC. In some embodiments of the invention the progenitor cells do not express CD74. In some embodiments of the invention the progenitor cells express CD74 at level that is lower than the level found on an MSC. In some embodiments the progenitor cell lines express one or more genes expressed by a chondrocyte or a chondrocyte precursor.

In certain embodiments the invention provides a progenitor cell chosen from 4D20.8, 4D20.9, 7PEND24, 7PEND30, 7SMOO32, E15, MEL2, SK5, SK11, SK44, SK50, EN18, EN26, EN27, EN31, EN47, E19, E44, E68, E69, E72, E75, E120, E163, F15, T7, T14, T20, T42, T44, U18, RAD20.5, SM22 and SM30. In some embodiments the progenitor cells may have the capability to differentiate into one or more tissue types or tissue type precursors such as bone, tendon, ligament, fat, muscle and chondrocytes.

In some embodiments the invention provides a progenitor cell line expressing one or more genes chosen from COL2A1, LHX8, BARX1, CD29 (ITGB1), CD45 (PTPRC), CD73 (NT5E), CD90 (THY1), CD105 (ENG), FGF18, CEBPD, CHRNA3, GRM1, LHX1, MSX2, BBOX1, DLK1, BMP5, EGFL6, AJAP1, ALDH1AZ, ZIC2, PITX1, PITX2, PPAR6, SNAI2, FOXF1, TWIST1, TBX15, HOXB2, HOXA2, HOXB2, HOXA6 and HOXB6. In certain embodiments the progenitor cell lines of the invention do not express one or more genes chosen from HOXA11, HOXB7, HOXC10, and HOXD4.

In yet other embodiments the invention provide a progenitor cell line that expresses one or more genes chosen from *FMO3, FOXF1, GABRB1, NEFM, POSTN, RGS1, SOD3, TFPI2,* and *ZIC2, PI16,* TNMD, COL2A1, *HOXA2* and *HOXB2.* The in vitro progeny of the progenitor cell line may be negative for the gene expression markers: *ALDH1A1, BARX1, CD24, CD69, CD74, FOXF2, FOXS1, GSC, LHX8, PAX9, PENK,* and *TBX15.* The progenitor cell line may have the potential to differentiate into a chondrocyte or tendon, or precursor thereof.

In still other embodiments the invention provides a progenitor cell line that expresses one or more genes chosen from: *FOXS1, KCNIP1, KRT17, TFAP2 C,* and *ZIC2.* In some embodiments the progenitor cell line does not express the genes *HOXA2* or *HOXB2.* In some embodiments the progenitor cell line may express *UGT2B7.* In other embodiments the progenitor cell line may not express *UGT2B7.* In some embodiments the progenitor cell line may express *NNAT,* while in other embodiments the progenitor cell line may not express *NNAT.*

In yet other embodiments the invention provides a progenitor cell line expressing one or more genes chosen from: *CD69, FOXF1, FOXF2.* In some embodiments the invention provides a progenitor cell that does not express one or more genes chosen from *NEFM,* or *ZIC2* and *HOXA2* or *HOXB2.* In some embodiments the progenitor cell line expressed the gene *HCLS1.* In other embodiments the progenitor cell line did not express *HCLS1.* In some embodiments the progenitor cell line expressed the gene *HEPH.* In other embodiments the progenitor cell line did not express while the line *HEPH.*

In further embodiments the invention provides a progenitor cell line expressing one or more genes chosen from: *CD69, CST1, FOXF1, OSR2,* and, ZIC2 and the *HOX* genes *HOXA2* and *HOXB2.* In some embodiments the progenitor cell line may not express one or more genes chosen from: *NEFM* and *TH.* In other embodiments the progenitor cell line may express low but detectable levels of *CD74* transcript, a gene abundantly expressed in MSCs but not most connective tissue cells. In further embodiments the progenitor cell line may not express the gene proenkephalin (*PENK*) which was expressed in MSCs.

In still other embodiments the invention provides a progenitor cell line expressing one or more genes chosen from: *EPHA5, HEY2, KCNIP1, KRT17, MKX, OLFML1, WDR72.* In some embodiments the progenitor cell line may not express one or more genes chosen from: the *HOX* genes *HOXA2* or *HOXB2.*

In certain embodiments the invention provides a progenitor cell line, e.g., a progenitor cell line having the ability to differentiate into one or more connective tissues, or connective tissue precursors, wherein the progenitor cell line expresses one or more genes chosen from *COL2A1, ALPL, IBSP* and *TNMD,* and not expressing one or more genes chosen from *BARX1, LHX8,* and *FOXF2.*

In yet other embodiments the invention provides a progenitor cell, e.g. a progenitor cell line having the ability to differentiate into smooth muscle, wherein the progenitor cell line expresses one or more genes chosen from *NR4A2, COL4A6, HGF, ELA2, GPR116, HAND2, VGF,* and *FOXF1.*

In further embodiments the invention provides a progenitor cell line expressing one or more of the genes chosen from TH, *CDH18, FGF13, ZIC4, HOXA2* and *HOXB2.* In certain embodiments the progenitor cell line may not express the genes *CST1* and *MKX.*

In still further embodiments the invention provides a progenitor cell line expressing one or more genes chosen from *EPHA5, HEY2, KCNIP1, KRT17, MKX,* and *WDR72.* In certain embodiments the progenitor cell line may not express the *HOX* genes *HOXA2* or *HOXB2.*

In other embodiments the invention provides a progenitor cell line expressing one or more genes chosen from *CST1, FOXF1,* and the HOX genes HOXA2 and HOXB2. In some embodiments the progenitor cell line may not express the gene *NEFM.*

In still other embodiments the invention provides a progenitor cell line expressing one or more genes chosen from *FOXF1, PAX9, WDR72* and the HOX genes *HOXA2* and *HOXB2.* In some embodiments the progenitor cell line may not express *ZIC2.*

In yet other embodiments the invention provides a progenitor cell line expressing one or more genes chosen from *GJB2, PAX8,* MX2. In some embodiments the progenitor cell line may not express the *HOX* genes *HOXA2* and *HOXB2* and may not express *UGT2B7.*

In other embodiments the invention provides a progenitor cell line expressing one or more genes chosen from *TFAP2C,* NTN4, GAP43, S100A6. In some embodiments the progenitor cell line may not express the *HOX* gene *HOXA2* and may not express *IAH1.*

In still other embodiments the invention provides a progenitor cell line expressing one or more genes chosen from *SPP1, DES, ZIC2, ACP5, MT3,* and the *HOX* genes *HOXA2* and HOXB2. In some embodiments the progenitor cell line may not express *NNAT.*

In further embodiments the invention provides a progenitor cell line expressing one or more genes chosen from *SPP1, DES, ZIC2, WDR72.* In some embodiments the progenitor cell line may not express *MT3* or *HOXA2.*

In other embodiments the invention provides a progenitor cell line expressing one or more genes chosen from *PROM1, S100A6, IAH1,* and *ZIC2.* In some embodiments the progenitor cell may not express *NEFM* or the *HOX* gene *HOXA2.*

In yet other embodiments the invention provides a progenitor cell line expressing one or more genes chosen from *NTN4, S100A6, TRIM4,* CD74, ACTG2 and *NPTX1.* In some embodiments the progenitor cell line may not express *AJAP1, PENK* or ZIC2.

In still other embodiments the invention provides a progenitor cell line expressing one or more genes chosen from *HOXA10,* SPP1, *HOXB6, HOXB7, HOXC8, APOE* and *WDR72.* In some embodiments the progenitor cell line may not express *TFAP2C PENK* or *PITX1.*

In further embodiments the invention provides a progenitor cell line expressing one or more genes chosen from *HOXA10, POSTN, KRT34, MKX, HAND2, HOXD11,* and *TBX15.* In some embodiments the progenitor cell line may not express *LHX8, FOXF2, AJAP1, PLXDC2, DLK1, HOXB6, HOXB7, HOXC9, HOXC10,* or *HOXC11.*

In further embodiments the invention provides a progenitor cell line expressing one or more genes chosen from *WDR72, P1TX1, MSX2, TFAP2C, HOXA2, HOXB2.* In some embodiments the progenitor cell line did not express *FOXF2,* or *CD24.*

In further embodiments the invention provides a progenitor cell line expressing one or more genes chosen from *CD24, PLXDC2, MKX, KRT17, KRT34,* and *NRG1.* In some embodiments the progenitor cell line may not express *HOXA2, HOXB2,* and *UGT2B7.*

In still further embodiments the invention provides a progenitor cell line expressing one or more genes chosen from *WDR72, FOXS1, GABRB1,* and *CD90.* In some embodiments the progenitor cell line may not express *HOXB2, ZIC2,* and *UGT2B7.*

In yet further embodiments the invention provides a progenitor cell line expressing one or more genes chosen from *S100A6, HEY2, FOXS1, GABRB1,* and *CD90.* In some embodiments the progenitor cell line may not express *HOXB2, ZIC2, UGT2B7* and *WDR72.*

In further embodiments the invention provides a progenitor cell line expressing one or more genes chosen from *TFAP2C, ZIC2, WDR72, RCAN2,* and *TBX1.* In some embodiments the progenitor cell line may not express *HOXA2,* or *HOXB2.*

In further embodiments the invention provides a progenitor cell line expressing one or more genes chosen from *TFAP2C, ZIC2, CD90, NNAT, TMEM119* and *SCG5.* In some embodiments the progenitor cell line may not express *HOXA2* or *HOXB2.*

In further embodiments the invention provides a progenitor cell line expressing one or more genes chosen from *CD90, PITX1, HOXB7, HOXC8, HOXB6, HOXC9* and *HOXC11.* In some embodiments the progenitor cell line may not express *HEY2, FOXS1,* or *CD74.*

In yet further embodiments the invention provides a progenitor cell line expressing one or more genes chosen from *WDR72, CST1, CD69,* and *TRPV2.* In some embodiments the progenitor cell line may not express *CD90.*

In still other embodiments the invention provides a progenitor cell line expressing one or more markers chosen from *HOXA10, POSTN, KRT34, MKX, HAND2, HOXD11, TBX15* and *PLXDC2.* In some embodiments the progenitor cell line may not express *LHX8, FOXF2, AJAPI , PLXDC2,* or *DLK1, HOXB7, HOXC8, HOXC9, HOXC10,* or *HOXC11.*

Any of the progenitor cell lines described infra may be used in the methods described infra. For example the progenitor cell lines may be contacted with a member of the TGF-β superfamily and induced to differentiate. The progenitor cell lines described infra may be contacted with retinoic acid and induced to differentiate. The progenitor cell line may be cultured in a hydrogel, as described infra, with or without a differentiation agent such as a member of the TGF-β superfamily or retinoic acid.

To improve the scalability of purified somatic progenitors from hPS cells, we previously reported the generation of a library of >140 diverse clonal human embryonic progenitor (hEP) cell lines as source of purified cell types with site-specific homeobox gene expression¹⁹. We designated these novel cell lines "embryonic progenitors" because they show the potential to be propagated extensively *in vitro* and can subsequently differentiate in response to diverse growth factors and inducers. The term therefore refers to cells with an intermediate differentiated state between pluripotent cells and terminally differentiated cell types.

After screening 100 diverse hEP lines for chondrogenic potential, we studied seven lines that showed the induction of the chondrocyte marker *COL2A1.* One of these lines, 4D20.8, showed expression of craniofacial mesenchyme markers such as *LHX8* and *BARX1.* We demonstrated long-term scalability of 4D20.8 in the undifferentiated state, and an ability to regenerate bone and cartilage when engrafted in articular defects in rat models²⁰.

In certain embodiments disclosed herein, the comparative site-specific gene expression of markers of all seven of these chondrogenic hEP lines is provided and along with the disclosure of their diverse responses when differentiated in the presence of one or more TFG beta family members, such as, TGFβ3, BMP2, 4, 6, and 7, and GDF5.

In still other embodiments the invention provides a cell culture comprising the progenitor cell line EN47 cultured in micromass or cultured in a hydrogel. The cell culture may comprise one or more TGFβ family members such as TGF-β3, BMP-2, BMP-4 BMP-7 and the like.

### Clonal Embryonic Progenitor Line Nomenclature:

Many of the human embryonic progenitor cell lines used in the work described infra have been previously described. (See, e.g., US Patent Publication Nos. 20120171171 and 20100184033 both of which are incorporated by reference in their entirety). Nomenclature of the lines includes their alternative designations along with synonyms that represent minor modifications that result from the manipulation of the names resulting from bioinformatics analysis, including the substitution of "-" for "." and vice versa, the inclusion of an "x" before cell line names beginning with an Arabic number, and suffixes such as "bio1" or "bio2" that indicate biological replicates of the same line which are examples of cases where a frozen ampule of the same line was thawed, propagated, and used in a parallel analysis and "Rep1" or "Rep2" which indicate technical replicates wherein RNA isolated from a given cell line is utilized a second time for a repeat analysis without thawing or otherwise beginning with a new culture of cells. Passage number (which is the number of times the cells have been trypsinized and replated) for the cell lines is usually designated by the letter "P" followed by an Arabic number, and in contrast, the population doubling number (which refers to the number of estimated doublings the cell lines have undergone in clonal expansion from one cell) is designated by the letters "PD" followed by an Arabic number. The number of PDs in a passage varied from experiment to experiment but generally each trypsinization and replating was at a 1:3 to 1:4 ratio (corresponding to an increase of PDs of 1.5 and 2 respectively). In the expansion of clones, the original colonies were removed from tissue culture plates with cloning cylinders, and transferred to 24-well plates, then 12-well, and 6-well as described above. First confluent 24 well is designated P1, the first confluent 12 well culture is P2, the first 6-well culture is P3, then the six well culture was then split into a second 6 well plate (P4) and a T25 (P4). The second 6 well at P4 is utilized for RNA extraction (see U.S. Patent Publication No. 20100184033) and represents about 18-21 PD of clonal expansion. Typical estimated subsequent passages and PDs are the following split to a T75 flask (19.5-22.5 PD), the P6 passage of the cells to a T225 flask (21-24 PD), then P7 being the transfer of the cells to a roller bottle (850cm², 23-26 PD), and P8 the split into 4 rollers (25-28 PD). The ranges shown above in parenthesis represent estimated ranges in cell counts due to cell sizes, attachment efficiency, and counting error.

### Propagation of Clonal, Pooled Clonal, Oligoclonal, and Pooled Oligoclonal Cell Lines.

Aspects of the invention provide methods for identifying and differentiating embryonic progenitor cell lines that are derived from a single cell (clonal) or cell lines that are "pooled clonal" meaning that cell lines cloned have indistinguishable markers, such as gene expression markers, and are combined to produce a single cell culture often for the purpose of increasing the number of cells in a culture, or are oligoclonal wherein a line is produced from a small number, typically 2-1,000 similar cells and expanded as a cell line, or "pooled oligoclonal" lines which are lines produced by combining two or more oligoclonal cell lines that have indistinguishable markers such as patterns of gene expression. Said clonal, pooled clonal, oligoclonal, or pooled oligoclonal cell lines are then propagated in vitro through removal of the cells from the substrate to which they are affixed, and the re-plating of the cells at a reduced density of typically 1/3 to 1/4 of the original number of cells, to facilitate further proliferation. Examples of said cell lines and their associated cell culture media is disclosed in U.S. patent application Ser. No. 12/504,630 filed on July 16, 2009 and titled "Methods to Accelerate the Isolation of Novel Cell Strains from Pluripotent Stem Cells and Cells Obtained Thereby"; and West et al., 2008, Regenerative Medicine vol. 3(3) pp. 287-308. The compositions and methods of the present invention relate to said cell lines cultured as described but for greater than 21 doublings of clonal expansion.

### Limb bud Mesenchyme

Unlike the human species, some invertebrate and vertebrate species show a profound capacity to regenerate any tissue damage that does not directly kill the organism. The most commonly-studied vertebrate organisms used in these studies are the Axolotls (Ambystoma mexicanum). While many tissues may be used in regeneration research in Axolotls, the most common studies involve the amputation of the limb and study of the formation of a blastema that recapitulates development in regenerating the entire functional limb. It is commonly believed that the blastema, being composed of relatively undifferentiated mesenchymal cells in a differentiated state functionally equivalent to primitive embryonic limb bud mesenchyme (ELBM) cells, is the source of these repair processes. These ELBM cells carry a pattern of site-specific homeobox gene such as HOX gene expression, that facilitate the cells then forming exactly the tissues that were removed. Methods to understand this process in the human species and to apply these insights into novel methods of tissue regeneration would have widespread clinical applications to not only the tissue engineering but even regeneration in situ for applications including but not limited to limbs lost from ischemic disease, amputation, trauma, or birth defects. In the certain embodiments of the instant invention, we describe clonally-purified, stable, and scalable human embryonic progenitor mesenchyme cell lines isolated from hES cells, displaying lateral plate mesoderm markers such as HOXB6, as well as limb markers such as HOXA10 and markers that discriminate between forelimb and hindlimb such as the hindlimb marker PITX1. The cells may be multipotent and capable of responding to morphogenetic signals within the developing human limb to form the complex array of differentiated cell types and tissue morphologies of the forelimb and hindlimb. ELBM cell lines are distinct from adult or fetal-derived tissues incapable of responding to these morphogenetic signals in causing limb regeneration. Examples of adult-derived stem cells incapable of displaying a complete regenerative phenotype are bone marrow-derived MSCs, skin-derived MSCs, adipose-derived MSCs or adipocyte stromal fraction cells, placenta and endometrium-derived MSCs, and umbilical cord-derived MSCs. Instead, the cell lines of the present invention are purified clonal, oligoclonal, pooled clonal or pooled oligoclonal lines of embryonic progenitor cells displaying a prenatal pattern of gene expression expressed in the embryonic phases of normal human development (i.e. 2-8 weeks post fertilization), such as dermal progenitors with a prenatal pattern of gene expression, displaying a capacity of scarless wound repair. Methods and uses of said cells with a prenatal pattern of gene expression including HOXA10-expression limb bud mesenchyme are described in the following: U.S. Patent Publication 20080070303, entitled "Methods to accelerate the isolation of novel cell strains from pluripotent stem cells and cells obtained thereby"; U.S. Patent Application Serial No. 20080070303 and PCT Application PCT/US2006/013519, filed on April 11, 2006, entitled "NOVEL USES OF CELLS WITH PRENATAL PATTERNS OF GENE EXPRESSION"). Since regeneration of a tissue such as a limb would require the reprogramming of the developmental cascade of homeobox gene expression, such as HOX gene expression, methods to manufacture large numbers of homogeneous cells in the embryonic progenitor state and with site-specific homeobox gene expression would be useful.

By way of non-limiting example, homogeneous populations of limb bud mesenchyme that still retains a prenatal pattern of gene expression, such as hES-derived monoclonal embryonic progenitor cell lines expressing the LPM marker HOXB6, the limb mesenchyme marker HOXA10, and the lower limb marker PITX1, could be used to generate mesenchyme capable of generating the complex structures of the leg. These cells may be formulated in isotonic solutions of disaggregated cells in a relatively undifferentiated (progenitor state), or more differentiated cells. Alternatively, the aforementioned cells may be formulated in hydrogels such as HyStem-C (BioTime, Inc. Alameda, CA), wherein the matrix comprises a thiol-modified gelatin and thiolated hyaluronan crosslinked in vivo or in vitro with (polyethylene glycol diacrylate (PEGDA). In other embodiments other known matrices may be used or the cells may be formulated in solution without said matrices. The cells formulated as described infra, may then be used in any of the applications described infra, including in research on stem cell biology, embryology, and tissue regeneration, or in therapy, such as to regenerate tissue function damaged by disease or trauma.

### Methods of Differentiating Progenitor Cells

In certain embodiments the invention provides a method of differentiating a progenitor cell in vitro, such as a hEP cell, to a more differentiated state (e.g., such as one or more of the differentiated progeny of progenitor cells described infra), relative to the starting progenitor cell, comprising contacting the progenitor cell with one or more members of the TGFβ super family. In some embodiments the TGFβ super family member may be chosen from TGFβ3, BMP2, BMP4, BMP6, BMP7, and GDF5. In some embodiments the more differentiated cell expresses one or more genes described infra as being expressed by an in vitro differentiated progeny of a progenitor cell. The progenitor cell may be any progenitor cell disclosed infra. In one embodiment the progenitor cell is chosen from 4D20.8, 4D20.9, 7PEND24, 7PEND30, 7SMOO32, E15, MEL2, SK5, SK11, SK44, SK50, EN18, EN26, EN27, EN31, EN47, E19, E44, E68, E69, E72, E75, E120, E163, F15, T7, T14, T20, T42, T44, U18, RAD20.5, SM22 and SM30.

In other embodiments the invention provides a method of differentiating a progenitor cell in vitro, such as a hEP cell, to a more differentiated state relative to the starting progenitor cell comprising contacting the progenitor cell with a retinol, such as retinoic acid. The progenitor cell may be chosen from 4D20.8, 4D20.9, 7PEND24, 7PEND30, 7SMOO32, E15, MEL2, SK5, SK11, SK44, SK50, EN18, EN26, EN27, EN31, EN47, E19, E44, E68, E69, E72, E75, E120, E163, F15, T7, T14, T20, T42, T44, U18, RAD20.5, SM22 and SM30. In some embodiments the more differentiated cell expresses one or more genes described infra as being expressed by an in vitro differentiated progeny of a progenitor cell.

In one embodiment of the methods disclosed infra the progenitor cell is comprised of a micromass i.e. is cultured under micromass conditions. In another embodiment the progenitor cell is in contact with a hydrogel. In some embodiments the progenitor cell is encapsulated within the hydrogel. The hydrogel may be comprised of hyaluronate. The hyaluronate may be thiolated. The hydrogel may be comprised of gelatin. The gelatin may be thiolated. The hydrogel may comprise a crosslinker. The crosslinker may be comprised of an acrylate. In one embodiment the acrylate is PEG diacrylate. In some embodiments the hydrogel is comprised of thiolated hyaluronate or thiolated carboxymethylhyaluronate in combination with thiolated gelatin or thiolated carboxymethylgelatin

In some embodiments the more differentiated cell expresses one or more genes described infra as being expressed by an in vitro differentiated progeny of a progenitor cell. In some embodiments the in vitro differentiated progeny express one or more genes expressed by a chondrocyte, e.g. COL2A1. In some embodiments the in vitro differentiated progeny express one or more genes expressed by a tendon, e.g. TNMD. In some embodiments the differentiated progeny express one or more genes expressed by an adipose cell. In some embodiments the differentiated progeny express one or more genes expressed by an osteoblast.

In certain embodiments the invention provides a method of differentiating a progenitor cell chosen from 4D20.8, 7PEND24, 7SMOO32, E15, MEL2, SK11, and SM30 into a cell expressing one or more chondrocyte related genes comprising culturing the progenitor cell under micromass conditions and contacting the cells with a media comprising exogenously added TGFβ3 thereby differentiating the progenitor cell into a cell expressing one or more chondrocyte related genes. The media may further comprise dexamethasone and insulin, transferrin and selenium (ITS). The one or more chondrocyte genes may include COL2A1, COL10A1, ACAN, and CRTAC1.

In other embodiments the invention provides a method of differentiating a progenitor cell chosen from 4D20.8, 7PEND24, 7SMOO32, E15, MEL2, SK11, and SM30 into a cell expressing one or more chondrocyte related genes comprising contacting the cells with a hydrogel and a media comprising one or more TGFβ super family members thereby obtaining a differentiated progeny of a progenitor cell wherein the differentiated progeny expresses one or more chondrocyte related genes. In some embodiments the progenitor cell is contacted with hydrogel before it is contacted with the TGFβ super family member. In other embodiments the progenitor cell is contacted with the TGFβ super family member before it is contacted with the hydrogel. In still other embodiments the progenitor cell is contacted simultaneously with the hydrogel and the TGFβ super family member. The TGFβ super family member may be chosen from TGFβ3, BMP2, BMP4, BMP6, BMP7, and GDF5. The one or more chondrocyte genes may include any of COL2A1, COL10A1, ACAN, and CRTAC1. In certain embodiments the method described in this paragraph produces a differentiated progeny of a progenitor cell that expresses more COL2A1 and less COL10A1compared to the same progenitor cell contacted with a TGFβ superfamily member grown under micromass conditions without the hydrogel. In some embodiments the cells are encapsulated by the hydrogel. In some embodiments the hydrogel may comprise hyaluronate. In some embodiments the hyaluronate is thiolated. In some embodiments the hydrogel may comprise gelatin. In some embodiments the gelatin is thiolated. In some embodiments the hydrogel may comprise an acrylate such as a PEG acrylate e.g. PEG diacrylate.

In further embodiments the invention provides a method of differentiating a progenitor cell into a cell expressing one or more genes chosen from COL2A1, COL10A1, ACAN, ALPL, IBSP and CRTAC1 comprising 1) contacting the progenitor cell with a hydrogel as described infra; 2) contacting the progenitor cell with TGFβ-3 and a second factor chosen from BMP-2, BMP-4, BMP-6, BMP-7 and GDFS, thereby differentiating the progenitor cell into a cell expressing one or more genes chosen from COL2A1, COL10A1, ACAN, ALPL, IBSP,ELN, PRG4, SPP1 and CRTAC1. In certain embodiments the second factor is BMP-4. In some embodiments the progenitor cell used in the method may express one or more genes chosen from *CST1, FOXF1,* and the HOX genes HOXA2 and HOXB2. In some embodiments the progenitor cell used in the method may express one or more genes chosen from *FOXS1, KCNIP1, KRT17, TFAP2C,* and *ZIC2.* In some embodiments the progenitor cell used in the method may express one or more genes chosen from *CD69, FOXF1, FOXF2.* In some embodiments the progenitor cell used in the method may express one or more genes chosen from *CD69, CST1, FOXF1, OSR2,* and, ZIC2 and the *HOX* genes *HOXA2* and *HOXB2.* In some embodiments the progenitor cell used in the method may express one or more genes chosen from *EPHA5, HEY2, KCNIP1, KRT17, MKX, OLFML1, WDR72.* In some embodiments the progenitor cell used in the method may express one or more genes chosen from CST1, FOXF1, and the HOX genes HOXA2 and HOXB2. In some embodiments the progenitor cell used in the method may express one or more genes chosen from FOXF1, *PAX9,* WDR72 and the HOX genes HOXA2 and HOXB2. In some embodiments the progenitor cell used in the method may express one or more genes chosen from GJB2, MKX, PAX8, MX2. In some embodiments the progenitor cell used in the method may express one or more genes chosen from: CD24, TFAP2C, NTN4, GAP43, S100A6. In some embodiments the progenitor cell used in the method may express one or more genes chosen from SPP1, DES, ZIC2, ACP5, MT3, and the HOX genes HOXA2 and HOXB2. In some embodiments the progenitor cell used in the method may express one or more genes chosen from SPP1, DES, ZIC2, WDR72. In some embodiments the progenitor cell used in the method may express one or more genes chosen from PROM1, S100A6, IAH1, and ZIC2. In some embodiments the progenitor cell used in the method may express one or more genes chosen from NTN4, S100A6, TRIM4, NPTX1 and CD74. In some embodiments the progenitor cell used in the method may express one or more genes chosen from HOXA10, S100A6, SPP1, HOXB6, and WDR72, HOXB7, and HOXC8. In some embodiments the progenitor cell used in the method may express one or more genes chosen from HOXA10, POSTN, KRT34, MKX, HAND2, HOXD11. In some embodiments the progenitor cell used in the method may express one or more genes chosen from HOXA10, POSTN, KRT34, MKX, HAND2, HOXD11, TBX15 PLXDC2. In some embodiments the progenitor cell used in the method may express one or more genes chosen from WDR72, PITX1, MSX2, TFAP2C, HOXA2, HOXB2. In some embodiments the progenitor cell used in the method may express one or more genes chosen from CD24, PLXDC2, MKX, KRT17, KRT34, and NRG1. In some embodiments the progenitor cell used in the method may express one or more genes chosen from WDR72, FOXS1, GABRB1, and CD90. In some embodiments the progenitor cell used in the method may express one or more genes chosen from S100A6, HEY2 , FOXS, GABRB1, and CD90. In some embodiments the progenitor cell used in the method may express one or more genes chosen from TFAP2C, ZIC2, WDR72, RCAN2, and TBX1. In some embodiments the progenitor cell used in the method may express one or more genes chosen from TFAP2C, ZIC2, CD90, NNAT, TMEM119, and SCG5. In some embodiments the progenitor cell used in the method may express one or more genes chosen from CD90, PITX1, and HOXB6. In some embodiments the progenitor cell used in the method may express one or more genes chosen from WDR72, CST1, CD69, and TRPV2.

In further embodiments the invention provides a method of differentiating a progenitor cell having osteochondral differentiation potential into a cell expressing SFRP4 comprising contacting the progenitor cell with retinoic acid and optionally dexamethasone thereby differentiating the progenitor cell into a cell expressing SFRP4. Any progenitor cell described infra having ostechondral differentiation potential may be used in the method. In certain embodiments the progenitor cell may be chosen from EN47, E68, 7PEND24, SK11, SM30 and EN7. The progenitor cell may be cultured under micromass conditions as described infra. The progenitor cell may be contacted with a hydrogel as described infra.

In yet further embodiments the invention provides a method of differentiating a progenitor cell into a cell expressing plexis epithelium TTR and optionally KRT7 comprising contacting the progenitor cell with a hydrogel and BMP4 thereby differentiating the progenitor cell into a cell expressing plexis epitheliun TTR and optionally KRT7. The progenitor cell may be chosen from E68 (ATCC Accession No: PTA-8119), E69, and T42 (ATCC Accession No: PTA-120383) or cell line having a similar marker profile.

In yet other embodiments the invention provides a method of differentiating a progenitor cell expressing *PPARG, DLK1, CEBPD, BARX1, LHX8, SNA12, TWIS1,* and *FOXF1* into a cell expressing one or more genes chosen from *LPL, CEBPD, PPARG, FABP4, PLIN1, DLK1, PPARGC1A, CEBPD, PPARG, FABP4, PRDM16, FOXC2, CRLF1, FOXF2, FBLN5, DPT, ITGBL1, COL2A1, COL6A3, DUSP1, FOXF1, TGFB3, PAX9, GSN, FMOD, PDE8B, COMP, ITGA10, SAA1, DTNA, PCDH9, EBF1, SORBS1, SORBS2, ZNF503,* and *MGST2* comprising contacting the progenitor cell with a hydrogel and BMP-4 thereby differentiating the progenitor cell into a cell expressing one or more genes chosen from *LPL, CEBPD, PPARG, FABP4,* and *PLIN1, DLK1, PPARGC1A, CEBPD, PPARG, FABP4, PRDM16, FOXC2, CRLF1, FOXF2, FBLN5, DPT, ITGBL1, COL2A1, COL6A3, DUSP1, FOXF1, TGFB3, PAX9, GSN, FMOD, PDE8B, COMP, ITGA10, SAA1, DTNA, PCDH9, EBF1, SORBS1, SORBS2, ZNF503, and MGST2.*

In yet other embodiments the invention provides a method of differentiating a progenitor cell expressing *PPARG, DLK1, CEBPD, BARX1, LHX8, SNAI2, TWIST1,* and *FOXF1* into a cell expressing one or more genes chosen from *DPT, COL2A1, OGN, FBLN5, FMOD, CRLF1, ITGBL1, COL6A3, TGFB3, FOXF2, GSN, HHIP, LRIG1, TRPS1, COMP, COL2A1, ACAN, LECT1, COL9A1, HAPLN1, ITGA10, OLFML3, PKDCC, FGFR3, CSPG4, COL9A3, ITGB5,DNM1 OGN, COL9A2, FMOD, LECT1, EPYC, CHAD, COL9A1, PPIB, SRPX2, MATN3, LUM, COL13A1, FKBP11,* MXRA8, *COL27A1, PELI2,* GPX7, *ANGPTL2, GXYLT2, KLF4, STEAP3, SLC39A14, PTH1R, FAM46A, FAM180A, SLC26A2, RUNX1, CTGF, COL9A3, PLEKHB1, FKBP7, HHIP, TNC, JAK2, CYTL1, KDELR3, ATP8B2, TRPS1; ELN, FMOD, EPYC, CHAD, COL9A1, PPIB, PHEX, SOX9, COL27A1, PELI2, ANGPTL2, GXYLT2, SLC39A14, P4HA3, SLC26A2, CTGF, MRC2, COL9A3, PLEKHB1, TNC, FRMD8, CYTL1,* and *ATP8B, COL2A1, SCRG1, FMOD, CHAD, COL9A1, MATN3, FOXF2, SMOC1, COL27A1, PELI2, SOX8, PTH1R, HTRA1, RUNX1, CTGF, PLEKHB1, RG9MTD1, CYTL1,* and *KLF2* comprising contacting the progenitor cell with a hydrogel and GDF-5 thereby differentiating the progenitor cell into a cell expressing one or more genes chosen from *DPT, COL2A1, OGN, FBLN5, FMOD, CRLF1, ITGBL1, COL6A3, TGFB3, FOXF2, GSN, HHIP, LRIG1, TRPS1, COMP, COL2A1, ACAN, LECT1, COL9A1, HAPLN1, ITGA10, OLFML3, PKDCC, FGFR3, CSPG4, COL9A3, ITGB5,DNM1 OGN, COL9A2, FMOD, LECT1, EPYC, CHAD, COL9A1, PPIB, SRPX2, MATN3, LUM, COL13A1, FKBP11, MXRA8, COL27A1, PELI2, GPX7, ANGPTL2, GXYLT2, KLF4, STEAP3, SLC39A14, PTH1R, FAM46A, FAM180A, SLC26A2, RUNX1, CTGF, COL9A3, PLEKHB1, FKBP7, HHIP, TNC, JAK2, CYTL1, KDELR3, ATP8B2, TRPS1; ELN, FMOD, EPYC, CHAD, COL9A1, PPIB, PHEX, SOX9, COL27A1, PELI2, ANGPTL2, GXYLT2, SLC39A14, P4HA3, SLC26A2, CTGF, MRC2, COL9A3, PLEKHB1, TNC, FRMD8, CYTL1,* and *ATP8B, COL2A1, SCRG1, FMOD, CHAD, COL9A1, MATN3, FOXF2, SMOC1, COL27A1, PELI2, SOX8, PTH1R, HTRA1, RUNX1, CTGF, PLEKHB1, RG9MTD1, CYTL1,* and *KLF2.*

In other embodiments the invention provides a method of differentiating a cell expressing one or more markers chosen from *FOXF1, MMP10, MSX2, FOXF2, FOXD1, MMP2, CDH11, TFAP2A, SOX9, COL1A1, FOXF2, SPP1, MSX2, FGFR3* into a cell expressing one or more markers chosen from *FABP4, LPL, CEBPB, PLIN1, PPARG, CEBPD, FABP4, PPARGC1A, DLK1, CEBPB, PPARG,* and *CEBPD* comprising contacting the progenitor cell with a hydrogel and TGFβ-3 thereby differentiating the progenitor cell into a cell expressing one or more genes chosen from *FABP4, LPL, CEBPB, PLIN1, PPARG, CEBPD, FABP4, PPARGC1A, DLK1, CEBPB, PPARG,* and *CEBPD.*

In other embodiments the invention provides a method of differentiating a cell expressing one or more markers chosen from *FOXF1, MMP10, MSX2, FOXF2, FOXD1, MMP2, CDH11, TFAP2A, SOX9, COL1A1, FOXF2, SPP1, MSX2, FGFR3* into a cell expressing one or more markers chosen from *DPT, CRLF1, FBLN5, COL2A1, OGN, ITGBL1, FOXF2, COL6A3, GSN, WIF1, TGFB3, LRIG1, CORO2B, ADAMTS15, IGFBP7, COMP, COL2A1, SPP1, KAZALD1, LECT1, ITGA10, MMP13, ACAN, HAPLN1, PHEX, PTH1R, COL11A1, IP6K2; COMP, COL2A1, PKDCC, LECT1, ITGA10, LTBP3, ACAN, HAPLN1, OLFML3, WIF1, ITGB5, IP6K2; ELN, CYTL1, LTBP3, PELI2, EPYC, PHEX, MRC2, CALY, GXYLT2, COL27A1, ANGPTL2, SOX9, GAA, TNC, LEPRE1, LTBP2, ARFGAP1; COL9A2, OGN, SRPX2, CYTL1, FAM46A, LECT1, LUM, LTBP3, PELI2, COL13A1, EPYC, MXRA8, RUNX1, CALY, PTH1R, FKBP11, STEAP3, CFH, SLC40A1, GXYLT2, COL27A1, GPX7, ANGPTL2, MATN3, FKBP7, GAA, TNC, LEPRE1,* and *LTBP2* comprising contacting the progenitor cell with a hydrogel and TGFβ-3 and GDF-5 thereby differentiating the progenitor cell into a cell expressing one or more genes chosen from *DPT, CRLF1, FBLN5, COL2A1, OGN, ITGBL1, FOXF2, COL6A3, GSN, WIF1, TGFB3, LRIG1, CORO2B, ADAMTS15, IGFBP7, COMP, COL2A1, SPP1, KAZALD1, LECT1, ITGA10, MMP13, ACAN, HAPLN1, PHEX, PTH1R, COL11A1, IP6K2; COMP, COL2A1, PKDCC, LECT1, ITGA10, LTBP3, ACAN, HAPLN1, OLFML3, WIF1, ITGB5, IP6K2; ELN, CYTL1, LTBP3, PELI2, EPYC, PHEX, MRC2, CALY, GXYLT2, COL27A1, ANGPTL2, SOX9, GAA, TNC, LEPRE1, LTBP2, ARFGAP1; COL9A2, OGN, SRPX2, CYTL1, FAM46A, LECT1, LUM, LTBP3, PELI2, COL13A1, EPYC, MXRA8, RUNX1, CALY, PTH1R, FKBP11, STEAP3, CFH, SLC40A1, GXYLT2, COL27A1, GPX7, ANGPTL2, MATN3, FKBP7, GAA, TNC, LEPRE1,* and *LTBP2.*

In further embodiments the invention provides a method of differentiating a progenitor cell expressing one or more genes chosen from *SOX9, VCAN, COL6A2 TFAP2A, CDH2, SIX1* into a cell expressing one or more markers chosen from *FABP4, PPARG, PLIN1, LPL, FABP4, PPARG, PRDM16, FGFR3, ITGA10, CSPG4, LTBP3, COMP, HAPLN1, PKDCC, CA12, GDF10, COL9A2, KLF4, PPIB, IRX5, ARHGAP24, and HHIP* comprising contacting the progenitor cell with a hydrogel and BMP-4 thereby differentiating the progenitor cell into a cell expressing one or more genes chosen from *FABP4, PPARG, PLIN1, LPL, FABP4, PPARG, PRDM16, FGFR3, lTGA10, CSPG4, LTBP3, COMP, HAPLN1, PKDCC, CA12, GDF10, COL9A2, KLF4, PPIB, IRX5, ARHGAP24,* and *HHIP.*

In still other embodiments the invention provides a method of differentiating a progenitor cell expressing one or more genes chosen from *SOX9, VCAN, COL6A2 TFAP2A, CDH2, SIX1* into a cell expressing one or more genes chosen from *COL2A1, FBLN5, DPT, FMOD, CRLF1, OGN, HHIP, ITGBL1, TGFB3, COL6A3, VCAN, DUSP1, TRPS1, GSN, ADAMTS6, ERG; COL10A1, EPYC, ELN, FMOD, SOX9, COL8A1, COL9A3, CHAD, CYTL1, PPIB, CTHRC1, PLEKHB1, SLC26A2, KANK1, SLC39A14, ATP8B2, TNC, LTBP2, GPC1, WWP2, P4HA3, BAMBI, COL27A1, CTGF, FGFRL1, SDC2; SCRG1, COL2A1, FMOD, MEF2C, MATN3, PTH1R, CHAD, ENPP2, CYTL1, CTHRC1, PLEKHB1, RUNX3, RUNX1, PRICKLE1, WWP2, HTRA1, COL27A1, CTGF, FGFRL1, SOX8, ERG, FAT3; COL9A2, COL10A1, EPYC, FMOD, PCOLCE2, OGN, MEF2C, MATN3, LUM, HHIP, PTH1R, COL8A1, COL9A3, DKK1, SLC40A1, KLF4, SRPX2, CHAD, CYTL1, PPIB, LECT1, FKBP11, CTHRC1, STEAP3, PLEKHB1, RUNX3, SLC26A2, KANK1, LOXL4, SLC39A14, ATP8B2, DUSP1, RUNX1, TNC, LEPR, LTBP2, TRPS1, GPC1, WWP2, CFH, BAMBI, CDH2, COL27A1, FAM46A, GPX7, CTGF, FGFRL1, SDC2,* and *PLCD1* comprising contacting the progenitor cell with a hydrogel and TGFβ-3 and GDF-5 thereby differentiating the progenitor cell into a cell expressing one or more genes chosen from *COL2A1, FBLN5, DPT, FMOD, CRLF1, OGN, HHIP, ITGBL1, TGFB3, COL6A3, VCAN, DUSP1, TRPS1, GSN, ADAMTS6, ERG; COL10A1, EPYC, ELN, FMOD, SOX9, COL8A1, COL9A3, CHAD, CYTL1, PPIB, CTHRC1, PLEKHB1, SLC26A2, KANK1, SLC39A14, ATP8B2, TNC, LTBP2, GPC1, WWP2, P4HA3, BAMBI, COL27A1, CTGF, FGFRL1, SDC2; SCRG1, COL2A1, FMOD, MEF2C, MATN3, PTH1R, CHAD, ENPP2, CYTL1, CTHRC1, PLEKHB1, RUNX3, RUNX1, PRICKLE1, WWP2, HTRA1, COL27A1, CTGF, FGFRL1, SOX8, ERG, FAT3; COL9A2, COL10A1, EPYC, FMOD, PCOLCE2, OGN, MEF2C, MATN3, LUM, HHIP, PTH1R, COL8A1, COL9A3, DKK1, SLC40A1, KLF4, SRPX2, CHAD, CYTL1, PPIB, LECT1, FKBP11, CTHRC1, STEAP3, PLEKHB1, RUNX3, SLC26A2, KANK1, LOXL4, SLC39A14, ATP8B2, DUSP1, RUNX1, TNC, LEPR, LTBP2, TRPS1, GPC1, WWP2, CFH, BAMBI, CDH2, COL27A1, FAM46A, GPX7, CTGF, FGFRL1, SDC2,* and *PLCD1.*

In further embodiments the invention provides a method of differentiating a progenitor cell expressing one or more genes chosen from *NKX3-2 SATB2, ALPL, MSX2, FRZB, HAND2, DLX5, DLX6, TWIST1, FOXD1, MSX2, TFAP2A,* into a cell expressing one or more markers chosen from *ALPL, BMP2, PTH1R, MSX2, DCN, SPP1, SATB2; DLX5, ALPL, PTH1R, MSX2, POSTN, TWIST1, PCDH20, SERPINA3, TAC1, EDNRA, SCRG1, CRABP2, SPOCK3, VCAN, BOC, ECM2, CRLF1, GAS1, TSPAN8, MFAP4, NFIA,* and *RASSF9* comprising contacting the progenitor cell with a hydrogel and BMP-4 thereby differentiating the progenitor cell into a cell expressing one or more genes chosen from *ALPL, BMP2, PTH1R, MSX2, DCN, SPP1, SATB2; DLX5, ALPL, PTH1R, MSX2, POSTN, TWIST1, PCDH20, SERPINA3, TAC1, EDNRA, SCRG1, CRABP2, SPOCK3, VCAN, BOC, ECM2, CRLF1, GAS1, TSPAN8, MFAP4, NFIA,* and *RASSF9.*

In still further embodiments the invention provides a method of differentiating a progenitor cell expressing one or more genes chosen from *NKX3-2 SATB2, ALPL, MSX2, FRZB, HAND2, DLX5, DLX6, TWIST1, FOXD1, MSX2, TFAP2A,* into a cell expressing one or more markers chosen from *COL2A1, FBLN5, DPT, FMOD, CRLF1, OGN, HHIP, ITGBL1, TGFB3, COL6A3, VCAN, DUSP1, TRPS1, GSN, ADAMTS6, ERG; COL10A1, EPYC, ELN, FMOD, SOX9, COL8A1, COL9A3, CHAD, CYTL1, PPIB, CTHRC1, PLEKHB1, SLC26A2, KANK1, SLC39A14, ATP8B2, TNC, LTBP2, GPC1, WWP2, P4HA3, BAMBI, COL27A1, CTGF, FGFRL1, SDC2; SCRG1, COL2A1, FMOD, MEF2C, MATN3, PTH1R, CHAD, ENPP2, CYTL1, CTHRC1, PLEKHB1, RUNX3, RUNX1, PRICKLE1, WWP2, HTRA1, COL27A1, CTGF, FGFRL1, SOX8, ERG, FAT3; COL9A2, COL10A1, EPYC, FMOD, PCOLCE2, OGN, MEF2C, MATN3, LUM, HHIP, PTH1R, COL8A1, COL9A3, DKK1, SLC40A1, KLF4, SRPX2, CHAD, CYTL1, PPIB, LECT1, FKBP11, CTHRC1, STEAP3, PLEKHB1, RUNX3, SLC26A2, KANK1, LOXL4, SLC39A14, ATP8B2, DUSP1, RUNX1, TNC, LEPR, LTBP2, TRPS1, GPC1, WWP2, CFH, BAMBI, CDH2, COL27A1, FAM46A, GPX7, CTGF, FGFRL1, SDC2,* and *PLCD1* comprising contacting the progenitor cell with a hydrogel and TGFβ-3 and GDF-5 thereby differentiating the progenitor cell into a cell expressing one or more genes chosen from *COL2A1, FBLN5, DPT, FMOD, CRLF1, OGN, HHIP, ITGBL1, TGFB3, COL6A3, VCAN, DUSP1, TRPS1, GSN, ADAMTS6, ERG; COL10A1, EPYC, ELN, FMOD, SOX9, COL8A1, COL9A3, CHAD, CYTL1, PPIB, CTHRC1, PLEKHB1, SLC26A2, KANK1, SLC39A14, ATP8B2, TNC, LTBP2, GPC1, WWP2, P4HA3, BAMBI, COL27A1, CTGF, FGFRL1, SDC2; SCRG1, COL2A1, FMOD, MEF2C, MATN3, PTH1R, CHAD, ENPP2, CYTL1, CTHRC1, PLEKHB1, RUNX3, RUNX1, PRICKLE1, WWP2, HTRA1, COL27A1, CTGF, FGFRL1, SOX8, ERG, FAT3; COL9A2, COL10A1, EPYC, FMOD, PCOLCE2, OGN, MEF2C, MATN3, LUM, HHIP, PTH1R, COL8A1, COL9A3, DKK1, SLC40A1, KLF4, SRPX2, CHAD, CYTL1, PPIB, LECT1, FKBP11, CTHRC1, STEAP3, PLEKHB1, RUNX3, SLC26A2, KANK1, LOXL4, SLC39A14, ATP8B2, DUSP1, RUNX1, TNC, LEPR, LTBP2, TRPS1, GPC1, WWP2, CFH, BAMBI, CDH2, COL27A1, FAM46A, GPX7, CTGF, FGFRL1, SDC2,* and *PLCD1.*

In other embodiments the invention provides a method of differentiating a progenitor cell expressing one or more genes chosen from *NKX3-2, SATB2, ALPL, MSX2 FRZB, HAND2, DLX5, DLX6, TWIST1, FOXD1, MSX2, TFAP2A,* into a cell expressing one or more genes chosen from *ALPL, BMP2, PTH1R, MSX2, DCN, SPP1,* and *SATB2; DLX5, ALPL, PTH1R, MSX2, POSTN, TWIST1, and SATB2; PCDH20, SERPINA3, TAC1, EDNRA, SCRG1, CRABP2, SPOCK3, VCAN, BOC, ECM2, CRLF1, GAS1, TSPAN8, MFAP4, NFIA,* and RASSF9 comprising contacting the progenitor cell with a hydrogel and BMP-4 thereby differentiating the progenitor cell into a cell expressing one or more genes chosen from *DLX5, ALPL, PTH1R, MSX2, POSTN, TWIST1, SATB2; PCDH20, SERPINA3, TAC1, EDNRA, SCRG1, CRABP2, SPOCK3, VCAN, BOC, ECM2, CRLF1, GAS1, TSPAN8, MFAP4, NFIA,* and *RASSF9.*

In other embodiments the invention provides a method of differentiating a progenitor cell expressing one or more genes chosen from *NKX3-2, SATB2, ALPL, MSX2 FRZB, HAND2, DLX5, DLX6, TWIST1, FOXD1, MSX2, TFAP2A,* into a cell expressing one or more genes chosen from *SPP1, BMP2, ALPL, PTH1R, FGFR3, DCN, MSX2, SATB2, NKX3-2, TWIST1*; *COMP, SERPINA3, ITGA10, EBF1, SORBS2, PCDH17, SOBP, ST8SIA4*; *COL10A1, COL9A2, OGN, MEF2C, ALPL, PTH1R, LUM, CFH, STEAP3, PELI2, LTBP2, SLC40A1, MXRA8, COL8A1, MATN3, PARD6G, GPC1, GPX7, FAM180A, CTHRC1, ATP8B2, BAMBI, DKK1, PLCD1, LTBR, SLC26A2, ANGPTL2, SATB2, FAM46A, DUSP1; COMP, ITGA10, CSPG4, FGFR3, CA12, ITGB5, OLFML3, COL2A1,* and *ACAN* comprising contacting the progenitor cell with a hydrogel and TGFβ-3 and GDF-5 thereby differentiating the progenitor cell into a cell expressing one or more genes chosen from *SPP1, BMP2, ALPL, PTH1R, FGFR3, DCN, MSX2, SATB2, NKX3-2, TWIST1; COMP, SERPINA3, ITGA10, EBF1, SORBS2, PCDH17, SOBP, ST8SIA4; COL10A1, COL9A2, OGN, MEF2C, ALPL, PTH1R, LUM, CFH, STEAP3, PELI2, LTBP2, SLC40A1, MXRA8, COL8A1, MATN3, PARD6G, GPC1, GPX7, FAM180A, CTHRC1, ATP8B2, BAMBI, DKK1, PLCD1, LTBR, SLC26A2, ANGPTL2, SATB2, FAM46A, DUSP1; COMP, ITGA10, CSPG4, FGFR3, CA12, ITGB5, OLFML3, COL2A1, and ACAN.*

In yet other embodiments the invention provides a method of differentiating a progenitor cell expressing one or more genes chosen from *SIX1, TWIST1, SNAI2* into a cell expressing one or more genes chosen from *FGFR3, ACAN, ITGA10, OLFML3, COMP, CSPG4, XIST, PKDCC, COL2A1, LTBP3, FUS; TGFB3, FBLN5, OGN, GAS1, ITGBL1, DPT, DUSP1, GSN, COL2A1, ARHGAP24, VCAN,* comprising contacting the progenitor cell with a hydrogel and BMP-4 thereby differentiating the progenitor cell into a cell expressing one or more genes chosen from *FGFR3, ACAN, ITGA10, OLFML3, COMP, CSPG4, XIST, PKDCC, COL2A1, LTBP3, FUS; TGFB3, FBLN5, OGN, GAS1, ITGBL1, DPT, DUSP1, GSN, COL2A1, ARHGAP24, VCAN.*

In other embodiments the invention provides a method of differentiating a progenitor cell expressing one or more genes chosen from *SIX1, TWIST1, SNAI2* into a cell expressing one or more genes chosen from *COMP, COL2A1, ACAN, SPP1, FGFR3, HAPLN1, COL11A1, PTH1R, LECT1, CHAD, COL11A2, COL9A1, ITGA10, COL9A3, CSPG4, ENPP1, MMP13, MMP2, XIST,* and *SERPINH1; COMP, COL2A1, ACAN, FGFR3, HAPLN1, LECT1, COL9A1, ITGA10, OLFML3, COL9A3, CSPG4, MMP2, PKDCC, XIST, ITGB5, SERPINH1, CA12, and DNM1; COL10A1, COL9A2, EPYC, OGN, PTH1R, MATN3, LECT1, FMOD, CHAD, COL9A1, HHIP, STEAP3, FKBP11, PCOLCE2, GPC1, GXYLT2, LUM, MEF2C, COL9A3, MXRA8, GPX7, RUNX1, FAM46A, CTHRC1, COL8A1, ANGPTL2, CFH, SLC39A14, COL27A1, IBSP, FKBP7, KLF4, P4HA2, ATP8B2, SRPRB, PELI2, METRNL, KDELR3, PARD6G,* and *LEPRE1* comprising contacting the progenitor cell with a hydrogel and TGFβ-3 and GDF-5 thereby differentiating the progenitor cell into a cell expressing one or more genes chosen from *COMP, COL2A1, ACAN, SPP1, FGFR3, HAPLN1, COL11A1, PTH1R, LECT1, CHAD, COL11A2, COL9A1, ITGA10, COL9A3, CSPG4, ENPP1, MMP13, MMP2, XIST, SERPINH1; COMP, COL2A1, ACAN, FGFR3, HAPLN1, LECT1, COL9A1, ITGA10, OLFML3, COL9A3, CSPG4, MMP2, PKDCC, XIST, ITGB5, SERPINH1, CA12,* and *DNM1; COL10A1, COL9A2, EPYC, OGN, PTH1R, MATN3, LECT1, FMOD, CHAD, COL9A1, HHIP, STEAP3, FKBP11, PCOLCE2, GPC1, GXYLT2, LUM, MEF2C, COL9A3, MXRA8, GPX7, RUNX1, FAM46A, CTHRC1, COL8A1, ANGPTL2, CFH, SLC39A14, COL27A1, IBSP, FKBP7, KLF4, P4HA2, ATP8B2, SRPRB, PELI2, METRNL, KDELR3, PARD6G,* and *LEPRE1.*

In still other embodiments the invention provides a method of differentiating a progenitor cell expressing one or more genes chosen from *FOXF1, SOX9, RUNX2*, and *NKX3-2* into a cell expressing one or more genes chosen from *COL2A1, ACAN, DCN, FOS, LUM, ITGA1, A2M, CTGF COL2A1, GSN, OGN, EMILIN3, ITGBL1, TGFB3, FBLN5, DUSP1, CRLF1; COL2A1, GSN, OGN, EMILIN3, ITGBL1, TGFB3, FBLN5, DUSP1, CRLF1; COL2A1, FGFR3, ACAN, ITGA10, CSPG4, PKDCC, COL9A3, HAPLN1, XIST, COL9A1, OLFML3, COMP, LTBP3, LECT1; COL9A2, PTH1R, CFH, EPYC, DKK1, MATN3, MEF2C, OGN, CD36, COL9A3, COL8A1, BAMBI, CHAD, LEPR, COL10A1, LUM, LPAR3, KLF4, COL9A1, ANGPTL2, PCOLCE2, MXRA8, LTBP3, ATP8B2, DUSP1, LTBP2, COL24A1, SLC40A1, LECT1, GPX7, FAM46A, CTGF, FGFRL1, CDH2; COL2A1, PTH1R, ENPP2, SCRG1, MATN3, MEF2C, CHAD, GDF10, COL9A1, FAT3, SERPINE2, SOX8, CTGF, FGFRL1; COL2A1, FGFR3, ACAN, PTH1R, COL11A1, SOX9, COL11A2, HAPLN1, NKX3-2, COMP; FRZB, FABP4, DLK1,* and *PPARG* comprising contacting the progenitor cell with a hydrogel and BMP-4 thereby differentiating the progenitor cell into a cell expressing one or more genes chosen from *COL2A1, ACAN, DCN, FOS, LUM, ITGA1, A2M, CTGF COL2A1, GSN, OGN, EMILIN3, ITGBL1, TGFB3, FBLN5, DUSP1, CRLF1; COL2A1, GSN, OGN, EMILIN3, ITGBL1, TGFB3, FBLN5, DUSP1, CRLF1; COL2A1, FGFR3, ACAN, ITGA10, CSPG4, PKDCC, COL9A3, HAPLN1, XIST, COL9A1, OLFML3, COMP, LTBP3, LECT1; COL9A2, PTH1R, CFH, EPYC, DKK1, MATN3, MEF2C, OGN, CD36, COL9A3, COL8A1, BAMBI, CHAD, LEPR, COL10A1, LUM, LPAR3, KLF4, COL9A1, ANGPTL2, PCOLCE2, MXRA8, LTBP3, ATP8B2, DUSP1, LTBP2, COL24A1, SLC40A1, LECT1, GPX7, FAM46A, CTGF, FGFRL1, CDH2; COL2A1, PTH1R, ENPP2, SCRG1, MATN3, MEF2C, CHAD, GDF10, COL9A1, FAT3, SERPINE2, SOX8, CTGF, FGFRL1*; *COL2A1*, *FGFR3, ACAN*, *PTH1R*, *COL11A1*, *SOX9*, *COL11A2*, *HAPLN1*, *NKX3-2, COMP; FRZB*, *FABP4*, *DLK1,* and *PPARG.*

In still other embodiments the invention provides a method of differentiating a progenitor cell expressing one or more genes chosen from *FOXF1*, *SOX9*, *RUNX2*, and *NKX3-2* into a cell expressing one or more genes chosen from *COL2A1*, *OGN*, *FBLN5*, *DPT, FMOD, CRLF1*, *TGFB3, ITGBL1*, *HHIP, GSN, ADAMTS6*, *TRPS1*, *VCAN; COL2A1*, *ACAN*, *COMP, LECT1*, *HAPLN1*, *FGFR3, COL9A1, ITGA10, COL9A3, CSPG4, XIST, PKDCC, OLFML3, WWP2, SUSD5, DNM1; EPYC, COL9A2, COL10A1, LECT1*, *CHAD, OGN*, *MATN3, COL9A1, PTH1R, PCOLCE2, COL9A3, FMOD, FKBP11, STEAP3, LUM, MEF2C, GPC1, COL27A1, ANGPTL2, CFH, HHIP, KLF4, GPX7, WWP2, FAM46A, DKK1, MXRA8, COL8A1,* ATP8B2, RUNX1, *TNC, PLCD1, FAM180A, SLC39A14, TRPS1*, *CTGF, PELI2, FGFRL1, PYCR1, GXYLT2, SLC40A1, LTBP2; EPYC, COL10A1, CHAD, COL9A1, COL9A3, FMOD, ELN, SOX9*, *GPC1, COL27A1, ANGPTL2, WWP2, COL8A1, ATP8B2, MRC2, TNC, SLC39A14, CTGF, PELI2, FGFRL1, GXYLT2, LTBP2; COL2A1*, *SCRG1, CHAD, MATN3, COL9A1, PTH1R*, *SOX8, FMOD, MEF2C, PRICKLE1, COL27A1, WWP2, ENPP2, RUNX1, CTGF, PELI2, FGFRL1, and KLF2; COL2A1*, *ACAN*, *COMP, LECT1, HAPLN1, CHAD, SPP1, FGFR3, COL11A2, COL11A1, COL9A1, PTH1R*, *ITGA10, COL9A3, CSPG4, XIST, WWP2, ENPP1, MMP13, SUSD5,* and *KAZALD1* comprising contacting the progenitor cell with a hydrogel and TGFβ-3 and GDF-5 thereby differentiating the progenitor cell into a cell expressing one or more genes chosen from *COL2A1*, *OGN*, *FBLN5, DPT, FMOD, CRLF1*, *TGFB3, ITGBL1*, *HHIP, GSN, ADAMTS6*, *TRPS1*, *VCAN; COL2A1*, *ACAN*, *COMP, LECT1*, *HAPLN1*, *FGFR3, COL9A1, ITGA10, COL9A3, CSPG4, XIST, PKDCC, OLFML3, WWP2, SUSD5, DNM1; EPYC, COL9A2, COL10A1, LECT1*, *CHAD, OGN*, *MATN3, COL9A1, PTH1R, PCOLCE2, COL9A3, FMOD, FKBP11, STEAP3, LUM, MEF2C, GPC1, COL27A1, ANGPTL2, CFH, HHIP, KLF4, GPX7, WWP2, FAM46A, DKK1, MXRA8, COL8A1, ATP8B2, RUNX1, TNC, PLCD1, FAM180A, SLC39A14, TRPS1*, *CTGF, PELI2, FGFRL1, PYCR1, GXYLT2, SLC40A1, LTBP2; EPYC, COL10A1, CHAD, COL9A1, COL9A3, FMOD, ELN, SOX9*, *GPC1, COL27A1, ANGPTL2, WWP2, COL8A1, ATP8B2, MRC2, TNC, SLC39A14, CTGF, PELI2, FGFRL1, GXYLT2, LTBP2; COL2A1*, *SCRG1, CHAD, MATN3, COL9A1, PTH1R*, *SOX8, FMOD, MEF2C, PRICKLE1, COL27A1, WWP2, ENPP2, RUNX1, CTGF, PELI2, FGFRL1, KLF2; COL2A1*, *ACAN*, *COMP, LECT1*, *HAPLN1, CHAD, SPP1, FGFR3, COL11A2, COL11A1, COL9A1, PTH1R*, *ITGA10, COL9A3, CSPG4, XIST, WWP2, ENPP1, MMP13, SUSD5,* and *KAZALD1.*

In yet other embodiments the invention provides a method of differentiating a T42 progenitor cell into a cell expressing one or more markers chosen from *ACTC1, CRYAB, EFHD1, FGFR3, MFAP5, TGFB3, DLK1, ACTA2, OCA2, AIF1L, GPC4, SCRG1, CNN1, HES6, KRT17, COL8A1, COL9A2, DCN, RASL11B, IGFBP3, EDN1, IGFBP2, ENC1, SFRP2, ACTG2, CKB, CSRP1, CSRP2, C5orf46, COL3A1, HEY1, TUBB2B, CALD1, KAL1, CD24, CDH2, MAMDC2, EFR3B, CDKN2B, HES4, TAGLN, CAP2, PMEPA1, CTGF, TPM1, TGFB2, CXXC5, COL4A1, PALLD, SOX11, OCIAD2, EML1, CCDC99; TTR, CCDC3, TGFB3, ZIC2, GPC4, POSTN, ID3, PODXL, FBLN5*, *KRT7, COL8A1, OGN*, *TINAGL1, LGMN, GPX7, ACTG2, C5orf46, ANGPTL4, FOXS1, TUBB2B, SLC4A2, SULF1, SLC16A9, CDH2, MAMDC2, ITGA10, ENPP2, NPR3, CDKN2B, TAGLN, INO80C, HTRA1, DHRS3, CTGF, IGFBP7, SERPINE1, PMEPA1, MRPS6, APOE, SMPD1, TPM1, STXBP2, TMEM108, IQCG, COL4A1, SPINT2, MXD3, TPD52L1, HEYL, CDH6, CYR61; EFHD1, CCDC3:, CFH , ZIC2, TIMP4, SYNM , AIF1L, ITGA1, EBF1, DCN, H19, DYSF , EDNRA, NDUFA4L2, ACTG2, COL3A1, CSPG4 , PRRX1, ITGA10, TMEFF2, TAGLN, ZBTB46 , HTRA1, IGFBP7 , ITGA8, APOE , OLAH , IGDCC4, FBLN1, COL4A2, GGT5, MAN1C1, COL4A1, RFTN2, STC2 , IGFBP1, TMEM119, CDH6; DKK1,* DKK2, *DKK3,* and *FAM198B* comprising contacting a micromass of the progenitor cell with BMP-4 thereby differentiating the progenitor cell into a cell expressing one or more genes chosen from *ACTC1, CRYAB, EFHD1, FGFR3, MFAP5, TGFB3, DLK1, ACTA2, OCA2, AIF1L, GPC4, SCRG1, CNN1, HES6, KRT17, COL8A1, COL9A2, DCN, RASL11B, IGFBP3, EDN1, IGFBP2, ENC1, SFRP2, ACTG2, CKB, CSRP1, CSRP2, C5orf46, COL3A1, HEY1, TUBB2B, CALD1, KAL1, CD24, CDH2, MAMDC2, EFR3B, CDKN2B, HES4, TAGLN, CAP2, PMEPA1, CTGF, TPM1, TGFB2, CXXC5, COL4A1, PALLD, SOX11, OCIAD2, EML1, CCDC99; TTR, CCDC3, TGFB3, ZIC2, GPC4, POSTN, ID3, PODXL, FBLN5*, *KRT7, COL8A1, OGN*, *TINAGL1, LGMN, GPX7, ACTG2, C5orf46, ANGPTL4, FOXS1, TUBB2B, SLC4A2, SULF1, SLC16A9, CDH2, MAMDC2, ITGA10, ENPP2, NPR3, CDKN2B, TAGLN, INO80C, HTRA1, DHRS3, CTGF, IGFBP7, SERPINE1, PMEPA1, MRPS6, APOE, SMPD1, TPM1, STXBP2, TMEM108, IQCG, COL4A1, SPINT2, MXD3, TPD52L1, HEYL, CDH6, CYR61; EFHD1, CCDC3: , CFH, ZIC2, TIMP4, SYNM, AIF1L, ITGA1, EBF1, DCN, H19, DYSF , EDNRA , ND UFA4L2 , ACTG2, COL3A1, CSPG4 , PRRX1, ITGA10, TMEFF2 , TAGLN, ZBTB46 , HTRA1, IGFBP7 , ITGA8, APOE , OLAH , IGDCC4, FBLN1, COL4A2, GGT5, MAN1C1, COL4A1, RFTN2 , STC2 , IGFBP1, TMEM119, CDH6; DKK1, DKK2, DKK3,* and *FAM198B.*

In further embodiments the invention provides a method of differentiating a progenitor cell expressing one or more genes chosen from *NR4A2, COL4A6, HGF, ELA2, GPR116, HAND2, VGF,* and *FOXF1* into a cell expressing one or more genes chosen from ACTG2, transgelin *TAGLN, FILIP1L, MYH11, CSRP2* comprising culturing the progenitor cell under micromass conditions and contacting the progenitor cell with BMP-4 thereby differentiating the progenitor cell in a cell expressing ACTG2, transgelin *TAGLN, FILIP1L, MYH11, CSRP2*.

In further embodiments the invention provides a method of differentiating a progenitor cell expressing one or more genes chosen from *TH, CDH18, FGF13, ZIC4, HOXA2* and *HOXB2* into a cell expressing one or more genes chosen from *COL2A1*, *PRG4, COL9A2, ELN* and *ACTA2* comprising culturing the progenitor cell under micromass conditions and contacting the progenitor cell with BMP-2 and TGFβ-3 thereby differentiating the progenitor cell into a cell expressing one or more genes chosen from *TH, CDH18, FGF13, ZIC4, HOXA2* and *HOXB2*

In further embodiments the invention provides a method of differentiating a progenitor cell expressing one or more genes chosen from *EPHA5, HEY2, KCNIP1, KRT17, MKX, OLFML1* and *WDR72* into a cell expressing one or more genes chosen from *ANGPTL7* and COL8A comprising culturing the progenitor cell under micromass conditions and contacting the progenitor cell with BMP-2 and TGFβ-3 thereby differentiating the progenitor cell into a cell expressing one or more markers chosen from ANGPTL7 and COL8A.

### Progeny of Progenitor Cell Lines

In certain embodiments the invention provides the progeny of a progenitor cell line. The progenitor cell line may be an embryonic progenitor cell line such as a human embryonic progenitor cell line (hEP). The progeny of the progenitor cell line may be the in vitro progeny of the progenitor cell line and may include one or more cells that are more differentiated compared to the parental progenitor cell line. The differentiation state of a cell may be determined by analyzing one or more genes expressed by the progeny cell relative to the parental progenitor cell line and/or accessing a database containing information regarding gene expression of cells at various stages of development, such as the LifeMap database. The progeny of the progenitor cell line may be a cell expressing one or more genes typically expressed by a cell in a developing mammalian embryo, such as a primate (e.g. a human). For example the progeny of the progenitor cell line may express one or more genes chosen from: *COL2A1*, *COL10A1, ACAN*, *CRTAC1, TNMD, ALPL, PENK, BGLAP, BMP-2, DLX5, GPC3, IHH, PRG4, CILP, EPYC,SPP1, TTR LPL, CEBPD, PPARG, FABP4, PLIN1, DLK1, PPARGC1A, CEBPD, PPARG, FABP4*, *PRDM16, FOXC2, CRLF1, FOXF2, FBLN5*, *DPT, ITGBL1*, *COL6A3, DUSP1, FOXF1, TGFB3, PAX9, GSN, FMOD, PDE8B, COMP, ITGA10, SAA1, DTNA, PCDH9, EBF1, SORBS1, SORBS2, ZNF503, MGST2, PNMT, DPT, OGN*, *FBLN5*, *FMOD, CRLF1*, *ITGBL1*, *TGFB3, GSN, HHIP, LRIG1, TRPS1, COMP, LECT1*, *COL9A1, HAPLN1*, *ITGA10, OLFML3, PKDCC, FGFR3, CSPG4, COL9A3, ITGB5, DNM1; OGN*, *COL9A2, EPYC, CHAD, PPIB, SRPX2, MATN3, LUM, COL13A1, FKBP11, MXRA8, COL27A1, PELI2, GPX7, ANGPTL2, GXYL.T2, KLF4, STEAP3, SLC39A14, PTH1R*, *FAM46A, FAM180A, SLC26A2, RUNX1, CTGF, PLEKHB1, FKBP7, TNC, JAK2, CYTL1, KDELR3, ATP8B2, TRPS1; ELN, PPIB, PHEX, SOX9*, *COL27A1, PELI2, ANGPTL2, GXYLT2, SLC39A14, P4HA3, SLC26A2, CTGF, MRC2, COL9A3, PLEKHB1, TNC, FRMD8, CYTL1, ATP8B2*; *SCRG1, MATN3, SMOC1, PELI2, PTH1R*, *HTRA1, RUNX1, CTGF, PLEKHB1, RG9MTD1*, *CYTL1,* and *KLF2, LPL, CEBPB, PLIN1, PPARG, PPARGC1A,, PPARG, GSN, WIF1, TGFB3, , CORO2B, ADAMTS15,* and *IGFBP7COMP, SPP1, KAZALD1, LECT1*, *MMP13, HAPLN1*, *PHEX, PTH1R*, *COL11A1,* and *IP6K2 PKDCC, LTBP3, HAPLN1*, *OLFML3, ITGB5, IP6K2, ELN, CYTL1, LTBP3, PELI2, EPYC, PHEX, MRC2, CALY, GXYL.T2, COL27A1, ANGPTL2, SOX9*, *GAA, TNC, LEPRE1, LTBP2, ARFGAP1, SRPX2, CYTL1, FAM46A, LUM, LTBP3, MXRA8, RUNX1, CALY, PTH1R*, *FKBP11, STEAP3, CFH, SLC40A1, GXYL.T2, COL27A1, GPX7, ANGPTL2, MATN3, FKBP7, GAA, TNC, LEPRE1,* and *LTBP2, PPARG, PLIN1, LPL, CSPG4, LTBP3, CA12, PPIB, IRX5, ARHGAP24, FBLN5*, *DPT, CRLF1*, *ITGBL1*, *TGFB3, COL6A3, VCAN, DUSP1, TRPS1, GSN, ADAMTS6*, *ERG; EPYC, ELN, COL8A1, COL9A3, CYTL1, PPIB, CTHRC1, PLEKHB1, SLC26A2, KANK1, SLC39A14, ATP8B2, TNC, LTBP2, GPC1, WWP2, P4HA3, BAMBI, FGFRL1, SDC2; SCRG1, MEF2C, MATN3, PTH1R*, *ENPP2, CYTL1, CTHRC1, PLEKHB1, RUNX3, RUNX1, PRICKLE1, WWP2, HTRA1, CTGF, FGFRL1, ERG, FAT3*; *COL9A2, PCOLCE2, MEF2C, MATN3, LUM, HHIP, PTH1R, SLC40A1, KLF4, SRPX2, CYTL1, FKBP11, CTHRC1, STEAP3, LOXL4, DUSP1, LTBP2, TRPS1, GPC1, CFH, BAMBI, CDH2, COL27A1, FAM46A, CTGF, SDC2, and PLCD1, FRZB, HAND2, DLX5, DLX6, FOXD1, MSX2, TFAP2A, ALPL, BMP2, MSX2, DCN, SPP1, SATB2, MSX2, POSTN PCDH20, SERPINA3, TAC1, EDNRA, SCRG1, CRABP2, SPOCK3, VCAN, BOC, ECM2, CRLF1*, *GAS1, TSPAN8, MFAP4, NFIA, RASSF9, DCN, SATB2, NKX3-2, EBF1, SORBS2, PCDH17, SOBP, ST8SIA4*; *MEF2C, ALPL, PTH1R*, *STEAP3, PELI2, SLC40A1, MXRA8, MATN3, PARD6G, GPX7, FAM180A, CTHRC1, ATP8B2, BAMBI, PLCD1, LTBR, SLC26A2, ANGPTL2, SATB2, FAM46A, DUSP1, CSPG4, , OLFML3, PKDCC, LTBP3, FUS, FBLN5*, *GAS1, DUSP1, GSN, ARHGAP24, VCAN, EMILIN3; PDE8B, SORBS2, PHACTR2, EBF1, PCDH9, MATN3, LTBP2, DUSP1, CDH2, SLC40A1, MXRA8, STEAP3, ANGPTL2, GPX7, TSPAN3, SDC2, ATP8B2, CD36, CTGF, ARHGAP24, FBLN2, SPP1, CSPG4, CSPG4, ENPP1, CA12, DNM1*; *GXYL.T2, ANGPTL2, METRNL, KDELR3, PARD6G*, and *LEPRE1*, *ACTC1, CRYAB, EFHD1, MFAP5, DLK1, ACTA2, OCA2, AIF1L, GPC4, SCRG1, CNN1, HES6, KRT17, RASL11B, IGFBP3, EDN1, ENC1, SFRP2, ACTG2, CKB, CSRP1, CSRP2, C5orf46, COL3A1, HEY1, TUBB2B, CALD1, KAL1, CD24, CDH2, MAMDC2, EFR3B, CDKN2B, HES4, TAGLN, CAP2, PMEPA1, CTGF, TPM1, TGFB2, CXXC5, COL4A1, PALLD, SOX11, OCIAD2, EML1, CCDC99, TTR, CCDC3, TGFB3, ZIC2, GPC4, POSTN, ID3, PODXL, FBLN5*, *KRT7, TINAGL1, LGMN, GPX7, ACTG2, ANGPTL4, TUBB2B, SLC4A2, SULF1, SLC16A9, CDH2, MAMDC2, , ENPP2, NPR3, CDKN2B, TAGLN, INO80C, HTRA1, DHRS3, CTGF, IGFBP7, , PMEPA1, MRPS6, APOE, SMPD1, TPM1, STXBP2, TMEM108, IQCG, COL4A1, SPINT2, MXD3, TPD52L1, HEYL, CDH6, CYR61, EFHD1, CCDC3: , CFH , ZIC2, TIMP4, SYNM , AIF1L, EBF1, H19, DYSF , EDNRA, NDUFA4L2, ACTG2, COL3A1, CSPG4 , PRRX1, TMEFF2 , TAGLN, ZBTB46 , HTRA1, IGFBP7 , APOE , OLAH , IGDCC4, FBLN1, GGT5, MAN1C1, RFTN2, STC2 , IGFBP1, TMEM119, CDH6, DKK2*, *DKK3, FAM198B,* ACTG2, *FILIP1L, MYH11*; *CSRP2 PRG4, AMELX, ENAM, SILV1, PITX1, FABP4*, *AMBN, CNN1, MYH11, ORM1* and *LIPASIN*

In certain embodiments the invention provides an in vitro progeny of progenitor cell line, wherein the progeny expresses one or more genes expressed by a chondrocyte, an adipose cell or an osteoblast.

In some embodiments the progeny of the progenitor cell line is a not a hypotrophic chondrocyte. In some embodiments of the invention the chondrocyte is a definitive chondrocyte. In some embodiments the definitive chondrocyte expresses COL2A1. In some embodiments of the invention the definitive chondrocyte expresses more COL2A1 than COL10A. In certain embodiments of the invention the ratio of expressed COL2A1 to COL10A in the definitive chondrocyte (relative to NHAC) is about 5, about 10, about 15, about 20, about 25 about 30, about 35, about 40, about 45, about 50, about 55, about 60 about 65, about 70, about 75, about 80, about 85, about 90, about 95, about 100. In some embodiments of the invention the ratio of expressed *COL2A1* to *COL10A* in the definitive chondrocyte (relative to NHAC) is greater than 100.

In other embodiments the invention provides an in vitro progeny of a progenitor cell having endochondral ossification capability. In other embodiments the invention provides an in vitro progeny of a progenitor cell wherein the progenitor cell expresses one or more gene markers found on one or more cells chosen from: brown adipocytes, white odipocytes, such as visceral white adipocytes, neural crest derived mesenchyme, intervertebral disc annulus fibrosis, mandibular condyle, endochondral head bones, limb long bones, glenoid fossa cells, endochondrial facial bones, thoracic rib bones, autopod long bones, osteoblasts, intramembraneous preosteoblasts, epiphyseal end limb bone, preosteoblast, lumbar vertebral body, intervertebral disc nucleus pulposis cells, head mesenchyme chondrocytes, glial cells, choroid plexus, brain vascular pericytes, and lens epithelium.

In certain embodiments the invention provides a cell culture comprising the in vitro progeny of a progenitor cell line such as a hEP cell line. In some embodiments the cell culture may comprise one or more growth factors, cytokines and/or mitogens. In certain embodiments the cell culture may comprise one or more members of the TGF-β superfamily. Exemplary members of the TGF-β superfamily include TGFβ3, BMP-2, 4, 6, and 7, and GDF5. In some embodiments the invention provides a micromass culture of progeny. In certain embodiments the cell culture may comprise a hydrogel. Suitable hydrogels may comprise one or more polymers. The polymers may include any polymer known to form a hydrogel including hyaluronate, gelatin, acrylate and the like. In some embodiments the hydrogel is comprised of thiolated hyaluronate. In some embodiments the hydrogel is comprised of thiolated gelatin. In some embodiments the hydrogel is comprise of acrylate crosslinker such PEG diacrylate. In other embodiments a cell matrix may be used. For example suitable matrices may comprise any of the following: agarose, alginate, fibrin, collagen, Matrigel®, e.g. a gelatinous protein mixture secreted by Engelbreth-Holm-Swarm (EHS) mouse sarcoma cells.

In some embodiments the invention provides a cell culture comprising the in vitro progeny of a progenitor cell line wherein the in vitro progeny of a progenitor cell line is chosen from a chondrocyte precursor, an osteoblast precursor and an adipose cell precursor. In certain embodiments of the invention the in vitro progeny of the progenitor cell line, e.g. a chondrocyte precursor, an osteoblast precursor, a connective tissue precursor, such as a tendon or ligament precursor, an adipose precursor. The in vitro progeny of a progenitor cell line may comprise about 5% of the cells in culture, about 10% of the cells in culture, about 15% of the cells in culture, about 20% of the cells in culture, about 25% cells in culture, about 30% of the cells in culture, about 35% of the cells in culture, about 40% of the cells in culture, about 45% of the cells in culture, about 50% of the cells in culture, about 55% of the cells in culture, about 60% of the cells in culture, about 65% of the cells in culture, about 70% of the cells in culture, about 75% of the cells in culture, about 80% of the cells in culture, about 85% of the cells in culture, about 90% of the cells in culture, about 95% of the cells in culture, about 99% of the cells in culture.

### Methods and Compositions for Cryo-Preserving Cells

In some embodiments the invention provides methods for cryo-preserving the cells described infra. In other embodiments the invention provides compositions comprising cryo-preserved cells, wherein the cell is one or more cells described infra.

In certain embodiments the invention provides a composition comprising a cryo-preserved progenitor cell, such as hEP cell described infra. The composition may comprise at least 1 progenitor cell, at least 10, at least 100, at least 1,000, at least 10,000, at least 100,00, at least 1, 000,000 viable cryo preserved progenitor cells. The composition may comprise about 1 progenitor cell, about 10, about 100, about 1,000, about 10,000, about 100,00, about 1, 000,000 viable cryo preserved progenitor cells. The cryopreserved progenitor cell may further comprise a hydrogel wherein the progenitor cell is seeded within the hydrogel. For example, the cell may be encapsulated in the hydrogel. The cryopreserved progenitor cell may include a suitable media containing one or more cryoprotectants, such as DMSO or EBS to facilitate freezing the cells. In one embodiment the invention provides a hEP cell cryopreserved in a hydrogel comprising hyaluronate. The hydrogel may further comprise gelatin. The hydrogel may further comprise an acrylate such as PEG acrylate. The acrylate may serve as a crosslinker. An example of a suitable media for cryo-preserving the cells in a hydrogel may comprise FBS that is 10% DMSO. The cells may frozen at -80° C.

In other embodiments the invention provides a composition comprising a cryo-preserved in vitro differentiated progeny of a progenitor cell, such as any of the in vitro progeny of progenitor cells described infra. The composition may comprise at least 1, at least 10, at least 100, at least 1,000, at least 10,000, at least 100,00, at least 1, 000,000 viable cryo preserved in vitro differentiated progeny of a progenitor cell. The composition may comprise about 1, about 100, about 1,000, about 10,000, about 100,00, about 1, 000,000 viable cryo preserved in vitro differentiated progeny of a progenitor cell. The cryopreserved in vitro differentiated progeny of a progenitor cell may further comprise a hydrogel wherein the in vitro differentiated progeny of a progenitor cell is seeded within the hydrogel. The cryopreserved in vitro differentiated progeny of a progenitor cell may include a suitable media containing one or more cryoprotectants, such as DMSO or FBS to facilitate freezing the cells. In one embodiment the invention provides the in vitro differentiated progeny of an hEP cell cryopreserved in a hydrogel comprising hyaluronate. The hydrogel may further comprise gelatin. The hydrogel may further comprise an acrylate such as PEG acrylate. The acrylate may serve as a crosslinker. An example of a suitable media for cryo-preserving the cells in a hydrogel may comprise FBS that is 10% DMSO. The cells may frozen at -80° C.

The cryopreserved compositions may be used in research and therapeutic applications. For example a subject in need of cell therapy may be treated with the cryopreserved composition described infra. The composition may be thawed and administered to a subject in need of treatment. The placement of the cells described infra in the hydrogel may facilitate both cryopreserving the cell and enhancing transplantation of the cell into a subject. For example the cryo-preserved cell may be stored and shipped frozen and thawed at just prior to administration to a subject.

In some embodiments the invention provides a method of cryo-preserving a cell comprising 1) contacting the cell with a hydrogel, 2) contacting the cell of 1) with a media comprising fetal bovine serum (FBS) and dimethyl sulfoxide (DMSO) and 3) freezing the cell of 2) at -80°C thereby cryo-preserving the cell.

In some embodiments the method described in the previous paragraph is practiced using one or more of the cells described infra. Thus the cell may be a hEP cell or the in vitro differentiated progeny of a hEP cell. The cell may be contacted with the hydrogel before the hydrogel has had a chance to solidify, e.g. the may be contacted with one or more liquid preparations comprising the hydrogel and after contacting the cell with the one or more liquid preparations comprising the hydrogel the hydrogel may be allowed to polymerize. The cell may be encapsulated within the hydrogel. The hydrogel may comprise hyaluronate, gelatin and a crosslinker such as an acrylate or methacrylate, e.g., PEG acrylate. The hyaluronate may be thiolated. The gelatin may be thiolated. (See US Patent Nos. 7,928,069; 7,981,871). The hydrogel may be seeded with about 100 cells, about 500 cells, about 1,000 cells, about 10,000 cells, about 100,000 cells, about 1, 000,000 cells, about 10, 000,000 cells. In some embodiments the hydrogel is seeded with about 10⁵-to about 10⁷ cells.

The media used in the method of cryo-preserving cells described infra may comprise any known media and a suitable cryoprotectant. Examples of suitable cryoprotectants include FBS, DMSO, glycerol, glucose and the like. In one embodiment the media is comprised of FBS that is made 10% DMSO. In another embodiment the media consists of FBS that is made 10% DMSO.

### Applications

The disclosed methods for the culture of animal cells and tissues are useful in generating cells or progeny thereof in mammalian and human cell therapy, such as, but not limited to, generating human cells useful in treating orthopedic disorders in humans and nonhuman animals. In some embodiments the progenitor cells and their in vitro derived differentiated progeny may be used to treat cartilage related diseases, such as arthritis or trauma and the like. In other embodiments the progenitor cells and their in vitro derived differentiated progeny may be used as tissue bulking agents, e.g. to treat incontinence.

In certain embodiments of the invention, single cell-derived and oligoclonal cell-derived cells and their differentiated progeny as described infra are utilized in research and/or treatment of disorders relating to cell biology. For example the hEP cells and their differentiated progeny may be used to generate cDNA libraries which in turn could be used to study gene expression in developing tissue, such bone, cartilage and fat. The hEP cells and their differentiated progeny can be used in drug screening. For example, the cell, such as a differentiated progeny of hEP cell, could be contacted with a test drug or compound and analyzed for toxicity by examining the cells under a microscope and observing their morphology or by studying their growth or survival in culture. The cells may also be screened for gene expression to determine the effects of the test drug or compound. For example, a comparison could be made between a differentiated progeny of hEP cell that has been contacted with the test drug or compound compared with the same differentiated progeny cell that has not been so contacted. The differentiated progeny of hEP cells may be used to screen for the effects of growth factors, hormones, cytokines, mitogens and the like to determine the effects of these test compounds on the differentiation status of the differentiated progeny of the hEP cells.

In certain embodiments of the invention, the differentiated progeny of the hEP cells may be introduced into the tissues in which they normally reside in order to exhibit therapeutic utility or alternatively to coax the cells to differentiate further. In certain embodiments of the invention, the differentiated progeny of the hEP cells described infra, may be utilized in inducing the differentiation of other pluripotent or multipotent stem cells. Cell-cell induction is a common means of directing differentiation in the early embryo. Cell types useful in the induction may mimic induction well known in the art to occur naturally in normal embryonic development.

Many potentially medically-useful cell types are influenced by inductive signals during normal embryonic development, including spinal cord neurons, cardiac cells, pancreatic beta cells, and definitive hematopoietic cells. Differentiated progeny of hEP cells may be cultured in a variety of in vitro, in ovo, or in vivo culture conditions to induce the differentiation of other pluripotent stem cells to become desired cell or tissue types. Induction may be carried out in a variety of methods that juxtapose the inducer cell with the target cell. By way of nonlimiting examples, the inducer cells may be plated in tissue culture and treated with mitomycin C or radiation to prevent the cells from replicating further. The target cells are then plated on top of the mitotically-inactivated inducer cells. Alternatively, the differentiated progeny of hEP cells may be cultured on a removable membrane from a larger culture of cells or from an original single cell-derived colony and the target cells may be plated on top of the inducer cells or a separate membrane covered with target cells may be juxtaposed so as to sandwich the two cell layers in direct contact. The resulting bilayer of cells may be cultured in vitro, transplanted into a SPF avian egg, or cultured in conditions to allow growth in three dimensions while being provided vascular support (see, for example, international patent publication number WO/2005/068610, published July 28, 2005). The inducer cells may also be from a source of differentiated progeny of hEP cells, in which a suicide construct has been introduced such that the inducer cells can be removed at will.

In certain embodiments of the invention, the differentiated progeny of hEP cells described infra, may be used as "feeder cells" to support the growth of other cell types, including pluripotent stem cells. The use of the differentiated progeny of hEP cells of the present invention as feeder cells alleviates the potential risk of transmitting pathogens from feeder cells derived from other mammalian sources to the target cells. The feeder cells may be inactivated, for example, by gamma ray irradiation or by treatment with mitomycin C, to limit replication and then co-cultured with the pluripotent stem cells.

In certain embodiments of the invention, the extracellular matrix (ECM) of the differentiated progeny of hEP cell disclosed infra, may be used to support less differentiated cells (see Stojkovic et al., Stem Cells (2005) 23(3):306-14). Certain cell types that normally require a feeder layer can be supported in feeder-free culture on a matrix (Rosler et al., Dev Dyn. (2004) 229(2):259-74). The matrix can be deposited by preculturing and lysing a matrix-forming cell line (see WO 99/20741), such as the STO mouse fibroblast line (ATCC Accession No. CRL-1503), or human placental fibroblasts.

In certain embodiments of the invention, the conditioned media of differentiated progeny of hEP cells may be collected, pooled, filtered and stored as conditioned medium. This conditioned medium may be formulated and used for research and therapy. The use of conditioned medium of cell cultures described infra may be advantageous in reducing the potential risk of exposing cultured cells to non-human animal pathogens derived from other mammalian sources (i.e. xenogeneic free).

In another embodiment of the invention, single cell-derived and oligoclonal cell-derived cells and their differentiated progeny described infra may be used as a means to identify and characterize genes that are transcriptionally activated or repressed as the cells undergo differentiation. For example, libraries of gene trap single cell-derived or oligoclonal cell-derived cells and/or their differentiated progeny may be made by methods of this invention, and assayed to detect changes in the level of expression of the gene trap markers as the cells differentiate in vitro and in vivo. The methods for making gene trap cells and for detecting changes in the expression of the gene trap markers as the cells differentiate are reviewed in Durick et al. (Genome Res. (1999) 9:1019-25). The vectors and methods useful for making gene trap cells and for detecting changes in the expression of the gene trap markers as the cells differentiate are also described in U.S. Patent No. 5,922,601 (Baetscher et al.), U.S. Patent No. 6,248,934 (Tessier-Lavigne) and in U.S. patent publication No. 2004/0219563 (West et al.). Methods for genetically modifying cells, inducing their differentiation in vitro, and using them to generate chimeric or nuclear-transfer cloned embryos and cloned mice are developed and known in the art. To facilitate the identification of genes and the characterization of their physiological activities, large libraries of gene trap cells having gene trap DNA markers randomly inserted in their genomes may be prepared. Efficient methods have been developed to screen and detect changes in the level of expression of the gene trap markers as the cells differentiate in vitro or in vivo. In vivo methods for inducing single cell-derived or oligoclonal cell-derived cells or their differentiated progeny to differentiate further include injecting one or more cells into a blastocyst to form a chimeric embryo that is allowed to develop; fusing a stem cell with an enucleated oocyte to form a nuclear transfer unit (NTU), and culturing the NTU under conditions that result in generation of an embryo that is allowed to develop; and implanting one or more clonogenic differentiated cells into an immune-compromised or a histocompatible host animal (e.g., a SCID mouse, or a syngeneic nuclear donor) and allowing teratomas comprising differentiated cells to form. In vitro methods for inducing single cell-derived or oligoclonal cell-derived cells to differentiate further include culturing the cells in a monolayer, in suspension, or in three-dimensional matrices, alone or in co-culture with cells of a different type, and exposing them to one of many combinations of chemical, biological, and physical agents, including co-culture with one or more different types of cells, that are known to capable of induce or allow differentiation.

In another embodiment of the invention, cell types that do not proliferate well under any known cell culture conditions may be induced to proliferate such that they can be isolated clonally or oligoclonally according to the methods of this invention through the regulated expression of factors that overcome inhibition of the cell cycle, such as regulated expression of SV40 virus large T-antigen (Tag), or regulated E1a and/or E1b, or papillomavirus E6 and/or E7, or CDK4 (see, e.g., U.S. patent application Ser. No. 11/604,047 filed on November 21, 2006 and titled "Methods to Accelerate the Isolation of Novel Cell Strains from Pluripotent Stem Cells and Cells Obtained Thereby").

In another embodiment of the invention, the factors that override cell cycle arrest may be fused with additional proteins or protein domains and delivered to the cells. For example, factors that override cell cycle arrest may be joined to a protein transduction domain (PTD). Protein transduction domains, covalently or non-covalently linked to factors that override cell cycle arrest, allow the translocation of said factors across the cell membranes so the protein may ultimately reach the nuclear compartments of the cells. PTDs that may be fused with factors that override cell cycle arrest include the PTD of the HIV transactivating protein (TAT) (Tat 47-57) (Schwarze and Dowdy 2000 Trends Pharmacol. Sci. 21: 45-48; Krosl et al. 2003 Nature Medicine (9): 1428-1432). For the HIV TAT protein, the amino acid sequence conferring membrane translocation activity corresponds to residues 47-57 (Ho et al., 2001, Cancer Research 61: 473-477; Vives et al., 1997, J. Biol. Chem. 272: 16010-16017). These residues alone can confer protein translocation activity.

In another embodiment of the invention, the PTD and the cycle arrest factor may be conjugated via a linker. The exact length and sequence of the linker and its orientation relative to the linked sequences may vary. The linker may comprise, for example, 2, 10, 20, 30, or more amino acids and may be selected based on desired properties such as solubility, length, steric separation, etc. In particular embodiments, the linker may comprise a functional sequence useful for the purification, detection, or modification, for example, of the fusion protein.

In another embodiment of the invention, single cell-derived or oligoclonal cell-derived cells or their differentiated progeny described infra may be reprogrammed to an undifferentiated state through novel reprogramming technique, as described in U.S. application no. 60/705,625, filed August 3, 2005, U.S. application no. 60/729,173, filed October 20, 2005; U.S. application no. 60/818,813, filed July 5, 2006. Briefly, the cells may reprogrammed to an undifferentiated state using at least a two, preferably three-step process involving a first nuclear remodeling step, a second cellular reconstitution step, and finally, a third step in which the resulting colonies of cells arising from step two are characterized for the extent of reprogramming and for the normality of the karyotype and quality. In certain embodiments, the single cell-derived or oligoclonal cell-derived cells or their differentiated progeny described infra may be reprogrammed in the first nuclear remodeling step of the reprogramming process by remodeling the nuclear envelope and the chromatin of a differentiated cell to more closely resemble the molecular composition of an undifferentiated or a germ-line cell. In the second cellular reconstitution step of the reprogramming process, the nucleus, containing the remodeled nuclear envelope of step one, is then fused with a cytoplasmic bleb containing requisite mitotic apparatus which is capable, together with the transferred nucleus, of producing a population of undifferentiated stem cells such as ES or ED-like cells capable of proliferation. In the third step of the reprogramming process, colonies of cells arising from one or a number of cells resulting from step two are characterized for the extent of reprogramming and for the normality of the karyotype and colonies of a high quality are selected. While this third step is not required to successfully reprogram cells and is not necessary in some applications, the inclusion of the third quality control step may be useful when reprogrammed cells are used in certain applications such as human transplantation. Finally, colonies of reprogrammed cells that have a normal karyotype but not sufficient degree of programming may be recycled by repeating steps one and two or steps one through three.

In another embodiment of the invention, the single cell-derived and oligoclonal cell-derived cells or their differentiated progeny may be used to generate ligands using phage display technology (see U.S. application no. 60/685,758, filed May 27, 2005, and PCT US2006/020552).

In some embodiments, a cell described infra could produce BMP2, BMP7, BMP3b or other members of the BMP family, and this cell could therefore be useful in inducing bone formation (as described below). For example the cells could be used to isolate one or more members of the BMP family. Alternatively, the cells could be put in contact with a cell capable of being induced to form bone.

The expression of genes of the cells of this invention may be determined. Measurement of the gene expression levels may be performed by any known methods in the art, including but not limited to, microarray gene expression analysis, bead array gene expression analysis and Northern analysis. The gene expression levels may be represented as relative expression normalized to the *ADPRT* (Accession number NM_001618.2), GAPD (Accession number NM_002046.2), or other housekeeping genes known in the art. The gene expression data may also be normalized by a median of medians method. In this method, each array gives a different total intensity. Using the median value is a robust way of comparing cell lines (arrays) in an experiment. As an example, the median may be found for each cell line and then the median of those medians may become the value for normalization. The signal from the each cell line may be made relative to each of the other cell lines. Based on the gene expression levels, one may be able to determine the expression of the corresponding proteins by the cells of the invention. For example, in the case of cell clone ACTC60 (or B-28) of Series 1, relatively high levels of *DKK1, VEGFC* and *IL1R1* were observed. Therefore, the ability to measure the bioactive or growth factors produced by said cells may be useful in research and in the treatment of disease.

In another embodiment of the invention, the single cell-derived or oligoclonal cell-derived cells, or their differentiated progeny, described infra may express unique patterns of CD antigen gene expression, which are cell surface antigens. The differential expression of CD antigens on the cell surface may be useful as a tool, for example, for sorting cells using commercially available antibodies, based upon which CD antigens are expressed by the cells. The expression profiles of CD antigens of some cells of this invention are shown in West et al., 2008, Regen Med vol. 3(3) pp. 287-308, incorporated herein by reference, including supplemental information. There are several CD antigens that are expressed in the relative more differentiated cells of this invention, but are not expressed in ES cells (or in some cases at markedly reduced levels). The antigens that fall into this category include: *CD73, CD97, CD140B, CD151, CD172A, CD230, CD280, CDw210b.* These antigens may be useful in a negative selection strategy to grow ES cells or alternatively to isolate certain cells described infra.

In another embodiment of the invention, the single cell-derived and oligoclonal cell-derived cells or their differentiated progeny, may be injected into mice to raise antibodies to differentiation antigens. Antibodies to differentiation antigens would be useful for both identifying the cells to document the purity of populations for cell therapies, for research in cell differentiation, as well as for documenting the presence and fate of the cells following transplantation. In general, the techniques for raising antibodies are well known in the art.

In another embodiment of the invention, the single cell-derived and oligoclonal cell-derived cells or the differentiated progeny thereof may be used for the purpose of generating increased quantities of diverse cell types with less pluripotentiality than the original stem cell type, but not yet fully differentiated cells. mRNA or miRNA can then be prepared from these cell lines and microarrays of their relative gene expression can be performed as described herein. In another embodiment of the invention, the single cell-derived and oligoclonal cell-derived cells or their differentiated progeny may be used in animal transplant models, e.g. transplanting escalating doses of the cells with or without other molecules, such as ECM components, to determine whether the cells proliferate after transplantation, where they migrate to, and their long-term differentiated fate in safety studies.

In another embodiment of the invention, the single cell-derived and oligoclonal cell-derived cells generated according to the methods of the present invention are useful for harvesting mRNA, microRNA, and cDNA from either single cells or a small number of cells (i.e., clones) to generate a database of gene expression information. This database allows researchers to identify the identity of cell types by searching for which cell types in the database express or do not express genes at comparable levels of the cell type or cell types under investigation. For example, the relative expression of mRNA may be determined using microarray analysis as is well known in the art. The relative values may be imported into a software such as Microsoft Excel and gene expression values from the different cell lines normalized using various techniques well known in the art such as mean, mode, median, and quantile normalization. Hierarchical clustering with the single linkage method may be performed with the software such as *The R Project for Statistical Computing* as is well known in the art. An example of such documentation may be found online. A hierarchical clustering analysis can then be performed as is well known in the art. These software programs perform a hierarchical cluster analysis using a group of dissimilarities for the number of objects being clustered. At first, each object is put in its own cluster, then iteratively, each similar cluster is joined until there is one cluster. Distances between clusters are computed by Lance-Williams dissimilarity update formula (Becker, R. A., Chambers, J. M. and Wilks, A. R. (1988) The New S Language. Wadsworth & Brooks/Cole. (S version.); Everitt, B. (1974). Cluster Analysis. London: Heinemann Educ. Books). Typically the vertical axis of the dendograms displays the extent of similarity of the gene expression profiles of the cell clones. That is, the farther down they branch apart, the more similar they are. The vertical axis is a set of n-1 non-decreasing real values. The clustering height is the value of the criterion associated with the clustering method for the particular agglomeration. In order to determine if a new cell line is identical to existing cell lines, two types of replicates are performed: biological and technical replicates. Biological replicates require that new cell lines be grown, mRNA harvested, and then the analysis compared. Technical replicates, on the other hand, analyze the same RNA twice. A line cutoff is then drawn just above where the replicates branch such that cells branching below the cutoff line are considered the same cell type. Another source of data for the database described above may be microRNA profiles of the single cell-derived and oligoclonal cell-derived cells or their differentiated progeny described infra. MicroRNAs (miRNA) are endogenous RNAs of ∼22 nucleotides that play important regulatory roles in animals & plants by targeting mRNAs for cleavage or translational repression. More than 700 miRNAs have been identified across species. Their expression levels vary among species and tissues. Low abundant miRNAs have been difficult to detect based on current technologies such as cloning, Northern hybridization, and the modified Invader® assay. In the present invention, an alternative approach using a new real-time quantitation method termed looped-primer RT-PCR may be used for accurate and sensitive detection of miRNAs as well as other non-coding RNA (ncRNA) molecules present in human embryonic stem cells and in cell lines differentiated from human embryonic stem cells.

In another embodiment of the invention, gene expression analysis may be used to identify the developmental pathways and cell types for in *vitro* differentiated hES cells. Gene expression analysis of single cells or a small number of cells from human or nonhuman embryonic or fetal tissues provides another means to generate a database of unique gene expression profiles for distinct populations of cells at different stages of differentiation. Gene expression analysis on single cells isolated from specific tissues may be performed as previously described by Kurimoto et al., Nucleic Acids Research (2006) Vol. 34, No. 5, e42. Thus, cellular miRNA profiles on their own or in conjunction with gene expression profiles, immunocytochemistry, and proteomics provide molecular signatures that can be used to identify the tissue and developmental stage of differentiating cell lines. This technique illustrates that the database may be used to accurately identify cell types and distinguish them from other cell types.

The cells of the present invention are also useful in providing a subset of gene expression markers that are expressed at relatively high levels in some cell lines while not be expressed at all in other cell lines as opposed to genes expressed in all cell lines but at different levels of expression. This subset of "all-or none" markers can be easily identified by comparing the levels of expression as measured for instance through the use of oligonucleotide probes or other means know in the art, and comparing the level of a gene's expression in one line compared to all the other lines of the present invention. Those genes that are expressed at relatively high levels in a subset of lines, and not at all in other lines, are used to generate a short list of gene expression markers. When applied to the cells and gene expression data described herein, where negative expression in Illumina 1 is <70 RFU and positive expression is >100 RFU.

Screening of secreted or extracellular matrix proteins for biological activity

The cell lines and their differentiated progeny of the present invention are also useful as a means of screening diverse embryonic secretomes for varied biological activities. The cell lines of the present invention cultured at 18-21 doublings of clonal expansion express a wide array of secreted soluble and extracellular matrix genes (see US Patent Application Publication 2010/0184033 entitled "METHODS TO ACCELERATE THE ISOLATION OF NOVEL CELL STRAINS FROM PLURIPOTENT STEM CELLS AND CELLS OBTAINED THEREBY" filed on July 16, 2009, incorporated herein by reference). At 21 or more doublings of clonal expansion, the cells of the present invention differentially express secreted soluble and extracellular matrix genes. These proteins, proteoglycans, cytokines, and growth factors may be harvested from the embryonic progenitor cell lines or their differentiated progeny of the present invention by various techniques known in the art including those described infra. These pools of secreted and extracellular matrix proteins may be further purified or used as mixtures of factors and used in varied in vitro or in vivo assays of biological activity as is known in the art. The secreted proteins could be used as an antigen to generate antibodies such as polyclonal or monoclonal antibodies. The antibodies in turn can be used to isolate the secreted protein. As an example, differentiated progeny expressing collagen such as COL2A1 or COL10 could be used to isolate collagen from the cells or to generate antibodies specific to collagen. The collagen could be used as a matrix for attaching and growing cells. The antibodies could be used to purify collagen from the cells described infra or other cells expressing collagen.

### Methods for Analyzing Embryonic Progenitor Cells and Their Differentiated Progeny

In some embodiments of the invention, described infra, the following methods may be useful in the analysis of embryonic progenitor cells and their differentiated progeny, e.g. their in vitro differentiated progeny.

### ELISA

The concentration of transthyretin protein in conditioned medium was determined by ELISA. E69, T42, and MEL2 cells were cultured for 14 days in micromass culture in the presence of BMP4 10 ng/mL and 10 ng/mL TGFβ3, (50 micromasses at 200,000 cells each in 10 cm dishes, with 20 mL serum-free differentiation medium). After three days, conditioned medium was removed, and concentrated using Amicon spin concentrators (Millipore Cat # UFC901024). Concentrates were assayed in duplicate at three dilutions (100x, 200x, and 400x for both T42 and E69; and 50x, 100x and 200x for MEL2) using an Abcam transthyretin ELISA kits (Cat# ab108895). The concentration (ng/mL) of transthyretin in conditioned medium was calculated upon taking into account the fold-concentration in Amicon concentrators.

### Isolation of RNA

RNA is prepared from cell lysates using the Rneasy mini kits (Qiagen) according to the manufacturer's instructions. Briefly, cell cultures (micromasses) are rinsed in PBS, then lysed in a minimal volume of the RLT lysis buffer. After incubation on ice, the cell debris is removed by centrifugation and the lysate is mixed with RLT buffer, after which ethanol is added to the mixture. The combined mixture is then loaded onto the Rneasy spin column and centrifuged; the loaded column is then washed and the purified RNA is released from the column with a minimal volume of DEPC-treated water (typically 30 ul or less). The concentration of RNA in the final eluate is determined by absorbance at 260 nm.

### cDNA Synthesis

cDNA synthesis is performed using the SuperScript First Strand cDNA kit (InVitrogen; Carlsbad, CA). Briefly, 2.5 ug of purified RNA is heat denatured in the presence of random hexamers. After cooling, the first strand reaction is completed using SuperSript reverse transcriptase enzyme and associated reagents from the kit. The resulting product is further purified using QIAquick PCR Purification kits (Qiagen) according to the manufacturer's instructions. Briefly, PB buffer is added to the first strand cDNA reaction products, then the mixture is loaded onto the QIAquick spin column and centrifuged. The column is washed with PE buffer and the purified cDNA is eluted from the column using a minimal volume of water (20 ul).

### qPCR Primers

qPCR primer pairs are synthesized for each target gene. Briefly, primer pairs for a target gene are designed to amplify only the target mRNA sequence and optimally have annealing temperatures for their target sequences that lie in the range of 65-80 °C and unique amplification products in the size range of 100-500 bp. Primer pairs are supplied at working concentrations (10 uM) to BioTrove, Inc. (Woburn, MA) for production of a custom qPCR Open Array plate. OpenArray plates are designed to accommodate 56-336 primer pairs and the final manufactured plate with dried down primer pairs is provided to the service provider. Purified cDNA reaction products (2.) and Syber green master mix are loaded into individual wells of the OpenArray plate using OpenArray autolader device (BioTrove). The plate is sealed and the qPCR and loaded into the NT Imager/Cycler device (BioTrove) for amplification. Ct values for each sample are calculated using the OpenArray application software.

Markers of differentiation are not those present in embryonic progenitor cell lines, but are present in later stages of differentiation. It is not obvious to what an effective array of such markers would be. For example, *COL2A1* is not expressed in the clonal embryonic progenitor cell lines, but is markedly induced > 100-fold in a subset of the cell lines of the present invention. Previous attempts to invent an array of differentiation markers were not useful in the context of the present invention because they included a majority of markers that were expressed in both embryonic progenitor cell types and in terminally-differentiated cell types (Luo, Y., Cai, J., Ginis, I., Sun, Y., Lee, S., Yu, S.X., Hoke, A., and Rao, M. 2003. Designing, testing, and validating a focused stem cell microarray for characterization of neural stem cells and progenitor cells. Stem Cells, 21:575-587). An example of a list of markers useful in determining that a particular differentiation condition induced terminal differentiation in embryonic progenitor cell lines a majority of which are not expressed in embryonic progenitor cell lines are disclosed in US Patent Publication Nos: 20120171171 and 20100184033 which discloses markers expressed by certain human embryonic progenitor cell lines.

### Secreted Protein Isolation Protocol 1 - Conditioned medium

Cells may be grown in either their normal propagation medium (West et al., 2008, Regen Med vol. 3(3) pp. 287-308) or the differentiation conditions described herein. To obtain conditioned medium on a smaller scale (typically 1-2 L or less), the cells may be grown in monolayer cultures in T150, T175 or T225 flasks (Corning or BD Falcon) in a 37°C incubator with 10% CO₂ atmosphere. For larger volume medium collections, the cells may be typically grown either in 2 L roller bottles, on microcarrier suspensions (porous such as Cytodex varieties from Sigma-Aldrich, St. Louis, MO, or non-porous such as from SoloHill Engineering, Ann Arbor, MI) in spinner flasks or other bioreactors, or in hollow fiber cartridge bioreactors (GE Healthcare, Piscataway, NJ). Prior to conditioned medium collection, the cultures may be rinsed twice with PBS and then incubated for 2 hours at 37°C in the presence of serum-free medium wherein the medium is the same basal medium as described herein for the propagation or differentiation of the cells, in order to remove fetal serum proteins. The serum-free medium may then be removed and replaced with fresh medium, followed by continued as described herein at 37°C for 24-48 hours.

The culture-conditioned medium may then be collected by separation from the cell-bound vessel surface or matrix (e.g., by pouring off directly or after sedimentation) and processed further for secreted protein concentration, enrichment or purification. As deemed appropriate for the collection volume, the culture medium may be first centrifuged at 500 to 10,000 xg to remove residual cells and cellular debris in 15 or 50 ml centrifuge tubes or 250 ml bottles. It then may be passaged through successive 1 µm or 0.45 µm and 0.2 µm filter units (Coming) to remove additional debris, and then concentrated using 10,000 MW cutoff ultrafiltration in a stirred cell or Centricon centrifuge filter (Amicon-Millipore) for smaller volumes, or using a tangential flow ultrafiltration unit (Amicon-Millipore) for larger volumes. The retained protein concentrate may then be dialyzed into an appropriate buffer for subsequent purification of specific proteins, and further purified using a combination of isoelectric focusing, size exclusion chromatography, ion exchange chromatography, hydrophobic or reverse phase chromatography, antibody affinity chromatography or other well-known methods appropriate for the specific proteins. During the various steps in the purification process, collection fractions may be tested for the presence and quantity of the specific secreted protein by ELISA (e.g., using BMP-2 or BMP-7 ELISA kits from R&D Systems, Minneapolis, MN). The purified proteins may then be kept in solution or lyophilized and then stored at 4 or minus 20-80°C.

### Secreted Protein Isolation Protocol 2 - Urea-mediated protein extraction

In the case of some secreted proteins, interactions with the cell or ECM components may reduce the simple diffusion of factors into the medium as described above in Secreted Protein Isolation Protocol 1. A simple comparison of the yield in the two protocols may suffice to determine which protocol provides the highest yield of the desired factors. In the case of Secreted Protein Isolation Protocol 2, a low concentration of urea may be added to facilitate the removal of factors. In the case of the examples provided, all urea extractions may be performed two days subsequent to feeding. On the second day, cell monolayers in T-150 cell culture flasks may be rinsed twice with CMF-PBS and then incubated for two hours at 37°C in the presence of serum-free medium. The rinse with CMF-PBS and the incubation in serum-free medium together may aid in the removal of fetal serum proteins from the surface of the cells. The serum-free medium may then be removed and 10 ml /T150 of freshly made 200 mM urea in CMF-PBS may be added. The flasks may then be placed on a rocker at 37°C. for 6.0 hours. The urea solution may then be removed and immediately frozen at -70°C.

### Extracellular Matrix Isolation Protocol- DOC-Mediated Preparation

Extracellular matrix proteins can be extracted using the method of Hedman et al, 1979 (Isolation of the pericellular matrix of human fibroblast cultures. J. Cell Bio. 81: 83-91). Cell layers may be rinsed three times with CMF-PBS buffer at ambient temperature and then washed with 30 mL of 0.5% sodium deoxycholate (DOC), 1 mM phenylmethylsulfonylfluride (PMSF, from 0.4M solution in EtOH), CMF-PBS buffer 3 X 10 min. on ice while on a rocking platform. The flasks may then be washed in the same manner with 2mM Tris-HCI, pH 8.0 and 1 mM PMSF 3 X 5 min. The protein remaining attached to the flask may then be removed in 2 mL of gel loading buffer with a rubber policeman.

### Safranin O Staining Assay

The well-known techniques of staining of formalin-fixed, paraffin-embedded tissue sections with Safranin O are commonly used in the detection of cartilage-related proteoglycans, however, the assay is not absolutely specific to cartilage since it also stains mucin, mast cell granules, and likely other substances in other cell types. A nonlimiting example of the protocol where cartilage and mucin may be stained orange to red, and the nuclei may be stained black and the background stained green uses formalin-fixed micromasses, pellets, or similar aggregations of cells. Reagents which may be used include Weigert's Iron Hematoxylin Solution: in which Stock Solution A composed of 1 gram of Hematoxylin in 100 ml of 95% Alcohol; Stock Solution B composed of 4 ml of 29% Ferric chloride in water diluted in 95 ml of Distilled water and 1.0 ml of concentrated Hydrochloric acid; Weigert's Iron Hematoxylin Working Solution composed of equal parts of stock solution A and B and used within four weeks; 0.001% Fast Green (FCF) Solution composed of 0.01 gram of Fast green, FCF, C.I. 42053 in 1000 ml Distilled water; 1% Acetic Acid Solution composed of 1.0 ml glacial acetic acid in 99 ml Distilled water; and 0.1% Safranin O Solution composed of 0.1 gram Safranin O, C.I. 50240 in 100 ml Distilled water. Samples may be deparaffinized and hydrated with distilled water. They may be stained with Weigert's iron hematoxylin working solution for 10 minutes, then washed in running tap water for 10 minutes, stained with fast green (FCF) solution for 5 minutes, rinsed quickly with 1% acetic acid solution for no more than 10 -15 seconds, stained in 0.1 % safranin **O** solution for 5 minutes, dehydrated and cleared with 95% ethyl alcohol, absolute ethyl alcohol, and xylene, using 2 changes each, 2 minutes each, mounted using resinous medium, and imaged and analyzed for stains as described above. Cartilage-related proteoglycan stains dark red-orange.

### Kits and Media

In certain embodiments the invention provides a kit for differentiating progenitor cell, such as hEG cells described infra.

In one embodiment the kit comprises a media supplemented with one or more exogenously added TGF-β superfamily member. The TGF-β superfamily member may include one or more of the following: TGFβ3, BMP2, BMP4, BMP6, BMP7, and GDF5. In some embodiments the media is supplemented with a plurality of exogenously added TGF-β superfamily members.

In one embodiment the media is supplemented with TGFβ3. In another embodiment the media is supplemented with exogenously added TGFβ3 and at least one other member of the TGF-β superfamily. In another embodiment the media is supplemented with exogenously added BMP2. In yet another embodiment the media is supplemented with exogenously added BMP4. In still another embodiment the media is supplemented with exogenously added BMP4. In a further embodiment the media is supplemented with exogenously added BMP6. In still other embodiments the media is supplemented with BMP7. In further embodiments the media is supplemented with GDF5.

One or more of the TGFβ superfamily members described in the preceding paragraphs may be provided in the media at a concentration of about 1 ng/ml, 5 ng/ml, 10 ng/ml, 15 ng/ml. 20 ng/ml, 25 ng/ml, 30 ng/ml, 40 ng/ml, 50 ng/ml, 60 ng/ml, 70 ng/ml, 80 ng/ml, 90ng/ml, 100 ng/ml, 200 ng/ml, 300 ng/ml, 400 ng/ml, 500 ng/ml, 600 ng/ml, 700 ng/ml, 800 ng/ml, 900 ng/ml, 1,000 ng/ml. In some embodiments of the invention the TGFβ superfamily members described in the preceding paragraphs may be provided in the media at a concentration of greater than 1,000 ng/ml. The TGFβ superfamily members may be chosen from TGFβ3, BMP2, BMP4, BMP6, BMP7, and GDF5.

In some embodiments the kit may comprise a media supplemented with an exogenously added retinol, such as retinoic acid. The exogenously added retinoic acid may be provided at a concentration of about 0.1µM, 0.2µM, 0.3µM, 0.4µM, 0.5µM, 0.6µM, 0.7µM, 0.8µM, 0.9µM, 1µM, 2µM, 3µM, 4µM, 5µM. In some embodiments the concentration of the exogenously added retinoic acid is greater than 5µM.

In some embodiments the kit may further comprise a hydrogel. The hydrogel may be comprised of hyaluronate. The hydrogel may be comprised of gelatin. The hydrogel may be comprised of an acrylate. The hyaluronate may be thiolated. The gelatin may be thiolated. The acrylate may be a PEG acrylate such as PEG diacrylate.

In certain embodiments of the invention the kit may further comprise a cell described infra. Thus, in some embodiments, the kit may further comprise a progenitor cell, such as a hEP cell. The hEP cell may have chondrogenic potential. In other embodiments, the kit may further comprise a differentiated progeny of a progenitor cell, such as an in vitro differentiated progeny of a progenitor cell described infra.

### Hydrogels and Matrices

Various embodiments described infra, including methods, compositions, cells, cell cultures and kits include a hydrogel. Alternatively, the methods, compositions, cells, cell cultures and kits described infra may compise a suitable matrix.

Any hydrogel known in the art may be used. Any matrix capable of supporting cell growth may be used. Suitable hydrogels may comprise one or more polymers. The polymers may include any polymer known to form a hydrogel including hyaluronate, gelatin, acrylate and the like. In some embodiments the hydrogel is comprised of thiolated hyaluronate. In some embodiments the hydrogel is comprised of thiolated gelatin. In some embodiments the hydrogel is comprise of acrylate crosslinker such PEG diacrylate.

In other embodiments a suitable matrix may be used. For example suitable matrices may comprise any of the following: agarose, alginate, fibrin, collagen, Matrigel®, e.g. a gelatinous protein mixture secreted by Engelbreth-Holm-Swarm (EHS) mouse sarcoma cells.

### Additional Embodiments of the Invention

1. A method of differentiating a human clonal progenitor cell into an osteochondral cell comprising contacting the human clonal progenitor cell with one or more TGFβ superfamily members.
2. The method of 1, wherein the human clonal progenitor cell is cultured under micromass conditions.
3. The method of 1, wherein the human clonal progenitor cell is cultured in contact with a hydrogel.
4. The method of 3, wherein the human clonal progenitor cell is encapsulated in the hydrogel.
5. The method of 3, wherein the hydrogel comprises hylauronate and gelatin.
6. The method of 5, wherein the hyaluronate is thiolated.
7. The method of 5 wherein the gelatin is thiolated.
8. The method of 3, wherein the hydrogel comprises acrylate.
9. The method of 8, wherein the acrylate is PEG Diacrylate.
10. The method of 3, wherein the one or more TGFβ super family member is chosen from TGFβ-3, BMP2, BMP-4, BMP-6, BMP-7 and GDF-5.
11. The method of 3, wherein the one or more TGFβ super family member is TGFβ-3 and BMP-2.
12. The method of 3, wherein the one or more TGFβ super family member is TGFβ-3 and BMP-4.
13. The method of 3, wherein the one or more TGFβ super family member is TGFβ-3 and GDF5.
14. A cellular composition comprising a hydrogel and the in vitro differentiated progeny of a human clonal progenitor cell, wherein the progeny of the human clonal progenitor cell is an osteochondral cell.
15. The composition of 14, wherein the hydrogel comprises hyaluronate and gelatin.
16. The composition of 15, wherein the hyaluronate is thiolated.
17. The composition of 15, wherein the gelatin is thiolated.
18. The composition of 14 further comprising one or more members of the TGFβ super family.
19. The composition of 18, wherein the one or more members of the TGFβ super family is chosen from TGFβ-3, BMP-2, BMP-4, BMP-6, BMP-7 and GDF-5.
20. The cellular composition of 14 wherein the differentiated progeny of the clonal human progenitor cell expresses one or more genes chosen from *COL2A1, COL10A1, ACAN, CRTAC1, TNMD, ALPL, PENK, BGLAP, BMP-2, DLX5, GPC3, IHH, PRG4, CILP, EPYC,SPP1, TTR LPL, CEBPD, PPARG, FABP4, PLIN1, DLK1, PPARGC1A, CEBPD, PPARG, FABP4, PRDM16, FOXC2, CRLF1, FOXF2, FBLN5, DPT, ITGBL1, COL6A3, DUSP1, FOXF1, TGFB3, PAX9, GSN, FMOD, PDE8B, COMP, ITGA10, SAA1, DTNA, PCDH9, EBF1, SORBS1, SORBS2, ZNF503, MGST2, PNMT, DPT, OGN, FBLN5, FMOD, CRLF1, ITGBL1, TGFB3, GSN, HHIP, LRIG1, TRPS1, COMP, LECT1, COL9A1, HAPLN1, ITGA10, OLFML3, PKDCC, FGFR3, CSPG4, COL9A3, ITGB5, DNM1; OGN, COL9A2, EPYC, CHAD, PPIB, SRPX2, MATN3, LUM, COL13A1, FKBP11, MXRA8, COL27A1, PELI2, GPX7, ANGPTL2, GXYLT2, KLF4, STEAP3, SLC39A14, PTH1R, FAM46A, FAM180A, SLC26A2, RUNX1, CTGF, PLEKHB1, FKBP7, TNC, JAK2, CYTL1, KDELR3, ATP8B2, TRPS1; ELN, PPIB, PHEX, SOX9, COL27A1, PELI2, ANGPTL2, GXYLT2, SLC39A14, P4HA3, SLC26A2, CTGF, MRC2, COL9A3, PLEKHB1, TNC, FRMD8, CYTL1, ATP8B2; SCRG1, MATN3, SMOC1, PELI2,*
   *PTH1R, HTRA1, RUNX1, CTGF, PLEKHB1, RG9MTD1, CYTL1,* and *KLF2, LPL, CEBPB, PLIN1, PPARG, PPARGC1A" PPARG, GSN, WIF1, TGFB3, , COR02B, ADAMTS15,* and *IGFBP7COMP, SPP1, KAZALD1, LECT1, MMP13, HAPLN1, PHEX, PTH1R, COL11A1,* and *IP6K2 PKDCC, LTBP3, HAPLN1, OLFML3, ITGB5, IP6K2, ELN, CYTL1, LTBP3, PELI2, EPYC, PHEX, MRC2, CALY, GXYLT2, COL27A1, ANGPTL2, SOX9, GAA, TNC, LEPRE1, LTBP2, ARFGAP1, SRPX2, CYTL1, FAM46A, LUM, LTBP3, MXRA8, RUNX1, CALY, PTH1R, FKBP11, STEAP3, CFH, SLC40A1, GXYLT2, COL27A1, GPX7, ANGPTL2, MATN3, FKBP7, GAA, TNC, LEPRE1,* and *LTBP2, PPARG, PLIN1, LPL, CSPG4, LTBP3, CA12, PPIB, IRX5, ARHGAP24, FBLN5, DPT, CRLF1, ITGBL1, TGFB3, COL6A3, VCAN, DUSP1, TRPS1, GSN, ADAMTS6, ERG; EPYC, ELN, COL8A1, COL9A3, CYTL1, PPIB, CTHRC1, PLEKHB1, SLC26A2, KANK1, SLC39A14, ATP8B2, TNC, LTBP2, GPC1, WWP2, P4HA3, BAMBI, FGFRL1, SDC2; SCRG1, MEF2C, MATN3, PTH1R, ENPP2, CYTL1, CTHRC1, PLEKHB1, RUNX3, RUNX1, PRICKLE1, WWP2, HTRA1, CTGF, FGFRL1, ERG, FAT3; COL9A2, PCOLCE2, MEF2C, MATN3, LUM, HHIP, PTH1R, SLC40A1, KLF4, SRPX2, CYTL1, FKBP11, CTHRC1, STEAP3, LOXL4, DUSP1, LTBP2, TRPS1, GPC1, CFH, BAMBI, CDH2, COL27A1, FAM46A, CTGF, SDC2, and PLCD1 , FRZB, HAND2, DLXS, DLX6, FOXD1, MSX2, TFAP2A, ALPL, BMP2, MSX2, DCN, SPP1, SATB2, MSX2, POSTN PCDH20, SERPINA3, TAC1, EDNRA, SCRG1, CRABP2, SPOCK3, VCAN, BOC, ECM2, CRLF1, GAS1, TSPAN8, MFAP4, NFIA, RASSF9, DCN, SATB2, NKX3-2, EBF1, SORBS2, PCDH17, SOBP, ST8SIA4; MEF2C, ALPL, PTH1R, STEAP3, PELI2, SLC40A1, MXRA8, MATN3, PARD6G, GPX7, FAM180A, CTHRC1, ATP8B2, BAMBI, PLCD1, LTBR, SLC26A2, ANGPTL2, SATB2, FAM46A, DUSP1, CSPG4, , OLFML3, PKDCC, LTBP3, FUS, FBLN5, GAS1, DUSP1, GSN, ARHGAP24, VCAN, EMILIN3; PDE8B, SORBS2, PHACTR2, EBF1, PCDH9, MATN3, LTBP2, DUSP1, CDH2, SLC40A1, MXRA8, STEAP3, ANGPTL2, GPX7, TSPAN3, SDC2, ATP8B2, CD36, CTGF, ARHGAP24, FBLN2, SPP1, CSPG4, CSPG4, ENPP1, CA12, DNM1; GXYLT2, ANGPTL2, METRNL, KDELR3, PARD6G,* and *LEPRE1, ACTC1, CRYAB, EFHD1, MFAPS, DLK1, ACTA2, OCA2, AIFIL, GPC4, SCRG1, CNN1, HES6, KRT17, RASL11B, IGFBP3, EDN1, ENC1, SFRP2, ACTG2, CKB, CSRP1, CSRP2, C5orf46, COL3A1, HEY1, TUBB2B, CALD1, KAL1, CD24, CDH2, MAMDC2, EFR3B, CDKN2B, HES4, TAGLN, CAP2, PMEPA1, CTGF, TPM1, TGFB2, CXXC5, COL4A1, PALLD, SOX11, OCIAD2, EML1, CCDC99, TTR, CCDC3, TGFB3, ZIC2, GPC4, POSTN, ID3, PODXL, FBLN5, KRT7, TINAGL1, LGMN, GPX7, ACTG2, ANGPTL4, TUBB2B, SLC4A2, SULF1, SLC16A9, CDH2, MAMDC2, , ENPP2, NPR3, CDKN2B, TAGLN, INO80C, HTRA1, DHRS3, CTGF, IGFBP7, , PMEPA1, MRPS6, APOE, SMPD1, TPM1, STXBP2, TMEM108, IQCG, COL4A1, SPINT2, MXD3, TPD52L1, HEYL, CDH6, CYR61, EFHD1, CCDC3: , CFH , ZIC2, TIMP4 , SYNM , AIF1L, EBF1, H19, DYSF , EDNRA, NDUFA4L2, ACTG2, COL3A1, CSPG4 , PRRX1, TMEFF2, TAGLN, ZBTB46 , HTRA1, IGFBP7 , APOE , OLAH , IGDCC4, FBLN1, GGTS5, MAN1C1, RFTN2 , STC2, IGFBP1, 7MEM119, CDH6, DKK2, DKK3, FAM198B,* ACTG2, *FILIP1L, MYH11; CSRP2 PRG4, AMELX, ENAM, SILV1, PITX1, FABP4, AMBN, CAW1, MYH11, ORM1* and *LIPASIN.*
21. A kit for differentiating human clonal progenitor cells into an osteochondral cell comprising a hydrogel and one or more members of the TGFβ super family.
22. The kit of 21, wherein one or more members of the TGFβ super family is chosen from TGFβ-3, BMP-2, BMP-4, BMP-6, BMP-7 and GDF-5.
23. The kit of 21, wherein the hydrogel is comprised of hyaluronate and gelatin.
24. The kit of 23 wherein the hydrogel further comprises an acrylate.
25. The kit of 24, wherein the acrylate is PEG diacrylate.
26. The kit of 23 wherein hyaluronate is thiolated.
27. The kit of 23, wherein the gelatin is thiolated.
28. A cryo-preserved human clonal progenitor cell and a hydrogel.
29. A cryo-preserved in vitro differentiated progeny of a human clonal progenitor cell and a hydrogel.
30. The kit of 22 wherein the one or more members of the TGFβ superfamily comprise TGFβ-3 and one other member of the TGFβ superfamily.
31. The kit of 30 wherein the one other member of the TGFβ superfamily is chosen from BMP-2, BMP-4 and GDF-5.

### Biological Deposits

Some cell lines described in this application have been deposited with the American Type Culture Collection ("ATCC"; P.O. Box 1549, Manassas, VA 20108, USA) under the Budapest Treaty. The cell line E68 (also known as ACTC207) at passage 15 was deposited at the ATCC on 1/3/07 and has ATCC Accession No. PTA-8119. The cell line T42 (also known as ACTC210) was deposited at the ATCC and has ATCC Accession No. PTA-120383.

### EXAMPLE 1: Analysis of chondrogenic hEP cell lines under differentiating culture conditions

### Cell lines and Growth Factors

The derivation of the hEP cell lines 4D20.8 (cat. #SCR220, Millipore Corporation, Temecula, CA, USA; cat. #ES-84, BioTime, Alameda, CA, USA), 7PEND24 (cat. #SCC122, Millipore Corporation; cat. #ES-283, BioTime), 7SMOO32 (cat. #ES-278, BioTime), E15 (cat. #ES-98, BioTime), MEL2 (cat. #ES-268, BioTime), SK11 (cat. #ES-250, BioTime), and SM30 (cat. #ES-256, BioTime) used in this study was previously described¹⁹. The hEP cell lines were routinely cultured in corresponding ESpan medium as recommended by manufacturer (BioTime, Alameda, CA, USA). Mesenchymal stem cells were (Lonza,Basel, Switzerland) and were propagated in growth medium (cat. #C-28010; PromoCell, Heidelberg, Germany) with pen:strep (100 U/m1:100 ug/ml). The cells lines were maintained in and all subsequent experiments were carried out at 37°C in an atmosphere of 10% CO₂ and 5% O₂ on gelatinized culture vessels, the relatively high concentration of CO₂ providing physiological pH in relatively low concentrations of O₂. The hEP cell lines were serially passaged as previously described while confluence was carefully prevented for more than 2 days to prevent differentiation. Cells were synchronized in quiescence by growing to confluence, then were switched to medium containing only 10% of the normal serum concentration and held for five days to induce quiescence. TGFβ3 was obtained from R&D Systems (Minneapolis, MN, USA) Sterile lyophilized TGFb3 (Lonza, PT-4124 or R&D systems (Minneapolis, MN, USA), Cat. No. 243-B3-010).. BMP2, BMP4, and BMP7 were obtained from Humanzyme (Chicago, IL, USA), BMP6 and GDF5 were obtained from PeproTech (Rocky Hill, NJ, USA).

### Differentiation in HyStem-C

HyStem-C (BioTime, Alameda, CA, USA) was reconstituted following the manufacturer's instructions. Briefly, the HyStem component (thiol modified hyaluronan, 10 mg) was dissolved in 1.0 ml degassed deionized water for about 20 minutes to prepare a 1% w/v solution. The Gelin-S component (thiol modified gelatin, 10 mg) was dissolved in 1 ml degassed deionized water to prepare a 1% w/v solution, and PEGDA (PEG diacrylate, 10 mg) was dissolved in 0.5 ml degassed deionized water to prepare a 2% w/v solution. Then, HyStem (1ml, 1% w/v) was mixed with Gelin-S (1 ml, 1% w/v) immediately before use. Pelleted cells were resuspended in recently prepared HyStem:Gelin-S (1:1 v/v) mix described above. Upon the addition of crosslinker PEGDA, the cell suspension, at a final concentration of 2.0 x 10⁷ cells/ml, was aliquoted at 25ul/aliquot four to five times into each well of 6 well plates (Corning 3516) after partial gelation. Following complete gelation (20 minutes), chondrogenic medium was added to each well (i.e about 4 ml/each well of a 6 well plate).

### Chondrogenic medium:

DMEM (CellGro Cat. No. 15-013-CV, or PromoCell, Heidelberg Germany C-71219), high glucose, Pyruvate, 1mM (Gibco Cat. 11360), Pen:Strep 100U/m1:100ug/ml (Gibco Cat. No. 504284), Glutamax 2mM (Gibco Cat. No. 35050), Dexamethasone O.1uM (Sigma, St. Louis, MO, Cat. No.D1756-100), L-Proline 0.35mM (Sigma Cat. No. D49752), 2-phospho-L-Ascorbic Acid 0.17mM (Sigma, Cat. No. 49792, Fluka), ITS Premix (BD, Franklin Lakes, NJ, sterile Cat. No. 47743-628) final concentration 6.25ug/ml insulin, 6.25ug/ml transferrin, 6.25ng/ml selenious acid, serum albumin 1.25mg/ml, 5.35 ug/ml linoleic acid and TGFb3 10ng/ml (R&D systems, Minneapolis MN, Cat. No. 243-B3-010).

Plates were then placed in a humidified incubator at 37°C, ambient O₂, 10% CO₂, and the cells were fed three times weekly. At the desired time point hydrogel constructs were either fixed and processed immunohistochemical analysis or lysed using RLT (Qiagen, Valencia CA) with 1% beta mercaptoethanol for total RNA to analyze transcript expression using qPCR and/or whole genome microarray.

### Pellet-induced chondrogenesis

Chondrogenic induction by pellet culture was performed according to the manufacturer's instructions (Chondrogenesis Differentiation Kit ES-K42; BioTime, Alameda, CA, USA). Briefly, cells were cultured in the undifferentiated state, trypsinized (0.25% w/v trypsin/EDTA (Invitrogen, Carlsbad, CA, USA), diluted 1:3 with PBS (Ca, Mg free), centrifuged at 150 x g for 5 min at room temperature. Then, the supernatant was aspirated, and the pellet resuspended in Factor-Free Chondrogenic Medium with supplements, less growth factors. Cells were centrifuged at 150 x g at room temperature, the supernatant was aspirated, and the pellet was resuspended with 1.0 ml Factor-Free Chondrogenic Medium per 7.5 x 10⁵ cells. The suspension was centrifuged again at 150 x g for 5 minutes and the cell pellets were resuspended in Factor-Containing Chondrogenic Medium at a concentration of 1.0 x 10⁶ cells/ml. Factor-Containing Chondrogenic Medium was made of BioTime Factor-Free Medium plus TGFβ3 or other TGFβ family members as described herein. Factor-Containing Chondrogenic medium was prepared just before use by the addition of TGFβ3 (final concentration, 10 ng/ml). An aliquot of 0.5 ml (5.0 x 10⁵ cells) of the cell suspension was placed into sterile 15ml polypropylene culture tubes. Cells were spun at 150 x g for 5 minutes at room temperature. Cell pellets were maintained in a humidified atmosphere of 10% CO₂ and medium was changed every 2-3 days with 0.5 ml of freshly prepared Factor-Containing Chondrogenic medium. Pellets were harvested at varying time points and prepared for histology by fixation with Neutral Buffered Formalin.

### Micromass-induced chondrogenesis

Chondrogenic induction by micromass culture was performed according to the manufacturer's instructions (Chondrogenesis Differentiation Kit - ES-K42; BioTime, Alameda, CA, USA). Briefly, cells were cultured in the undifferentiated state, trypsinized (0.25% w/v trypsin/EDTA (Invitrogen, Carlsbad, CA, USA), diluted 1:3 with PBS (Ca, Mg free), and resuspended at a cell density of 2.0 x 10⁷ cells/ml in their respective growth medium. Twenty-five or more micromass aliquots (200,000 cells/10 ul aliquot) were seeded onto Coming Tissue Culture-treated polystyrene plates or dishes. The seeded micromasses were placed in a humidified incubator at 37°C with 5% O₂ and 10% CO₂ for 90 minutes to 2 hours for attachment. The growth medium for each respective cell line was added, aspirated the following morning, and the cells were rinsed with PBS (Ca, Mg free). Then, the media were replaced with Factor-Containing Chondrogenic medium (prepared as described above for the pellet micromasses). Cells were maintained in a humidified incubator at 37°C with 5% O₂, 10% CO₂ in Factor-Containing Chondrogenic medium, which was replaced with freshly prepared medium every 2-3 days. At the designated periods of time, RNA was extracted using Qiagen RNeasy kits (Qiagen, Valencia, CA, USA cat. #74104) according to the manufacturer's instructions. The RNA yield was maximized using Qiagen's QiaShredder (Qiagen, Valencia, CA, USA cat. #79654) to homogenize samples following the lysis of the micromasses with RLT buffer prior to RNA extraction.

### In vivo studies

The methods for the preparation of cellular implants, osetochondral deficit rat model and histology were described previously²⁰

### In vitro cartilage tissue engineering

Each cell line was analyzed for its in vitro potential to differentiate and form mechanically functional cartilage tissue. Tissue engineered constructs were formed by encapsulating each hEP cell line and MSCs within HyStem-C (BioTime, Inc. Alameda, CA), a well-characterized hydrogel consisting of thiolated hyaluronan and porcine gelatin with a polyethylene glycol diacrylate (PEGDA) crosslinker (BioTime, Alameda, CA, USA). Pelleted cells were resuspended (5.0 x 10⁷/mL) in the liquid hyaluronan and gelatin components of HyStem-C (BioTime, Inc. Alameda, CA) before combining with the PEGDA crosslinking agent. The cell-hydrogel slurry was distributed into a custom 2.3 mm thick agarose gel mold with 5 mm diameter wells. Cell-laden hydrogels were allowed one hour to completely polymerize before transferring them into ultra-low adherence 6-well plates. To maximize the delivery of chondrogenic medium including growth factors (10ng/mL TGF-B3; 100ng/mL GDF-5) throughout the constructs, medium (2 mL/construct) was replenished 3/week and cultures were maintained under dynamic conditions (orbital shaker) in atmospheric oxygen with 10% CO₂ for 42 days.

The equilibrium and dynamic moduli were assessed with a testing device consisting of a motor-driven impermeable platen and load cell used to apply a 2 gram tare load (300 seconds) while monitoring displacement within a PBS bath⁵⁴. Subsequently, a single 10% compressive strain was applied (0.05%/second) followed by 1000 seconds of relaxation. The equilibrium modulus was calculated from the stress and strain values at equilibrium using the construct dimensions before testing. Lastly, dynamic testing consisted of a sinusoidal 1% strain compression at 1 Hertz for 5 cycles. The dynamic modulus was calculated using the slope of the dynamic stress-strain curve as described⁵⁵. The data are presented as mean ± standard deviation for n=3-6 samples and a one-way ANOVA with Tukey's honestly significant difference *post hoc* analysis was performed (SYSTAT 13, Chicago, IL, USA).

### Gene expression analysis

Total RNA was extracted directly from cells growing in 6-well plates or 10cm tissue culture dishes using Qiagen RNeasy mini kits according to the manufacturer's s instructions. RNA concentrations were measured using a Beckman DU530 or Nanodrop spectrophotometer and RNA quality was determined by denaturing agarose gel electrophoresis or using an Agilent 2100 bioanalyzer. Whole-genome expression analysis was carried out using Illumina Human Ref-8v3 or Human HT-12 v4 BeadArrays, and RNA levels for certain genes were confirmed by qRT-PCR. For the Illumina BeadArrays, total RNA was linearly amplified and biotin-labeled using Illumina TotalPrep kits (Life Technologies, Temecula, CA, USA), and cRNA was quality-controlled using an Agilent 2100 Bioanalyzer. The cRNA was hybridized to Illumina BeadChips, processed, and read using a BeadStation array reader according to the manufacturer's instructions (Illumina, San Diego, CA, USA). Values of less than 100 relative fluorescence units (RFUs) were considered as nonspecific background signal.

### Quantitative real-time PCR (qRT-PCR) analysis

Samples for testing (template) were prepared in standard Optical 96-well reaction plates (Applied Biosystems Carlsbad, CA, PN 4306737) consisting of 30ng of RNA equivalent of cDNA, 0.8uM per gene-specific custom oligonucleotide primer set (Invitrogen), ultra-pure distilled water (Invitrogen Cat. # 10977015), diluted 1:1 with 12.5ul of Power SYBR Green PCR Master Mix (Applied Biosystems Carlsbad, CA, Cat. # 4367659) incorporating AmpliTaq Gold DNA polymerase in a total reaction volume of 25ul. Real-Time qPCR was run using Applied Biosystems 7500 Real-Time PCR System employing SDS2.0.5 software. Amplification conditions were set at 50°C for 2 min. (stage 1), 95°C for 10 min. (stage 2), 40 cycles of 95°C for 15 sec then 60°C for 1 min (stage 3), with a dissociation stage (stage 4) at 95°C for 15 sec, 60°C for 1 min, and 95°C for 15 sec. Ct values of amplicons were normalized to the average Ct value of 3 housekeeping genes (GAPD, RPS10, and GUSB), and normalized gene expression of samples calculated relative to that of early passage knee-Normal Human Articular Chondrocytes (Lonza). For the genes: *AJAP1, ALDH1A2, BARX1, BMP5, CD74, HAND2, HOXB2, LHX1, LHX8, PITX1, TBX15* and *ZIC2,* Ct values for the amplification products of genes of interest were normalized to the average Ct value of 3 housekeeping genes *(GAPDH, RPS10,* and *GUSB)* to calculate relative gene expression across samples.

### Primers Used:

*ACAN* (NM_013227.2) f. TGAGTCCTCAAGCCTCCTGT (SEQ ID NO: 1), r. CCTCTGTCTCCTTGCAGGTC (SEQ ID NO: 2) (185bp); *ALDH1A2* (NM_170697.1)
f. AGTGTTTTCCAACGTCACTGATGATATGC, (SEQ ID NO: 3) r. AAAGGGGCTCTGGGCATTTAAGGC (SEQ ID NO: 4) (244 bp); *AJAP1* (NM_018836.3)
f. GTGCCCGTGTACACCGATGAGAC, (SEQ ID NO: 5) r. GGCCAGTCAGCAGGAGATTTCAAAC (SEQ ID NO: 6) (150 bp); *BARX1* (NM_021570.3) f. AGAAGTACCTTTCCACGCCGGAC, (SEQ ID NO: 7) r. CTTGGTGGGAGACTCCAGGC(SEQ ID NO: 8) (146 bp); *BMP5* (NM_021073.2)
f. GCAATAAATCCAGCTCTCATCAGGAC, (SEQ ID NO: 9)r. CAGTCCTGCCATCCCAGATCCC (SEQ ID NO: 10) (133 bp); *CD74* (NM_001025159.1) f. TGCTGCCCAATCTCCATCTGTCAAC (SEQ ID NO: 11), r. GGGTCTGGGTGTAGGGTTATCC(SEQ ID NO: 12) (173 bp); *COL2A1* (NM_001844.4) f. TGGCCTGAGACAGCATGA (SEQ ID NO: 13), r. AGTGTTGGGAGCCAGATTG (SEQ ID NO: 14) (373 bp); *COL10A1* (NM_000493.3) f. GGGCCTCAATGGACCCACCG (SEQ ID NO: 15), r. CTGGGCCTTTGGCCTGCCTT (SEQ ID NO: 16) (150bp); *CRTAC1* (NM_018058.4) f. ATCCGTAGAGAGCACGGAGA(SEQ ID NO: 17), r. GGACTCTCCATGGGACAAGA (SEQ ID NO: 18) (144bp); *GAPDH* (NM_002046.3) f. GGCCTCCAAGGAGTAAGACC (SEQ ID NO: 19), r. AGGGGTCTACATGGCAACTG (SEQ ID NO: 20) (147bp); *GUSB* (NM_000181.2) f. AAACGATTGCAGGGTTTCAC (SEQ ID NO: 21), r. CTCTCGTCGGTGACTGTTCA (SEQ ID NO: 22) (171bp); *HAND2* (NM_021973.2) f. GGACGCCGAGCGCTGAGGC (SEQ ID NO: 23), r. GAAAACCACCTACCAGACTCATTTCGC (SEQ ID NO: 24) (111 bp); *HOXB2* (NM_002145.2)
f. CTCCTGTCTCCAGCTATCCG (SEQ ID NO: 25), r. GCACAGAGCGTACTGGTGAA (SEQ ID NO: 26) (111 bp);
*LHX1* (NM_005568.3) f. AAACTCTACTGCAAGAACGACTTCTTCC (SEQ ID NO: 27), r. TGCAGGTGAAGCAGTTCAGGTGAAAC (SEQ ID NO: 28) (133 bp); *LHX8* (NM_001001933.1) f. CTCGGACCAGCTTTACAGCAGATC (SEQ ID NO: 29), r. ACGTGTTTCTTGTGGCGTGCTCTAC (SEQ ID NO: 30) (169 bp); *RPS10* (NM_001014.3) f. ATTTGGTCGTGGACGTGGT (SEQ ID NO: 31), r. TTTGGCTGTAAGTTTATTCAATGC(SEQ ID NO: 32) (77bp); *PITX1* (NM_002653.3)
f. ACGACCCAGCCAAGAAGAAGAAGC (SEQ ID NO: 33), r. TGCTGGTTACGCTCGCGCTTAC (SEQ ID NO: 34) (214 bp); *TBX15* (NM_152380.2) f. AGATTCAGGTGGAGCTGCAATGTGC (SEQ ID NO: 35), r. CCACTTGGAGCTATGATACACATATC (SEQ ID NO: 36) (212 bp); and *ZIC2* (NM_007129.3) f. GACCCACACAGGGGAGAAGCC (SEQ ID NO: 37), r. GTGTAGGACTTGTCGCACATCTTGC (SEQ ID NO: 38) (144 bp).

### mRNA expression in undifferentiated MSCs and seven novel clonal chondrogenic hEP cell lines

A previously-reported screen of 100 diverse hES-derived clonal hEP cell lines for collagen type II, alpha I (COL2A1) mRNA expression identified seven responsive lines: 4D20.8, 7PEND24, 7SMOO32, E15, MEL2, SK11, and SM30²⁰. To compare gene expression in the undifferentiated state, all seven lines were expanded *in vitro* then rendered quiescent at confluence in low serum culture to synchronize cell cycle status. All lines displayed an overall mesenchymal morphology with subtle cell line specific features (Figure 1 (A-H)). HumanRef-8 v3 and human HT-12 v4 Microarray analysis of mRNA expression revealed differences between the lines and bone marrow-derived MSCs including CD antigens commonly used as markers of MSCs such as *CD29 (ITGB1), CD45 (PTPRC), CD73 (NTSE), CD90 (THY1),* and *CD105 (ENG)²¹,* but these same markers were also expressed in a majority of diverse primary cultures of epithelial and mesenchymal somatic cell types and all but one of the seven chondrogenic lines described in this report (Figure 2). In contrast, the expression of the MSC marker *CD74*²¹ was confirmed in MSCs but not most other somatic cell types, and was not expressed in the seven hEP cell lines with chondrogenic potential (Figure 2).

Further comparisons of MSCs and hEP cell lines showed differential expression of several site-specific developmentally-regulated genes (Figure 3). We will discuss these along with information provided by the LifeMap Discovery™ (LMD) a database that describes cellular differentiation during embryonic development and includes embryonic progenitor gene expression profiles to assist in the elucidation of gene expression profiles. In addition to *CD74,* MSCs, but not the seven chondrogenic hEP cell lines, expressed distal *HOX* genes such as *HOXA11, HOXB7, HOXC10,* and *HOXD4* as expected from cells derived from the iliac crest. The lines 7SMOO32, MEL2, SK11, and SM30 lacked *HOX* gene expression, while the most distal *HOX* expression of 4D20.8 was *HOXB2,* E15 was *HOXA2, HOXB2,* and the line 7PEND24 expressed only *HOXA6* and *HOXB6.*

A subset of the differentially-expressed genes shown in Figure 3 were confirmed by qPCR (Figure 4). The line 4D20.8, and to a lesser degree, 7PEND24, expressed *LHX8,* a gene expressed in orofacial mesenchyme²²⁻²⁴ and as shown in LMD, expressed selectively in neural crest populating branchial arch 1 including the maxillary process. 4D20.8 and 7PEND24 express *BARX1,* a gene expressed in proximal oral ectomesenchyme that is regulated during molar vs incisor development^{25,26} and as shown in LMD expressed selectively in neural crest populating both mandibular and hyoid arches. In addition, 4D20.8, MEL2, and SM30 cells specifically expressed FGF18, a gene implicated in regulating cranial base and mandibular development²⁷ and as shown in LMD expressed in neural crest-derived preosteoblasts. Only 7SMOO32 expressed *LHX1,* a gene critical in head development 28. The line 7SMOO32 also expressed metabotropic glutamate receptor (GRM1), nicotinic cholinergic receptor CHRNA3, the transcription factor *MSX2,* shown in LMD to be expressed in differentiating intramembranous and endochondral osteoblasts and the genes BBOX1, Dex1, and the secreted proteins BMP5, EGFL6 (normally expressed in brain and lung tumors and fetal tissues, but not adult tissues²⁹). *AJAP1* associated with epithelial cell junctions was expressed in the line E15 and weakly in MSCs, but not detectable in the other hEP lines. The line MEL2 specifically expressed the retinoid metabolizing enzyme *ALDHIA2* and as shown in LMD expressed selectively in the maxillary and mandibular processes and HAND2, a gene expressed in branchial arch mesenchyme³⁰ and as shown in LMD expressed in cranial and cardiac neural crest cells. The lines SK1 1 and SM30, and to a lessor extent E15, shared expression of *ZIC2,* a gene associated with neural and axial skeleton development and as shown in LMD expressed selectively in somitic dermomyotome and sclerotome cells, developing maxillary and mandibular mesenchyme, and limb bud mesenchyme. The *paired-related* homeobox gene *PITX1* is expressed in hind limb mesenchyme³¹ and derivatives of the first branchial arch³² and was expressed in MSCs, SK11, and SM30 while *PITX2* was expressed in MSCs and SK11, but not SM30. *TBX15,* normally expressed in limb bud mesenchyme³³ lateral nasal, mandibular, and caudal maxillary mesenchyme^{34,35} was expressed in MSCs and the lines E15, SK11, and 7PEND24.

### Comparative gene expression profiles in micromass conditions

To examine the comparative gene expression in the lines when differentiated in micromass conditions³⁶, 10 µl aliquots at 2.0 x 10⁷ cells/mL growth medium were seeded for attachment, then incubated in a chondrogenic cocktail containing TGFβ3, dexamethasone, and ITS for 14 days. MSCs differentiating in micromasses typically condense into spheres mimicking embryological condensing mesenchyme³⁷. All lines, except 7PEND24, were observed to undergo condensation comparable to MSCs (Figure 1 (I-P) and expressed one or more chondrocyte-related genes including *COL2A1, COL10A1,* aggrecan *(ACAN),* and cartilage acidic protein 1 *(CRTAC1*)³⁸ (Figure 5). The lines E15 and SM30 showed robust induction of *COL2A1* expression that was comparable to that seen with MSCs.

### Comparative gene expression when differentiated in HyStem-C hydrogels in the presence of combinations of TGFβ family members

We next compared MSCs and the seven chondrogenic hEP cell lines in both undifferentiated conditions (five days of quiescence (CTRL)) and in cells differentiated for 14 days in HyStem-C (BioTime, Inc. Alameda, CA), a biocompatible hydrogel composed of thiolated hyaluronan and thiolated porcine gelatin crosslinked with a polyethylene glycol diacrylate (PEGDA) crosslinker. Cells were incorporated into the HyStem-C (BioTime, Inc. Alameda, CA) hydrogel (BioTime, Inc. Alameda, CA) and then maintained in chondrogenic medium containing combinations of the TGFβ family members TGFβ3, BMP2, BMP4, BMP6, BMP7, and GDF5. As shown in and Figure 6, combinations of TGFβ-related growth factors markedly altered chondrocyte-related gene expression in all cell lines. In the case of MSCs, TGFβ3 in combination with other TGFβ family members increased *COL2A1* expression. The lines 4D20.8, E15, and SK11 expressed relatively more *COL2A1, ACAN,* and *CRTAC1* transcript than MSCs when cultured in TGFβ3 in combination with BMP2, 4, 6, 7 and GDF5. As previously reported, 4D20.8 showed the lowest levels of *COL10A1* expression in the presence of TGFβ3 and GDF5, consistent with the formation of non-hypertrophic definitive chondrocytes²⁰. The lines 7PEND24 and 7SMOO32 similarly showed an up-regulation of *COL2A1, ACAN,* and *CRTAC1* but markedly lower relative expression of *COL10A1* while the line MEL2 expressed low but detectable levels of *COL2A1, COL10A1, ACAN,* and *CRTAC1. The* lines SM30 and 7SMOO32 also robustly expressed *COL2A1, COL10A1, ACAN,* but failed to induce *CRTAC1* expression. These alterations in *COL2A1, COL10A2, ACAN,* and *CRTAC1* gene expression were confirmed by qPCR (Supplementary Figures 21-22); the line SM30 showed the highest induction of COL2A1 expression compared to cultured NHACs (35-50,000 fold induction).

To better quantify the hypertrophic phenotype, we plotted the mean of the ratios of *COL2A1*/*COL10A1* expressed by qPCR in all the lines and conditions. As shown in Figure 7, relative *COL10A1* expression was pronounced in MSCs, MEL2, and SK11 while expression in the other lines showed patterns where the ratios could shift depending on the growth factor stimulus. As previously reported, the condition most favoring a high *COL2A1*/*COL10A1* ratio for the line 4D20.0 was the combination of TGFβ3 and GDF5 while the lowest ratio (most hypertrophic) for this line was observed with the combination of TGFβ3 and BMP2.

We next analyzed the expression of site-specific or cell type-specific markers under these same differentiation conditions. Apolipoprotein D *(APOD),* a gene specifically expressed in embryonic cephalic condensing mesenchyme including that of the otic capsule and ossicles, Meckel's cartilage, basioccipital bone, and primitive vertebral bodies³⁹, and reportedly expressed at higher levels in middle zone as apposed to surface joint cartilage⁴⁰ was expressed at the highest levels in 7SMOO32 and was essentially undetectable in the line 7PEND24 (Figure 8 and Supplementary Table III). Remarkably, 7PEND24 a line that showed little if any condensation in micromass conditions, markedly up-regulated the tendon marker tenomodulin *(TNMD)* when differentiated in HyStem-C (BioTime, Inc. Alameda, CA) with BMP2, 4, or 7 alone without TGFβ3. Lower levels of *TNMD* were also observed in So11 in the presence of BMP4, 6, and GDF5 alone. Both 4D20.8 and 7PEND24 expressed *BARX1,* a protein implicated in molar development, and *FGF18,* normally expressed in Meckel's cartilage⁴¹.

The expression of tissue-nonspecific alkaline phosphatase *(ALPL),* commonly associated with bone mineralization, was expressed at high levels in MSCs and MEL2, but was expressed at lower or undetectable levels in the other hEP cell lines. *PENK,* a gene recently associated with bone mineralization⁴² was expressed at the highest levels in 4D20.8 and MEL2. Additional markers of osteogenesis expressed by MEL2 included: bone gamma-carboxyglutamate (gla) protein (osteocalcin) *(BGLAP),* bone morphogenic protein 2 *(BMP2),* distal-less homeobox 5 *(DLX5),* and glipican 3 *(GPC3),* the latter gene associated with calvarial development⁴³. However, the marker of endochondral ossification marker Indian hedgehog *(IHH),* was expressed by MSCs, 4D20.8, SK11, and SM30, especially in the presence of TGFβ3 plus TGFβ family members, but not expressed in 7PEND24, 7SMOO32, or MEL2, suggesting MEL2 undergoes intramembranous ossification. Lubricin *(PRG4)* and cartilage intermediate-layer protein *(CILP),* genes expressed at higher levels in surface articular chondrocytes⁴⁰, was abundantly expressed in 7PEND24 and 7SMOO32, but almost undetectable in the line E15. The neural crest marker Phenylethanolamine-N-methyltransferase *(PNMT)* was expressed at the highest level in 4D20.8, 7PEND24, and 7SMOO32, but not expressed in MSCs or the other hEP cell lines. Low-affinity nerve growth factor receptor (NGFR) has been reported to be expressed in neural crest derivatives⁴⁴, however, we did not detect NGFR in MSCs or the hEP cell lines in either the undifferentiated or differentiated conditions by microarray analysis.

### Analysis of histology, proteoglycan, and collagen II content during in vitro differentiation of MSCs and chondrogenic hEP cell lines

MSCs and the seven chondrogenic hEP cell lines were collected by centrifugation (∼500,000 cells) and maintained in chondrogenic conditions for 14 and 21 days. Samples were then assessed with H&E, Safranin O staining, and with anti-collagen II antibody as a measure of general histology, the presence of proteoglycans, and collagen II respectively. Most samples showed evidence of cartilage-like extracellular matrix accumulation in H&E sections, however there was variable levels of Safranin-O and collagen II staining between the lines and different conditions (Figure 9, Figure 22). At day 21, the combination of TGFβ3 and GDF5 resulted in robust cartilage staining in 4D20.8 (as previously reported) and also in 7PEND24, 7SMOO32, E15, MEL2, SK11, and SM30 lines.

### In vitro tissue engineering

The potential for these cell lines to be used for the in vitro engineering of functional cartilage tissue was assessed by encapsulating cells within HyStem-C (BioTime, Inc. Alameda, CA) (BioTime, Inc Alameda, CA). Each cell type was encapsulated at 5.0 x 10⁷ cells/mL in HyStem-C (BioTime, Inc. Alameda, CA) in disc-shaped constructs (5mm diameter), which were cultured for 42 days in chondrogenic medium containing both TGF-β3 and GDF-5. The equilibrium modulus of 7SMOOS32, 7PEND24, SK11, and MSC cell lines did not attain a level that was measurable by our methods. The MEL2, SM30, and E15 lines reached equilibrium modulus values of 0.05, 0.28, and 8.35 kPa, respectively. The 4D20.8 cell line reached an equilibrium modulus of 65 kPa, similar to what has previously been observed²⁰. The dynamic modulus values ranged from 35-95 kPa for all cell lines with the exception of 4D20.8 which reached 368 kPa.

### Repair of trochlear osteochondral defects in rNU/rNU 6-8 week old rats using MSCs and the hEP cell lines 7PEND24 and E15

We previously described the repair of cartilage tissue *in vivo* using the hEP line 4D20.8²⁰. Here we analyzed the performance of two additional lines, 7PEND24 and E15 since these had relatively high dymamic modulus readings. We prepared trochlear groove defects in the knees of immune deficient (rNU/rNU) rats then treated these with differentiated MSCs, 7PEND24, and E15 cell pellet and hydrogel cultures as described previously²⁰. The *in vitro* prepared cell pellets and hydrogel encapsulated cell samples were press-fit into the lesion using a small amount of fibrin glue on the defect for adhesion. After four weeks following implantion, regeneration of the defect was observed on a macroscopic level using the hEP cell lines 7PEND24 and E15 compared to hydrogel controls (Figure 10). While MSCs typically showed profound Safranin-O and collagen-II staining throughout the graft area, consistent with a uniformly hypertrophic response, the 7PEND24 graft showed what appeared to be a potential for regeneration of the original architecture of the joint surface in that structure approximating developing bone in the subchondral space was clearly visible by four weeks and a rim of Safranin-O and collagen II positive cells similar to the original joint surface was apparent (cells determined by anti-human mitochondrial staining (data not shown)). MSCs and the line E15 yielded more disordered differentiation.

### DISCUSSION

We previously reported the generation of a library of over 140 diverse clonal progenitor cell lines produced from hES cells. From 100 lines chosen from this library, seven lines showed detectable expression of *COL2A1* when cultured in the presence of TGFβ3 in micromass conditions. One of these lines, 4D20.8 was previously reported to be a novel LHX8+,BARX1+ line that showed increased expression of chondrogenic and/or osteogenic markers when cultured in the presence of combinations of TGFβ family members²⁰. In this study we compared the gene expression profiles of all seven lines in the undifferentiated as well as differentiated states to shed light on the normal embryological mesenchyme to which these lines correspond, and to determine their differentiation potential.

We conclude that each of the seven novel clonal chondrogenic hEP cell lines represents different embryonic anlagen of osteochondral mesenchyme that are distinct from bone marrow-derived MSCs. We base this conclusion on three criteria. First, the expression level of the CD antigens commonly used as markers of bone marrow MSCs, such as *CD29⁺, CD45⁻, CD73⁺, CD90⁺* and *CD105⁺,* while useful for discriminating between various blood cell types and MSCs, are not useful in discriminating MSCs from diverse somatic cell types nor hEP cell lines. However, *CD74,* a marker reported to specify MSCs from other fibroblastic cell types, was expressed in all MSCs used in this study, but was not expressed in the seven chondrogenic hEP cell lines or in most other fibroblastic-like hEP cell lines. Second, markers associated with site-specific anatomical locations, such as the *HOX* homeobox genes, and genes such as *LHX1, LHX8, PITX1, BARX1, ZIC2, TBX15,* and *HAND2* were differentially expressed among undifferentiated chondrogenic hEP cell lines and MSCs and indeed, and among the hEP cell lines themselves. Lastly, when exposed to combinations of TGFβ family members, these lines were found to display unique differentiation markers.

We utilized LifeMap Discovery™ (LMD) a database that describes cellular differentiation during embryonic development and includes embryonic progenitor gene expression profiles to assist in the elucidation of the gene expression profiles. The lines 4D20.8 and 7PEND24 shared similar markers in that both expressed *LHX8* and *BARX1,* as well as *FGF18* reported in humans to be expressed only in cephalic mesenchyme, in particular, mesenchyme surrounding developing Meckel's cartilage⁴¹. However, unlike 4D20.8, 7PEND24 expressed FOXF1 and showed an ability to induce the tendon marker TNMD when cultured in the presence of TGFβ3 and BMP2, 4, and 7.

The line MEL2 showed robust expression of osteogenic markers such as and *IBSP* and *BGLAP,* but consistently low *COL2A1* and *IHH* levels. This fact in combination with the lack of *HOX* gene expression in the line and the markers *FABP3* and *DLX5,* suggest that MEL2 may correspond to cells capable of undergoing cranial intramembranous ossification.

The lines SK11 and SM30 expressed *ZIC2,* a gene reportedly expressed in sclerotome and dermatome as well as limb bud mesenchyme⁴⁵ and mandibular and maxillary mesenchyme (LMD). In addition, both lines expressed *PITX1* and SK11 but not SM30 expressed *TBX15,* both genes commonly associated with limb developmene^{33,46}_ENREF_40. However, the lack of *HOX* gene expression in SK11 and SM30, lead us to provisionally assign the lines to cranial rather than limb mesenchyme. SK11, but not SM30 also expressed *PITX2. PITX1* and *PITX2* are members of a multigene family with similar but distinguishable patterns of expression in vertebrate embryology³². *PITX1* is expressed in the lateral mesenchyme of the first branchial arch, while *PITX2* is expressed more broadly in other tissues. We therefore provisionally assign SK11 to lateral mandibular mesenchyme^{47,48}. The lateral mandibular mesenchyme will give rise to both bone and cartilage to that part of the jaw giving rise to molars as well as the ossicles of the middle ear.

The relatively rostral pattern of HOX gene expression in the lines with no line showing a more caudal pattern than *HOXB2,* and the abundant expression of other homeobox genes commonly associated with craniofacial mesenchyme such as *DLX, PITX, LHX, MSX,* and *BARX,* leads us to conclude that these diverse lines likely correspond to diverse progenitors of the connective tissues of the head and neck. Being transient embryonic structures, the branchial arch mesenchyme contributes to the development of the cranium, lower face, pharynx, inner, and outer ears. The arches are commonly believed to possess an inner core of mesodermal cells surrounded by migratory neural crest originating from the hindbrain rhombomeres^{49,50}. The observation that in avian transplant experiments, neural crest cell fates are determined even before migration⁵¹, combined with the diversity of homeobox gene expression observed in clonal hEP cell lines, suggests that hES cells may induce these site-specific genes during differentiation in vitro making it possible to generate corresponding cell lines using clonal propagation with correspondingly diverse phenotypes.

The pellets and hydrogels of MSCs, 7PEND24, and E15 that were transplanted into experimentally-induced defects of articular condyles of rats were differentiated prior to transplantation *in vitro* only in the presence of TGFβ3. Both MSCs and E15 showed a disordered accumulation of cells in the graft area, while the line 7PEND24 gave what appeared to be regenerating articular architecture similar to that previously reported with the line 4D20.8²⁰. This is consistent with the markers *PRG4* and *CILP* that are relatively highly expressed in 7PEND24 and superficial as opposed to middle zone joint cartilage⁴⁰. *APOD,* a marker expressed higher in middle zone joint cartilage than surface tissue, while expressed at relatively high levels in lines such as 7SMOO32, was essentially undetectable in 7PEND24. The marked up-regulation of *COL2A1* and *ACAN* observed in the presence of TGFβ3 and related family members indicates the advisability of differentiating with these combinations prior to transplantation and/or administration of these factors into the joint space at or after the administration of the cells *in vivo* to improve regeneration of the tissue.

One consideration in the analysis of these chondrogenic hEP cell lines is their potential to synthesize proteins that assemble to form a functional tissue matrix. For cartilage, this means being capable of bearing compressive loads. Constructs from each cell line were created under specific conditions that have previously led to the formation of cartilage-like tissue using the 4D20.8 cell line²⁰ In this study, the potential of the 4D20.8 cell line was again confirmed as it reached a mean equilibrium modulus value of 65 kPa, nearly two-fold greater than previously reported²⁰. This value of equilibrium compressive modulus is superior to other published reports using human MSCs^{52,53} and comparable to recent work that achieved 55 kPa with human MSCs seeded in a methacrylated hyaluronic acid hydrogel for 10 weeks. In addition to the hEP cell lines SM30, MEL2, and E15 also generated equilibrium mechanical properties; albeit at substantially lower levels than those seen with 4D20.8. This analysis demonstrated the functional diversity of these hEP lines through a simple approach of *in vitro* tissue engineering and mechanical testing.

To our knowledge, this is the first report of the comparative properties of purified hPS-derived site-specific mesenchymal progenitors. The scalability of these lines combined with the ability to define their identity and purity may simplify manufacturing therapeutic products from hES and induced pluripotent stem cells.

### EXAMPLE 2: Discovery of novel clonal human embryonic progenitor cell lines capable of cartilage and tendon differentiation when differentiated in the presence of TGFβ3 together with: BMP2, BMP4, BMP6, BMP7, or GDF5.

Additional clonal human embryonic progenitor cell lines previously disclosed (See US Patent Application Serial Nos. 12/504,630; 13/456,400) and incorporated by reference that did not show *COL2A1* expression when differentiated in micromass conditions in the presence of 10 ng/mL of TGFβ3 alone, were differentiated for either 14 days in micromass conditions with combinations of 10 ng/mL of TGFβ3 together with one of the following other members of the TFG beta family: BMP2 (50ng/mL), BMP4 (10ng/mL), BMP6 (30 ng/mL), and BMP7 (100 ng/mL), and GDF5 (100 ng/mL) or alternatively, the cells were differentiated for 14 or 21 days in the presence of HyStem-C (BioTime, Inc. Alameda, CA) hydrogel as described herein supplemented with combinations of 10 ng/mL of TGFβ3 together with one of the following other members of the TGF beta family: BMP2 (50ng/mL), BMP4 (10ng/mL), BMP6 (30 ng/mL), and BMP7 (100 ng/mL), and GDF5 (100 ng/mL).

The cell line EN47 at passage 13 when cultured in the undifferentiated state, displayed the gene expression markers at 13-21 doublings of clonal expansion: *FM03* (accession number NM_001002294.1), *FOXF1* (accession number NM_001451.2), *GABRB1* (accession number NM_000812.2), *NEFM* (accession number NM_005382.1), *POSTN* (accession number NM_006475.1), *RGS1* (accession number NM_002922.3), *SOD3* (accession number NM_003102.2), *TFPI2* (accession number NM_006528.2), and *ZIC2* (accession number NM_007129.2), and distal *HOX* genes expressed being *HOXA2* and *HOXB2,* and negative for the gene expression markers: *ALDH1A1, BARX1, CD24, CD69, CD74, FOXF2, FOXS1, GSC, LHX8, PAX9, PENK,* and *TBX15,* and unlike the cell line EN7, the line EN47 expressed the gene *PI16* (accession number NM_153370.2). As shown in FIGURE 12, the cell line EN47 expressed relatively high levels of *COL2A1* when cultured in micromass conditions in 10 ng/mL of TGFβ3 together with BMP2 (50ng/mL) as determined by qPCR and Illumina microarray analysis compared to the previously disclosed chondrogenic hEP cell line 4D20.8 (WO11/009106) and in addition, the cell line EN47 expressed the tendon marker *TNMD.* In the presence of 10 ng/mL of BMP4 alone and in micromass differentiation conditions, *TNMD* was expressed minimal *COL2A1* expression. Surprisingly, while there was no evidence of *COL2A1* expression in EN47 in the presence of only TGFβ3 in micromass conditions as described (Sternberg et al, A human embryonic stem cell-derived clonal progenitor cell line with chondrogenic potential and makers of craniofacial mesenchyme. Regen Med. 2012 Apr 23. [Epub ahead of print], 2012), and indeed, only seven of 100 clonal progenitor cell lines responded to TGFβ3 in micromass conditions with chondrogenic differentiation (WO11/009106), nevertheless, in the presence of 50 ng/mL of BMP2 together with 10 ng/mL of TGFβ3, there was an average of 1,177 times more *COL2A1* expression as determined by qPCR than cultured NHACs. In its expression of both *COL2A1* and *TNMD,* the line EN47 resembled 7PEND24, however, unlike 7PEND24, EN47 when cultured in the undifferentiated state lacked expression of *BARX1, LHX8,* and *FOXF2,* while 7PEND24 expressed these transcripts. Based on these observations, the cell line EN47 or another cell line isolated from hPS cells with the above-described gene expression markers, are useful in studying the differentiation of cartilage and tendon, as well as regenerating these tissues, such as in the case of degenerative disease (intervertebral disk degeneration and osteoarthritis in the case of cartilage and tendon tears respectively).

### EXAMPLE 3: Osteochondral potential of clonal human embryonic progenitor cell lines when differentiated in HyStem-C in the presence of BMP4 and TGFβ3.

Additional clonal human embryonic progenitor cell lines previously disclosed (See US Patent Application Serial Nos. 12/504,630; 13/456,400 incorporated by reference) were differentiated for either 14 or 21 days in the presence of HyStem-C (BioTime, Inc. Alameda, CA) hydrogel as described herein supplemented with combinations of the TGF beta family.

The cell line EN8 at passage 13 displayed the gene expression markers at 13-21 doublings of clonal expansion: *CST1* (accession number NM_001898.2), *FOXF1* (NM_001451.2), *NEFM* (accession number NM_005382.1), and *ZIC2* (accession number NM_007129.2) and distal *HOX* genes expressed being *HOXA2* and *HOXB2,* and unlike the cell lines EN7 and EN47, the line EN8 expressed low or undetectable RGS 1 and did not express *TH* (tyrosine hydroxylase, accession number NM_199293.2, Illumina probe ID 1990068). As shown in FIGURE 11, as measured by qPCR, the cell line EN8 when cultured for 21 days in HyStem with 10 ng/mL of TGFβ3 together with BMP4 (10ng/mL) expressed high levels of *COL2A1* and *ACAN,* low levels of the definitive cartilage marker *CRTAC1,* as well as the hypertrophic marker *COL7CA1,* markers of cells capable of robust endochondral ossification. Surprisingly, while there was no evidence of *COL2A1* expression in EN8 in the presence of to TGFB3 in micromass conditions as described (Sternberg et al, A human embryonic stem cell-derived clonal progenitor cell line with chondrogenic potential and makers of craniofacial mesenchyme. Regen Med. 2012 Apr 23. [Epub ahead of print], 2012), indeed, only seven of 100 clonal progenitor cell lines responded to TGFβ3 in micromass conditions with chondrogenic differentiation, nevertheless, in the presence of 10 ng/mL of BMP4 together with 10 ng/mL of TGFβ3, in HyStem, there was an average of approximately 207,000 times more *COL2A1* expression as determined by qPCR than cultured NHACs by 21 days. The hypertrophic marker *COL10A1* is evidence of the novel use of the line EN8 to induce high levels of endochondral ossification for use in regenerating bone for the treatment of osteoporosis, bone fractures, fusion of bones such as in the fusion of vertebrates, osteonecrosis, and other applications where the induction of new bone is therapeutic. The cells may be formulated in hydrogels such as HyStem-C (BioTime, Inc., Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and/or therapeutic applications.

The cell lines E68 (P15) and E69 (P18) displayed the gene expression markers at 15-21 doublings of clonal expansion: *FOXS1* (accession number NM_004118.3, Illumina probe ID 3610102), *KCNIP1* (accession number NM_001034838.1, Illumina Probe ID 6960259), *KRT17* (accession number NM_000422.1, Illumina probe ID 3840445), *TFAP2C* (accession number NM_003222.3, Illumina probe ID 6450075), and *ZIC2* (accession number NM_007129.2) and no expression of the *HOX* genes *HOXA2* (accession number NM_006735.3, Illumina ID 2060471) or *HOXB2* (accession number NM_002145.3, Illunina ID 3460097). However, the cell lines E68 and E69 differed in a subset of genes. By way of non-limiting example the cell line E68 expressed the gene *UGT2B7* (accession number XM_001128725.1, Illumina probe ID 5420450) whereas the line E69 did not express *UGT2B7.* The cell lines E68 and E69 also differed in that the line E69 expressed *NNAT* (accession number NM_181689.1, Illumina probe ID 4010709) while the cell line E68 did not express *NNAT as* measured on the aforementioned Illumina microarray. As shown in FIGURE 13, as measured by qPCR, the cell line E68 at passage 23 when cultured for 21 days in HyStem with 10 ng/mL of TGFfβ3 together with BMP4 (10ng/mL) expressed high levels of *COL2A1* and *ACAN,* abundant levels of the definitive cartilage marker *CRTAC1,* as well as the hypertrophic marker *COL10A1,* markers of cells capable of robust endochondral ossification. Surprisingly, while there was no evidence of *COL2A1* expression in E68 or E69 in the presence of TGFfβ3 in micromass conditions as described (Sternberg et al, A human embryonic stem cell-derived clonal progenitor cell line with chondrogenic potential and makers of craniofacial mesenchyme. Regen Med. 2012 Apr 23. [Epub ahead of print], 2012), indeed, only seven of 100 clonal progenitor cell lines responded to TGFfβ3 in micromass conditions with chondrogenic differentiation, nevertheless, in the presence of 10 ng/mL of BMP4 together with 10 ng/mL of TGFβ3, in HyStem, there was an average of approximately 6,442 and 88,159 times more *COL2A1* expression as determined by qPCR than cultured NHACs by 21 days respectively. When the cell lines E68 (P23) and E69 (P15) were cultured as described above in HyStem for 21 days in the presence of 10 ng/mL of TGFβ3 together with BMP4 (10ng/mL), strong markers of endochondral ossification were also observed by Illumina microarray analysis, including a robust induction of *COL2A1, ACAN,* and the ossification markers *ALPL* (bone alkaline phosphatase accession number NM_000478.3, Illumina probe ID 6100356), and *IBSP* (bone sialoprotein II, accession number NM_004967.2, Illumina probe ID 6110142). The line E69 (P17) when cultured for 21 days in Hystem supplemented with GDF5 (100ng/ml) and TGFβ3 (10ng/ml), also markedly up-regulated *COL2A1* and additional cartilage markers, but with markedly reduced ossification markers such as *ALPL* (bone alkaline phosphatase accession number NM_000478.3, Illumina probe ID 6100356), and *IBSP* (bone sialoprotein II, accession number NM_004967.2, providing a novel method of generating cartilage rather than bone from this novel cultured cell type. The hypertrophic markers are evidence of the novel use of the lines E68 and E69 or lines with the above-described markers to induce high levels of endochondral ossification for use in regenerating bone for the treatment of osteoporosis, bone fractures, fusion of bones such as in the fusion of vertebrates, osteonecrosis, and other applications where the induction of new bone is therapeutic. The cells may be formulated in hydrogels such as HyStem-C (BioTime, Inc. Alameda CA) wherein the matrix is thiol-modified gelatin and thiolated hyaluronan crosslinked *in vivo* or *in vitro* with (polyethylene glycol diacrylate (PEGDA), or in alternative matrices or in solution without said matrices.

Additional clonal human embryonic progenitor cell lines previously disclosed (See US Patent Application Serial Nos. 12/504,630; 13/456,400) and incorporated by reference were differentiated for either 14 or 21 days in the presence of HyStem-C (BioTime, Inc. Alameda, CA) hydrogel as described herein supplemented with combinations of the TGF beta family. The cell lines EN26 (P13) and EN27 (P13) displayed the gene expression markers at 13-21 doublings of clonal expansion: *CD69* (accession number NM_001781.1, Illumina probe ID 2710575), *FOXF1* (NM_001451.2, Illumina probe ID 3800554), *FOXF2* (NM_001452.1, Illumina probe ID 1660470)), but did not express *NEFM* (accession number NM_005382.1, Illumina probe ID 1660767), or *ZIC2* (accession number NM_007129.2, Illumina probe ID 510368) and no expression of the *HOX* genes *HOXA2* (accession number NM_006735.3, Illumina ID 2060471) or *HOXB2* (accession number NM_002145.3, Illumina ID 3460097). However, the cell lines EN26 and EN27 differed in that cell line EN26 expressed the gene *HCLS1* (accession number NM_005335.3, Illumina ID 1300408), whereas the cell line EN27 did not express *HCLS1,* and the line EN27 expressed the gene *HEPH* (accession number NM_138737.1, Illumina probe ID 1850349), while the line EN26 did not express *HEPH* as determined using this microarray probe. As shown in FIGURE 14, as measured by Illumina microarray, the cell lines EN26 and EN27 when cultured for 21 days in HyStem with 10 ng/mL of TGFfβ3 together with BMP4 (10ng/mL) expressed *COL2A1* as well as other cartilage markers (not shown). These cells are therefore useful in repairing cartilage and bone as described herein. In the examples of research and therapeutic formulations, said cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices.

Additional clonal human embryonic progenitor cell lines previously disclosed (See US Patent Application Serial Nos. 12/504,630; 13/456,400) and incorporated by reference were differentiated for either 14 or 21 days in the presence of HyStem-C (BioTime, Inc. Alameda, CA) hydrogel as described herein supplemented with combinations of the TGF beta family. The cell line EN31 at passage 12 displayed the gene expression markers at 12-21 doublings of clonal expansion: *CD69* (accession number NM_001781.1, Illumina probe ID 2710575), *CST1* (accession number NM_001898.2, Illumina probe ID 6370541), *FOXF1* (NM_001451.2, Illumina probe ID 3800554), *OSR2* (accession number XM_001126824.1, Illumina probe ID 5420452), and *ZIC2* (accession number NM_007129.2, Illumina probe ID 510368) and the *HOX* genes *HOXA2* and *HOXB2,* but did not express *NEFM* (accession number NM_005382.1, Illumina probe ID 1660767) or *TH* (accession number NM_199293.2). The cell line EN31 expressed low but detectable levels of *CD74* transcript, a gene abundantly expressed in MSCs but not most connective tissue cells, however, numerous genes were strikingly expressed in MSCs and not in EN31 in similar culture conditions including, but not limited to the gene proenkephalin *(PENK),* which was expressed in MSCs, but not the line EN31, and *ZIC2,* which was expressed in the line EN31, but was not detected in the MSCs. As shown in FIGURE 14, as measured by Illumina microarrays, the cell line EN31 when cultured for 21 days in HyStem with 10 ng/mL of TGFfβ3 together with BMP4 (10ng/mL) expressed extremely high levels of *COL2A1.* The line also expressed high levels of the bone mineralization markers *IBSP, ALPL,* and *COL10A1* (not shown), markers of cells capable of robust endochondral ossification. These mineralization markers are evidence of the novel use of the line EN31 to induce high levels of ossification for use in regenerating bone for the treatment of osteoporosis, bone fractures, fusion of bones such as in the fusion of vertebrates, osteonecrosis, and other applications where the induction of new bone is therapeutic. In the examples of research and therapeutic formulations, said cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices.

Additional clonal human embryonic progenitor cell lines previously disclosed (See US Patent Application Serial Nos. 12/504,630; 13/456,400) and incorporated by reference were differentiated for either 14 or 21 days in the presence of HyStem-C (BioTime, Inc. Alameda, CA) hydrogel as described herein supplemented with combinations of the TGF beta family. The cell lines T42 at passage 13 displayed the gene expression markers at 13-21 doublings of clonal expansion: *EPHA5* (accession number NM_004439.4, Illumina probe ID 5360408), *HEY2* (NM_012259.1, Illumina probe ID 2350685), *KCNIP1* (accession number NM_001034838.1, Illumina probe ID 6960259), *KRT17* (accession number NM_000422.1, Illumina probe ID 3840445), *MKX* (accession number NM_173576.1, Illumina probe ID 6620017) and does not express the *HOX* genes *HOXA2* or *HOXB2.* The cell line T42 expressed the gene *OLFML1* (accession number NM_198474.2, Illumina probe ID 130390) but the cell line E68 did not express *OLFML1.* The line T42 expressed *WDR72* (accession number NM_182758.1, Illumina ID 2810451) unlike the lines E68 and E69 which did not express *WDR72.* As shown in FIGURE 14, as measured by Illumina gene expression microarray, the cell line T42 when cultured for 21 days in HyStem with 10 ng/mL of TGFfβ3 together with BMP4 (10ng/mL) expressed high levels of *COL2A1, EPYC,* and *ACAN,* and relatively high levels of the bone markers *ALPL,* and osteopontin *(SPP1)* (accession number NM_000582.2), markers of cells capable of robust endochondral ossification. Surprisingly, while there was no evidence of *COL2A1* expression in T42 in the presence of to TGFB3 in micromass conditions as described (Sternberg et al, A human embryonic stem cell-derived clonal progenitor cell line with chondrogenic potential and makers of craniofacial mesenchyme. Regen Med. 2012 Apr 23. [Epub ahead of print], 2012), indeed, only seven of 100 clonal progenitor cell lines responded to TGFfβ3 in micromass conditions with chondrogenic differentiation, nevertheless, at passage 17 and cultured in the presence of 10 ng/mL of BMP4 together with 10 ng/mL of TGFβ3, in HyStem, there was approximately 88,000-fold more *COL2A1* expression as determined by qPCR than cultured NHACs by 14 days of differentiation. The osteogenic markers are evidence of the novel use of the line T42 to induce high levels of endochondral ossification for use in regenerating bone for the treatment of osteoporosis, bone fractures, fusion of bones such as in the fusion of vertebrates, osteonecrosis, and other applications where the induction of new bone is therapeutic.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for therapeutic and research applications.

### EXAMPLE 4: Use of all-trans retinoic acid to induce SFRP4 in osteochondral progenitors and therapeutic uses thereof.

Wnt inhibitors such as the secreted frizzled-related proteins have been implicated in a number of developmental and pathological processes, in particular, in generating pattern formation during development. The gene *SFRP4* has been reported to inhibit bone formation in part through transcriptional inhibition of BMP2 (Zhang, R. 2012. Wnt/β-catenin signaling activates bone morphogenetic protein 2 expression in osteoblasts, Bone 52: 145-156). This suggests that wnt signaling may underlie the patterning of connective tissue formation in the body, for instance, generating the sharp boundaries between calvarial bone and underlying meningeal membranes. Surprisingly, we observed that while the clonal embryonic progenitors that display osteochondral differentiation potential in response to some combination of TGFβ family members show a wide array of different markers in the undifferentiated state, when said clonal osteochondral embryonic progenitors together with clonal embryonic progenitors not capable of osteochondral differentiation are differentiated in the presence of retinoic acid, we observe that every osteochondral progenitor line robustly induced the expression of *SFRP4,* a potent inhibitor of wnt signaling, while little or no SFRP4 expression was observed in the lines incapable of osteochondral differentiation (FIGURE 15).

While combinations of TGFfβ3 and other members of the TGFβ family can be used as described herein to induce cartilage, bone, and tendon formation, alternatively the osteochondral progenitors described herein may be exposed to retinoic acid while cultured *in vitro* with or without matrix support to obtain cells expressing high levels of SFRP4 expression. These SFRP4-expressing cells may be used to express the protein *in vitro* for research and therapy that is, as a method of manufacturing the protein, or as a means of manufacturing the protein *in vivo* to treat disease wherein the SFRP4-expressing cells which may or may not be mitotically inactivated, are transplanted in the body and express the protein for a therapeutic effect. Examples of such treatments include the cell-based expression of SFRP4 around the tendons of the hands: 1) to prevent fibrosis and contractures as occurs in Duypuytren's disease; 2) to reduce fibrosis or scarring in the heart following infarction; 3) to inhibit wnt signaling in tumors thereby inhibiting tumor growth and tumor angiogenesis; and 4) to prevent inappropriate ossification of tissue.

The osteochondral progenitor cells may be formulated with a hydrogel comprising one or more polymers, such as polymers of hyaluronic acid (HyStem) or hyaluronic acid and gelatin (HyStem-C (BioTime, Inc. Alameda, CA)). In addition, bilaminar constructs may be generated wherein the osteochondral progenitors described herein capable of differentiating into cartilage, bone, or tendon are so differentiated and incorporated into one layer of hydrogel to induce corresponding tissue regeneration, while an opposing layer of hydrogel containing retinoic acid-induced free SFRP4 or SFRP4-expressing cells are applied wherein the SFRP4 containing surface inhibits fibrosis, chondrogenesis, or osteogenesis occurring on the opposing side. As a result, said biologically active bilayers may induce for example tendon healing on one side of the membrane/hydrogel while inhibiting adhesions and fibrosis on the other side. Such membrane may be useful in treating tendon tears such as commonly occur in the rotator cuff tendons.

Many malignancies generate disordered tissue structure, abnormal cell proliferation, and tumor-associated angiogenesis through the activation of wnt signaling pathways, often through mutations in wnt inhibitors. The cells of the present invention that activate SFRP4 can also be used to inhibit wnt signaling in tumors. For example, cells expressing *SFRP4* in response to retinoic acid, and cells also expressing *FOXS1,* a marker of perivascular cells, may be introduced into the resection area following tumor removal, to increase wnt signaling thereby reducing proliferation of remaining tumor cells and tumor angiogenesis. The cells expressing *FOXS1* of the present invention include the cell lines 7PEND24, E68, and E69. Moreover, the cells used to inhibit tumor growth may also be genetically modified as previously described for mesenchymal stem cells in treating glioma (Binello, E. 2012. Stem cells as therapeutic vehicles for the treatment of high-grade gliomas, Neuro Oncol. 2012 Mar;14(3):256-65. Epub 2011 Dec 13).

### EXAMPLE 5: Identification and uUse of TTR-expressing cell lines for regenerating choroid plexus functionormation and delivering protein into theproducing cerebrospinal fluid in vivo or in vitro.

The cell lines of the present invention E68, E69 (derived from the hES cell line ACT03 (MA03)), and T42 (derived from the hES cell line H9) were differentiated in the presence of BMP4. The cell lines E68 (P15) and E69 (P18) displayed the gene expression markers at 15-21 doublings of clonal expansion: FOXS1 (accession number NM_004118.3, Illumina probe ID 3610102), KCNIP1 (accession number NM_001034838.1, Illumina Probe ID 6960259), KRT17 (accession number NM_000422.1, Illumina probe ID 3840445), TFAP2C (accession number NM_003222.3, Illumina probe ID 6450075), and ZIC2 (accession number NM_007129.2) and no expression of the HOX genes HOXA2 (accession number NM_006735.3, Illumina ID 2060471) or HOXB2 (accession number NM_002145.3, Illunina ID 3460097). However, the cell lines E68 and E69 differed in a subset of genes. By way of non-limiting example the cell line E68 expressed the gene UGT2B7 (accession number XM_001128725.1, Illumina probe ID 5420450) whereas the line E69 did not express UGT2B7. The cell lines E68 and E69 also differed in that the line E69 expressed NNAT (accession number NM_181689.1, Illumina probe ID 4010709) while the cell line E68 did not express NNAT as measured on the aforementioned Illumina microarray. The cell line T42 in the undifferentiated state expressed the gene expression markers at 13-21 doublings of clonal expansion: EPHA5 (accession number NM_004439.4, Illumina probe ID 5360408), HEY2 (accession number NM_012259.1, Illumina probe ID 2350685), KCNIP1 (accession number NM_001034838.1, Illumina probe ID 6960259), KRT17 (accession number NM_000422.1, Illumina probe ID 3840445), MKX (accession number NM_173576.1, Illumina probe ID 6620017) and does not express the HOX genes HOXA2 or HOXB2. The cell line T42 also expressed the gene OLFML1 (accession number NM_198474.2, Illumina probe ID 130390) and WDR72 (accession number NM_182758.1, Illumina ID 2810451).

When E68, E69, and T42 were differentiated in the presence of 10 ng/mL of BMP4 in HyStem for 14 days expressed the marker for choroid plexus cellsepithelium TTR (FIGURE 16). This is consistent with the cranial neural crest markers of the cells as is the expression of TFAP2A in the absence of distal HOX gene expression such as HOXA2 or HOXB2*.* The cell lines E68, E69 and T42 also expressed KRT7 on an RNA level, though only significant expression levels of keratin 17 protein as detected by immunocytochemistry was detected in the line E69 (not shown). The clones E68 and E69 (passages ranging from 14-22) expressed mRNA detectable on Illumina microarrays as well as qPCR for KRT17, TRIM4, and ZIC2 while T42 (passages ranging from 17-20) expressed TRIML2, EPDR1, ZIC2, and lower but detectable levels of KRT17.

Differentiation of the clonal lines E69 and T42 in micromass cultures or differentiation of confluent monolayers of the cells as opposed to HyStem beads for 7 to 14 days also led to the expression of TTR, though micromass and confluent cultures led to TTR in the absence of the adipocyte marker FABP4, which therefore may be a preferred method to yield TTR positive choroid plexus cells in the absence of adipocytes.

To determine whether transthretin was also expressed on a protein level, three day serum-free conditioned medium was collected from parallel cultures of E69 (P16), T42 (P17), and a clonal progenitor line capable of chondrogenesis designated MEL2 (P19) in micromass conditions in differentiation media supplemented with 10 ng/mL of BMP4, and analyzed by ELISA for the presence of soluble secreted protein. As shown in Figure 24, the line T42 expressed the highest levels of secreted protein, reaching approximately 8.9 ng/ml at three days, E69 reaching approximately 0.7 ng/ml, while no transthyretin was detectable in the media of cultured MEL2 cells.

TTR is believed to be expressed relatively rarely in vivo, in tissues such as the retina, liver, and choroid plexus of the brain. We therefore examined RNA from>130 diverse cultured somatic cell types and observed TTR expression in cells from only these tissues. Specifically, cultured hES-derived retinal pigment epithelial (RPE) cells expressed high levels of TTR, while we did not observe TTR expression in undifferentiated hES cells or human iris pigment epithelial (RPE) cells. We also observed TTR expression in primary cultures of human choroid plexus epithelium and human choroid plexus-derived stromal cells, but did not observe expression in cultured human choroid plexus vascular endothelial cells. Lastly, we observed TTR expression in primary hepatocytes, but not hepatic sinusoidal endothelial or stellate cells. Since neither E69 or T42 cells differentiated in BMP4 in either HyStem or micromass conditions expressed the numerous common markers of RPE cells such as TYRP1 or TYR, or markers of hepatocytes such as FOXA2 or FGG, it would appear that they do not correspond to either RPE cells or hepatocytes, but rather choroid plexus cells.

All three cell lines (E68, E69, and T42) also up-regulated KIT in response to differentiation in the presence of 10 ng/mL of BMP4. To examine whether activation of KIT affected E69 and T42 cell differentiation, we differentiated E69 and T42 cells for 14 days in differentiation media supplemented with stem cell factor (SCF) or SCF and BMP4 with each condition being performed in both micromass and HyStem-4D beads. As shown in Figure 25, culture in SCF alone resulted in a marked induction of the leptomeningeal markers PTGDS and ISLR in both E69 and T42 in both micromass and HyStem-4D bead arrays. PTGDS encodes a glutathione-independent PGD synthase converting PGH2 to PGD2 in the brain where it is also known as β-trace, the second most abundant protein of cerebral spinal fluid (CSF) after albumin. In humans, it appears to be expressed at highest levels in the arachnoid barrier cells, followed by arachnoid trabecular and pia cells, but is not expressed in the dura mater. T42 appeared to differ from E69 cells in a relatively high induction of SLC6A1 and APOD compared to E69 when differentiated in the presence of SCF without BMP4.

The combination of BMP4 and SCF led to an outcome similar to BMP4 and TGFβ3, namely TTR-expressing cells in the relative absence of leptomeningeal cells. As before, HyStem-C in these conditions led to a higher expression of the adipocyte markers FABP4 and CD36 compared to differentiation in micromass culture.

It has been reported that cultured neural crest cells express low-affinity NGFR, and that such expression is a requirement of the mammalian neural crest stem cells. However, we did not detect NGFR in E69, T42, or MEL2 in any differentiation condition by microarray analysis.

Culture of the lines E69 and T42 in confluent conditions in the presence of differentiation media supplemented with 250 ng/mL growth differentiation factor 5 (GDF5) also led to the induction of KIT and TTR in E69 and T42 cells, though at lower levels than that observed with cultured in 10 ng/mL of BMP4.

A facile means of scaling purified progenitors to choroid plexus or leptomeninges cells will have numerous applications in human and veterinary therapy. Cells having this genetic profile differentiated from clonally purified progenitors such as the line E68, E69, and T42 or derived from other sources such as hPS cell lines and having similar progenitor markers, may be useful in regenerating choroid plexus function in vivo by the transplantation of the cells into the choroid plexus. Dosages and vehicle to delivery of the cells will vary based on the degree of degeneration present in the tissue and the size of the recipient, but common dosages in humans would be expected to be 0.1, 0.5, or 1.0 x 107 cells per ventricle. The vehicle can be various solutions known in the art such as physiological saline. However, preferably, the cells are transplanted in a liquid matrix capable of crosslinking and promoting survival while limiting inappropriate migration of the cells, such as can be achieved by using the vehicle HyStem-C (BioTime, Inc. Alameda, CA) described herein.

The transplanted cells can be used to increase cerebral spinal fluid (CSF) circulation. This application may be beneficial to treat trauma to the choroid plexus, or age-related neurodegeneration as occurs in Alzheimer's disease and Parkinson's disease. The cells may be genetically modified to secrete desired proteins or other molecules into the CSF. An example would be the genetic modification of the cells with enzymes deficient in a patient such as lysosomal storage disorders or mucopolysaccharide storage disorders.

In addition, as shown in this example, adherent cells cultured in vitro, can also be differentiated as described herein, then subsequently used to express transthyretin- and/or β-trace conditioned medium. When that medium is serum-free saline solution corresponding to the physiological concentrations of the salts in normal cerebral spinal fluid instead of normal culture medium as described above, the cells of the present invention such as E68, E69, T42, or cells with similar patterns of gene expression can be differentiated to express transthyretin by differentiating confluent, micromass, or HyStem bead cultures of E68, E69, or T42 cells or cells with similar gene expression in 10 ng/mL BMP4 for typically 7-14 days, or β-trace by similarly culturing the cells in the presence of 10 ng/mL of SCF. Alternatively, conditioned artificial CSF can be mixed from the BMP4 and SCF conditions described above at a ratio of approximately 50% conditioned medium from TTR-expressing cells and 50% conditioned medium from PTGDS-expressing cells. Physiological salt concentrations for artificial cerebral spinal fluid is known in the art (Davson, H. Physiology of the Cerebrospinal Fluid, J. & A. Churchill, Ltd., London, 1967 and Biology Data Book, Volume III, 2nd ed., Fed. Am. Soc. Exper. Biol., Washington D.C., 1974). The CSF-specific concentrations of electrolytes in humans for example are 150 mM Na, 3.0 mM K, 1.4 mM Ca, 0.8 mM Mg, 1.0 mM P, and 155 mM Cl. Standard cell culture medium (such as dulbecco's modified minimal essential media) without added serum, normal physiological glucose (100 mg/dL) but with the CSF-specific mineral concentrations, when incubated with the differentiated cells of the present invention to create 24, 48, or 72 hour conditioned medium as described in this example, will result in artificial cerebral spinal fluid containing proteins such as transthyretin and β-trace. This more physiological artificial cerebral spinal fluid will have utility in replacing the fluid in the central nervous system lost from trauma or disease.

### EXAMPLE 6: Analysis of Progenitor Cell Line Potential Under Various Differentiation Protocols

### Mapping in vitro cells to in vivo entities

Since pluripotent stem or progenitor cells undergoing *in vitro* differentiation generally express similar gene expression patterns to corresponding *in vivo* cells, methods to correlate cells generated *in vitro* with those *in vivo,* i.e. it's localization inside the developmental ancestry tree and its location vis-a-vis anatomical or tissue classification, may be useful. Matching or mapping may be performed utilizing the LifeMap database (available online), using the accumulated and statistically analyzed data collected for cells, anatomical compartments and tissues. The matched entity is represented by the experimental measurement of the gene expression of these *in vitro* cells, and typically a reference dataset. These data are commonly obtained by high-throughput techniques such as hybridization microarrays or more recently, RNA-seq. Mapping may be performed from essentially any entity with experimental gene expression, for the purpose of illustration, an *in vitro* cell (e.g. a specific BioTime cell line) may be mapped to *in vivo* entities - cells, tissues or anatomical compartments.

The mapping process takes place in four parts:
1. Database pre-processing: all available gene expression data that is stored in the LifeMap database in the various levels is analyzed. Genes are scored based on overall or local abundance, available expression level. Genes are grouped or clustered based on similarity (unsupervised) or biological context (supervised). Each method will yield a unique collection of gene sets.
2. Sample preparation: the experimental gene expression data of the *in vitro* cell to be mapped needs to be provided as intensity ranked list of genes (or other methods yielding normalized transcript count measure).
3. Matching: a number of methods and algorithms are applied to match the provided ranked gene list of step 2, to the various gene sets prepared in step 1. Each such process will yield a list of matches with scores and measures of statistical significance where available (e.g. P-values or FDR scores).
4. Consolidation: the underlying gene expression data is qualitative, heterogeneous and usage of different gene set groups and multiple algorithms yield results that are directly incomparable. Since each such method produces lists of matched entities, it is necessary to consolidate the information and reshuffle the statistically significant results to the top. The end result is a distilled list of matches and scores.

The mapping application concludes its functionality in providing the user with both a detailed list and a graphical display of the mapped entities (in this example mapping to organ/tissue entities).

### Experimental

Gene expression was assayed with Illumina Human HT-12 v4 microarrays. Raw data was background corrected using negative control probes, quantile normalized and log2 transformed.

Enrichment analysis evaluates the likelihood of any constructed set of genes to have significant presence in an experimental gene expression study, rather than examining single genes. The LifeMap expression information was hence used to create sets of genes (GeneSets) that contain lists of known or inferred genes in every developing cell and anatomical compartment. These GeneSets were used as input for the Score Matrix Classification (SMC) analysis (LifeMap, unpublished), and Gene Set Enrichment Analysis (GSEA) algorithm (Subramanian et. al. PNAS October 25, 2005 vol. 102 no. 43 15545-15550). The GSEA input was modified such that we may assign more weight for matches of highly specific marker genes in the GeneSets. Specifically, genes that are highly specific have been doubled in occurrence, while the rest of the genes appear once. We obtained for each result its leading edge gene list to account for the gene matching. Results with too few genes (less than 3) in the leading edge analysis, or results that did not show highly statistical significance (p value> 0.05 and FDR >0.25) were ignored.

The cell lines of the present invention designated 7PEND24, 7SMOO32, E15, MEL2, SK11, SM30 and T42 were differentiated for 14 days in HyStem differentiation conditions as described herein supplemented with 10 ng/ml of BMP4, or 10 ng/ml TGFβ3 and 100 ng/ml GDF5, or in micromass conditions as described herein supplemented with 10 ng/ml BMP4. Upon microarray analysis, the raw data from the progenitor cell lines was background corrected and normalized. Gene Set Enrichment Analysis (GSEA) was used to evaluate the likelihood of any constructed set of genes to have biological significance vs. other subset of progenitors as described above in the methods for mapping in vitro cells to in vivo entities.

The cell line 7PEND24 in the undifferentiated state showed markers of early adipocytes (*PPARG, DLK1, CEBPD*) in addition to *BARX1, LHX8, SNAI2, TWIST1,* and *FOXF1,* previously mentioned as markers of the line consistent with it being of neural crest-derived mesenchyme origin. When differentiated in the presence of BMP4, in Hystem the line demonstrated the potential to differentiate into both brown and white adipocytes, expressing the markers of visceral white adipocytes *LPL, CEBPD, PPARG, FABP4,* and *PLIN1,* as well as markers of brown adipocytes *DLK1, PPARGC1A, CEBPD, PPARG, FABP4, PRDM16,* and *FOXC2.* The cell line also showed the potential to develop into intervertebral disc annulus fibrosis cells expressing the markers *CRLF1, FOXF2, FBLN5, DPT, ITGBL1, COL2A1, COL6A3, DUSP1, FOXF1, TGFB3, PAX9, GSN,* and *FMOD,* and vertebrae body cells expressing the markers *PDE8B, COMP, ITGA10, SAA1, DTNA, PCDH9, EBF1, SORBS1, SORBS2, ZNF503,* and *MGST2.* When the line 7PEND24 was differentiated for 14 days in HyStem supplemented with GDF5 and 10 ng/ml TGFβ3, the cells showed markers of intervertebral disc annulus fibrosis cells such as *DPT, COL2A1, OGN, FBLN5, FMOD, CRLF1, ITGBL1, COL6A3, TGFB3, FOXF2, GSN, HHIP, LRIG1,* and *TRPS1;* as well as markers of mandibular condyle: *COMP, COL2A1, ACAN, LECT1, COL9A1, HAPLN1, ITGA10, OLFML3, PKDCC, FGFR3, CSPG4, COL9A3, ITGB5,* and *DNM1;* markers of thoracic rib bone: *OGN, COL9A2, FMOD, LECT1, EPYC, CHAD, COL9A1, PPIB, SRPX2, MATN3, LUM, COL13A1, FKBP11, MXRA8, COL27A1, PELI2, GPX7, ANGPTL2, GXYLT2, KLF4, STEAP3, SLC39A14, PTH1R, FAM46A, FAM180A, SLC26A2, RUNX1, CTGF, COL9A3, PLEKHB1, FKBP7, HHIP, TNC, JAK2, CYTL1, KDELR3, ATP8B2,* and *TRPS1;* endochondral head bones: *ELN, FMOD, EPYC, CHAD, COL9A1, PPIB, PHEX, SOX9, COL27A1, PELI2, ANGPTL2, GXYLT2, SLC39A14, P4HA3, SLC26A2, CTGF, MRC2, COL9A3, PLEKHB1, TNC, FRMD8, CYTL1,* and *ATP8B2;* and limb long bones: *COL2A1, SCRG1, FMOD, CHAD, COL9A1, MATN3, FOXF2, SMOC1, COL27A1, PELI2, SOX8, PTH1R, HTRA1, RUNX1, CTGF, PLEKHB1, RG9MTD1, CYTL1,* and *KLF2.*

The cell line 7SMOO32 in the undifferentiated state expressed markers of axial and appendicular-derived mesenchyme cell-specific gene *markers (FOXF1, MMP10, MSX2, FOXF2, FOXD1, MMP2, CDH11, TFAP2A),* chondrogenic markers *(SOX9, COL1A1, FOXF2),* and osteogenic markers *(SPP1, MSX2, FGFR3).* When differentiated in Hystem for 14 days supplemented with 10 ng/ml of BMP4, the line expressed markers of white adipocytes such as: *FABP4, LPL, CEBPB, PLIN1, PPARG,* and *CEBPD,* as well as brown adipocytes: *FABP4, PPARGC1A, DLK1, CEBPB, PPARG,* and *CEBPD.* When differentiated for 14 days in HyStem supplemented with 10 ng/ml TGFfβ3 and 100 ng/ml GDF5, the line 7SMOO32 expressed markers of intervertebral disc annulus fibrosis cells such as: *DPT, CRLF1, FBLN5, COL2A1, OGN, ITGBL1, FOXF2, COL6A3, GSN, WIF1, TGFB3, LRIG1, CORO2B, ADAMTS15,* and *IGFBP7*; markers of glenoid fossa cells such as: *COMP, COL2A1, SPP1, KAZALD1, LECT1, ITGA10, MMP13, ACAN, HAPLN1, PHEX, PTH1R, COL11A1,* and *IP6K2;* markers of mandibular condyle such as: *COMP, COL2A1, PKDCC, LECT1, ITGA10, LTBP3, ACAN, HAPLN1, OLFML3, WIF1, ITGB5,* and *IP6K2;* endochondral facial bones such as: *ELN, CYTL1, LTBP3, PELI2, EPYC, PHEX, MRC2, CALY, GXYLT2, COL27A1, ANGPTL2, SOX9, GAA, TNC, LEPRE1, LTBP2,* and *ARFGAP1*; and markers of thoracic rib bone such as: *COL9A2, OGN, SRPX2, CYTL1, FAM46A, LECT1, LUM, LTBP3, PELI2, COL13A1, EPYC, MXRA8, RUNX1, CALY, PTH1R, FKBP11, STEAP3, CFH, SLC40A1, GXYLT2, COL27A1, GPX7, ANGPTL2, MATN3, FKBP7, GAA, TNC, LEPRE1,* and *LTBP2.*

The cell line E15 which in the undifferentiated state expressed markers including *SOX9, VCAN,* and *COL6A2* and markers of axial and appendicular-derived mesenchymal cells *(TFAP2A, CDH2, SIX1*) when differentiated in HyStem supplemented with 10 ng/ml BMP4 expressed the markers of white adipocytes: *FABP4, PPARG, PLIN1*, *LPL;* brown preadipocyte cells: *FABP4, PPARG, PRDM16*; markers of mandibular condyle: *FGFR3, ITGA10, CSPG4, LTBP3, COMP, HAPLN1, PKDCC,* and *CA12*; and the markers of endochondral facial bones: *GDF10, COL9A2, KLF4, PPIB, IRX5, ARHGAP24,* and *HHIP.* The line E15 when differentiated for 14 days in HyStem supplemented with 10 ng/ml TGFβ3 and 100 ng/ml GDF5 expressed the markers of intervertebral disc annulus fibrosis cells: *COL2A1, FBLN5, DPT, FMOD, CRLF1, OGN, HHIP, ITGBL1, TGFB3, COL6A3, VCAN, DUSP1, TRPS1, GSN, ADAMTS6,* and ERG; markers of endochondral facial bones: *COL10A1, EPYC, ELN, FMOD, SOX9, COL8A1, COL9A3, CHAD, CYTL1, PPIB, CTHRC1, PLEKHB1, SLC26A2, KANK1, SLC39A14, ATP8B2, TNC, LTBP2, GPC1, WWP2, P4HA3, BAMBI, COL27A1, CTGF, FGFRL1,* and *SDC2*; autopod long bone: *SCRG1, COL2A1, FMOD, MEF2C, MATN3, PTH1R, CHAD, ENPP2, CYTL1, CTHRC1, PLEKHB1, RUNX3, RUNX1, PRICKLE1, WWP2, HTRA1, COL27A1, CTGF, FGFRL1, SOX8, ERG,* and *FAT3;* and thoracic rib bone: *COL9A2, COL10A1, EPYC, FMOD, PCOLCE2, OGN, MEF2C, MATN3, LUM, HHIP, PTH1R, COL8A1, COL9A3, DKK1, SLC40A1, KLF4, SRPX2, CHAD, CYTL1, PPIB, LECT1, FKBP11, CTHRC1, STEAP3, PLEKHB1, RUNX3, SLC26A2, KANK1, LOXL4, SLC39A14, ATP8B2, DUSP1, RUNX1, TNC, LEPR, LTBP2, TRPS1, GPC1, WWP2, CFH, BAMBI, CDH2, COL27A1, FAM46A, GPX7, CTGF, FGFRL1, SDC2,* and *PLCD1.*

The line MEL2 which expresses *NKX3-2* and the osteogenic markers *SATB2, ALPL, MSX2* in the undifferentiated state, also expresses markers of neural crest- and lateral plate-derived head and limb mesenchymal cells such as *FRZB, HAND2, DLX5, DLX6, TWIST1, FOXD1, MSX2, TFAP2A.* When differentiated in HyStem supplemented with 10 ng/mL BMP4 expressed the markers of osteoblasts: *ALPL, BMP2, PTH1R, MSX2, DCN, SPP1,* and *SATB2*; intramembranous preosteoblasts: *DLX5, ALPL, PTH1R, MSX2, POSTN, TWIST1,* and *SATB2*; and epiphyseal end limb bone: *PCDH20, SERPINA3, TAC1, EDNRA, SCRG1, CRABP2, SPOCK3, VCAN, BOC, ECM2, CRLF1, GAS1, TSPAN8, MFAP4, NFIA,* and *RASSF9.* When MEL2 is differentiated in HyStem supplemented with 10 ng/ml TGFβ3 and 100 ng/ml GDF5, the line expressed markers of preosteoblasts: *SPP1, BMP2, ALPL, PTH1R, FGFR3, DCN, MSX2, SATB2, NKX3-2,* and *TWIST1*; lumbar vertebral body: *COMP, SERPINA3, ITGA10, EBF1, SORBS2, PCDH17, SOBP,* and *ST8SIA4*; thoracic rib bone: *COL10A1, COL9A2, OGN, MEF2C, ALPL, PTH1R, LUM, CFH, STEAP3, PELI2, LTBP2, SLC40A1, MXRA8, COL8A1, MATN3, PARD6G, GPC1, GPX7, FAM180A, CTHRC1, ATP8B2, BAMBI, DKK1, PLCD1, LTBR, SLC26A2, ANGPTL2, SATB2, FAM46A,* and *DUSP1*; and mandibular condyle: *COMP, ITGA10, CSPG4, FGFR3, CA12, ITGB5, OLFML3, COL2A1,* and *ACAN.*

The line SK11 that expresses in addition to the previously-described markers expresses markers of axial and appendicular level-derived mesenchyme cells (*SIX1, TWIST1, SNA12).* When differentiated in HyStem supplemented with 10 ng/ml BMP4, the line expressed the markers of mandibular condyle: *FGFR3, ACAN, ITGA10, OLFML3, COMP, CSPG4, XIST, PKDCC, COL2A1, LTBP3,* and *FUS;* intervertebral disc annulus fibrosis cells: *TGFB3, FBLN5, OGN, GAS1, ITGBL1, DPT, DUSP1, GSN, COL2A1, ARHGAP24, VCAN,* and *EMILIN3*; lumbar vertebrae body: *PDE8B, SORBS2, ITGA10, PHACTR2, COMP, EBF1, ST8SIA4,* and *PCDH9;* thoracic rib bone: *PTH1R, CFH, DKK1, ADAMTS1, COL9A2, COL8A1, OGN, MEF2C, BAMBI, TNMD, FAM46A, MATN3, LTBP2, DUSP1, FKBP11, CDH2, SLC40A1, MXRA8, STEAP3, ANGPTL2, GPX7, COL27A1, LUM, TSPAN3, SDC2, ATP8B2, CD36, CTGF, LTBP3, ARHGAP24,* and *FBLN2;* and glenoid fossa cells: *SPP1, FGFR3, PTH1R, COL11A1, ACAN, ITGA10, COMP, CSPG4, XIST, COL2A1,* and *FUS.* The line SK11 when differentiated in HyStem in the presence of 10 ng/ml TGFβ3 and 100 ng/ml GDF5, the line expressed the markers of glenoid fossa cells: *COMP, COL2A1, ACAN, SPP1, FGFR3, HAPLN1, COL11A1, PTH1R, LECT1, CHAD, COL11A2, COL9A1, ITGA10, COL9A3, CSPG4, ENPP1, MMP13, MMP2, XIST,* and *SERPINH1*; mandibular condyle: *COMP, COL2A1, ACAN, FGFR3, HAPLN1, LECT1, COL9A1, ITGA10, OLFML3, COL9A3, CSPG4, MMP2, PKDCC, XIST, ITGB5, SERPINH1, CA12,* and *DNM1*; and thoracic rib: *COL10A1, COL9A2, EPYC, OGN, PTH1R, MATN3, LECT1, FMOD, CHAD, COL9A1, HHIP, STEAP3, FKBP11, PCOLCE2, GPC1, GXYLT2, LUM, MEF2C, COL9A3, MXRA8, GPX7, RUNX1, FAM46A, CTHRC1, COL8A1, ANGPTL2, CFH, SLC39A14, COL27A1, IBSP, FKBP7, KLF4, P4HA2, ATP8B2, SRPRB, PELI2, METRNL, KDELR3, PARD6G,* and *LEPRE1.*

The line SM30 which expressed the markers of axial and appendicular mesenchyme cells *FOXF1, SOX9, RUNX2,* and *NKX3-2,* when differentiated in HyStem supplemented with 10 ng/ml BMP4, expressed the markers of intervertebral disc nucleus pulposis cells: *COL2A1, ACAN, DCN, FOS, LUM, ITGA1, A2M,* and *CTGF* intervertebral disc nucleus pulposis cells: *COL2A1, GSN, OGN, EMILIN3, ITGBL1, TGFB3, FBLN5, DUSP1,* and *CRLF1*; intervertebral annulus fibrosis cells: *COL2A1, GSN, OGN, EMILIN3, ITGBL1, TGFB3, FBLN5, DUSP1,* and *CRLF1*; mandibular condyle cells: *COL2A1, FGFR3, ACAN, ITGA10, CSPG4, PKDCC, COL9A3, HAPLN1, XIST, COL9A1, OLFML3, COMP, LTBP3,* and *LECT1*; thoracic rib cell markers: *COL9A2, PTH1R, CFH, EPYC, DKK1, MATN3, MEF2C, OGN, CD36, COL9A3, COL8A1, BAMBI, CHAD, LEPR, COL10A1, LUM, LPAR3, KLF4, COL9A1, ANGPTL2, PCOLCE2, MXRA8, LTBP3, ATP8B2, DUSP1, LTBP2, COL24A1, SLC40A1, LECT1, GPX7, FAM46A, CTGF, FGFRL1, CDH2; limb long bone markers: COL2A1, PTH1R, ENPP2, SCRG1, MATN3, MEF2C, CHAD, GDF10, COL9A1, FAT3, SERPINE2, SOX8, CTGF,* and *FGFRL1*; head mesenchyme chondrocytes: *COL2A1, FGFR3, ACAN, PTH1R, COL11A1, SOX9, COL11A2, HAPLN1, NKX3-2,* and *COMP;* and white preadipocytes: *FRZB, FABP4, DLK1,* and *PPARG.* The line SM30 when differentiated in HyStem supplemented with of 10 ng/ml TGFfβ3 and 100 ng/ml GDF5 expressed markers of intervertebral disc annulus fibrosis cells: *COL2A1, OGN, FBLN5, DPT, FMOD, CRLF1, TGFB3, ITGBL1, HHIP, GSN, ADAMTS6, TRPS1,* and *VCAN;* mandibular condyle cells: *COL2A1, ACAN, COMP, LECT1, HAPLN1, FGFR3, COL9A1, ITGA10, COL9A3, CSPG4, XIST, PKDCC, OLFML3, WWP2, SUSD5,* and *DNM1*; thoracic rib bone cells: *EPYC, COL9A2, COL10A1, LECT1, CHAD, OGN, MATN3, COL9A1, PTH1R, PCOLCE2, COL9A3, FMOD, FKBP11, STEAP3, LUM, MEF2C, GPC1, COL27A1, ANGPTL2, CFH, HHIP, KLF4, GPX7, WWP2, FAM46A, DKK1, MXRA8, COL8A1, ATP8B2, RUNX1, TNC, PLCD1, FAM180A, SLC39A14, TRPS1, CTGF, PELI2, FGFRL1, PYCR1, GXYLT2, SLC40A1,* and *LTBP2*; endochondral facial bones: *EPYC, COL10A1, CHAD, COL9A1, COL9A3, FMOD, ELN, SOX9, GPC1, COL27A1, ANGPTL2, WWP2, COL8A1, ATP8B2, MRC2, TNC, SLC39A14, CTGF, PELI2, FGFRL1, GXYLT2,* and *LTBP2;* limb long bone: *COL2A1, SCRG1, CHAD, MATN3, COL9A1, PTH1R, SOX8, FMOD, MEF2C, PRICKLE1, COL27A1, WWP2, ENPP2, RUNX1, CTGF, PELI2, FGFRL1, and KLF2;* and glenoid fossa cells: *COL2A1, ACAN, COMP, LECT1, HAPLN1, CHAD, SPP1, FGFR3, COL11A2, COL11A1, COL9A1, PTH1R, ITGA10, COL9A3, CSPG4, XIST, WWP2, ENPP1, MMP13, SUSD5,* and *KAZALD1.*

The line T42 when cultured in micromass conditions in the presence of 10 ng/ml BMP4 expressed markers of glial cells: *ACTC1, CRYAB, EFHD1, FGFR3, MFAP5, TGFB3, DLK1, ACTA2, OCA2, AIF1L, GPC4, SCRG1, CNN1, HES6, KRT17, COL8A1, COL9A2, DCN, RASL11B, IGFBP3, EDN1, IGFBP2, ENC1, SFRP2, ACTG2, CKB, CSRP1, CSRP2, C5orf46, COL3A1, HEY1, TUBB2B, CALD1, KAL1, CD24, CDH2, MAMDC2, EFR3B, CDKN2B, HES4, TAGLN, CAP2, PMEPA1, CTGF, TPM1, TGFB2, CXXC5, COL4A1, PALLD, SOX11, OCIAD2, EML1,* and *CCDC99;* markers of Choroid Plexus (LATERAL VENTRICLE METENCEPHALON FIBROBLASTS): *TTR, CCDC3, TGFB3, ZIC2, GPC4, POSTN, ID3, PODXL, FBLN5, KRT7, COL8A1, OGN, TINAGL1, LGMN, GPX7, ACTG2, CSorf46, ANGPTL4, FOXS1, TUBB2B, SLC4A2, SULF1, SLC16A9, CDH2, MAMDC2, ITGA10, ENPP2, NPR3, CDKN2B, TAGLN, INO80C, HTRA1, DHRS3, CTGF, IGFBP7, SERPINE1, PMEPA1, MRPS6, APOE, SMPD1, TPM1, STXBP2, TMEM108, IQCG, COL4A1, SPINT2, MXD3, TPD52L1, HEYL, CDH6,* and *CYR61*; brain vascular pericytes: *EFHD1, CCDC3: , CFH , ZIC2, TIMP4, SYNM , AIF1L, ITGA1, EBF1, DCN, H19*, *DYSF , EDNRA , ND UFA4L2 , ACTG2, COL3A1, CSPG4 , PRRX1, IFGA10, TMEFF2, TAGLN, ZBTB46 , HTRA1, IGFBP7 , ITGA8, APOE , OLAH , IGDCC4, FBLN1, COL4A2, GGT5, MW1C1, COL4A1, RFTN2, STC2, IGFBP1, 7MEM119,* and *CDH6;* lens epithelium: *DKK1, DKK2, DKK3,* and *FAM198B.*

### Example 6: Smooth Muscle Cells from Clonal Progenitor Cell Line

The cell line of the present invention designated 7PEND12 (P13) in the undifferentiated state expressed the markers: *NR4A2, COL4A6, HGF, ELA2, GPR116, HAND2, VGF,* and *FOXF1* and when cultured in micromass conditions for 14 days supplemented with 10 ng/ml BMP4 expressed markers of smooth muscle including enteric smooth muscle actin ACTG2 (accession number NM_001615.3; Illumina probe ID number ILMN_1795325), transgelin *TAGLN* (accession number NM_003186.3; Illumina probe ID number ILMN_2400935), *FILIP1L* (accession number NM_182909.2; Illumina probe ID number ILMN_1730906), *MYH11* (accession number NM_002474.2; Illumina probe ID number ILMN_1660086); the smooth muscle LIM protein *CSRP2* (accession number NM_001321.1; Illumina probe ID ILMN_1660806) reported to be expressed at relatively high levels in aortic smooth muscle and to play a role in embryonic vasculogenesis and heart formation *(*J Biol Chem. 1996 Apr 26;271(17):10194-9). Therefore, the line 7PEND24, or cells such as clonal or oligoclonal lines or heterogeneous cultures of cells derived from pluripotent stem cells with these gene expression markers in the undifferentiated state when differentiated into smooth muscle, are useful in repairing damaged large arteries such as the aorta, or when cultured in combination with vascular endothelial cells or generating tissue engineered arteries such as aorta for the repair of aneurysms or other similar uses in the surgical repair of large arteries. In the examples of research and therapeutic formulations, said cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices.

### EXAMPLE 7: Cartilage Expressing Cells from Clonal Progenitor Cell Line

The cell line SM22 in the undifferentiated state expressed the gene expression markers *TH* (accession number NM_199293.2, Illumina ID 1990068), *CDH18* (accession number NM_004934.2, Illumina ID 7330561), *FGF13* (accession number NM_004114.2, Illumina ID 7380239), and *ZIC4* (accession number NM_032153.3, Illumina ID 4490288), and the line expressed the HOX genes *HOXA2* and *HOXB2.* The cell line SM22 did not express the genes *CST1* (accession number NM_001898.2, Illumina ID 6370541) or *MKX* (accession number NM_173576.1, Illumina ID 6620017) as measured by the aforementioned Illumina gene expression microarray.

When the line SM22 at P12 was cultured in micromass conditions supplemented with 50 ng/ml BMP2 and 10 ng/ml TGFβ3, it expressed markers of cartilage including *COL2A1, PRG4,* and *COL9A2,* but also relatively high levels of the elastic cartilage markers *ELN* and *ACTA2.* This is surprising since MSCs express abundant *COL2A1* transcript in the presence of TGF beta family members alone when cultured in micromass conditions, and the line SM22 required an additional BMP family member such as BMP2. The line or clonal or oligoclonal cells or heterogeneous cultures of cells with these and other markers described herein for the line, is therefore useful in repairing damaged elastic cartilage including that of the outer ear, trachea, intervertebral disc, or nose, as well as generating tissue engineered elastic cartilage such as for repair of the outer ear, trachea, intervertebral disc, or nose.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for therapeutic and research applications.

### EXAMPLE 8: Corneal Endothelium from Clonal Progenitor Cell Line

The cell line T42 in the undifferentiated state expressed the gene expression markers at 13-21 doublings of clonal expansion: *EPHA5* (accession number NM_004439.4, Illumina probe ID 5360408), *HEY2* (accession number NM_012259.1, Illumina probe ID 2350685), *KCNIP1* (accession number NM_001034838.1, Illumina probe ID 6960259), *KRT17* (accession number NM_000422.1, Illumina probe ID 3840445), *MKX* (accession number NM_173576.1, Illumina probe ID 6620017) and does not express the *HOX* genes *HOXA2* or *HOXB2.* The cell line T42 also expressed the gene *OLFML1* (accession number NM_198474.2, Illumina probe ID 130390) and *WDR72* (accession number NM_182758.1, Illumina ID 2810451).

When the line T42 at P20 when cultured in micromass conditions supplemented with 50 ng/ml BMP2 and 10 ng/ml TGFβ3, it expressed markers of corneal endothelium, including *ANGPTL7* (Illumina probe ID number ILMN_1813361) and *COL8A2* (Illumina probe ID number ILMN_2102330). The line is therefore useful in generating corneal endothelial cells or corneal endothelial progenitor cells useful in repairing injured or diseased cornea *in vivo* by injection into the anterior chamber of the eye, or for the tissue engineering of corneas in vitro in conjunction with corneal epithelium and a matrix or blebs of corneal tissue derived from pluripotent stem cell cultures that would otherwise lack the neural crest-derived endothelial cell layer.

### EXAMPLE 9: Additional clonal human embryonic progenitor cell lines with osteochondral potential when differentiated in HyStem-C in the presence of BMP4 and TGFβ3.

Additional clonal human embryonic progenitor cell lines previously disclosed (See US Patent Application Serial Nos. 12/504,630; 13/456,400) and incorporated by reference were differentiated for either 14 or 21 days in the presence of HyStem-C (BioTime, Inc. Alameda, CA) hydrogel as described herein supplemented with combinations of the TGF beta family. The cell line EN18 at passage 13 displayed the gene expression markers: *CST1* (accession number NM_001898.2, Illumina probe ID 6370541), *FOXF1* (NM_001451.2), *TBX1* (accession number NM_080647.1, Illumina ID 460575), and the HOX genes HOXA2 and HOXB2. However, the line EN18 did not express *NEFM* (accession number NM_005382.1) or *ZIC2* (accession number NM_007129.2).

When the cell line EN18 was cultured for 21 days in HyStem with 10 ng/mL of TGFβ3 together with BMP4 (10ng/mL), the differentiated cells expressed relatively high levels of *COL2A1* and *ACAN,* but relatively low levels of the bone sialoprotein IBSP and ALPL. Surprisingly, while there was no evidence of *COL2A1* expression in EN18 in the presence of to TGFB3 in micromass conditions as described (Sternberg et al, A human embryonic stem cell-derived clonal progenitor cell line with chondrogenic potential and makers of craniofacial mesenchyme. Regen Med. 2012 Apr 23. [Epub ahead of print], 2012), indeed, only seven of 100 clonal progenitor cell lines responded to TGFβ3 in micromass conditions with chondrogenic differentiation, nevertheless, in the presence of 10 ng/mL of BMP4 together with 10 ng/mL of TGFβ3, in HyStem, there was a robust expression of *COL2A1* and related cartilage gene expression as determined by Illumina microarray analysis at 21 days of differentiation. The line is therefore useful in research and in regenerating cartilage and for undergoing endochondral ossification to repair cartilage and bone. The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line EN42 at passage 12 displayed the gene expression markers: *FOXF1* (accession number NM_001451.2, Illumina Probe ID 3800554), *PAX9* (accession number NM_006194.2, Illumina ID 6350138), *WDR72* (accession number NM_182758.1, Illumina ID 2810451) and the HOX genes *HOXA2* and *HOXB2.* However, the line EN42 did not express *NEFM* (accession number NM_005382.1, Illumina probe ID 1660767) or *ZIC2* (accession number NM_007129.2).

When the cell line EN42 at passage 12 was cultured for 21 days in HyStem with 10 ng/mL of TGFβ3 together with BMP4 (10ng/mL), the differentiated cells expressed relatively high levels of *COL2A1, ACAN,* elastin *(ELN),* and lubricin *(PRG4),* but relatively low levels of the bone sialoprotein *IBSP* and *ALPL.* Surprisingly, while there was no evidence of *COL2A1* expression in EN42 in the presence of to TGFB3 in micromass conditions as described (Sternberg et al, A human embryonic stem cell-derived clonal progenitor cell line with chondrogenic potential and makers of craniofacial mesenchyme. Regen Med. 2012 Apr 23. [Epub ahead of print], 2012), indeed, only seven of 100 clonal progenitor cell lines responded to TGFβ3 in micromass conditions with chondrogenic differentiation, nevertheless, in the presence of 10 ng/mL of BMP4 together with 10 ng/mL of TGFβ3, in HyStem, there was a robust expression of *COL2A1* and related cartilage gene expression as determined by Illumina microarray analysis at 21 days of differentiation. The high levels of *ELN* and *PRG4* expression by the line makes it particularly useful in repairing damaged elastic cartilage including that of the outer ear, trachea, intervertebral disc, or nose, as well as generating tissue engineered elastic cartilage such as for repair of the outer ear, trachea, intervertebral disc, or nose.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for therapeutic and research applications.

The cell line E120 at passage 13 displayed the gene expression markers: *GJB2* (NM_004004.4, Illumina ID 5260095), *MKX* (accession number NM_173576.1, Illumina ID 6620017), *PAX8* (accession number NM_003466.3, Illumina ID 1090451), MX2 (accession number NM_002463.1, Illumina ID 5490470) and the line E120 did not express the *HOX* genes *HOXA2* and *HOXB2* and did not express *UGT2B7* (accession number XM_001128725.1, Illumina ID 1190064).

When the cell line E120 at passage 15 was cultured for 21 days in HyStem with 10 ng/mL of TGFβ3 together with BMP4 (10ng/mL), the differentiated cells expressed relatively high levels of *COL2A1,* as well as the bone differentiation markers *IBSP* and *ALPL.* Surprisingly, while there was no evidence of *COL2A1* expression in E120 in the presence of to TGFB3 in micromass conditions as described (Sternberg et al, A human embryonic stem cell-derived clonal progenitor cell line with chondrogenic potential and makers of craniofacial mesenchyme. Regen Med. 2012 Apr 23. [Epub ahead of print], 2012), indeed, only seven of 100 clonal progenitor cell lines responded to TGFβ3 in micromass conditions with chondrogenic differentiation, nevertheless, in the presence of 10 ng/mL of BMP4 together with 10 ng/mL of TGFβ3, in HyStem, there was a robust expression of *COL2A1* and related cartilage and bone gene expression as determined by Illumina microarray analysis at 21 days of differentiation. The high levels of bone markers as measured by Illumina microarrays in the cell line E120 when cultured for 21 days in HyStem with 10 ng/mL of TGFβ3 together with BMP4 (10ng/mL) indicate that the cells when differentiated in the presence of TGFb3 and BMP4 are capable of robust endochondral ossification. These mineralization markers are evidence of the novel use of the line E120 to induce high levels of ossification for use in regenerating bone for the treatment of osteoporosis, bone fractures, fusion of bones such as in the fusion of vertebrates, osteonecrosis, and other applications where the induction of new bone is therapeutic.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line T20 at passage 10 displayed the gene expression markers: *CD24* (NM_013230.2, Illumina ID 610437), *TFAP2C* (accession number NM_003222.3, Illumina ID 6450075), NTN4 (accession number NM_021229.3, Illumina ID 3190021), GAP43 (accession number NM_002045.2, Illumina ID 4670204), S100A6 (accession number NM_014624.3, Illumin ID 2810315), and the line E120 did not express the *HOX* gene *HOXA2* and did not express *IAH1* (accession number NM_001039613.1, Illumina ID 1300743).

When the cell line T20 at passage 13 was cultured for 21 days in HyStem with 10 ng/mL of TGFβ3 together with BMP4 (10ng/mL), the differentiated cells expressed relatively high levels of *COL2A1,* as well as the bone differentiation markers *IBSP* and *ALPL.* Surprisingly, while there was no evidence of *COL2A1* expression in T20 in the presence of to TGFB3 in micromass conditions as described (Sternberg et al, A human embryonic stem cell-derived clonal progenitor cell line with chondrogenic potential and makers of craniofacial mesenchyme. Regen Med. 2012 Apr 23. [Epub ahead of print], 2012), indeed, only seven of 100 clonal progenitor cell lines responded to TGFβ3 in micromass conditions with chondrogenic differentiation, nevertheless, in the presence of 10 ng/mL of BMP4 together with 10 ng/mL of TGFβ3, in HyStem, there was a robust expression of *COL2A1* and related cartilage and bone gene expression as determined by Illumina microarray analysis at 21 days of differentiation. The high levels of endochondral ossification markers as measured by Illumina microarrays in the cell line T20 when cultured for 21 days in HyStem with 10 ng/mL of TGFβ3 together with BMP4 (10ng/mL) indicate that the cells when differentiated in the presence of TGFb3 and BMP4 are capable of robust endochondral ossification. These mineralization markers are evidence of the novel use of the line T20 to induce high levels of ossification for use in regenerating bone for the treatment of osteoporosis, bone fractures, fusion of bones such as in the fusion of vertebrates, osteonecrosis, and other applications where the induction of new bone is therapeutic.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line SK50 at passage 9 displayed the gene expression: *SPP1* (accession number NM_000582.2, Illumina ID 3460070), *DES* (accession number NM_001927.3, Illumina ID 10296), *ZIC2* (accession number NM_007129.2, Illumina ID 510368), *ACP5* (accession number NM_001611.2, Illumina ID 7050082), *MT3* (accession number NM_005954.2, Illumin ID 3060273), and the *HOX* genes *HOXA2* and *HOXB2,* but the line SK50 did not express *NNAT* (accession number NM_181689.1, Illumina ID 4010709).

When the cell line SK50 at passage 9 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed relatively high levels of *COL2A1,* as well as the bone differentiation markers osteopontin *(SPP1), IBSP* and *ALPL.* Surprisingly, while there was no evidence of *COL2A1* expression in SK50 in the presence of to TGFB3 in micromass conditions as described (Sternberg et al, A human embryonic stem cell-derived clonal progenitor cell line with chondrogenic potential and makers of craniofacial mesenchyme. Regen Med. 2012 Apr 23. [Epub ahead of print], 2012), indeed, only seven of 100 clonal progenitor cell lines responded to TGFβ3 in micromass conditions with chondrogenic differentiation, nevertheless, in the presence of 10 ng/mL of BMP4 together with 10 ng/mL of TGFβ3, or 50 ng/mL of BMP2 together with 10 ng/mL of TGFβ3in HyStem, there was a robust expression of *COL2A1* and related cartilage and bone gene expression as determined by Illumina microarray analysis at 21 days of differentiation. The high levels of endochondral ossification markers as measured by Illumina microarrays in the cell line SK50 when cultured for 21 days in HyStem with the aforementioned growth factors, indicate that the cell line has the surprising potential for robust endochondral ossification. These mineralization markers are evidence of the novel use of the line SK50, or pluripotent stem cell-derived cells with similar markers, to induce high levels of ossification for use in regenerating bone for the treatment of osteoporosis, bone fractures, fusion of bones such as in the fusion of vertebrates, osteonecrosis, and other applications where the induction of new bone is therapeutic.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line SK44 at passage 9 displayed the gene expression markers: *SPP1* (accession number NM_000582.2, Illumina ID 3460070), *DES* (accession number NM_001927.3, Illumina ID 10296), *ZIC2* (accession number NM_007129.2, Illumina ID 510368), *WDR72* (accession number NM_182758.1, Illumina ID 2810451), but unlike the cell line SK50 described above, the cell line SK44 did not express *MT3* (accession number NM_005954.2, Illumin ID 3060273) or the *HOX* gene *HOXA2.*

When the cell line SK44 at passage 9 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed relatively high levels of *COL2A1,* and expressed, though at lower levels than the cell line SK50 the bone differentiation markers *IBSP and ALPL.* Surprisingly, while there was no evidence of *COL2A1* expression in SK44 in the presence of to TGFB3 in micromass conditions as described (Sternberg et al, A human embryonic stem cell-derived clonal progenitor cell line with chondrogenic potential and makers of craniofacial mesenchyme. Regen Med. 2012 Apr 23. [Epub ahead of print], 2012), indeed, only seven of 100 clonal progenitor cell lines responded to TGFβ3 in micromass conditions with chondrogenic differentiation, nevertheless, in the presence of 10 ng/mL of BMP4 together with 10 ng/mL of TGFβ3, or 50 ng/mL of BMP2 together with 10 ng/mL of TGFβ3in HyStem, there was a robust expression of *COL2A1* and related cartilage and bone gene expression as determined by Illumina microarray analysis at 21 days of differentiation. The high levels of endochondral ossification markers as measured by Illumina microarrays in the cell line SK44 when cultured for 21 days in HyStem with the aforementioned growth factors, indicate that the cell line has the surprising potential for robust endochondral ossification. These mineralization markers are evidence of the novel use of the line SK44, or pluripotent stem cell-derived cells, including clonal or oligoclonal cell lines with similar markers, to induce high levels of ossification for use in regenerating bone for the treatment of osteoporosis, bone fractures, fusion of bones such as in the fusion of vertebrates, osteonecrosis, and other applications where the induction of new bone is therapeutic.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line E44 at passage 20 displayed the gene expression markers: *PROM1* (accession number NM_006017.1, Illumina ID 7400452), *S100A6* (accession number NM_014624.3, Illumina ID 2810315), *IAH1* (accession number NM_001039613.1, Illumina ID 1300743), and *ZIC2* (accession number NM_007129.2, Illumina ID 510368), but did not express *NEFM* (accession number NM_005382.1, Illumin ID 1660767) or the *HOX* gene *HOXA2.*

When the cell line E44 at passage 20 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed relatively low but detectable levels of *COL2A1* transcript, the tendon marker *TNMD* (accession number NM_022144.1, Illumina ID 290445), and expressed low to undetectable levels of *IBSP* and *ALPL.* Surprisingly, while there was no evidence of *COL2A1* expression in E44 in the presence of to TGFB3 in micromass conditions as described (Sternberg et al, A human embryonic stem cell-derived clonal progenitor cell line with chondrogenic potential and makers of craniofacial mesenchyme. Regen Med. 2012 Apr 23. [Epub ahead of print], 2012), indeed, only seven of 100 clonal progenitor cell lines responded to TGFβ3 in micromass conditions with chondrogenic differentiation, nevertheless, in the presence of 10 ng/mL of BMP4 together with 10 ng/mL of TGFβ3, or 50 ng/mL of BMP2 together with 10 ng/mL of TGFβ3 in HyStem, there was an induction of expression of *COL2A1* and related cartilage and tendon/ligament gene expression as determined by Illumina microarray analysis at 21 days of differentiation. The high levels of cartilage and tendon/ligament markers as measured by Illumina microarrays in the cell line E44 when cultured for 21 days in HyStem with the aforementioned growth factors, indicate that the cell line has the surprising potential for the use of the line E44, or pluripotent stem cell-derived cells, including clonal or oligoclonal cell lines with similar markers, to induce high levels of ossification for use in regenerating cartilage and joint ligaments for the treatment of tears of ligament and associated cartilage and bone, and other applications where the induction of such tissue is therapeutic.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line T7 at passage 16 displayed the gene expression markers: *PROM1* (accession number NM_006017.1, Illumina ID 7400452), *S100A6* (accession number NM_014624.3, Illumina ID 2810315), *IAH1* (accession number NM_001039613.1, Illumina ID 1300743), and *ZIC2* (accession number NM_007129.2, Illumina ID 510368), but did not express *NEFM* (accession number NM_005382.1, Illumin ID 1660767) or the *HOX* gene *HOXA2.*

When the cell line T7 at passage 13 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed relatively low but detectable levels of *COL2A1* transcript, and expressed no detectable levels of *IBSP* and did express *ALPL.* Surprisingly, while there was no evidence of *COL2A1* expression in T7 in the presence of to TGFB3 in micromass conditions as described (Sternberg et al, A human embryonic stem cell-derived clonal progenitor cell line with chondrogenic potential and makers of craniofacial mesenchyme. Regen Med. 2012 Apr 23. [Epub ahead of print], 2012), indeed, only seven of 100 clonal progenitor cell lines responded to TGFβ3 in micromass conditions with chondrogenic differentiation, nevertheless, in the presence of 10 ng/mL of BMP4 together with 10 ng/mL of TGFβ3, or 50 ng/mL of BMP2 together with 10 ng/mL of TGFβ3 in HyStem, there was an induction of expression of *COL2A1* and related cartilage gene expression as determined by Illumina microarray analysis at 21 days of differentiation. The high levels of cartilage markers as measured by Illumina microarrays in the cell line T7 when cultured for 21 days in HyStem-C (BioTime, Inc. Alameda, CA) with the aforementioned growth factors, indicate that the line T7, or pluripotent stem cell-derived cells, including clonal or oligoclonal cell lines with the above-mentioned markers, has the potential to induce high levels of cartilage formation and is therefore useful in research to study cell differentiation, screening for drugs that modify chondrogenesis, and other applications where the induction of such tissue is therapeutic such as in trauma or degenerative diseases of cartilaginous tissues such as osteoarthritis of the knee and hip, meniscal tears, or repair of the intervertebral disc.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line F15 at passage 18 displayed the gene expression markers: *NTN4* (accession number NM_021229.3, Illumina ID 3190021), *S100A6* (accession number NM_014624.3, Illumina ID 2810315), *TRIM4* (accession number NM_033091.1, Illumina ID 2810674), and *NPTX1* (accession number NM_002522.2, Illumina ID 6100468), but did not express *AJAP1* (accession number NM_018836.3, Illumin ID 1300647) or ZIC2 (accession number NM_007129.2, Illumina ID 510368). The line F15 expressed low but detectable levels of the gene CD74 similar to the abundant expression of CD74 in cultured MSCs, however numerous genes were observed to be strikingly differently expressed in F15 compared to MSCs grown in the same culture conditions. By way of non-limiting example, MSCs abundantly expressed proenkephalin *(PENK)* whereas the cell line F15 did not express *PENK.* And the cell line F15 abundantly expressed the gene *ACTG2,* whereas MSCs in the same conditions did not express detectable *ACTG2.*

When the cell line F15 at passage 18 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed relatively high levels of *COL2A1* transcript and expressed no detectable levels of *IBSP* or *ALPL.* Surprisingly, while there was no evidence of *COL2A1* expression in F15 in the presence of to TGFB3 in micromass conditions as described (Sternberg et al, A human embryonic stem cell-derived clonal progenitor cell line with chondrogenic potential and makers of craniofacial mesenchyme. Regen Med. 2012 Apr 23. [Epub ahead of print], 2012), indeed, only seven of 100 clonal progenitor cell lines responded to TGFβ3 in micromass conditions with chondrogenic differentiation, nevertheless, in the presence of 10 ng/mL of BMP4 together with 10 ng/mL of TGFβ3, or 50 ng/mL of BMP2 together with 10 ng/mL of TGFβ3 in HyStem, there was an induction of expression of *COL2A1* and related cartilage gene expression with no detectable osteogenic markers such as *ALPL* and *IBSP* as determined by Illumina microarray analysis at 21 days of differentiation. When cultured in HyStem-C (BioTime, Inc. Alameda, CA) for 21 days in the presence of 10 ng/mL of BMP4 but without 10 ng/mL of TGFβ3, the cell line did not express *COL2A1* but instead expressed relatively high levels of *TNMD,* a marker of tendons and ligaments. The high levels of cartilage markers as measured by Illumina microarrays in the cell line F15 when cultured for 21 days in HyStem with the aforementioned growth factors, indicate that the cell line has the surprising potential for the use of the line F15, or pluripotent stem cell-derived cells, including clonal or oligoclonal cell lines with these markers, to induce cartilage differentiation in the absence of ossification for use in regenerating cartilage in joints, intervertebral joints, or other cartilaginous tissues, and other applications where the induction of such tissue is therapeutic, or alternatively, when differentiated in the presence of BMP4 only as described herein, to differentiate into tissues with high tensile strength such as tendon and ligament useful in the repair of such tissues damaged by injury or disease.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line SK5 at passage 12 displayed the gene expression markers: *HOXA10* (accession number NM_153715.2, Illumina ID 3290427), *S100A6* (accession number NM_014624.3, Illumina ID 2810315), *SPP1* (accession number NM_000582.2, Illumina ID 3460070), the lateral plate mesoderm marker *HOXB6* (Accession number NM_018952.4, Illumina ID 6220189), and *WDR72* (accession number NM_182758.1, Illumina ID 2810451), but did not express *TFAP2C* (accession number NM_003222.3, Illumin ID 6450075) or *PITX1* (accession number NM_002653.3, Illumina ID 2000373). In addition to *HOXB6* expression, the line expressed relatively distal *HOX* genes such as *HOXB7* and *HOXC8* similar to cultured MSCs from the iliac crest. However, the cell line SK5 was markedly distinct from MSCs cultured in the same conditions in that MSCs abundantly expressed the gene proenkephalin *(PENK)* while the cell line SK5 in the same culture conditions did not express *PENK.* In addition, the cell line SK5 expressed the gene *APOE,* while MSCs in the same culture conditions did not express *APOE.*

We examined diverse differentiation conditions to determine whether conditions could be identified wherein the cells would commit to hind limb bud differentiation as evidenced by the expression of *PITX1.* We observed that the culture of the line SK5 at passage 12 in HyStem-C (BioTime, Inc. Alameda, CA) supplemented with 1.0 uM all-trans retinoic acid, induced the expression *of PITX1.*

When the cell line SK5 at passage 12 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed relatively low but detectable levels of *COL2A1* transcript and expressed very high levels of *TNMD,* a marker of tendon and ligaments. Surprisingly, while there was no evidence of *COL2A1* expression in SK5 in the presence of to TGFB3 in micromass conditions as described (Sternberg et al, A human embryonic stem cell-derived clonal progenitor cell line with chondrogenic potential and makers of craniofacial mesenchyme. Regen Med. 2012 Apr 23. [Epub ahead of print], 2012), indeed, only seven of 100 clonal progenitor cell lines responded to TGFβ3 in micromass conditions with chondrogenic differentiation, nevertheless, in the presence of 10 ng/mL of BMP4 together with 10 ng/mL of TGFβ3, or 50 ng/mL of BMP2 together with 10 ng/mL of TGFβ3 in HyStem, there was an induction of expression of *COL2A1* as well as *TNMD* as determined by Illumina microarray analysis at 21 days of differentiation. When cultured in HyStem-C (BioTime, Inc. Alameda, CA) for 21 days in the presence of 10 ng/mL of BMP4 but without 10 ng/mL of TGFβ3, the cell line did not express *COL2A1* and expressed relatively low levels of TNMD, and instead upregulated the expression of *SILV,* a marker of preosteogenic mesenchyme. The markers of caudal lateral plate mesoderm, inducibility of *PITX1,* and multipotency of the line SK5 or hPS-derived cells including clonal, oligoclonal, or pooled clonal or oligoclonal lines with the aforementioned markers, capable of differentiating into muscle, cartilage, tendon, or bone, indicate that the cell line has the surprising potential for multiple applications in research in lateral plate mesoderm differentiation and cell-based therapy where the induction of the corresponding tissues are therapeutic, such as when differentiated in the presence of BMP4 and TGFβ3 as described herein, to differentiate into tissues with high tensile strength such as tendon and ligament useful in the repair of such tissues damaged by injury or disease.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line E72 at passage 11 displayed the gene expression markers: *HOXA10* (accession number NM_153715.2, Illumina ID 3290427), *POSTN* (accession number NM_006475.1, Illumina ID 510246), *KRT34* (accession number NM_021013.3, Illumina ID 3710168), *MKX* (accession number NM_173576.1, Illumina ID 6620017), *HAND2* (accession number NM_021973.2, Illumina probe ID 4640563), the relatively rarely-expressed *HOX* gene *HOXD11* (accession number NM_021192.2, Illumina probe ID 5290142) implicated in forelimb development, and *TBX15* (accession number NM_152380.2, Illumina probe ID 6060113), but did not express *LHX8* (accession number NM_001001933.1, Illumina ID 2900343), *FOXF2* (accession number NM_001452.1, Illumina ID 1660470), *AJAP1* (accession number NM_018836.3, Illumina ID 1300647), *PLXDC2* (accession number NM_032812.7, Illumina ID 5900497), *DLK1* (accession number NM_003836.4, Illumina ID 6510259), or the lateral plate mesoderm marker *HOXB6* (Accession number NM_018952.4, Illumina ID 6220189). In addition to not expressing *HOXB6,* the line did not express relatively distal *HOX* genes such as *HOXB7* (accession number NM_004502.2, Illumina probe ID 2470328), and *HOXC8* (accession number NM_022658.3, Illumina probe ID 4640059) expressed by cultured MSCs from the iliac crest, or the HOX genes *HOXC9, HOXC10,* or *HOXC11* expressed in hindlimb, but not forelimb bud mesenchyme. The expression of *HOXA10* (a marker of forelimb and hindlimb bud mesenchyme, but the lack of many distal *HOX* genes such as *HOXB7* or *HOXC8,* and the lack of expression of *HOXC9, HOXC10,* or *HOXC11* provides evidence of the commitment of the cell line E72 or cells with the same gene expression markers of being forelimb bud mesenchyme.

When the cell line E72 at passage 11 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed relatively high levels of markers of endochondral ossification including *COL2A1, ALPL, IBSP,* and osteopontin *(SPP1*), such expression of osteogenic markers being comparable to early passage differentiating cultured MSCs.

We observed that the culture of the line E72 at passage 11 in HyStem-C (BioTime, Inc. Alameda, CA) supplemented with 1.0 uM all-trans retinoic acid, induced the expression of *HOXB6,* a marker of lateral plate mesenchyme. The lack of distal *HOX* gene expression such as *HOXB7* or *HOXC8,* the expression of *HOXA10* and *HOXD11,* and the inducibility of *HOXB6* with retinoic acid provide evidence that the line E72 is mesodermal with potential to develop into forelimb bud mesenchyme. In addition, and the line was unusual in that when differentiated in HyStem in the presence of BMP4 and TGFβ3 or BMP4 and TGFβ3 as described herein, the line expressed the markers normally associated with enamel, including enamelin *(ENAM,* accession number NM_031889.1, Illumina probe ID 7160598) and amelogenin *(AMELX,* accession number NM_001142.2, Illumina probe ID). When the line E72 was at passage 12 was cultured for 21 days in HyStem with 10 ng/mL of BMP4, the differentiated cells expressed relatively high levels of markers of adipocyte markers *FABP4* and *CD36.* Such lateral plate mesoderm progenitors, especially those from the forelimb region, are useful in the production of brown fat cells and cells that expression lipasin, a regulator of lipid metabolism and beta cell proliferation. When differentiated in BMP4 and TGFβ3, these marked osteogenic markers as well as markers of hard bone such as *ENAM,* or differentiated in BMP4 only to yield lipasin-expressing adipocytes, provide a useful and unique research model of osteogenesis as well a scalable source of novel cells useful in the repair of bone, such as conditions of osteonecrosis, fractures, repair of bone following surgical resection of tumors, osteoporosis, and spinal vertebrae fusion and for the scalable production of lipasin-expressing brown fat cells useful in the regulation of lipids and beta cell proliferation, the later being useful in the treatment of both type I and II diabetes.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications. For example the cells may be used in transplantation for the treatment of lipid disorders, such as for the treatment of hyperlipidemia or for the induction of beta cell proliferation as a therapeutic modality for type I or type II diabetes and formulated in HyStem-C (BioTime, Inc. Alameda, CA) and transplanted subcutaneously at dosages calculated to cause a therapeutically useful reduction in lipids or induction of beta cells and associated insulin.

The cell lines E75 and E163 at passage 11 and 12 respectively, displayed gene expression markers similar but slightly different from E72. The line E75 expressed: *HOXA10* (accession number NM_153715.2, Illumina ID 3290427) abundantly expressed in forelimb and hindlimb bud mesenchyme, *POSTN* (accession number NM_006475.1, Illumina ID 510246), *KRT34* (accession number NM_021013.3, Illumina ID 3710168), *MKX* (accession number NM_173576.1, Illumina ID 6620017), *HAND2* (accession number NM_021973.2, Illumina probe ID 4640563), the relatively rarely-expressed *HOX* gene *HOXD11* (accession number NM_021192.2, Illumina probe ID 5290142) implicated in forelimb development, and *TBX15* (accession number NM_152380.2, Illumina probe ID 6060113), but unlike the line E72 which did not express *PLXDC2* (accession number NM_032812.7, Illumina probe ID 5900497), the lines E75 and E163 did express *PLXDC2.* The line E75 did not express *LHX8* (accession number NM_001001933.1, Illumina ID 2900343), *FOXF2* (accession number NM_001452.1, Illumina ID 1660470), *AJAP1* (accession number NM_018836.3, Illumina ID 1300647), or *DLK1* (accession number NM_003836.4, Illumina ID 6510259), or the lateral plate mesoderm marker *HOXB6* (Accession number NM_018952.4, Illumina ID 6220189). In addition to not expressing *HOXB6,* the line did not express relatively distal *HOX* genes such as *HOXB7* (accession number NM_004502.2, Illumina probe ID 2470328), and *HOXC8* (accession number NM_022658.3, Illumina probe ID 4640059) expressed by cultured MSCs from the iliac crest, or the HOX genes *HOXC9, HOXC10,* or *HOXC11* expressed in hindlimb, but not forelimb bud mesenchyme.

We observed that the culture of the line E72 at passage 11 in HyStem-C (BioTime, Inc. Alameda, CA) supplemented with 1.0 uM all-trans retinoic acid, induced the expression of *HOXB6,* a marker of lateral plate mesenchyme. The lack of distal *HOX* gene expression such as *HOXB7* or *HOXC8,* the expression of *HOXA10* (a marker of forelimb and hindlimb bud mesenchyme), and *HOXD11* (being a marker of forelimb bud mesenchyme), the lack of expression of distal HOX genes including *HOXC9, HOXC10,* or *HOXC11,* and the inducibility of *HOXB6* with retinoic acid provide evidence that the line E72 is mesodermal with potential to develop into forelimb bud mesenchyme.

When the cell line E75 at passage 11 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed relatively high levels of markers of endochondral ossification including *COL2A1, ALPL, IBSP,* and osteopontin *(SPP1),* such expression of osteogenic markers being comparable to early passage differentiating cultured MSCs. In addition, and the line was unusual in that when differentiated in HyStem in the presence of BMP4 and TGFβ3 or BMP4 and TGFβ3 as described herein, the line expressed the markers normally associated with enamel, including enamelin *(ENAM,* accession number NM_031889.1, Illumina probe ID 7160598) and amelogenin (AMELX, accession number NM_001142.2, Illumina probe ID). Unlike the line E72 described herein, the line E75 in differentiated for 21 days in the presence of HyStem and BMP4 and TGFβ3 as described herein, expressed the additional marker normally associated with enamel called ameloblastin (AMBN) (accession number NM_016519.4, Illumina probe ID 6400438).

When the lines E75 and E163 at passages 11 and 12 respectively were cultured for 21 days in HyStem with 10 ng/mL of BMP4, the differentiated cells expressed relatively high levels of markers of adipocyte markers *FABP4* and *CD36.* Such lateral plate mesoderm progenitors, especially those from the forelimb region, are useful in the production of brown fat cells and cells that expression lipasin, a regulator of lipid metabolism and beta cell proliferation. These marked osteogenic markers as well as markers of hard bone such as present in the enamel of teeth in one differentiation condition, or alternatively in different differentiation conditions the markers of adipocyte differentiation from the interscapular region of the back, provide a useful and unique research model of osteogenesisand adipogenesis, in particular of lipasin-secreting adipocytes, as well a scalable source of novel cells useful in the repair of bone, such as conditions of osteonecrosis, fractures, repair of bone following surgical resection of tumors, osteoporosis, and spinal vertebrae fusion and for the scalable production of brown fat cells and cells capable of secreting lipasin useful in the regulation of lipids and beta cell proliferation, the later being useful in the treatment of both type I and II diabetes.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications. For example for transplantation of cells for the treatment of lipid disorders, such as for the treatment of hyperlipidemia or for the induction of beta cell proliferation as a therapeutic modality for type I or type II diabetes, the cells may be formulated in HyStem-C (BioTime, Inc. Alameda, CA) and transplanted subcutaneously at dosages calculated to cause a therapeutically useful reduction in lipids or induction of beta cells and associated insulin.

The cell line 4D20.9 at passage 12 displayed the gene expression markers: *WDR72* (accession number NM_182758.1, Illumina ID 2810451), *PITX1* (accession number NM_002653.3, Illumina ID 2000373), *MSX2* (accession number NM_002449.4, Illumina ID 5960243), *TFAP2C* (accession number NM_003222.3, Illumina ID 6450075), *HOXA2* (accession number NM_006735.3, Illumina ID 2060471), *HOXB2* (accession number NM_002145.3, Illumina ID 3460097), but did not express *FOXF2* (accession number NM_001452.1, Illumina ID 1660470), or *CD24* (accession number NM_013230.2, Illumina probe ID 610437).

When the cell line 4D20.9 at passage 12 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed abundant levels of *COL2A1* transcript as well as transcript for the osteogenic marker *IBSP.* When cultured in HyStem-C (BioTime, Inc. Alameda, CA) for 21 days in the presence of 10 ng/mL of BMP4 but without 10 ng/mL of TGFβ3, the cell line expressed essentially undetectable levels of *COL2A1* and instead expressed relatively high levels of adipocyte markers such as *FABP4* and *CD36.*

The cell line 4D20.9 or cells with the markers described herein for the line, or pluripotent stem cell-derived clonal, oligoclonal, or pooled clonal or oligoclonal lines with the aforementioned markers, capable of differentiating into cartilage, bone, or adipose tissue, make the cells useful in research in cellular differentiation and cell-based therapy where the induction of the aforementioned cell types are therapeutic, such in the repair of cartilage and bone, or reconstruction of subcutaneous fat.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line E19 at passage 11 displayed the gene expression markers: *CD24* (accession number NM_013230.2, Illumina probe ID 610437), *PLXDC2* (accession number NM_032812.7, Illumina ID 5900497), *MKX* (accession number NM_173576.1, Illumina ID 6620017), *KRT17* (accession number NM_000422.1, Illumina probe ID 3840445), *KRT34* (accession number NM_021013.3, Illumina ID 3710168), and *NRG1* (accession number NM_013962.2, Illumina ID 1940128), and negative for the markers: *HOXA2* (accession number NM_006735.3, Illumina ID 2060471), *HOXB2* (accession number NM_002145.3, Illumina ID 3460097), and *UGT2B7* (accession number XM_001128725.1, Illumina ID 1190064).

When the cell line E19 at passage 11 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed abundant levels of *COL2A1* transcript without detectable levels of the osteogenic marker *IBSP.* When cultured in HyStem-C (BioTime, Inc. Alameda, CA) for 21 days in the presence of 10 ng/mL of BMP4 but without 10 ng/mL of TGFβ3, the cell line expressed essentially undetectable levels of *COL2A1* and instead expressed relatively high levels of adipocyte markers such as *FABP4* and *CD36* as well as the choroid plexus marker *TTR.*

The cell line E19, or cells with the markers described herein for the line, or pluripotent stem cell-derived clonal, oligoclonal, or pooled clonal or oligoclonal lines with the aforementioned markers, capable of differentiating into cartilage, choroid plexus, or adipose tissue, are useful to make the cells for use in research in cellular differentiation and for use in cell-based therapy where the induction of the aforementioned cell types are therapeutic, such in the repair of cartilage, or reconstruction of subcutaneous fat, repairing diseased choroid plexus, using such transplanted choroid plexus cells or choroid plexus progenitors for delivering proteins into the cerebral spinal fluid including cases where said proteins are recombinant exogenously introduced constructs to deliver therapeutic proteins into the CSF, and the transplantation of choroid plexus cells or choroid plexus progenitors into aged brain to increase turnover of the CSF to improve the physiology of the brain including the removal of amyloidogenic proteins to prevent or treat Alzheimer's disease.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line T14 at passage 11 displayed the gene expression markers: *WDR72* (accession number NM_182758.1, Illumina ID 2810451), *FOXS1* (accession number NM_004118.3, Illumina probe ID 3610102), *GABRB1* (accession number NM_000812.2, Illumina ID 6130692), and *CD90* (accession number NM_006288.2, Illumina ID 6480204), and negative for the markers: *HOXB2* (accession number NM_002145.3, Illumina ID 3460097), *ZIC2* (accession number NM_007129.2, Illumina ID 510368), and *UGT2B7* (accession number XM_001128725.1, Illumina ID 1190064).

When the cell line T14 at passage 11 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed abundant levels of expression of the cartilage marker *COL2A1* and the osteogenic markers *IBSP, ALPL, and osteopontin (SPP1).* When cultured in HyStem-C (BioTime, Inc. Alameda, CA) for 21 days in the presence of 10 ng/mL of BMP4 but without 10 ng/mL of TGFβ3, the cell line expressed essentially undetectable levels of *COL2A1* and instead expressed relatively high levels of smooth muscle markers such as *MYH11* and *CNN1.*

The cell line T14, or cells with the markers described herein for the line, or pluripotent stem cell-derived clonal, oligoclonal, or pooled clonal or oligoclonal lines with the aforementioned markers, capable of differentiating into cartilage, bone, or smooth muscle tissue, are useful to make the cells for use in research in cellular differentiation and for use in cell-based therapy where the induction of the aforementioned cell types are therapeutic, such in the repair of cartilage and bone, or reconstruction of smooth muscle in diseased tissues or manufactured tissue engineered arteries.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line T44 at passage 11 displayed the gene expression markers: *S100A6* (accession number NM_014624.3, *HEY2* (NM_012259.1, Illumina probe ID 2350685), *FOXS1* (accession number NM_004118.3, Illumina probe ID 3610102), *GABRB1* (accession number NM_000812.2, Illumina ID 6130692), and *CD90* (accession number NM_006288.2, Illumina ID 6480204), and did not express the markers: *HOXB2* (accession number NM_002145.3, Illumina ID 3460097), *ZIC2* (accession number NM_007129.2, Illumina ID 510368), *UGT2B7* (accession number XM_001128725.1, Illumina ID 1190064) and *WDR72* (accession number NM_182758.1, Illumina ID 2810451).

When the cell line T44 at passage 11 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed abundant levels of expression of the cartilage marker *COL2A1* and the osteogenic markers *IBSP, ALPL, and osteopontin (SPP1).* When cultured in HyStem-C (BioTime, Inc. Alameda, CA) for 21 days in the presence of 10 ng/mL of BMP4 but without 10 ng/mL of TGFβ3, the cell line expressed relatively low levels of *COL2A1* and instead expressed relatively high levels of adipocyte markers such as *FABP4* and *CD36.*

The cell line T44, or cells with the markers described herein for the line, or pluripotent stem cell-derived clonal, oligoclonal, or pooled clonal or oligoclonal lines with the aforementioned markers, capable of differentiating into cartilage, bone, or adipose tissue, are useful to make the cells for use in research in cellular differentiation and for use in cell-based therapy where the induction of the aforementioned cell types are therapeutic, such in the repair of cartilage and bone, or reconstruction of subcutaneous fat in diseased tissues.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line U18 at passage 12 displayed the gene expression markers: *TFAP2C* (accession number NM_003222.3, Illumina ID 6450075), *ZIC2* (accession number NM_007129.2, Illumina ID 510368), *WDR72* (accession number NM_182758.1, Illumina ID *2810451), RCAN2* (accession number NM_005822.2, Illumina probe ID 630161), and *TBX1* (accession number NM_080647.1, Illumina ID 460575), and did not express the markers: *HOXA2* (accession number NM_006735.3, Illumina ID 2060471), or *HOXB2* (accession number NM_002145.3, Illumina ID 3460097).

When the cell line U18 at passage 11 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed abundant levels of expression of the cartilage marker *COL2A1* but low to undetectable levels of the osteogenic markers *IBSP* and *ALPL.* When cultured in HyStem-C (BioTime, Inc. Alameda, CA) for 21 days in the presence of 10 ng/mL of BMP4 but without 10 ng/mL of TGFβ3, the cell line expressed relatively low but detectable levels of *COL2A1* as well as cranial perivascular markers such as *MYH11, RGS5,* and *CNN1.*

The cell line U18, or cells with the markers described herein for the line, or pluripotent stem cell-derived clonal, oligoclonal, or pooled clonal or oligoclonal lines with the aforementioned markers, capable of differentiating into cartilage or perivascular cells, are useful to make the cells for use in research in cellular differentiation and for use in cell-based therapy where the induction of the aforementioned cell types are therapeutic, such in the repair of cartilage in the nose, joint surface, ear, and intervertebral joint, and providing support for developing vasculature such as in the vascular choroid of the retina often adversely affected in diabetic retinopathy, or in combination with vascular endothelial cells, such as those produced from pluripotent stem cells to stabilize and promote the survival of transplanted vascular-forming cells capable of supplying ischemic or traumatized tissues with increased vascular support.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line EN26 at passage 10 displayed the gene expression markers: *TFAP2C* (accession number NM_003222.3, Illumina ID 6450075), *ZIC2* (accession number NM_007129.2, Illumina ID 510368), *CD90* (accession number NM_006288.2, Illumina ID 6480204), *NNAT* (accession number NM_181689.1, Illumina ID 4010709), *TMEM119* (accession number NM_181724.1, Illumina probe ID 3830762), and *SCG5* (accession number NM_003020.1, Illumina ID 5260343), and did not express the markers: *HOXA2* (accession number NM_006735.3, Illumina ID 2060471), or *HOXB2* (accession number NM_002145.3, Illumina ID 3460097).

When the cell line EN26 at passage 11 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed very high levels of expression of the cartilage marker *COL2A1* high levels of the osteogenic markers *IBSP* and *ALPL,* but unlike the majority of the cell lines of the present invention, when differentiated for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the cell line EN26 expressed very high levels of orosomucoid 1 (ORM1), an angiogenic factor associated with highly vascularized bone structures such as the axial skeleton and the pulp-forming dentin of the teeth. When cultured in HyStem-C (BioTime, Inc. Alameda, CA) for 21 days in the presence of 10 ng/mL of BMP4 but without 10 ng/mL of TGFβ3, the cell line also expressed *COL2A1,* but did not express *IBSP,* and *ALPL.*

The cell line EN26, or cells with the markers described herein for the line, or pluripotent stem cell-derived clonal, oligoclonal, or pooled clonal or oligoclonal lines with the aforementioned markers, capable of differentiating into cartilage or bone-forming cells, are useful to make the cells for use in research in cellular differentiation and for use in cell-based therapy where the induction of the aforementioned cell types are therapeutic, such in the repair of bone of the axial skeleton such as vertebral bone, and the dentin of teeth.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line RAD20.5 at passage 16 displayed the gene expression markers: *CD90* (accession number NM_006288.2, Illumina ID 6480204), *PITX1* (accession number NM_002653.3, Illumina ID 2000373), and *HOXB6* (Accession number NM_018952.4, Illumina ID 6220189), but did not express *HEY2* (NM_012259.1, Illumina probe ID 2350685), *FOXS1* (accession number NM_004118.3, Illumina probe ID 3610102), or the mesenchymal stem cell marker *CD74* (accession number NM_001025159.1, Illumina ID 1240070). In addition to *HOXB6* expression, the line expressed relatively distal *HOX* genes such as *HOXB7* and *HOXC8* similar to cultured MSCs from the iliac crest, expressed the *HOX* genes *HOXC9* and *HOXC11,* markers of lower limb bud mesenchyme.

When the cell line RAD20.5 at passage 16 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed abundant levels of *COL2A1* transcript and relatively low levels of the osteogenic markers *IBSP* and *ALPL.* The markers of caudal lateral plate mesoderm and multipotency of the line RAD20.5 or hPS-derived cells including clonal, oligoclonal, or pooled clonal or oligoclonal lines with the aforementioned markers, capable of differentiating into derivatives of lower limb mesenchyme such as articular hyaline cartilage, trabecular and compact bone, ligament, tendon, periosteum, meniscus, synovial membrane, and other connective tissues of the leg, indicate that the cell line has the surprising potential for multiple applications in research and therapy.

In research, the use of such novel and scalable cell lines can be used to study lateral plate mesoderm differentiation, including research models of limb development and limb regeneration in which conflicting models of the molecular basis of development currently exist including the progress zone model and the two signal model, and the use of the purified forelimb or hindlimb-specific limb but mesenchyme of the present invention allows *in vitro* models to precisely test theories with cells of the human species, and cell-based therapy where the induction of the corresponding tissues are therapeutic, such as when differentiated in the presence of BMP4 and TGFβ3 as described herein.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

The cell line 7PEND30 at passage 13 displayed the gene expression markers: *WDR72* (accession number NM_182758.1, Illumina ID 2810451), *CST1* (accession number NM_001898.2, Illumina ID 6370541), *CD69* (accession number NM_001781.1, Illumina ID 2710575), and *TRPV2* (accession number NM_016113.3, Illumina ID 4390301), and did not express the marker *CD90* (accession number NM_006288.2, Illumina ID 6480204).

When the cell line 7PEND30 at passage 13 was cultured for 21 days in HyStem with 10 ng/mL of TGFβ3 together with BMP4 (10ng/mL), or alternatively, 21 days in HyStem with 10 ng/mL of TGFβ3 together with BMP2 (50ng/mL), the differentiated cells expressed low but detectable levels of expression of the cartilage marker *COL2A1,* and relatively high levels of the marker lubricin *(PRG4). PRG4* is synthesized by chondrocytes located at the surface of articular cartilage, to lubricate the joint surface and also by some synovial lining cells.

The cell line 7PEND30, or cells with the markers described herein for the line, or pluripotent stem cell-derived clonal, oligoclonal, or pooled clonal or oligoclonal lines with the aforementioned markers, capable of differentiating into cartilage or bone-forming cells, are useful to make the cells for use in research in cellular differentiation and for use in cell-based therapy where the induction of the aforementioned cell types are therapeutic, such in the repair cartilage in the nose, joint surface, ear, and intervertebral joint where high levels of lubricin are useful.

The cells may be formulated in hydrogels such as HyStem-C (BioTime, Inc. Alameda, CA) wherein the matrix is thiol-modified gelatin and thiolated hyaluronan crosslinked *in vivo* or *in vitro* with (polyethylene glycol diacrylate (PEGDA), or in alternative matrices or in solution without said matrices for research and therapeutic applications.

### EXAMPLE 10: Use of HOXB6+ HOXD10+ limb bud progenitors to recapitulate embryonic limb development.

Embryonic progenitor cells of the present invention display a prenatal pattern of gene expression not shared with differentiated cells of the adult, that are useful in tissue regeneration. An example of this phenotype is that expressed in the non-human animal species such as the Axolotls capable of profound tissue regeneration, including the entire regeneration of an amputated limb. However, human model systems have hitherto not been available to model *in vitro,* or to be implemented *in vivo* for tissue regeneration. The use of certain cells of the present invention, such as RAD20.5, SK5, EN72, or EN75 cells or clonal, oligoclonal, pooled clonal, or pooled oligoclonal cells with the same markers as RAD20.5, SK5, EN72, or EN75, may be incorporated into a semisolid construct including but not limited to HyStem hydrogel which is a semisolid matrix composed of matrix is thiol-modified gelatin and thiolated hyaluronan crosslinked *in vivo* or *in vitro* with (polyethylene glycol diacrylate (PEGDA) to provide such a human model. Because cell lines such as RAD20.5, SK5, EN72, and EN75 or cells with similar markers are purified cells with prenatal patterns of gene expression and site-specific markers of lateral plate mesenchyme, when incorporated into a semisolid matrix, with, for instance, a cylindrical shape to mimic the limb bud, and then exposed to factors such as the slowly-released factors produced by the Apical Ectodermal Ridge (AER) including but not limited to FGF4, 8, 9, and 17, the distal proliferation and differentiation of the cells can be studied (Figure 17).

Similarly, factors that induce the interzone cells that will lead to the morphology of the joint, can be studied by introducing factors such as GDF5, Wnt14, or noggin, (or cells already exposed to such factors) within the construct. Furthermore, factors that play a role in instituting the posterior/anterior axis can be modeled by slowly releasing factors including but not limited to sonic hedgehog on one axis to mimic the Zone of Polarizing Activity (ZPA), or Wnt-7A to mimic dorsalizing factors. Said constructs containing human embryonic limb progenitors and measuring their responses to the aforementioned morphogens are also useful in studying the parallel lack of responsiveness of mesenchymal cell types obtained from adult sources such as MSCs, and for assaying to the effects of teratogens as a component of the safety testing of potential small molecule drug testing.

In addition, the mapping of the fate of certain cells of the present invention, such as RAD20.5, SK5, EN72, or EN75 cells or clonal, oligoclonal, pooled clonal, or pooled oligoclonal cells with the same markers as RAD20.5, SK5, EN72, or EN75, may be accomplished by incorporating the cells into a semisolid construct including but not limited to HyStem (BioTime, Inc. Alameda, CA) hydrogel which is a semisolid matrix composed of matrix is thiol-modified gelatin and thiolated hyaluronan crosslinked *in vivo* into embryonic murine limb buds. Limb buds from rodent limbs, for example murine, are explanted on days 9.5 - 12 of gestation and cultured in a cell culture medium for periods of time up to 6-9 days. At or near the beginning of this period, the cells of the present invention such as RAD20.5, SK5, EN72, or EN75 cells or clonal, oligoclonal, pooled clonal, or pooled oligoclonal cells with the same markers and with prenatal patterns of gene expression and site-specific markers of lateral plate mesenchyme, are incorporated into a semisolid matrix, at the excised proximal aspect or within a hollowed-out portion of the proximal aspect of the limb bud, and then cultured *in vitro* for 6-9 days. Histological analysis including the analysis of the incorporation of the human cells into the murine limb in response to the morphogens naturally produced in the adjacent murine AER or ZPA is then studied by tracking the human cells with transgenes or reagents that specifically recognize human cells such as anti-human mitochondrial antibodies known in the art.

### EXAMPLE 11: Use HyStem-C to cryopreserve cells in beads and to modify MYH11 and FABP4 gene expression in human cells.

HyStem-C (BioTime, Inc. Alameda, CA) is a matrix composed of thiol-modified gelatin and thiolated hyaluronan crosslinked *in vivo* or *in vitro* with (polyethylene glycol diacrylate (PEGDA). We observed that clonal human embryonic progenitor cell lines such as those described in the present invention, could be frozen and thawed within beads of polymerized HyStem-C (BioTime, Inc. Alameda, CA) such as 25µl aliquots of 2.0 x 10⁷ cells/mL (in FBS that is 10% DMSO) in 1% w/v HyStem-C (BioTime, Inc. Alameda, CA) (500,000 cells/bead). This facilitates the accumulation of large numbers of beads with large numbers of diverse hEP cell types that can be simultaneously thawed and assayed such as in high throughput robotic systems wherein the beads are exposed to diverse differentiation conditions and their differentiation assayed by gene expression microarray or other means known in the art. It also makes possible the thawing of large numbers of cryopreserved beads and the incubation of combinations of beads with diverse types of embedded cells and subsequent analysis of changes of differentiated state such as gene expression microarray or other means known in the art.

In addition, the incubation of hEP cell lines in HyStem-C (BioTime, Inc. Alameda, CA), allowed the accumulation of a large amount of data on the biological influence of HyStem-C (BioTime, Inc. Alameda, CA) on diverse cell types. With Illumina gene expression microarray data from more than 3,000 differentiation experiments, we searched for genes frequently up- and down-regulated in HyStem-C (BioTime, Inc. Alameda, CA) beads and compared those profiles to those obtained under micromass conditions. For example, we observed that cells cultured in HyStem-4D (Biotime, Inc. Alameda, CA) beads with BMP4 frequently exhibited a marked decrease in myofibroblast markers such as *MYH11,* and increased expression of adipocyte markers such as *FABP4* and anti-inflammatory markers such as *TIMP4.* A representative experiment is shown in Figure 18. The cell line E15, which in other conditions was shown to have chondrogenic potential and the line W10 strongly induced *MYH11* in micromass conditions supplemented with 10 ng/mL BMP4, but this induction was essentially ablated in HyStem-C (BioTime, Inc. Alameda, CA) culture supplemented with BMP4. Instead, in HyStem-C (BioTime, Inc. Alameda, CA) beads, the line markedly upregulated expression of *DCN,* a marker of meninges. This physiological effect on myofibroblastic differentiation seen in many lines cultured in HyStem-C (BioTime, Inc. Alameda, CA) beads (i.e., the strong reduction *in MYH11* expression) has therapeutic implications *in vivo,* such as in inhibiting fibrosis or adhesions. It also is of benefit in surgical settings where cells could be transplanted to regenerate tissue function while inhibiting adhesions and related fibrotic process at the surgical site.

### EXAMPLE 12: Isolation of purified lipasin-expressing adipocytes and uses thereof.

As described herein, the diverse clonal embryonic progenitor cell lines of the present invention show correspondingly diverse differentiation responses to growth factors such as members of the TGF beta family described herein. In some cases, including but not limited to the culture of the cells in HyStem-C (BioTime, Inc. Alameda, CA) beads in the presence of BMP4, some cell lines strongly express markers of adipocytes such as *FABP4* and *CD36.* Because the clonal progenitor cell lines capable of adipocyte differentiation represent mesenchymal anlagen of diverse anatomical origin, the corresponding adipocytes may represent fat-forming cells with diverse phenotypes. Some of these diverse phenotypes offer novel therapeutic opportunities as described herein.

We disclose herein that one subset of therapeutically-useful adipocytes are those expressing the gene *LIPASIN* (also known as *C19orf80,* also known as *LOC55908,* also known as *BETATROPHIN,* Accession number NM_018687.3. These cells express upper limb markers such as *HOXA10* and *HOXD11,* but lack distal *HOX* genes such as *HOXC9, HOXC10,* or *HOXC11.* The cell lines of the present invention that display this pattern of gene expression include E72, E75 and E163. The cell line E72 expresses *HOXA10* (accession number NM_153715.2, Illumina ID 3290427), *POSTN* (accession number NM_006475.1, Illumina ID 510246), *KRT34* (accession number NM_021013.3, Illumina ID 3710168), *MKX* (accession number NM_173576.1, Illumina ID 6620017), *HAND2* (accession number NM_021973.2, Illumina probe ID 4640563), the relatively rarely-expressed *HOX* gene *HOXD11* (accession number NM_021192.2, Illumina probe ID 5290142) implicated in forelimb development, and *TBX15* (accession number NM_152380.2, Illumina probe ID 6060113), but does not express *LHX8* (accession number NM_001001933.1, Illumina ID 2900343), *FOXF2* (accession number NM_001452.1, Illumina ID 1660470), *AJAP1* (accession number NM_018836.3, Illumina ID 1300647), *PLXDC2* (accession number NM_032812.7, Illumina ID 5900497), or *DLK1* (accession number NM_003836.4, Illumina ID 6510259). The line E72 did not express relatively distal *HOX* genes such as *HOXB7* (accession number NM_004502.2, Illumina probe ID 2470328), and *HOXC8* (accession number NM_022658.3, Illumina probe ID 4640059) expressed by cultured MSCs from the iliac crest, or the *HOX* genes *HOXC9, HOXC10,* or *HOXC11* expressed in hindlimb, but not forelimb bud mesenchyme. The cell lines E75 and E163 expressed the same markers as E72, but unlike the line E72 which did not express *PLXDC2* (accession number NM_032812.7, Illumina probe ID 5900497), the lines E75 and E163 did express *PLXDC2.*

The cell lines E72, E75, and E163, or cells with a similar pattern of gene expression, are capable of differentiating into Lipasin-expressing adipocytes when exposed to adipogenic differentiation conditions such as Differentiation in HyStem-C (BioTime, Inc. Alameda, CA) as described herein with chondrogenic medium supplemented with 10 ng/mL BMP4 for 14-21 days, but without TGFβ3. As shown in Figure 19, bone marrow-derived mesenchymal stem cells (MSCs), differentiated in HyStem-C (BioTime, Inc. Alameda, CA) beads in the presence of 50 ng/mL of BMP2, or 10 ng/mL of BMP4, or 100 ng/mL of BMP7 for 14 days caused the differentiation of the cells into adipocytes as evidenced by their expression of *FABP4,* whereas in the undifferentiated state, the MSCs did not express detectable *FABP4.* Similarly, the lines E72, E75, E163, as well as clonal hES-derived embryonic progenitors corresponding to other anatomic locations, also differentiated into *FABP4*-expressing cells. However, only MSCs, E72, E75, and E163 cells induced Lipasin/Betatrophin expression upon differentiation. Interestingly, the lines described herein capable of differentiating into Lipasin-expressing adipocytes also induced the expression of HEPACAM upon differentiation, a marker potentially useful in assays of purity or useful in purifying said cells by methods such as affinity purification.

The *HEPACAM*+*, LIPASIN*+ adipocytes of the present invention are useful in producing the secreted protein Lipasin which in turn is useful in inducing the proliferation of pancreatic beta cells either *in vitro* or *in vivo.* Said beta cells may be beta cells cultured *in vitro* wherein said cells are derived from mammalian pancreas or derived from cultured pluripotent stem cells such as hES or human iPS cells.

The lipasin-expressing adipocytes of the present invention including said adipocytes derived by differentiating bone marrow-derived MSCs, or the hES-derived clonal embryonic progenitor cell lines E72, E75, or E163, or adipocytes differentiated from pluripotent stem cell-derived cells with the above-described pattern of gene expression, are also useful in treating type I and type II diabetes. In said therapeutic applications, the lipasin-expressing adipocytes of the present invention may be injected into the body, by way of nonlimiting example, the cells in a concentration of 2.5 x 10⁵ cells/ml to 1.0 x 10⁸ cells/ml in HyStem-C (BioTime, Inc. Alameda, CA), preferably 1.0 x 10⁷ cells/ml, or at these concentrations in other matrices useful in promoting cell engraftment. The site of engraftment may vary, but by way of example, the cells may be injected subcutaneously at the normal site of brown fat cells in humans such as in the interscapular region of the back. The cells may or may not also be genetically-modified, with modifications to increase lipasin expression, such as those that down-regulate the insulin receptor gene, or allow the inducible apoptosis of the engrafted cells, or modification to promote the allogeneic histocompatibility of said cells.

In some applications, said lipasin-expressing adipocytes are mitotically inactivated as described herein to limit their lifespan and lead to a transient expression of lipasin to transiently induce the proliferation of pancreatic beta cells.

In aged patients, pancreatic beta cell proliferation in response to the transplanted lipasin-secreting adipocytes or alternatively, the pancreatic beta cell proliferation in response to administered lipasin protein, or simply the pancreatic beta cell proliferation in response hypoinsulinemia, can be facilitated by the extension of telomere length in the beta cells or beta cell precursors by the exogenous expression of the catalytic component of telomerase reverse transcriptase, such as human *TERT.* The telomerase catalytic component of telomerase may be introduced by varied methods known in the art such as viral gene therapy, including but not limited to adenoviral vectors.

### EXAMPLE 13: Regenerative and Anti-Inflammatory Effect of Clonal Human Embryonic Progenitor Cell line Injections in a Whole-Organ Intervertebral Disc Culture System

Intervertebral disc (IVD) degeneration is associated with progression of various degenerative spinal diseases, which directly and/or indirectly causes low back pain and sciatica. In the process of ongoing disc degeneration, decrease in the number of viable cells with subsequent loss of their producing extracellular matrix components consisting of primarily type II collagen and aggrecan is evident. In addition, degenerative IVDs produce a multitude of inflammatory, degradative and catabolic molecules, including proteolytic enzymes, oxygen free radicals, nitric oxide, interleukins and prostaglandins. In particular, interleukin-1 (IL-1) and its regulator, tumor necrosis factor-α (TNF-α), IL-6, proteases like A Disintegrin And Metalloproteinase with Thrombospondin Motifs (ADAMTS) 4, 5 and metalloproteinase (MMP) 3 are thought to play a major role in IVD degeneration. In order to supply viable cells to re-organize the IVD microenvironment, cell transplantation has shown regenerative and anti-inflammatory effects in animal models (Sakai et al., (2005) Spine 30:2379)(Sakai et al., (2008) J. Orthop. Res. 26:589) (Huang et al., (2013) Spine J. 13:352). Although mesenchymal stem cells (MSCs) has been widely reported as candidate cell source, there is no clear evidence that conventional MSCs consisting of heterogenic cells are best suited. Clonal human embryonic progenitor cell lines with chondrogenic potential in comparison to MSCs have shown potential for availability for transplantation site-specific differentiation (Sternberg et al., (2013) Biomatter e24496). The purpose of the current study is to assess whether injection of clonal human embryonic progenitor cell lines affect the normal or degenerate microenvironment in a rabbit whole-organ IVD culture system.

### Methods:

All experiments were performed upon approval by the Animal Care and Use Committee at our institution. Cell preparation: Seven clonal human embryonic progenitor cell lines selected from 100 cell lines which were confirmed to possess chondrogenic potential (4D20.8, 7PEND24, 7SMOO32, E15, MEL2, SK11, SM30) and human MSCs (hMSCs) were used.

Organ culture: Whole spines were harvested from mature New Zealand white rabbits (n=8, 14-20 months old) after euthanasia. Soft tissue surrounding the spine was asceptically removed with care and vertebra was cut in the axial direction close to the chphalad and caudal end plates with a rotary saw to make endplate-disc-endplate units (IVD units) as previously described {Haschtmann, 2006 #347}. Fifty-four IVD units from T11/12-L7/S1 were washed with PBS containing antibiotics and pre-cultured in 12-well multi plates in DMEM/F12/10%FBS media in 5%O₂-5% CO₂, 37°C condition for 24 hours. IVD units were randomly separated in to normal or degenerate groups. Thrombin (10µl; 100 U/disc, 10000 U/ml) was injected in to the IVD with 30G micro-syringe in order to initiate degenerate microenvironment in the degenerate group. Each of the cell lines and hMSCs were then injected (2x10⁴ cells/10µl saline) in a controlled allocation to minimize IVD level/size effect to normal and degenerate IVD units. All cell lines were injected in triplets and 10µl saline was injected as sham in normal group whereas in the degenerate group, sham only received thrombin injection. IVD units were cultured for 7 days in DMEM/F12/10%FBS media in 5%O₂-5% CO₂, 37°C condition and recovered. IVD units were separated to nucleus pulposus (NP) and annulus fibrosus (AF) and total RNA was extracted. qPCR: Gene expression for type II collagen, aggrecan, IL-1, TNF-α, MMP3, ADAMTS4 and 5 were analyzed by quantitative real-time PCR with standards. Statistical Analyses: Two-way ANOVA and the Scheffe's test was used.

### Results:

Regarding type II collagen gene, 4D20.8 and E15 cells up-regulated expression in both NP and AF in normal IVDs whereas hMSC only up-regulated expression in the AF. While in degenerative IVD, four of the seven cell lines (4D20.8, E15, SM30, SK11) and hMSC all up-regulated expression in both NP and AF. No cell injections up-regulated aggrecan gene expression in both NP and AF in normal IVDs, however, in degenerate IVDs, likewise to type II collagen results, 4D20.8, E15, SM30, SK11 and hMSC all up-regulated expression in both NP and AF. Regarding IL-1, IL-6, TNF-α, MMP3, ADAMTS4 and 5, none of the cell injections up-regulated expression of these genes in normal IVDs and demonstrated a similar expression level to saline injected control. On the other hand, in degenerate IVDs, elevated gene expression in all of these inflamatory cytokines were seen with thrombin injection; importantly, these are significantly down-regulated with 4D20.8, E15, SM30, SK11 and hMSC injection in both AF and NP. In particular, down-regulation of *IL-1* and *ADAMTS4* gene by 4D20.8 and SK11 injection was significantly greater than that of hMSC injection (P<0.01). Fibroblast injections show no significant effect except *COLII* in the AF (Figure 20).

### Discussion:

While increasing evidence in experimental studies promise cell therapy as an option in inhibition of degenerative disc disease, selection of an optimal cell source remains to be tuned. MSCs may be ideal however, since they are heterogenic and conventional isolation methods do not warrant clonal stem cell properties. Clonal human embryonic progenitor cell lines are committed progenitor clones that minimize the risk for tumorigenesis and ethical issues compared to ES or iPS cells. The result of the current study demonstrates that, among the seven tested clonal human embryonic progenitor cell lines possessing chondrogenic differentiation capability, four cell lines were capable of facilitating regenerative and antiinflammatory effects in degenerative IVDs. Moreover, two cell lines achieved superior antiinflammatory effects compared to hMSCs, which demonstrate that in cell therapy for degenerative disc disease, donor cells should be selected upon condition of the IVD pathology and microenvironment.

### References

1. Kim, M. et al. Age-related alterations in mesenchymal stem cells related to shift in differentiation from osteogenic to adipogenic potential: implication to age-associated bone diseases and defects. Mechanisms of ageing and development 133, 215-225 (2012).
2. Caplan, A.I. Mesenchymal stem cells. Journal of orthopaedic research : official publication of the Orthopaedic Research Society 9, 641-650 (1991).
3. Caplan, A.I. Review: mesenchymal stem cells: cell-based reconstructive therapy in orthopedics. Tissue engineering 11, 1198-1211 (2005).
4. Pittenger, M.F. et al. Multilineage potential of adult human mesenchymal stem cells. Science 284, 143-147 (1999).
5. Nombela-Arrieta, C., Ritz, J. & Silberstein, L.E. The elusive nature and function of mesenchymal stem cells. Nature reviews. Molecular cell biology 12, 126-131 (2011).
6. Beltrami, A.P. et al. Multipotent cells can be generated in vitro from several adult human organs (heart, liver, and bone marrow). Blood 110, 3438-3446 (2007).
7. Bianco, P., Robey, P.G. & Simmons, P.J. Mesenchymal stem cells: revisiting history, concepts, and assays. Cell stem cell 2, 313-319 (2008).
8. Pelttari, K. et al. Premature induction of hypertrophy during in vitro chondrogenesis of human mesenchymal stem cells correlates with calcification and vascular invasion after ectopic transplantation in SCID mice. Arthritis and rheumatism 54, 3254-3266 (2006).
9. Grant, W.T., Wang, G.J. & Balian, G. Type X collagen synthesis during endochondral ossification in fracture repair. J Biol Chem 262, 9844-9849 (1987).
10. Gebhard, S. et al. BAC constructs in transgenic reporter mouse lines control efficient and specific LacZ expression in hypertrophic chondrocytes under the complete Col10al promoter. Histochemistry and cell biology 127, 183-194 (2007).
11. Mak, K.K., Kronenberg, H.M., Chuang, P.T., Mackem, S. & Yang, Y. Indian hedgehog signals independently of PTHrP to promote chondrocyte hypertrophy. Development 135, 1947-1956 (2008).
12. Topping, R.E., Bolander, M.E. & Balian, G. Type X collagen in fracture callus and the effects of experimental diabetes. Clinical orthopaedics and related research, 220-228 (1994).
13. Mao, J.J. et al. Craniofacial tissue engineering by stem cells. Journal of dental research 85, 966-979 (2006).
14. Creuzet, S., Couly, G., Vincent, C. & Le Douarin, N.M. Negative effect of Hox gene expression on the development of the neural crest-derived facial skeleton. Development 129, 4301-4313 (2002).
15. Ishii, M. et al. Combined deficiencies of Msx1 and Msx2 cause impaired patterning and survival of the cranial neural crest. Development 132, 4937-4950 (2005).
16. Levi, G. et al. Msx1 and D1x5 act independently in development of craniofacial skeleton, but converge on the regulation of Bmp signaling in palate formation. Mechanisms of development 123, 3-16 (2006).
17. Depew, M.J., Lufkin, T. & Rubenstein, J.L. Specification of jaw subdivisions by Dlx genes. Science 298, 381-385 (2002).
18. Bonilla-Claudio, M. et al. Bmp signaling regulates a dose-dependent transcriptional program to control facial skeletal development. Development 139, 709-719 (2012).
19. West, M.D. et al. The ACTCellerate initiative: large-scale combinatorial cloning of novel human embryonic stem cell derivatives. Regen Med 3, 287-308 (2008).
20. Sternberg, H. et al. A human embryonic stem cell-derived clonal progenitor cell line with chondrogenic potential and markers of craniofacial mesenchyme. Regen Med 7, 481-501 (2012).
21. Boxall, S.A. & Jones, E. Markers for characterization of bone marrow multipotential stromal cells. Stem cells international 2012, 975871 (2012).
22. Zhao, Y. et al. Isolated cleft palate in mice with a targeted mutation of the LIM homeobox gene lhx8. Proc Natl Acad Sci U S A 96, 15002-15006 (1999).
23. Zhang, Y. et al. Comparison of the expression patterns of two LIM-homeodomain genes, Lhx6 and L3/Lhx8, in the developing palate. Orthodontics & craniofacial research 5, 65-70 (2002).
24. Grigoriou, M., Tucker, A.S., Sharpe, P.T. & Pachnis, V. Expression and regulation of Lhx6 and Lhx7, a novel subfamily of LIM homeodomain encoding genes, suggests a role in mammalian head development. Development 125, 2063-2074 (1998).
25. Tissier-Seta, J.P. et al. Barx1, a new mouse homeodomain transcription factor expressed in cranio-facial ectomesenchyme and the stomach. Mechanisms of development 51, 3-15 (1995).
26. Tucker, A.S., Matthews, K.L. & Sharpe, P.T. Transformation of tooth type induced by inhibition of BMP signaling. Science 282, 1136-1138 (1998).
27. Nie, X. Developmentally regulated expression of MSX1, MSX2 and Fgfs in the developing mouse cranial base. The Angle orthodontist 76, 990-995 (2006).
28. Shawlot, W. & Behringer, R.R. Requirement for Lim1 in head-organizer function. Nature 374, 425-430 (1995).
29. Yeung, G. et al. Cloning of a novel epidermal growth factor repeat containing gene EGFL6: expressed in tumor and fetal tissues. Genomics 62, 304-307 (1999).
30. Thomas, T. et al. A signaling cascade involving endothelin-1, dHAND and msx1 regulates development of neural-crest-derived branchial arch mesenchyme. Development 125, 3005-3014 (1998).
31. DeLaurier, A., Schweitzer, R. & Logan, M. Pitx1 determines the morphology of muscle, tendon, and bones of the hindlimb. Developmental biology 299, 22-34 (2006).
32. Mina, M. Regulation of mandibular growth and morphogenesis. Critical reviews in oral biology and medicine : an official publication of the American Association of Oral Biologists 12, 276-300 (2001).
33. King, M., Arnold, J.S., Shanske, A. & Morrow, B.E. T-genes and limb bud development. American journal of medical genetics. PartA 140, 1407-1413 (2006).
34. Agulnik, S.I., Papaioannou, V.E. & Silver, L.M. Cloning, mapping, and expression analysis of TBX15, a new member of the T-Box gene family. Genomics 51, 68-75 (1998).
35. Gong, S.G., Gong, T.W. & Shum, L. Identification of markers of the midface. Journal of dental research 84, 69-72 (2005).
36. Johnstone, B., Hering, T.M., Caplan, A.I, Goldberg, V.M. & Yoo, J.U. In vitro chondrogenesis of bone marrow-derived mesenchymal progenitor cells. Exp Cell Res 238, 265-272 (1998).
37. Leonard, C.M. et al. Role of transforming growth factor-beta in chondrogenic pattern formation in the embryonic limb: stimulation of mesenchymal condensation and fibronectin gene expression by exogenenous TGF-beta and evidence for endogenous TGF-beta-like activity. Developmental biology 145, 99-109 (1991).
38. Steck, E. et al. Chondrocyte expressed protein-68 (CEP-68), a novel human marker gene for cultured chondrocytes. The Biochemical journal 353, 169-174 (2001).
39. Wilson, R. et al. Changes in the chondrocyte and extracellular matrix proteome during post-natal mouse cartilage development. Molecular & cellular proteomics : MCP 11, M111 014159 (2012).
40. Amanatullah, D.F., Yamane, S. & Reddi, A.H. Distinct patterns of gene expression in the superficial, middle and deep zones of bovine articular cartilage. Journal of tissue engineering and regenerative medicine (2012).
41. Cormier, S., Leroy, C., Delezoide, A.L. & Silve, C. Expression of fibroblast growth factors 18 and 23 during human embryonic and fetal development. Gene Expr Patterns 5, 569-573 (2005).
42. Seitz, S. et al. Preproenkephalin (Penk) is expressed in differentiated osteoblasts, and its deletion in Hyp mice partially rescues their bone mineralization defect. Calcified tissue international 86, 282-293 (2010).
43. Coussens, A.K. et al. Unravelling the molecular control of calvarial suture fusion in children with craniosynostosis. BMC genomics 8, 458 (2007).
44. Anderson, D.J. Molecular control of cell fate in the neural crest: the sympathoadrenal lineage. Annual review of neuroscience 16, 129-158 (1993).
45. Nagai, T. et al. The expression of the mouse Zic1, Zic2, and Zic3 gene suggests an essential role for Zic genes in body pattern formation. Developmental biology 182, 299-313 (1997).
46. Duboc, V. & Logan, M.P. Regulation of limb bud initiation and limb-type morphology. Dev Dyn 240, 1017-1027 (2011).
47. Liu, W., Selever, J., Lu, M.F. & Martin, J.F. Genetic dissection of Pitx2 in craniofacial development uncovers new functions in branchial arch morphogenesis, late aspects of tooth morphogenesis and cell migration. Development 130, 6375-6385 (2003).
48. Balic, A., Adams, D. & Mina, M. Prx1 and Prx2 cooperatively regulate the morphogenesis of the medial region of the mandibular process. Dev Dyn 238, 2599-2613 (2009).
49. Fukiishi, Y. & Morriss-Kay, G.M. Migration of cranial neural crest cells to the pharyngeal arches and heart in rat embryos. Cell and tissue research 268, 1-8 (1992).
50. Imai, H., Osumi-Yamashita, N., Ninomiya, Y. & Eto, K. Contribution of early-emigrating midbrain crest cells to the dental mesenchyme of mandibular molar teeth in rat embryos. Developmental biology 176, 151-165 (1996).
51. Noden, D.M. The role of the neural crest in patterning of avian cranial skeletal, connective, and muscle tissues. Developmental biology 96, 144-165 (1983).
52. Bahney, C.S., Hsu, C.W., Yoo, J.U., West, J.L. & Johnstone, B. A bioresponsive hydrogel tuned to chondrogenesis of human mesenchymal stem cells. FASEB journal : official publication of the Federation of American Societies for Experimental Biology 25, 1486-1496 (2011).
53. Bian, L., Zhai, D.Y., Zhang, E.C., Mauck, R.L. & Burdick, J.A. Dynamic Compressive Loading Enhances Cartilage Matrix Synthesis and Distribution and Suppresses Hypertrophy in hMSC-Laden Hyaluronic Acid Hydrogels. Tissue engineering. Part A 18, 715-724 (2012).
54. Mauck, R.L., Yuan, X. & Tuan, R.S. Chondrogenic differentiation and functional maturation of bovine mesenchymal stem cells in long-term agarose culture. Osteoarthritis and cartilage / OARS, Osteoarthritis Research Society 14, 179-189 (2006).
55. Park, S., Hung, C.T. & Ateshian, G.A. Mechanical response of bovine articular cartilage under dynamic unconfined compression loading at physiological stress levels. Osteoarthritis and cartilage / OARS, Osteoarthritis Research Society 12, 65-73 (2004).

## Claims

1. A method of differentiating a human clonal progenitor cell into an osteochondral cell comprising contacting the human clonal progenitor cell with one or more TGFβ superfamily members.

2. A method according to claim 1, wherein the human clonal progenitor cell is cultured under micromass conditions.

3. A method according to claim 1, wherein the human clonal progenitor cell is cultured in contact with a hydrogel.

4. A method according to claim 3, wherein the human clonal progenitor cell is encapsulated in the hydrogel.

5. A method according to claim 3, wherein the hydrogel comprises hyaluronate and gelatin.

6. A method according to claim 5, wherein the hyaluronate and/or the gelatin is thiolated.

7. A method according to claim 3, wherein the hydrogel comprises acrylate, optionally PEG Diacrylate.

8. A method according to claim 3, wherein the one or more TGFβ super family member is selected from the group consisting of: TGFβ-3, BMP2, BMP-4, BMP-6, BMP-7 and GDF-5 and combinations thereof.

9. A method according to claim 3, wherein the one or more TGFβ super family member is TGFβ-3 and BMP-2.

10. A method according to claim 3, wherein the one or more TGFβ super family member is TGFβ-3 and BMP-4.

11. A method according to claim 3, wherein the one or more TGFβ super family member is TGFβ-3 and GDF5.

12. A cellular composition comprising a hydrogel and the in vitro differentiated progeny of a human clonal progenitor cell, wherein the progeny of the human clonal progenitor cell is an osteochondral cell.

13. A composition according to claim 12, wherein the hydrogel comprises hyaluronate and gelatin.

14. The composition of claim 13, wherein the hyaluronate and/or the gelatin is thiolated.

15. A composition according to claim 12 further comprising one or more members of the TGFβ super family.

16. A composition according to claim 15, wherein the one or more members of the TGFβ super family is selected from the group consisting of: TGFβ-3, BMP-2, BMP-4, BMP-6, BMP-7 and GDF-5 and combinations thereof.

17. A cellular composition according to claim 12 wherein the differentiated progeny of the clonal human progenitor cell expresses one or more genes selected from the group consisting of: *COL2A1, COL10A1, ACAN, CRTAC1, TNMD, ALPL, PENK, BGLAP, BMP-2, DLX5, GPC3, IHH, PRG4, CILP, EPYC,SPP1, TTR LPL, CEBPD, PPARG, FABP4, PLIN1, DLK1, PPARGC1A, CEBPD, PPARG, FABP4, PRDM16, FOXC2, CRLF1, FOXF2, FBLN5, DPT, ITGBL1, COL6A3, DUSP1, FOXF1, TGFB3, PAX9, GSN, FMOD, PDE8B, COMP, ITGA10, SAA1, DTNA, PCDH9, EBF1, SORBS1, SORBS2, ZNF503, MGST2, PNMT, DPT, OGN, FBLN5, FMOD, CRLF1, ITGBL1, TGFB3, GSN, HHIP, LRIG1, TRPS1, COMP, LECT1, COL9A1, HAPLN1, ITGA10, OLFML3, PKDCC, FGFR3, CSPG4, COL9A3, ITGB5, DNM1; OGN, COL9A2, EPYC, CHAD, PPIB, SRPX2, MATN3, LUM, COL13A1, FKBP11, MXRA8, COL27A1, PELI2, GPX7, ANGPTL2, GXYLT2, KLF4, STEAP3, SLC39A14, PTH1R, FAM46A, FAM180A, SLC26A2, RUNX1, CTGF, PLEKHB1, FKBP7, TNC, JAK2, CYTL1, KDELR3, ATP8B2, TRPS1; ELN, PPIB, PHEX, SOX9, COL27A1, PELI2, ANGPTL2, GXYLT2, SLC39A14, P4HA3, SLC26A2, CTGF, MRC2, COL9A3, PLEKHB1, TNC, FRMD8, CYTL1, ATP8B2; SCRG1, MATN3, SMOC1, PELI2, PTH1R, HTRA1, RUNX1, CTGF, PLEKHB1, RG9MTD1, CYTL1,* and *KLF2, LPL, CEBPB, PLIN1, PPARG, PPARGC1A,, PPARG, GSN, WIF1, TGFB3, , CORO2B, ADAMTS15,* and *IGFBP7COMP, SPP1, KAZALD1, LECT1, MMP13, HAPLN1, PHEX, PTH1R, COL11A1,* and *IP6K2 PKDCC, LTBP3, HAPLN1, OLFML3, ITGB5, IP6K2, ELN, CYTL1, LTBP3, PELI2, EPYC, PHEX, MRC2, CALY, GXYLT2, COL27A1, ANGPTL2, SOX9, GAA, TNC, LEPRE1, LTBP2, ARFGAP1, SRPX2, CYTL1, FAM46A, LUM, LTBP3, MXRA8, RUNX1, CALY, PTH1R, FKBP11, STEAP3, CFH, SLC40A1, GXYLT2, COL27A1, GPX7, ANGPTL2, MATN3, FKBP7, GAA, TNC, LEPRE1,* and *LTBP2, PPARG, PLIN1, LPL, CSPG4, LTBP3, CA72, PPIB, IRX5, ARHGAP24, FBLN5, DPT, CRLF1, ITGBL1, TGFB3, COL6A3, VCAN, DUSP1, TRPS1, GSN, ADAMTS6, ERG; EPYC, ELN, COLBA1, COL9A3, CYTL1, PPIB, CTHRC1, PLEKHB1, SLC26A2, KANK1, SLC39A14, ATP8B2, TNC, LTBP2, GPC1, WWP2, P4HA3, BAMB1, FGFRL1, SDC2; SCRG1, MEF2C, MATN3, PTH1R, ENPP2, CYTL1, CTHRC1, PLEKHB1, RUNX3, RUNX1, PRICKLE1, WWP2, HTRA1, CTGF, FGFRL1, ERG, FAT3; COL9A2, PCOLCE2, MEF2C, MATN3, LUM, HHIP, PTH1R, SLC40A1, KLF4, SRPX2, CYTL1, FKBP11, CTHRC1, STEAP3, LOXL4, DUSP1, LTBP2, TRPS1, GPC1, CFH, BAMBI, CDH2, COL27A1, FAM46A, CTGF, SDC2,* and *PLCD1, FRZB, HAND2, DLX5, DLX6, FOXD1, MSX2, TFAP2A, ALPL, BMP2, MSX2, DCN, SPP1, SATB2, MSX2, POSTN PCDH20, SERPINA3, TAC1, EDNRA, SCRG1, CRABP2, SPOCK3, VCAN, BOC, ECM2, CRLF1, GAS1, TSPAN8, MFAP4, NFIA, RASSF9, DCN, SATB2, NKX3-2, EBF1, SORBS2, PCDH17, SOBP, ST8SIA4; MEF2C, ALPL, PTH1R, STEAP3, PELI2, SLC40A1, MXRA8, MATN3, PARD6G, GPX7, FAM180A, CTHRC1, ATP8B2, BAMB1, PLCD1, LTBR, SLC26A2, ANGPTL2, SATB2, FAM46A, DUSP1, CSPG4, , OLFML3, PKDCC, LTBP3, FUS, FBLN5, GAS1, DUSP1, GSN, ARHGAP24, VCAN, EMILIN3; PDE8B, SORBS2, PHACTR2, EBF1, PCDH9, MATN3, LTBP2, DUSP1, CDH2, SLC40A1, MXRA8, STEAP3, ANGPTL2, GPX7, TSPAN3, SDC2, ATP8B2, CD36, CTGF, ARHGAP24, FBLN2, SPP1, CSPG4, CSPG4, ENPP1, CA12, DNM1; GXYLT2, ANGPTL2, METRNL, KDELR3, PARD6G,* and *LEPRE1*, *ACTC1, CRYAB, EFHD1, MFAP5, DLK1, ACTA2, OCA2, AIF1L, GPC4, SCRG1, CNN1, HES6, KRT17, RASL11B, IGFBP3, EDN1, ENC1, SFRP2, ACTG2, CKB, CSRP1, CSRP2, C5orf46, COL3A1, HEY1, TUBB2B, CALD1, KAL1, CD24, CDH2, MAMDC2, EFR3B, CDKN2B, HES4, TAGLN, CAP2, PMEPA1, CTGF, TPM1, TGFB2, CXXC5, COL4A1, PALLD, SOX11, OCIAD2, EML1, CCDC99, TTR, CCDC3, TGFB3, ZIC2, GPC4, POSTN, ID3, PODXL, FBLN5, KRT7, TINAGL1, LGMN, GPX7, ACTG2, ANGPTL4, TUBB2B, SLC4A2, SULF1, SLC16A9, CDH2, MAMDC2, , ENPP2, NPR3, CDKN2B, TAGLN, INO80C, HTRA1, DHRS3, CTGF, IGFBP7, , PMEPA1, MRPS6, APOE, SMPD1, TPM1, STXBP2, TMEM108, IQCG, COL4A1, SPINT2, MXD3, TPD52L1, HEYL, CDH6, CYR61, EFHD1, CCDC3: , CFH , ZIC2, TIMP4, SYNM , AIF1L, EBF1, H19, DYSF , EDNRA , NDUFA4L2, ACTG2, COL3A1, CSPG4 , PRRX1, TMEFF2 , TAGLN, ZBTB46 , HTRA1, IGFBP7 , APOE , OLAH , IGDCC4, FBLN1, GGT5, M4MC1, RFTN2 , STC2 , IGFBPI , 7MEM119, CDH6, DKK2, DKK3, FAM198B,* ACTG2, *FILIPIL, MYH11; CSRP2 PRG4, AMELX, ENAM, SILV1, PITX1, FABP4, AMBN, CNN1, MYH11, ORM1* and *LIPASIN.*

18. A kit for differentiating human clonal progenitor cells into an osteochondral cell comprising a hydrogel and one or more members of the TGFβ super family.

19. A kit according to claim 18, wherein one or more members of the TGFβ super family is selected from the group consioting of: TGFβ-3, BMP-2, BMP-4, BMP-6, BMP-7 and GDF-5 and combinations thereof.

20. A kit according to claim 18, wherein the hydrogel is comprised of hyaluronate and gelatin; and optionally further comprises an acrylate, optionally PEG diacrylate.

21. A kit according to claim 20, wherein hyaluronate and/or the gelatin is thiolated.

22. A cryo-preserved human clonal progenitor cell and a hydrogel.

23. A cryo-preserved *in vitro* differentiated progeny of a human clonal progenitor cell and a hydrogel.


SEQUENCE LISTING

<110>  BIOTIME, INC

<120>  DIFFERENTIATED PROGENY OF CLONAL PROGENITOR CELL LINES

<130>  PX212121EP (BIOT-062)

<140>  61/711,161
<141>  2012-10-08

<140>  61/718,647
<141>  2012-10-25

<140>  61/725,866
<141>  2012-11-13

<140>  61/808,578
<141>  2013-04-04

<140>  61/817,260
<141>  2013-04-29

<140>  61/874,316
<141>  2013-09-05

<160>  38

<170>  PatentIn version 3.5

<210>  1
<211>  20
<212>  DNA
<213>  Homo sapiens

<400>  1
tgagtcctca agcctcctgt                                                    20


<210>  2
<211>  20
<212>  DNA
<213>  Homo sapiens

<400>  2
cctctgtctc cttgcaggtc                                                    20


<210>  3
<211>  29
<212>  DNA
<213>  Homo sapiens

<400>  3
agtgttttcc aacgtcactg atgatatgc                                          29


<210>  4
<211>  24
<212>  DNA
<213>  Homo sapiens

<400>  4

aaaggggctc tgggcattta aggc                                          24


<210>  5
<211>  23
<212>  DNA
<213>  Homo sapiens

<400>  5
gtgcccgtgt acaccgatga gac                                           23


<210>  6
<211>  25
<212>  DNA
<213>  Homo sapiens

<400>  6
ggccagtcag caggagattt caaac                                         25


<210>  7
<211>  23
<212>  DNA
<213>  Homo sapiens

<400>  7
agaagtacct ttccacgccg gac                                           23


<210>  8
<211>  20
<212>  DNA
<213>  Homo sapiens

<400>  8
cttggtggga gactccaggc                                               20


<210>  9
<211>  26
<212>  DNA
<213>  Homo sapiens

<400>  9
gcaataaatc cagctctcat caggac                                        26


<210>  10
<211>  22
<212>  DNA
<213>  Homo sapiens

<400>  10
cagtcctgcc atcccagatc cc                                            22


<210>  11
<211>  25
<212>  DNA
<213>  Homo sapiens

<400>  11

tgctgcccaa tctccatctg tcaac                                          25

<210>  12
<211>  22
<212>  DNA
<213>  Homo sapiens

<400>  12
gggtctgggt gtagggttat cc                                             22

<210>  13
<211>  18
<212>  DNA
<213>  Homo sapiens

<400>  13
tggcctgaga cagcatga                                                  18

<210>  14
<211>  19
<212>  DNA
<213>  Homo sapiens

<400>  14
agtgttggga gccagattg                                                 19

<210>  15
<211>  20
<212>  DNA
<213>  Homo sapiens

<400>  15
gggcctcaat ggacccaccg                                                20

<210>  16
<211>  20
<212>  DNA
<213>  Homo sapiens

<400>  16
ctgggccttt ggcctgcctt                                                20

<210>  17
<211>  20
<212>  DNA
<213>  Homo sapiens

<400>  17
atccgtagag agcacggaga                                                20

<210>  18
<211>  20
<212>  DNA
<213>  Homo sapiens

<400>  18

ggactctcca tgggacaaga                                                    20

<210>  19
<211>  20
<212>  DNA
<213>  Homo sapiens

<400>  19
ggcctccaag gagtaagacc                                                    20

<210>  20
<211>  20
<212>  DNA
<213>  Homo sapiens

<400>  20
aggggtctac atggcaactg                                                    20

<210>  21
<211>  20
<212>  DNA
<213>  Homo sapiens

<400>  21
aaacgattgc agggtttcac                                                    20

<210>  22
<211>  20
<212>  DNA
<213>  Homo sapiens

<400>  22
ctctcgtcgg tgactgttca                                                    20

<210>  23
<211>  19
<212>  DNA
<213>  Homo sapiens

<400>  23
ggacgccgag cgctgaggc                                                     19

<210>  24
<211>  27
<212>  DNA
<213>  Homo sapiens

<400>  24
gaaaaccacc taccagactc atttcgc                                            27

<210>  25
<211>  20
<212>  DNA
<213>  Homo sapiens

<400>  25

ctcctgtctc cagctatccg                                                    20

<210>  26
<211>  20
<212>  DNA
<213>  Homo sapiens

<400>  26
gcacagagcg tactggtgaa                                                    20

<210>  27
<211>  28
<212>  DNA
<213>  Homo sapiens

<400>  27
aaactctact gcaagaacga cttcttcc                                           28

<210>  28
<211>  26
<212>  DNA
<213>  Homo sapiens

<400>  28
tgcaggtgaa gcagttcagg tgaaac                                             26

<210>  29
<211>  24
<212>  DNA
<213>  Homo sapiens

<400>  29
ctcggaccag ctttacagca gatc                                               24

<210>  30
<211>  25
<212>  DNA
<213>  Homo sapiens

<400>  30
acgtgtttct tgtggcgtgc tctac                                              25

<210>  31
<211>  19
<212>  DNA
<213>  Homo sapiens

<400>  31
atttggtcgt ggacgtggt                                                     19

<210>  32
<211>  24
<212>  DNA
<213>  Homo sapiens

<400>  32

tttggctgta agtttattca atgc                                              24


<210>  33
<211>  24
<212>  DNA
<213>  Homo sapiens

<400>  33
acgacccagc caagaagaag aagc                                              24


<210>  34
<211>  22
<212>  DNA
<213>  Homo sapiens

<400>  34
tgctggttac gctcgcgctt ac                                                22


<210>  35
<211>  25
<212>  DNA
<213>  Homo sapiens

<400>  35
agattcaggt ggagctgcaa tgtgc                                             25


<210>  36
<211>  26
<212>  DNA
<213>  Homo sapiens

<400>  36
ccacttggag ctatgataca catatc                                            26


<210>  37
<211>  21
<212>  DNA
<213>  Homo sapiens

<400>  37
gacccacaca ggggagaagc c                                                 21


<210>  38
<211>  25
<212>  DNA
<213>  Homo sapiens

<400>  38
gtgtaggact tgtcgcacat cttgc                                             25